# EUROPÄISCHE PATENTSCHRIFT

(11) **EP 2 493 886 B1**
(45) Veröffentlichungstag und Bekanntmachung des Hinweises auf die Patenterteilung: **26.11.2014**
(21) Anmeldenummer: 10768944.0
(22) Anmeldetag: 26.10.2010
(51) Int. Cl.: C07D 417/04, C07D 417/12, C07D 417/14, A01N 43/78

(54) **HETEROARYLPIPERIDIN UND -PIPERAZIN DERIVATE**
HETEROARYL PIPERIDINE AND PIPERAZINE DERIVATES
DÉRIVÉS D'HÉTÉROARYL PIPÉRIDINE ET PIPÉRAZINE

(30) Priorität: 30.10.2009 EP 09174614; 02.11.2009 US 257243 P
(43) Veröffentlichungstag der Anmeldung: 05.09.2012
(73) Patentinhaber: Bayer CropScience AG, 40789 Monheim (DE)
(72) Erfinder: CRISTAU, Pierre, F-69009 Lyon (FR); RAHN, Nicola, 40589 Düsseldorf (DE); TSUCHIYA, Tomoki, 40227 Düsseldorf (DE); KLUTH, Joachim, 40764 Langenfeld (DE); WASNAIRE, Pierre, 40225 Düsseldorf (DE); HOFFMANN, Sebastian, 41470 Neuss (DE); BENTING, Jürgen, 42799 Leichlingen (DE); WACHENDORFF-NEUMANN, Ulrike, 56566 Neuwied (DE); SEITZ, Thomas, 40764 Langenfeld (DE)
(86) Internationale Anmeldenummer: PCT/EP2010/066098
(87) Internationale Veröffentlichungsnummer: WO 2011/051244

(56) Entgegenhaltungen:
- WO-A1-2009/132785
- WO-A2-2007/014290
- WO-A2-2008/091580
- WO-A2-2008/091594
- WO-A2-2009/094445

## Beschreibung

Die vorliegende Erfindung betrifft neue Heteroarylpiperidin und -piperazin Derivate, Verfahren zum Herstellen dieser Verbindungen, Mittel enthaltend diese Verbindungen, sowie deren Verwendung als biologisch aktive Verbindungen, insbesondere zur Bekämpfung von schädlichen Mikroorganismen im Pflanzenschutz und im Materialschutz.

Es ist bereits bekannt, dass bestimmte Heteroarylpiperidin und -piperazin Derivate, z.B. Piperidinylsubstituierte Thiazol-4-carbonsäureamide als fungizide Pflanzenschutzmittel benutzt werden können (siehe WO-A-2007/014290, WO-A-2008/091594, WO-A-2008/013925, WO-A-2008/013622, WO-A-2008/091580, WO-A-2009/055514, WO-A-2009/094407, WO-A-2009/094445, WO-A-2009/132785, WO-A-2010/037479). Die fungizide Wirksamkeit dieser Verbindungen ist jedoch gerade bei niedrigeren Aufwandmengen nicht immer ausreichend. Desweiteren ist das Wirkungsspektrum dieser Amide in vielen Fällen nicht ausreichend.

In WO-A-2004/058750, WO-A-2004/058751, US-A-0,234,033, WO-A-2005/003128, WO-A-2005/116653, FR-A-2856685, WO-A-2006/132436 und WO-A-2008/069313 werden weitere Heteroarylpiperidin und -piperazin Derivate beschrieben, die ebenfalls medizinisch genutzt werden können. Eine Wirkung auf pilzliche Pathogene wird jedoch nicht beschrieben.

Da sich die ökologischen und ökonomischen Anforderungen an moderne Wirkstoffe, z.B. Fungizide, laufend erhöhen, beispielsweise bezüglich Wirkspektrum, Toxizität, Selektivität, Aufwandmenge, Rückstandsbildung und günstige Herstellbarkeit, und außerdem z.B. Probleme mit Resistenzen auftreten können, besteht die ständige Notwendigkeit, neue fungizide Mittel zu entwickeln, die zumindest in Teilbereichen Vorteile gegenüber den bekannten aufweisen.

Gegenstand der Erfindung sind Verbindungen der Formel (**I**), 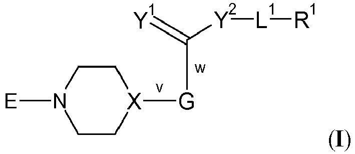 in welcher die Symbole folgende Bedeutungen haben:
- E: steht für 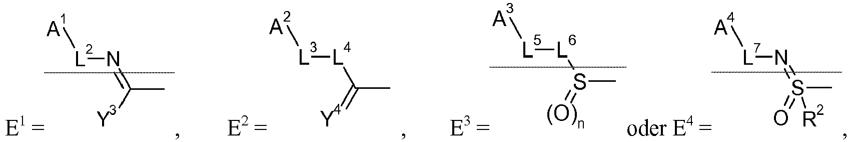
- A²,: stehen für ein Phenyl, das bis zu drei Substituenten enthalten kann, wobei die Substituenten unabhängig voneinander ausgewählt sind aus folgender Liste:
Halogen, Cyano, Hydroxy, SH, Amino, Nitro, C₁-C₆-Alkyl, C₂-C₆-Alkenyl, C₂-C₆-Alkinyl, C₃-C₁₀-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, C₂-C₈-Dialkylamino, C₃-C₆-Cycloalkylamino, C₄-C₁₀-Cycloalkylalkoxy, C₂-C₄-Alkoxyalkyl, C₂-C₆-Alkoxyalkoxy, C₁-C₄-Hydroxyalkyl, C₂-C₄-Alkylcarbonyl, C₂-C₆-Alkoxycarbonyl, C₂-C₆-Alkylcarbonyloxy, C₂-C₆-Alkylcarbonylthio, C₂-C₆-Alkylaminocarbonyl, C₃-C₈-Dialkylaminocarbonyl, Tri(C₁-C₄-alkyl)silyl, C(=O)H, CR³=NOR⁴, Phenyl oder Benzyl
oder
- A²,: stehen für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu drei Substituenten enthalten kann, wobei die Substituenten unabhängig voneinander ausgewählt sind aus folgender Liste:
Substituenten am Kohlenstoff:
Halogen, Cyano, Hydroxy, SH, Amino, Nitro, C₁-C₆-Alkyl, C₂-C₆-Alkenyl, C₂-C₆-Alkinyl, C₃-C₁₀-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, C₂-C₈-Dialkylamino, C₃-C₆-Cycloalkylamino, C₄-C₁₀-Cycloalkylalkoxy, C₂-C₄-Alkoxyalkyl, C₂-C₆-Alkoxyalkoxy, C₁-C₄-Hydroxyalkyl, C₂-C₄-Alkylcarbonyl, C₂-C₆-Alkoxycarbonyl, C₂-C₆-Alkylcarbonyloxy, C₂-C₆-Alkylcarbonylthio, C₂-C₆-Alkylaminocarbonyl, C₃-C₈-Dialkylaminocarbonyl, Tri(C₁-C₄-alkyl)silyl, C(=O)H, CR³=NOR⁴, Phenyl oder Benzyl
Substituenten am Stickstoff:
C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkylsulfonyl, C(=O)H, C(=O)Me, C(=O)OMe, oder C₂-C₄-Alkoxyalkyl,
- L¹: steht für (C(R¹⁵)₂)ₚ,
- p: steht für 0, 1 oder 2,
stehen für eine direkte Bindung, C(=O) oder S(=O)₂,
- L³: steht für eine direkte Bindung,
- L⁴: steht für Sauerstoff, CHR⁵, NR⁶ oder C(=O),
steht für CHR⁵ oder NR⁶,
- L⁸: steht für eine direkte Bindung, -O, -C(=O), -S(O)ₘ, -CHR¹⁶ oder -NR¹⁷,
- Y¹, Y², Y⁴: stehen unabhängig voneinander für Schwefel oder Sauerstoff,
- Y³: steht für OR⁷, SR⁸, NR⁹R¹⁰ oder R¹¹,
- m: steht für 0, 1 oder 2,
- n: steht für 0, 1 oder 2,
- X: steht für CR¹² oder Stickstoff,
- G: steht für 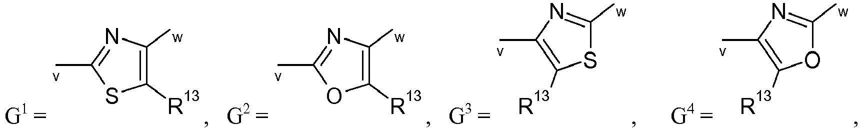 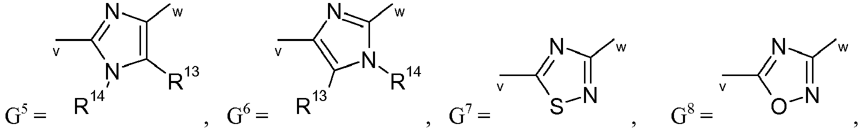 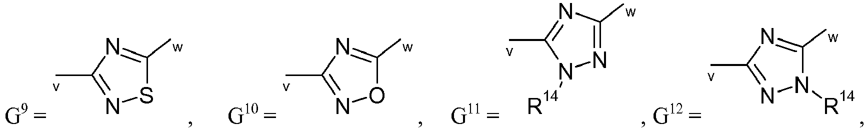 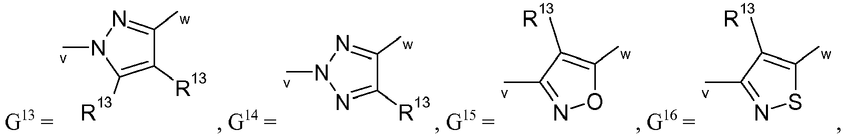 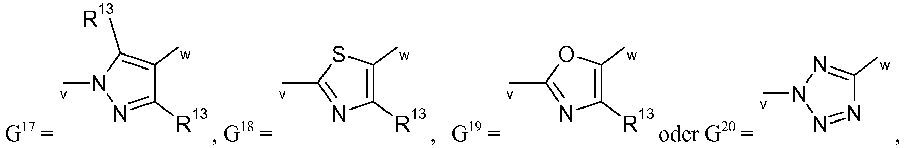
wobei die Bindung, die mit "v" identifiziert ist, direkt an X gebunden ist, und wobei die Bindung, die mit "w" identifiziert ist, direkt an C(=Y¹)Y²L¹R¹ gebunden ist,
- R¹: steht für unsubstituiertes oder substituiertes C₃-C₁₀-Cycloalkyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenoxy, Hydroxy, Oxo, C₂-C₆-Alkoxycarbonyl, C₁-C₆-Alkylcarbonyloxy, C₁-C₆-Alkylcarbonyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio oder - Q,
oder
- R¹: steht für unsubstituiertes oder substituiertes C₅-C₁₀-Cycloalkenyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Aklthio oder C₁-C₆-Halogenalkylthio,
oder
- R¹: steht für unsubstituiertes oder substituiertes Phenyl, wobei die Substituenten unabhängig voneinander ausgewählt sind aus folgender Liste:
Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆- Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₁₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -L⁸Q,
oder
- R¹: steht für gesättigtes oder teilweise oder vollständig ungesättigtes, unsubstituiertes oder substituietes, Naphthyl oder Indenyl, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
- R¹: steht für einen unsubstituierten oder substituierten 5- oder 6-gliedrigen Heteroarylrest, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -L⁸Q,
Substituenten am Stickstoff: C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
- R¹: steht für benzokondensiertes unsubstituiertes oder substituiertes 5- oder 6- gliedriges Heteroaryl, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
- R¹: steht für unsubstituiertes oder substituiertes C₅-C₁₅-Heterocyclyl, wobei L¹ an einem Kohlenstoff vom Heterocyclylrest gebunden ist und mögliche Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfmyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
- Q: steht für ein Phenyl, das bis zu zwei Substituenten enthalten kann, wobei die Substituenten unabhängig voneinander ausgewählt sind aus folgender Liste:
Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
oder
- Q: steht für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe oder Phenyl,
- R²: steht für C₁-C₄-Alkyl, C₁-C₄-Halogenalkyl oder C₂-C₄-Alkoxyalkyl,
- R³, R⁴: stehen unabhängig voneinander für Wasserstoff, C₁-C₄-Alkyl, C₂-C₆-Alkenyl, C₂-C₆-Alkinyl, C₁-C₃-Halogenalkyl, C₃-C₆-Cycloalkyl, Benzyl oder Phenyl,
- R⁵: steht für Wasserstoff, Halogen, Cyano, Hydroxy, C(=O)H, OC(=O)H, OC(=O)Me, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂-C₄-Halogenalkylcarbonyl, C₂-C₅-Alkoxycarbonyl, C₃-C₅-Alkoxycarbonylalkyl, C₂-C₅-Alkylaminocarbonyl, C₃-C₅-Dialkylaminocarbonyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
- R⁶: steht für Wasserstoff, C(=O)H, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂-C₄-Halogenalkylcarbonyl, C₂-C₅-Alkoxycarbonyl, C₃-C₅-Alkoxycarbonylalkyl, C₂-C₅-Alkylaminocarbonyl, C₃-C₅-Dialkylaminocarbonyl, C₁-C₄-Alkylsulfonyl oder C₁-C₄-Halogenalkylsulfonyl,
- R⁷, R⁸: sind unabhängig voneinander ausgewählt aus folgender Liste:
C₁-C₆-Akl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₈-Alkylcycloalkyl, C₄-C₈-Cycloalkylalkyl, C₄-C₈-Halogencycloalkylalkyl, C₅-C₈-Alkylcycloalkylalkyl, C₂-C₆-Alkoxyalkyl, C₄-C₈-Cycloalkoxyalkyl, C₃-C₆-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₆-Dialkylaminoalkyl, C₂-C₆-Halogenalkylaminoalkyl, C₄-C₈-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxylcarbonyl, C₂-C₆-Alkylaminocarbonyl, C₃-C₈-Dialkylaminocarbonyl, Tri(C₁-C₄-alkyl)silyl oder C₄-C₈-Cycloalkylaminocarbonyl,
- R⁹: steht für Wasserstoff, Cyano, Hydroxy, Amino, C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₄-C₈-Cycloalkylalkyl, C₂-C₆-Alkoxyalkyl, C₁-C₆-Alkoxy, C₁-C₆-Halogenalkoxy, C₁-C₆-Alkylsulfonyl, C₁-C₆-Halogenalkylsulfonyl, C₂-C₆-Alkylcarbonyl, C₂-C₆-Halogenalkylcarbonyl, C₁-C₆-Alkylamino, C₂-C₈-Dialkylamino, C₁-C₆-Halogenalkylamino oder C₂-C₈-Halogendialkylamino,
- R¹⁰: steht für Wasserstoff, C₁-C₆-Alkyl, C₂-C₆-Alkenyl, C₂-C₆-Alkinyl, C₁-C₆-Halogenalkyl oder C₃-C₆-Cycloalkyl,
oder
- R⁹, R¹⁰: bilden zusammen einen -(CH₂)₄-, -(CH₂)₅-, -(CH₂)₂S(CH₂)₂-, -(CH₂)₂S(=O)(CH₂)₂-, - (CH₂)₂S(=O)₂(CH₂)₂-, -(CH₂)₂NR³CH₂)₂- oder -(CH₂)₂O(CH₂)₂- Rest,
- R¹¹: steht für Wasserstoff, Halogen, Cyano, C₁-C₄-Alkyl, C₁-C₄-Halogenalkyl, C₂-C₄-Alkoxylalkyl, C₂-C₄-Alkylcarbonyl, C₂-C₄-Alkoxycarbonyl, C₂-C₃-Alkylaminocarbonyl oder C₃-C₆-Dialkylaminocarbonyl,
- R¹²: steht für Wasserstoff, Halogen, Cyano, Hydroxy, OC(=O)Me, OC(=O)H, C(=O)H, C(=O)OH, C(=O)OMe, C(=O)Me, C₁-C₄-Alkyl, C₂-C₄-Alkenyl, C₂-C₄-Alkinyl, C₁-C₄-Halogenalkyl, C₁-C₄-Alkoxy oder C₁-C₄-Halogenalkoxy,
- R¹³: steht für Wasserstoff, C₁-C₃-Alkyl, C₁-C₃-Halogenalkyl oder Halogen,
- R¹⁴: steht für Wasserstoff oder C₁-C₃-Alkyl, C(=O)H, C(=O)Me oder C(=O)OMe,
- R¹⁵: steht gleich oder verschieden unabhängig voneinander für Wasserstoff, Halogen, C₁-C₄-Alkyl, C₂-C₄-Alkenyl, C₂-C₄-Alkinyl, C₁-C₄-Alkoxy, Oxo, C₃-C₆-Cycloalkyl, C₁-C₄-Halogenalkyl oder Phenyl
- R¹⁶: steht für Wasserstoff, C₁-C₄-Alkyl oder C₁-C₄-Halogenalkyl,
- R¹⁷: steht für Wasserstoff, C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
sowie agrochemisch wirksame Salze davon,
wobei folgende Verbindungen ausgenommen sind:
a) Verbindungen in denen,
   - A²: für Pyrazol-1-yl steht,
   - X: für CH steht,
   - G: für G¹ steht,
b) Verbindungen in denen,
   - X: für Stickstoff steht,
   - G: für G¹³, G¹⁴, G¹⁷ oder G²⁰ steht,
c) Verbindungen, in denen,
   - Y⁴: für Schwefel steht,
   - L⁴: für C(=O) steht,

Je nach Art der oben definierten Substituenten weisen die Verbindungen der Formel (I) saure oder basische Eigenschaften auf und können mit anorganischen oder organischen Säuren oder mit anorganischen oder organischen Basen oder mit Metallionen Salze, gegebenenfalls auch innere Salze oder Addukte bilden. Als Metallionen kommen insbesondere die Ionen der Elemente der zweiten Hauptgruppe, insbesondere Calcium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkylresten, Mono-, Di- und Trialkanolamine von C₁-C₄-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden. Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf.

Die erfindungsgemäß verwendbaren Heteroarylpiperidin und -piperazin Derivate sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte (siehe auch unten unter "Erläuterungen der Verfahren und Zwischenprodukte) gleichermaßen.
- E: steht besonders bevorzugt für E²,
- A²: stehen außerdem bevorzugt für Phenyl, das bis zu zwei Substituenten enthalten kann, wobei die Substituenten unabhängig voneinander ausgewählt sind aus folgender Liste:
Halogen, Cyano, Hydroxy, Amino, Nitro, C₁-C₆-Alkyl, C₂-C₆-Alkenyl, C₂-C₆-Alkinyl, C₃-C₁₀-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenaklthio, C₁-C₄-Halogenalkylsulfonyl, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylcarbonyloxy, C(=O)H oder CR³=NOR⁴
- A²: stehen außerdem bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus folgender Liste:
Substituenten am Kohlenstoff:
Halogen, Cyano, Hydroxy, Amino, Nitro, C₁-C₆-Alkyl, C₂-C₆-Alkenyl, C₂-C₆-Alkinyl, C₃-C₁₀-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylcarbonyloxy, C(=O)H, CR³=NOR⁴ oder Phenyl
Substituenten am Stickstoff:
C₁-C₆-Alkyl, C₁-C₄-Halogenalkyl, C₂-C₆-Alkenyl, C₂-C₆-Halogenalkenyl, C₂-C₆-Alkinyl oder C₂-C₆-Halogenalkinyl.
- A²: stehen außerdem besonders bevorzugt für Phenyl, das bis zu zwei Substituenten enthalten kann, wobei die Substituenten unabhängig voneinander ausgewählt sind aus folgender Liste:
Halogen, Cyano, Nitro, C₁-C₃-Alkyl, C₁-C₃-Halogenalkyl, Cyclopropyl, C₁-C₃-Alkoxy, C₁-C₃-Halogenalkoxy, C₁-C₃-Alkylthio oder C₁-C₃-Halogenalkylthio,
- A²: stehen außerdem besonders bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus folgender Liste:
Substituenten am Kohlenstoff:
Halogen, Cyano, Nitro, C₁-C₃-Alkyl, C₁-C₃-Halogenalkyl, Cyclopropyl, C₁-C₃-Alkoxy, C₁-C₃-Halogenalkoxy, C₁-C₃-Alkylthio, C₁-C₃-Halogenalkylthio oder Phenyl,
Substituenten am Stickstoff:
C₁-C₃-Alkyl oder C₁-C₃-Halogenalkyl,
- A²: stehen außerdem ganz besonders bevorzugt für Phenyl, das bis zu zwei Substituenten enthalten kann, wobei die Substituenten unabhängig voneinander ausgewählt sind aus folgender Liste:
Chlor, Brom, Fluor, Iod, Methyl oder Trifluormethyl,
- A²: stehen außerdem ganz besonders bevorzugt für einen heteroaromatischen Rest ausgewählt aus folgender Gruppe: Furan-2-yl, Thiophen-2-yl, Thiophen-3-yl, Oxazol-4-yl, Thiazol-4-yl, Pyrazol-1-yl, Pyrazol-4-yl, 1,2,4-Triazol-1-yl, oder Pyridin-2-yl, der bis zu zwei Substituenten enthalten kann, wobei die Substituenten unabhängig voneinander ausgewählt sind aus folgender Liste:
Substituenten am Kohlenstoff:
Chlor, Methyl, Difluormethyl, Trifluormethyl oder Phenyl,
Substituenten am Stickstoff:
Methyl
- L¹: steht bevorzugt für eine direkte Bindung, -CH₂- oder -CH(CH₃)-,
- L¹: steht besonders bevorzugt für eine direkte Bindung oder -CH₂-,
- L¹: steht ganz besonders bevorzugt für eine direkte Bindung,
- L²: steht bevorzugt für eine direkte Bindung,
- L³: steht bevorzugt für eine direkte Bindung,
- L⁴: steht bevorzugt für Sauerstoff, CHR⁵, NR⁶,
- L⁴: steht besonders bevorzugt für CHR⁵, NR⁶,
steht besonders bevorzugt für CHR⁵
- L⁸: steht bevorzugt für eine direkte Bindung, -O-,
- Y¹: steht bevorzugt für Sauerstoff, Schwefel,
- Y¹: steht besonders bevorzugt für Sauerstoff,
- Y²: steht bevorzugt für Sauerstoff, Schwefel,
- Y²: steht besonders bevorzugt für Sauerstoff,
- Y³: steht bevorzugt für OR⁷, SR⁸,
- Y⁴: steht bevorzugt für Sauerstoff, Schwefel,
- Y⁴: steht besonders bevorzugt für Sauerstoff,
- X: steht Bevorzugt für CH, CF, N,
- X: steht besonders bevorzugt für CH,
- G: steht Bevorzugt für G¹, G², G³, G¹³ G¹⁴ oder G¹⁸
- G: steht besonders bevorzugt für G¹, G¹⁸
- R¹: steht außerdem bevorzugt für unsubstituiertes oder substituiertes C₃-C₁₀-Cycloalkyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio oder -Q,
- R¹: steht außerdem bevorzugt für unsubstituiertes oder substituiertes C₅-C₁₀-Cycloalkenyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
- R¹: steht außerdem bevorzugt für unsubstituiertes oder substituiertes Phenyl, wobei die Substituenten unabhängig voneinander ausgewählt sind aus folgender Liste:
Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆- Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkyl-sulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -L⁸Q,
- R¹: steht außerdem bevorzugt für gesättigtes oder teilweise oder vollständig ungesättigtes, unsubstituiertes oder substituietes, Naphthyl oder Indenyl, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
- R¹: steht außerdem bevorzugt für einen unsubstituierten oder substituierten 5- oder 6-gliedrigen Heteroarylrest, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -L⁸Q,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
- R¹: steht außerdem bevorzugt für benzokondensiertes unsubstituiertes oder substituiertes 5- oder 6- gliedriges Heteroaryl, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
- R¹: steht außerdem bevorzugt für unsubstituiertes oder substituiertes C₅-C₁₅-Heterocyclyl, wobei L¹ an einem Kohlenstoff vom Heterocyclylrest gebunden ist und wobei mögliche Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
- R¹: steht besonders bevorzugt für C₅-C₁₀-Cycloalkyl, das bis zu zwei Substituenten enthalten kann, wobei die Substituenten unabhängig voneinander ausgewählt sind aus folgender Liste:
Cyano, Halogen, C₁-C₄-Alkyl, C₁-C₃-Halogenalkyl, C₃-C₆-Cycloalkyl, C₂-C₄-Alkenyl, C₂-C₄-Halogenalkenyl, C₂-C₄-Alkinyl, C₂-C₄-Halogenalkinyl, Tri(C₁-C₄-alkyl)silyl, Phenyl, Hydroxy, Oxo, C₁-C₄-Alkoxy, C₁-C₃-Halogenalkoxy, C₂-C₄-Alkenyloxy, C₂-C₄-Alkinyloxy, C₁-C₄-Alkylthio oder C₁-C₃-Halogenalkylthio,
- R¹: steht außerdem besonders bevorzugt für C₅-C₁₀-Cycloalkenyl, das bis zu zwei Substituenten enthalten kann, wobei die Substituenten unabhängig voneinander ausgewählt sind aus folgender Liste:
Cyano, Halogen, C₁-C₄-Alkyl, C₁-C₃-Halogenalkyl, C₃-C₆-Cycloalkyl, C₂-C₄-Alkenyl, C₂-C₄-Halogenalkenyl, C₂-C₄-Alkinyl, C₂-C₄-Halogenalkinyl, Tri(C₁-C₄-alkyl)silyl, Phenyl, Hydroxy, Oxo, C₁-C₄-Alkoxy, C₁-C₃-Halogenalkoxy, C₂-C₄-Alkenyloxy, C₂-C₄-Alkinyloxy, C₁-C₄-Alkylthio oder C₁-C₃-Halogenalkylthio,
- R¹: steht außerdem besonders bevorzugt für Phenyl, das bis zu drei Substituenten enthalten kann, wobei die Substituenten unabhängig voneinander ausgewählt sind aus folgender Liste:
Halogen, Cyano, Nitro, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-Alkylthio, C₁-C₆-Halogenalkylthio, C₁-C₆-Alkylsulfinyl, C₁-C₆-Halogenalkylsulfinyl, C₁-C₆-Aklsulfonyl, C₁-C₆-Halogenalkylsulfonyl, Tri(C₁-C₄-alkyl)silyl oder -L⁸Q,
- R¹: steht außerdem besonders bevorzugt für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, das bis zu drei Substituenten enthalten kann, wobei die Substituenten unabhängig voneinander ausgewählt sind aus folgender Liste:
Cyano, Nitro, Halogen, C₁-C₄-Alkyl, C₁-C₃-Halogenalkyl, C₃-C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, Hydroxy, C₁-C₄-Alkoxy, C₁-C₃-Halogenalkoxy, C₂-C₄-Alkenyloxy, C₂-C₄-Alkinyloxy, C₁-C₄-Alkylthio oder C₁-C₄-Halogenalkylthio,
- R¹: steht außerdem besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste:
Substituenten am Kohlenstoff: Halogen, Cyano, Nitro, C₁-C₆-Akl, C₂-C₆-Alkenyl, C₂-C₆-Alkinyl, C₁-C₆-Halogenalkyl, C₂-C₆-Halogena!kenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₁-C₄-Alkoxy, C₁-C₄-Halogenalkoxy, C₂-C₆-Alkylcarbonyloxy, C₂-C₆-Alkylcarbonylthio, C₁-C₄-Akylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -L⁸Q,
Substituenten am Stickstoff: C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl oder Phenyl,
- R¹: steht außerdem besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, das bis zu zwei Substituenten enthalten kann, wobei die Substituenten unabhängig voneinander ausgewählt sind aus folgender Liste:
Substituenten am Kohlenstoff: Halogen, Cyano, Nitro, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₁-C₄-Alkoxy, C_{I}-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄ Alkylsulfonyl, C₁-C₄-Halogenalkylsulfonyl, Tri(C₁-C₄-alkyl)silyl oder Phenyl,
Substituenten am Stickstoff: C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl oder Phenyl,
- R¹: steht außerdem besonders bevorzugt für C₅-C₁₀-Heterocyclyl, wobei L¹ an einem Kohlenstoff vom Heterocyclylrest gebunden ist und der Heterocyclylrest bis zu zwei Substituenten enthalten kann, wobei die Substituenten unabhängig voneinander ausgewählt sind aus folgender Liste:
Substituenten am Kohlenstoff: Halogen, Cyano, Hydroxy, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₂-C₄-Alkoxyalkyl, C₁-C₄-Alkoxy, C₁-C₄-Halogenalkoxy, C₁-C₄-Alkylthio, C₁-C₄-Halogenalkylthio, Tri(C₁-C₄-alkyl)silyl oder Phenyl,
Substituenten am Stickstoff: C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl oder Phenyl,
- R¹: steht ganz besonders bevorzugt für Cyclopentyl, Cyclohexyl, Cycloheptyl oder Cyclooctyl
- R¹: steht außerdem ganz besonders bevorzugt für Cyclopent-2-en-1-yl, Cyclopent-3-en-1-yl, Cyclohex-1-en-1-yl, Cyclohex-2-en-1-yl, Cyclohex-3-en-1-yl, Cyclohept-1-en-1-yl, Cyclohept-2-en-1-yl, Cyclohept-3-en-1-yl oder Cyclohept-4-en-1-yl,
- R¹: steht außerdem ganz besonders bevorzugt für Phenyl, das bis zu zwei Substituenten enthalten kann, wobei die Substituenten unabhängig voneinander ausgewählt sind aus folgender Liste:
Chlor, Fluor, Brom, Iod, Cyano, Nitro, -CH₃, -CH₂CH₃, -CH₂CH₂CH₃, -CH(CH₃)₂,-CH₂CH₂CH₂CH₃, -CH(CH₃)CH₂CH₃, -CH₂CH(CH₃)CH₃, -C(CH₃)₃, -CH=CH₂,-CH=CHCH₃, -CH₂CH=CH₂, -CH=CHCH₂CH₃, -CH₂CH=CHCH₃,-CH₂CH₂CH=CH₂, -C=CH, -C=CCH₃, -CH₂C=CH, - C=CCH₂CH₃, -CH₂C=CCH₃,-CH₂CH₂C=CH, -CF₃, -CFH₂, -CF₂H, -CF₂CF₃, CCm₃, Cyclopropyl, Cyclobutyl, Cyclopentyl, Cyclohexyl, -CH₂OCH₃, -CH₂OCH₂CH₃, -CH₂CH₂OCH₃,-CH₂OCH₂CH₂CH₃, -CH₂CH₂OCH₂CH₃, -CH₂CH₂CH₂OCH₃, -C(=O)CH₃,-C(=O)CH₂CH₃, C(=O)CH₂CH₂CH₃, C(=O)CH(CH₃)₂, -C(=O)CF₃, -C(=O)OCH₃,-C(=O)OCH₂CH₃, -C(=O)OCH₂CH₂CH₃, -C(=O)OCH(CH₃)₂, -OCH₃, -OCH₂CH₃,-OCH₂CH₂CH₃, -OCH(,CH₃)₂, -OCH₂CH₂CH₂CH₃, -OCH₂CH(CH₃)₂,-OCH(CH₃)CH₂CH₃, -OC(CH₃)₃, -OCF₃, -OCF₂H, -OCH₂CF₃, -OCF₂CF₃, O-Cyclohexyl, O-Cyclopentyl, O-Cyclopropyl, -SCH₃, -SCH₂CH₃, -SCH₂CH₂CH₃,-SCH(CH₃)₂, -SCH₂CH₂CH₂CH₃, -SCH₂CH(CH₃)₂, -SCH(CH₃)CH₂CH₃, -SC(CH₃)₃, -SCF₃, -SCF₂H, -SCH₂CF₃, -SCF₂CF₃, -S(=O)Me, -S(O)CF₃, -S(=O)₂Me,-S(O)₂CF₃, -OCH₂CH=CH₂, -OCH₂C=CH, -OCH₂OCH₃, -OCH₂OCH₂CH₃,-OCH₂CH₂OCH₃, -OCH₂OCH(CH₃)₂, Trimethylsilyl, Phenyl oder Phenoxy
- R¹: steht außerdem ganz besonders bevorzugt für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
- R¹: steht außerdem ganz besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste:
Substituenten am Kohlenstoff: Chlor, Fluor, Brom, Iod, Cyano, Nitro, -CH₃,-CH₂CH₃, -CH₂CH₂CH₃, -CH(CH₃)₂, -CH₂CH₂CH₂CH₃, -CH(CH₃)CH₂CH₃,-CH₂CH(CH₃)CH₃, -C(CH₃)₃, -CH=CH₂, -CH=CHCH₃, -CH₂CH=CH₂,-CH=CHCH₂CH₃, -CH₂CH=CHCH₃, -CH₂CH₂CH=CH₂, -C=CH, -C=CCH₃,-CH₂C=CH, - C=CCH₂CH₃, -CH₂C=CCH₃, -CH₂CH₂C=CH, -CF₃, -CFH₂, -CF₂H,-CF₂CF₃, -CCl₃, Cyclopropyl, Cyclobutyl, Cyclopentyl, Cyclohexyl, -CH₂OCH₃,-CH₂OCH₂CH₃, -CH₂CH₂OCH₃, -CH₂OCH₂CH₂CH₃, -CH₂CH₂OCH₂CH₃,-CH₂CH₂CH₂OCH₃, -OCH₃, -OCH₂CH₃, -OCH₂CH₂CH₃, -OCH(CH₃)₂,-OCH₂CH₂CH₂CH₃, -OCH₂CH(CH₃)₂, -OCH(CH₃)CH₂CH₃, -OC(CH₃)₃, -CF3,-OCF₂H, -OCH₂CF₃, -OCF₂CF₃, -SCH₃, -SCH₂CH₃, -SCH₂CH₂CH₃, -SCH(CH₃)₂,-SCH₂CH₂CH₂CH₃, -SCH₂CH(CH₃)₂, -SCH(CH₃)CH₂CH₃, -SC(CH₃)₃, -CF3,-SCF₂H, -SCH₂CF₃, -SCF₂CF₃, -S(=0)Me, -S(O)CF₃, -S(=O)₂Me,-S(O)₂CF₃,Trimethylsilyl, Phenyl oder Phenoxy
Substituenten am Stickstoff: -CH₃, -CH₂CH₃, -CH₂CH₂CH₃, -CH(CH₃)₂,-CH₂CH₂CH₂CH₃, -CH(CH₃)CH₂CH₃, -CH₂CH(CH₃)CH₃, -C(CH₃)₃, -CH=CH₂,-CH=CHCH₃, -CH₂CH=CH₂, -CH=CHCH₂CH₃, -CH₂CH=CHCH₃,-CH₂CH₂CH=CH₂, C=CH, -C=CCH₃, -CH₂C=CH, - C=CCH₂CH₃, -CH₂C=CCH₃,-CH₂CH₂C=CH, -CF₃, -CFH₂, -CF₂H, -CF₂CF₃, -CCl₃, Cyclopropyl, Cyclobutyl, Cyclopentyl, Cyclohexyl oder Phenyl,
- R¹: steht außerdem ganz besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl,
- R¹: steht außerdem ganz besonders bevorzugt für Piperidin-1-yl, Piperidin-2-yl, Piperidin-3-yl, Piperidin-4-yl, Piperazin-1-yl, Piperazin-2-yl, Piperazin-3-yl, Morpholin-1-yl, Morpholin-2-yl, Morpholin-3-yl, Tetrahydropyran-2-yl, Tetrahydropyran-3-yl, Tetrahydropyran-4-yl, 1,2,3,4-Tetrahydrochinolin-1-yl, 1,2,3,4-Tetrahydroisochinolin-2-yl, 1,2,3,4-Tetrahydrochinoxalin-1-yl, Indolin-1-yl, Isoindolin-2-yl, Decahydrochinolin-1-yl oder Decahydroisochinolin-2-yl,
- R¹: steht insbesondere bevorzugt für Cyclohexyl,
- R¹: steht außerdem insbesondere bevorzugt für Phenyl, das bis zu zwei Substituenten enthalten kann, wobei die Substituenten unabhängig voneinander ausgewählt sind aus folgender Liste:
Fluor, Chlor, Trifluormethyl, Methoxy,
- R¹: steht außerdem insbesondere bevorzugt für Naphthalen-1-yl, Naphthalen-2-yl, 2,3-Dihydro-1H-Inden-2-yl, 1,2,3,4-Tetrahydronaphthalen-1-yl oder 1,2,3,4-Tetrahydronaphthalen-2-yl,
- R¹: steht außerdem insbesondere bevorzugt für Chinolin-8-yl,
- Q: steht bevorzugt für Phenyl, das bis zu zwei Substituenten enthalten kann, wobei die Substituenten unabhängig voneinander ausgewählt sind aus folgender Liste:
Halogen, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
- Q: steht außerdem bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Iso-thiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste:
Substituenten am Kohlenstoff:
Halogen, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Substituenten am Stickstoff: C₁-C₆-Alkyl, C₂-C₆-Alkenyl, C₂-C₆-Alkinyl, C₃-C₆-Cycloalkyl oder Phenyl,
- Q: steht besonders bevorzugt für Phenyl,
- Q: steht außerdem besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
- Q: steht ganz besonders bevorzugt für Phenyl,
- R³, R⁴: stehen unabhängig voneinander bevorzugt für Wasserstoff,
- R³, R⁴: stehen unabhängig voneinander außerdem bevorzugt für Methyl, Ethyl, Propyl, *iso-*Propyl, Butyl, *iso*-Butyl oder *tert*-Butyl,
- R⁵: steht bevorzugt für Wasserstoff,
- R⁵: steht außerdem bevorzugt für Methyl, Ethyl, Propyl, *iso*-Propyl, Butyl, *iso*-Butyl oder *tert*-Butyl,
- R⁵: steht besonders bevorzugt für Wasserstoff,
- R⁵: steht außerdem besonders bevorzugt für Methyl,
- R⁶: steht bevorzugt für Wasserstoff,
- R⁶: steht außerdem bevorzugt für Methyl, Ethyl, Propyl, *iso*-Propyl, Butyl, *iso*-Butyl oder *tert*-Butyl,
- R⁶: steht besonders bevorzugt für Wasserstoff,
- R⁶: steht außerdem besonders bevorzugt für Methyl,
- R⁷: steht bevorzugt für Methyl, Ethyl, Propyl, *iso-*Propyl, Butyl, *iso*-Butyl oder *tert-*Butyl,
- R⁷: steht besonders bevorzugt für Methyl,
- R⁸: steht bevorzugt für Methyl, Ethyl, Propyl, *iso*-Propyl, Butyl, *iso*-Butyl oder *tert-*Butyl,
- R⁸: steht besonders bevorzugt für Methyl,
- R¹³: steht bevorzugt für Wassertoff,

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Ganz besonders bevorzugt sind solche Verbindungen der Formel (I), in welcher alle Reste jeweils die oben genannten ganz besonders bevorzugten Bedeutungen haben.

Insbesondere bevorzugt sind solche Verbindungen der Formel (I), in welcher alle Reste jeweils die oben genannten insbesondere bevorzugten Bedeutungen haben.

Weiterhin bevorzugt sind Verbindungen der Formel (I), in welcher E für (2,5-Dibromphenyl)acetyl steht.

Weiterhin bevorzugt sind Verbindungen der Formel (I), in welcher E für (2,5-Dichlorphenyl)acetyl steht.

Weiterhin bevorzugt sind Verbindungen der Formel (I), in welcher E für (2,5-Difluorbenzyl)sulfonyl steht.

Weiterhin bevorzugt sind Verbindungen der Formel (I), in welcher E für (2,5-Dimethyl-1,3-thiazol-4-yl)acetyl steht.

Weiterhin bevorzugt sind Verbindungen der Formel (I), in welcher E für (2,5-Dimethylthiophen-3-yl)acetyl steht.

Weiterhin bevorzugt sind Verbindungen der Formel (I), in welcher E für (3,5-Dimethyl-1H-1,2,4-triazol-1-yl)acetyl steht.

Weiterhin bevorzugt sind Verbindungen der Formel (I), in welcher E für (3-Chlorphenyl)acetyl steht.

Weiterhin bevorzugt sind Verbindungen der Formel (I), in welcher E für (5-Brom-2-methylphenyl)acetyl steht.

Weiterhin bevorzugt sind Verbindungen der Formel (I), in welcher E für (5-Chlor-1-methyl-1H-pyrazol-4-yl)acetyl steht.

Weiterhin bevorzugt sind Verbindungen der Formel (I), in welcher E für (5-Iod-2-methylphenyl)acetyl steht.

Weiterhin bevorzugt sind Verbindungen der Formel (I), in welcher E für (5-Methyl-2-phenyl-1,3-oxazol-4-yl)acetyl steht.

Weiterhin bevorzugt sind Verbindungen der Formel (I), in welcher E für (5-Methyl-2-phenyl-1,3-thiazol-4-yl)acetyl steht.

Weiterhin bevorzugt sind Verbindungen der Formel (I), in welcher E für [2,5-Bis(trifluormethyl)phenyl] acetyl steht.

Weiterhin bevorzugt sind Verbindungen der Formel (I), in welcher E für [2-Brom-5-(trifluormethyl)phenyl]acetyl steht.

Weiterhin bevorzugt sind Verbindungen der Formel (I), in welcher E für [2-Chlor-5-(trifluormethyl)phenyl]acetyl steht.

Weiterhin bevorzugt sind Verbindungen der Formel (I), in welcher E für [2-Methyl-5-(trifluormethyl)phenoxy]carbonyl steht.

Weiterhin bevorzugt sind Verbindungen der Formel (I), in welcher E für [3-(Trifluormethyl)phenyl]acetyl steht.

Weiterhin bevorzugt sind Verbindungen der Formel (I), in welcher E für [3-(Trifluoromethyl)cyclohexyl] acetyl steht.

Weiterhin bevorzugt sind Verbindungen der Formel (I), in welcher E für [3-Chlor-6-(trifluormethyl)pyridin-2-yl] acetyl steht.

Weiterhin bevorzugt sind Verbindungen der Formel (I), in welcher E für [5-Chlor-2-(trifluormethyl)phenyl]acetyl steht.

Weiterhin bevorzugt sind Verbindungen der Formel (I), in welcher E für [5-Methyl-3-(trifluormethyl)-1H-pyrazol-1-yl]acetyl steht.

Weiterhin bevorzugt sind Verbindungen der Formel (I), in welcher E für 5,5,6,6,6-Pentafluorohexanoyl steht.

Weiterhin bevorzugt sind Verbindungen der Formel (I), in welcher E für Heptanoyl steht.

Weiterhin bevorzugt sind Verbindungen der Formel (I), in welcher E für (2,5-Dimethylphenyl)carbamoyl steht.

Weiterhin bevorzugt sind Verbindungen der Formel (I), in welcher E für Furan-2-yl(oxo)acetyl steht.

Weiterhin bevorzugt sind Verbindungen der Formel (I), in welcher E für Oxo(thiophen-2-yl)acetyl steht.

Weiterhin bevorzugt sind Verbindungen der Formel (I), in welcher E für Pentanoyl steht.

Weiterhin bevorzugt sind Verbindungen der Formel (I), in welcher E für Thiophen-3-ylacetyl steht.

Weiterhin bevorzugt sind Verbindungen der Formel (I), in welcher E für [2-Chlor-5-(trifluormethyl)phenyl]carbamoyl steht.

Weiterhin bevorzugt sind Verbindungen der Formel (I), in welcher E für (2-Chlor-5-methylphenyl)carbamoyl steht.

Weiterhin bevorzugt sind Verbindungen der Formel (I), in welcher E für (2,5-Dimethylphenyl)carbamothioyl steht.

Weiterhin bevorzugt sind Verbindungen der Formel (I), in welcher E für (2-Fluor-5-methylphenyl)carbamoyl steht.

Weiterhin bevorzugt sind Verbindungen der Formel (I), in welcher E für (3,5-Dimethylphenyl)acetyl steht.

Weiterhin bevorzugt sind Verbindungen der Formel (I), in welcher E für (2,4-Dimethylphenyl)acetyl steht.

Weiterhin bevorzugt sind Verbindungen der Formel (I), in welcher E für (2,5-Difluorphenyl)carbamothioyl steht.

Weiterhin bevorzugt sind Verbindungen der Formel (I), in welcher E für [3,5-Bis(difluormethyl)-1H-pyrazol-1-yl]acetyl steht.

Weiterhin bevorzugt sind Verbindungen der Formel (I), in welcher E für (Z)-{[2-Chlor-5-(trifluormethyl)phenyl]imino} (methoxy)methyl steht.

Weiterhin bevorzugt sind Verbindungen der Formel (I), in welcher E für (2-Methoxy-5-methylphenyl)carbamoyl steht.

Weiterhin bevorzugt sind Verbindungen der Formel (I), in welcher E für (5-Chlor-2-methylphenyl)carbamoyl steht.

Weiterhin bevorzugt sind Verbindungen der Formel (I), in welcher E für (3,5-Dimethyl-1,2-oxazol-4-yl)carbamothioyl steht.

Weiterhin bevorzugt sind Verbindungen der Formel (I), in welcher E für (5-Fluor-2-methylphenyl)carbamoyl steht.

Weiterhin bevorzugt sind Verbindungen der Formel (I), in welcher E für (5-Chlor-2-methoxyphenyl)carbamoyl steht.

Weiterhin bevorzugt sind Verbindungen der Formel (I), in welcher E für (2,5-Dimethylphenyl)carbamothioyl steht.

Weiterhin bevorzugt sind Verbindungen der Formel (I), in welcher E für (2,5-Dimethoxyphenyl)carbamoyl steht.

Weiterhin bevorzugt sind Verbindungen der Formel (I), in welcher E für Cyclopentylacetyl steht.

Weiterhin bevorzugt sind Verbindungen der Formel (I), in welcher E für (2,5-Difluorphenyl)carbamoyl steht.

Weiterhin bevorzugt sind Verbindungen der Formel (I), in welcher E für (1,3-Dimethyl-1H-pyrazol-5-yl)acetyl steht.

Weiterhin bevorzugt sind Verbindungen der Formel (I), in welcher E für (1,5-Dimethyl-1H-pyrazol-3-yl)acetyl steht.

Weiterhin bevorzugt sind Verbindungen der Formel (I), in welcher E für (2-Methyl-5-nitrophenyl)carbamoyl steht.

Weiterhin bevorzugt sind Verbindungen der Formel (I), in welcher E für (2,5-Dichlorphenyl)carbamoyl steht.

Weiterhin bevorzugt sind Verbindungen der Formel (I), in welcher E für (2,5-Dimethylphenyl)acetyl steht.

Weiterhin bevorzugt sind Verbindungen der Formel (I), in welcher E für (3,5-Dimethyl-1,2-oxazol-4-yl)carbamoyl steht.

Weiterhin bevorzugt sind Verbindungen der Formel (I), in welcher E für {[1-Methyl-3-(trifluormethyl)-1H-pyrazol-5-yl]oxy}carbonyl steht.

Weiterhin bevorzugt sind Verbindungen der Formel (I), in welcher E für [2-Fluor-5-(trifluormethyl)phenyl]carbamoyl steht.

Weiterhin bevorzugt sind Verbindungen der Formel (I), in welcher E für (5-Chlor-2-methylphenyl)carbamothioyl steht.

Weiterhin bevorzugt sind Verbindungen der Formel (I), in welcher E für (2,5-Dichlorphenyl)carbamothioyl steht.

Weiterhin bevorzugt sind Verbindungen der Formel (I), in welcher E für (2-Brom-5-fluorphenyl)carbamothioyl steht.

Weiterhin bevorzugt sind Verbindungen der Formel (I), in welcher E für (5-Chlor-2-fluorphenyl)carbamothioyl steht.

Weiterhin bevorzugt sind Verbindungen der Formel (I), in welcher E für (5-Fluor-2-methylphenyl)carbamothioyl steht.

Weiterhin bevorzugt sind Verbindungen der Formel (I), in welcher E für [2-Fluor-5-(trifluormethyl)phenyl]carbamothioyl steht.

Weiterhin bevorzugt sind Verbindungen der Formel (I), in welcher E für (2-Methoxyethoxy)acetyl steht.

Weiterhin bevorzugt sind Verbindungen der Formel (I), in welcher Y¹ für Sauerstoff steht.

Weiterhin bevorzugt sind Verbindungen der Formel (I), in welcher Y² für Sauerstoff steht.

Weiterhin bevorzugt sind Verbindungen der Formel (I), in welcher Y² für Schwefel steht.

Weiterhin bevorzugt sind Verbindungen der Formel (I), in welcher X für CH steht.

Weiterhin bevorzugt sind Verbindungen der Formel (I), in welcher X für CF steht.

Weiterhin bevorzugt sind Verbindungen der Formel (I), in welcher X für Stickstoff steht.

Weiterhin bevorzugt sind Verbindungen der Formel (I), in welcher G für G¹ steht.

Weiterhin bevorzugt sind Verbindungen der Formel (I), in welcher G für G¹⁸ steht.

Weiterhin bevorzugt sind Verbindungen der Formel (I), in welcher L¹ für eine direkte Bindung steht.

Weiterhin bevorzugt sind Verbindungen der Formel (I), in welcher L¹ für -CH₂- steht.

Weiterhin bevorzugt sind Verbindungen der Formel (I), in welcher L¹ für -CHCH₃- steht.

Weiterhin bevorzugt sind Verbindungen der Formel (I), in welcher R¹ für Cyclohexyl steht.

Weiterhin bevorzugt sind Verbindungen der Formel (I), in welcher R¹ für (3-Trifluormethyl)cyclohexyl steht.

Weiterhin bevorzugt sind Verbindungen der Formel (I), in welcher R¹ für 2-Chlorphenyl steht.

Weiterhin bevorzugt sind Verbindungen der Formel (I), in welcher R¹ für 2,4-Dichlorophenyl steht.

Weiterhin bevorzugt sind Verbindungen der Formel (I), in welcher R¹ für 2,5-Dichlorphenyl steht.

Weiterhin bevorzugt sind Verbindungen der Formel (I), in welcher R¹ für 2,6-Difluorphenyl steht.

Weiterhin bevorzugt sind Verbindungen der Formel (I), in welcher R¹ für 2,4-Difluorphenyl steht.

Weiterhin bevorzugt sind Verbindungen der Formel (I), in welcher R¹ für 2-Chlor-5-(trifluormethyl)phenyl steht.

Weiterhin bevorzugt sind Verbindungen der Formel (I), in welcher R¹ für 4-Methoxyphenyl steht.

Weiterhin bevorzugt sind Verbindungen der Formel (I), in welcher R¹ für Naphthalen-1-yl steht.

Weiterhin bevorzugt sind Verbindungen der Formel (I), in welcher R¹ für Naphthalen-2-yl steht.

Weiterhin bevorzugt sind Verbindungen der Formel (I), in welcher R¹ für 2,3-Dihydro-1H-inden-2-yl steht.

Weiterhin bevorzugt sind Verbindungen der Formel (I), in welcher R¹ für 1,2,3,4-Tetrahydronaphthalen-2-yl steht.

Weiterhin bevorzugt sind Verbindungen der Formel (I), in welcher R¹ für 1,2,3,4-Tetrahydronaphthalen-1-yl steht.

Weiterhin bevorzugt sind Verbindungen der Formel (I), in welcher R¹ für Chinolin-8-yl steht.

Weiterhin bevorzugt sind Verbindungen der Formel (I), in welcher R¹ für (1R)-1,2,3,4-Tetrahydronaphthalen-1-yl steht.

Weiterhin bevorzugt sind Verbindungen der Formel (I), in welcher R¹ für (1R,2S)-2-Phenylcyclohexyl steht.

Weiterhin bevorzugt sind Verbindungen der Formel (I), in welcher R¹ für (1S,2R)-2-Phenylcyclohexyl steht.

Weiterhin bevorzugt sind Verbindungen der Formel (I), in welcher R¹ für (1R,2S)-2-(3,4-Difluorphenyl)cyclohexyl steht.

Weiterhin bevorzugt sind Verbindungen der Formel (I), in welcher R¹ für 2,3-Dimethylphenyl steht.

Weiterhin bevorzugt sind Verbindungen der Formel (I), in welcher R¹ für 2,6-Dibromphenyl steht.

Weiterhin bevorzugt sind Verbindungen der Formel (I), in welcher R¹ für 2,6-Dichlorphenyl steht.

Weiterhin bevorzugt sind Verbindungen der Formel (I), in welcher R¹ für 2-Brom-6-fluorphenyl steht.

Weiterhin bevorzugt sind Verbindungen der Formel (I), in welcher R¹ für 2-Brom-6-methoxyphenyl steht.

Weiterhin bevorzugt sind Verbindungen der Formel (I), in welcher R¹ für 2-Brom-6-methylphenyl steht.

Weiterhin bevorzugt sind Verbindungen der Formel (I), in welcher R¹ für 2-Bromphenyl steht.

Weiterhin bevorzugt sind Verbindungen der Formel (I), in welcher R¹ für 2-Chlor-6-fluorphenyl steht.

Weiterhin bevorzugt sind Verbindungen der Formel (I), in welcher R¹ für 2-Chlor-6-methylphenyl steht.

Weiterhin bevorzugt sind Verbindungen der Formel (I), in welcher R¹ für 2-Fluor-6-methoxyphenyl steht.

Weiterhin bevorzugt sind Verbindungen der Formel (I), in welcher R¹ für 2-Fluorphenyl steht.

Weiterhin bevorzugt sind Verbindungen der Formel (I), in welcher R¹ für 2-Iodphenyl steht.

Weiterhin bevorzugt sind Verbindungen der Formel (I), in welcher R¹ für 3-Chlorpyridin-4-yl steht.

Weiterhin bevorzugt sind Verbindungen der Formel (I), in welcher R¹³ für Wassertoff steht.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Iod;
**Alkyl:** gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkenyl:** ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl; "Alkenyl" umfasst ebenfalls Polyene, z.B. (aber nicht beschränkt auf) C₃-C₆-Polyene wie 1,2-Propadienyl und 2,4-Hexadienyl;
**Alkinyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl; "Alkinyl" umfasst ebenfalls Fragmente mit mehreren Dreifachbindungen, z.B. (aber nicht beschränkt auf) C₄-C₆-Polyene wie 2,5 Hexadiinyl;
**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy; "Alkoxy" umfasst ebenfalls "Alkenyloxy", z.B. (aber nicht beschränkt auf) C₂-C₆-Alkenyloxy wie H₂C=CHCH₂O, (CH₃)₂C=CHCH₂O, CH₃CH=CHCH₂O, CH₃CH=C(CH₃)CH₂O und CH₂=CHCH₂CH₂O; "Alkoxy" umfasst ebenfalls "Alkinyloxy", z.B. (aber nicht beschränkt auf) C₂-C₆-Alkinyloxy wie HC ≡CCH₂O, CH₃C≡CCH₂O, und CH₃C≡CCH₂CH₂O;
**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl; Alkylsulfinyl umfasst beide Enantiomere der Alkylsulfinyl Fragmente.
**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome: :** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;

### Erläuterung der Verfahren und Zwischenprodukte

Die Heteroarylpiperidin und -piperazin Derivate der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

### Verfahren A

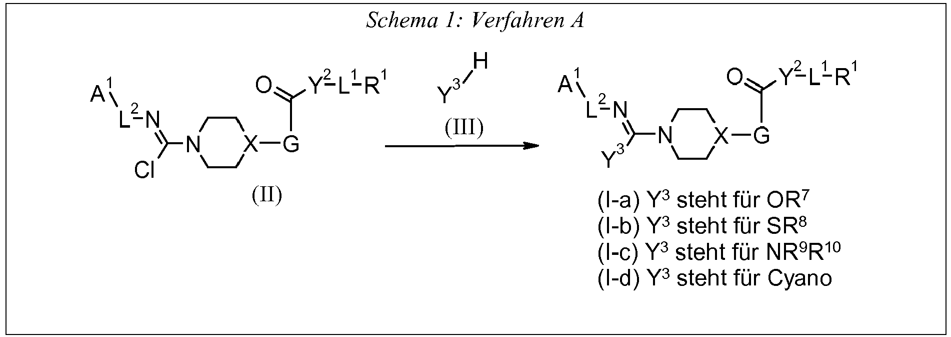

Die bei der Durchführung des erfindungsgemäßen Verfahrens A als Ausgangstoffe benötigten Verbindungen der Struktur (II) werden weiter unten bei Verfahren M beschrieben.

Das Verfahren A wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die bei der Durchführung des erfindungsgemäßen Verfahrens A erhaltenen Verbindungen der Struktur (I) können alternativ teilweise auch ohne Verwendung eines Säureakzeptors als korrespondierende Säurechloride [(I)-HCl] erhalten werden. Bei Bedarf erfolgt die Freisetzung der Verbindungen der Struktur (I) nach literaturbeschriebenen Methoden.

Die bei der Durchführung des erfindungsgemäßen Verfahrens A erhaltenen Produkte der Formel I-a, I-c und I-d können teilweise auch durch Reaktion einer Verbindung I-b mit einer Verbindung III erhalten werden. So ist beispielsweise die Synthese von Thiouronium-Salzen und deren Reaktion zu Guanidinen literaturbeschrieben (Synthesis (1988), 6, 460-466).

Das erfindungsgemäße Verfahren A wird vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan;

Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens A in einem größeren Bereich variiert werden. Im Allgemeinen arbeitet man bei Temperaturen von -78 °C bis +150 °C, vorzugsweise bei Temperaturen von -78 °C bis +50 °C, ganz besonders bevorzugt bei 0 °C bis 30 °C.

Zur Durchführung der Reaktion des erfindungsgemäßen Verfahrens A setzt man pro Mol an Verbindung der Formel (II) im Allgemeinen 0,5 bis 20 Mol, vorzugsweise 1 bis 5 Mol an Verbindung (III) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

Die bei der Durchführung des erfindungsgemäßen Verfahrens A erhaltenen Produkte I-a, I-b, I-c und I-d lassen sich allgemein als Isoharnstoffe, Isothioharnstoffe, Guanidine und Cyanoamidine bezeichnen. Ausgewählte Refernzen dieser Verbindungen sind Organic Preparations and Procedures International (1980), 12 (5), 309-326, Comprehensive Organic Chemistry, vol. 2, Pergamon Press, Oxford, Rodd's Chemistry of Carbon Compounds, vol. 1C, Elsevier, New York und Journal of Organic Chemistry (2004), 69, 309-313.

### Verfahren B

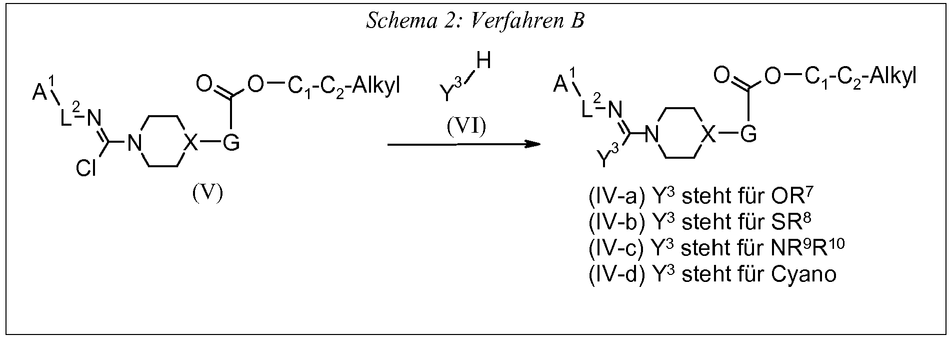

Die bei der Durchführung des erfindungsgemäßen Verfahrens B als Ausgangstoffe benötigten Verbindungen der Struktur (V) werden weiter unten bei Verfahren N beschrieben.

Das Verfahren B wird analog dem Verfahren A durchgeführt.

### Verfahren C

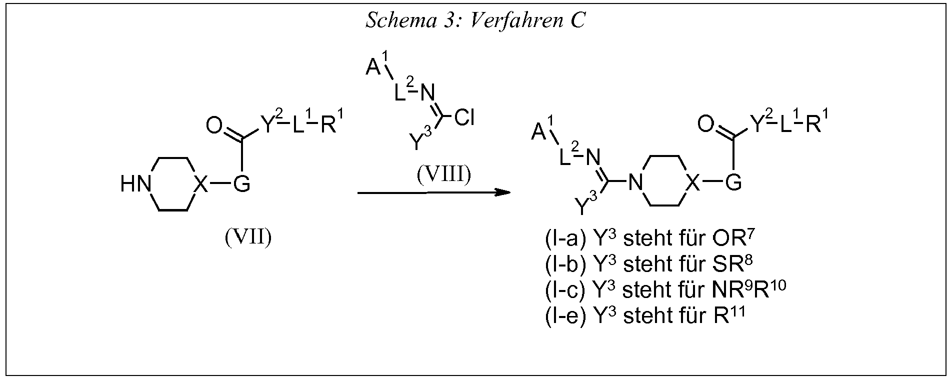

Die bei der Durchführung des erfindungsgemäßen Verfahrens C als Ausgangstoffe benötigten Verbindungen (VII) werden weiter unten bei Verfahren AI beschrieben.

Die bei der Durchführung des erfindungsgemäßen Verfahrens C als Ausgangstoffe benötigten Imidoyl-chloride der Formel (VIII) sind teilweise neu. Imidoylchloride der Formel (VIII) lassen sich mittels bekannter Methoden herstellen (z.B. The Chemistry of the Carbon-Nitrogen Double Bond, S. Patei, Interscience Publishers und dort genannte Referenzen). Einige Imidoylchloride der Formel (VIII) sind kommerziell erhältlich (z.B. mit A¹ = Phenyl, substituiertes Phenyl oder Alkyl, L² = direkte Bindung und Y² = OMe, SMe oder N(Me)₂) und lassen sich mit Hilfe literaturbeschriebener Methoden herstellen.

Die Reaktionsbedingungen sind analog Verfahren A zu wählen.

### Verfahren D

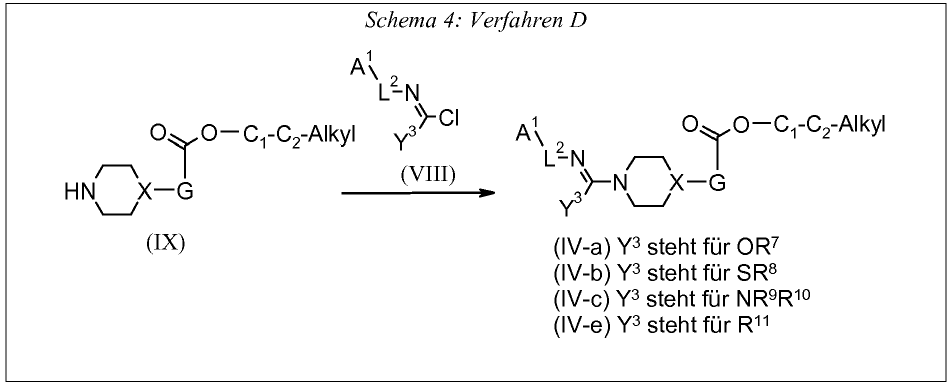

Die bei der Durchführung des erfindungsgemäßen Verfahrens D als Ausgangstoffe benötigten Verbindungen (IX) werden weiter unten bei Verfahren AJ beschrieben.

Das Verfahren D wird analog dem Verfahren C durchgeführt.

### Verfahren E

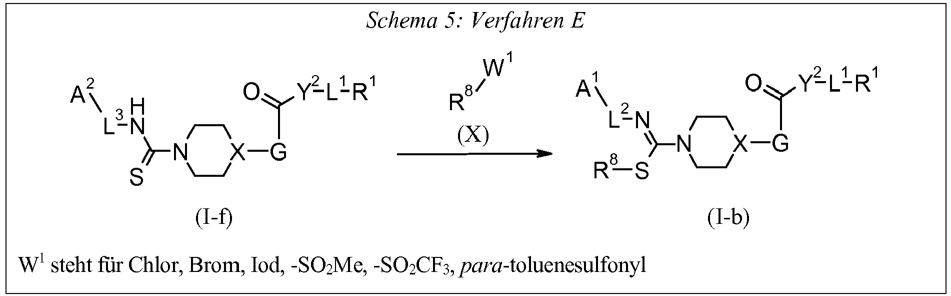

Die bei der Durchführung des erfindungsgemäßen Verfahrens E als Ausgangstoffe benötigten Verbindungen (I-f) werden weiter unten bei Verfahren K und M beschrieben.

Methoden zur Herstellung von Verbindungen der Struktur (I-b) sind bereits in den erfindungsgemäßen Verfahren A und C beschrieben.

Die Synthese von Thiouronium-Salzen und deren Reaktionen sind literaturbeschrieben (z.B. Synthesis (1988), 6, 460-466).

Das Verfahren E beschreibt die Herstellung von Verbindungen der Strukturen (I-b) durch Reaktion von Thioharnstoffen (I-f) mit Alkylierungs- und Acylierungsmitteln der Struktur (X).

Das Verfahren E wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, N,N-Diisopropylethylamin, Tributylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU).

Die bei der Durchführung des erfindungsgemäßen Verfahrens E erhaltenen Verbindungen (I-b) können alternativ teilweise auch ohne Verwendung eines Säureakzeptors als korrespondierende Salze [(I-b)-HW¹] erhalten werden. Bei Bedarf erfolgt die Freisetzung der Verbindungen (I-b) nach üblichen Methoden.

Das erfindungsgemäße Verfahren E wird vorzugsweise unter Verwendung eines oder mehrerer Verdünnungsmittel durchgeführt. Bei der Durchführung des erfindungsgemäßen Verfahrens E kommen vorzugsweise folgende Lösungsmittel infrage: Alkohole, wie z.B. Methanol, Ethanol oder Isopropanol; aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Die Reaktionstemperaturen können bei der Durchführung des erfindungsgemäßen Verfahrens E in einem größeren Bereich variiert werden. Im Allgemeinen arbeitet man bei Temperaturen von -78 °C bis +150 °C, vorzugsweise bei Temperaturen von -20 °C bis +150 °C, ganz besonders bevorzugt bei 0 °C bis 100 °C.

Zur Durchführung der Reaktion des erfindungsgemäßen Verfahrens E setzt man pro Mol an Verbindung der Formel (I-f) im Allgemeinen 0,5 bis 20 Mol, vorzugsweise 1 bis 5 Mol an Verbindung (X) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren F

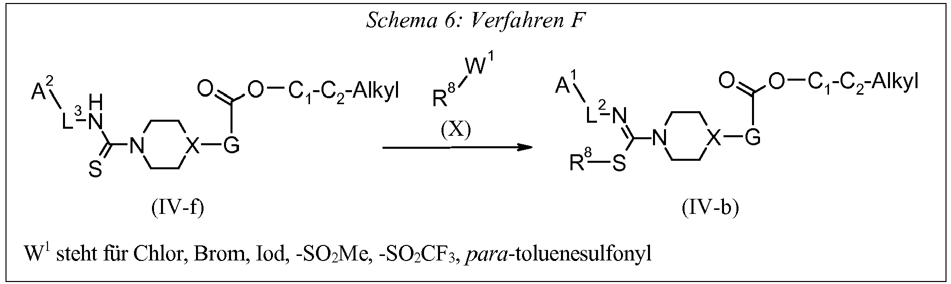

Die bei der Durchführung des erfindungsgemäßen Verfahrens F als Ausgangstoffe benötigten Verbindungen (IV-f) werden weiter unten bei Verfahren L und N beschrieben.

Methoden zur Herstellung von Verbindungen der Strukturen (IV-b) sind bereits in den erfindungsgemäßen Verfahren B und D beschrieben.

Das Verfahren F wird analog dem Verfahren E durchgeführt.

### Verfahren G

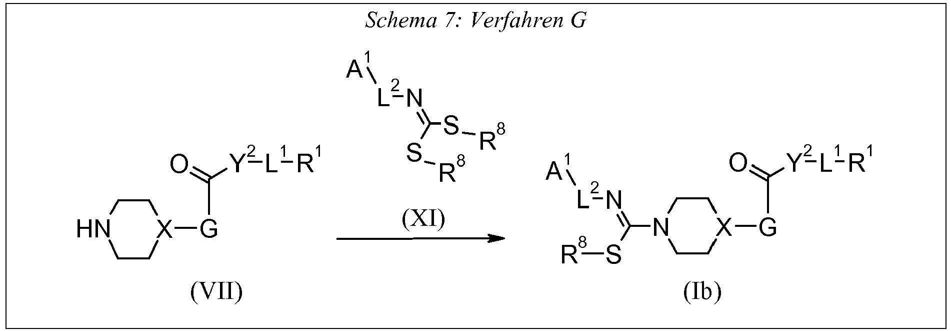

Methoden zur Herstellung von Verbindungen der Strukturen (I-b) sind bereits in den erfindungsgemäßen Verfahren A, C und E beschrieben.

Die bei der Durchführung des erfindungsgemäßen Verfahrens G als Ausgangsstoffe benötigten Dithiocarbamat-Derivate der Formel (XI) lassen sich mittels bekannter Methoden herstellen (z.B. Organic Preparations and Procedures (1991), 23(5), 611-616). Ausgehend von den korrespondierenden Aminen, Schwefelkohlenstoff, zwei Äquivalenten einer geeigneten Base und anschließender Alkylierung können die Verbindungen der Formel (XI) hergestellt werden.

Das Verfahren G beschreibt die Herstellung von Verbindungen der Strukturen (I-b) durch Reaktion von Aminen (VII) mit Dithiocarbamat-Derivaten der Struktur (XI).

Das erfindungsgemäße Verfahren G wird vorzugsweise unter Verwendung eines oder mehrerer Verdünnungsmittel durchgeführt. Bei der Durchführung des erfindungsgemäßen Verfahrens G kommen vorzugsweise folgende Lösungsmittel infrage: Alkohole, wie z.B. Methanol, Ethanol oder Isopropanol; aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Die Reaktionstemperaturen können bei der Durchführung des erfindungsgemäßen Verfahrens G in einem größeren Bereich variiert werden. Im Allgemeinen arbeitet man bei Temperaturen von -78 °C bis +150 °C, vorzugsweise bei Temperaturen von -20 °C bis +150 °C, ganz besonders bevorzugt bei 0 °C bis 100 °C.

Zur Durchführung der Reaktion des erfindungsgemäßen Verfahrens G setzt man pro Mol an Verbindung der Formel (VII) im Allgemeinen 0,5 bis 20 Mol, vorzugsweise 1 bis 5 Mol an Verbindung (XI) ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren H

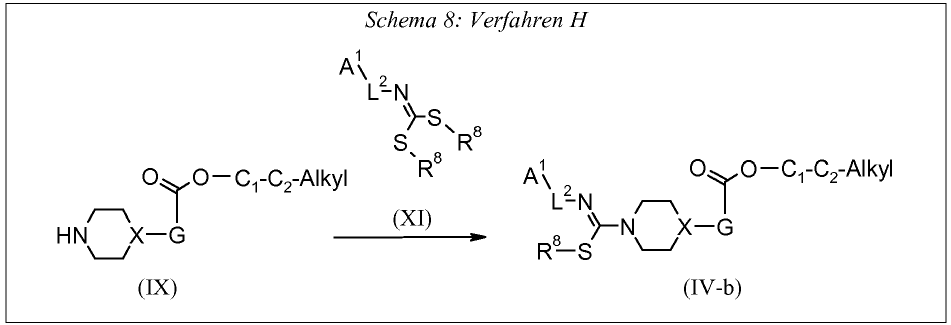

Methoden zur Herstellung von Verbindungen der Strukturen (IV-b) sind bereits in den erfindungsgemäßen Verfahren B, D und F beschrieben.

Die bei der Durchführung des erfindungsgemäßen Verfahrens H als Ausgangsstoffe benötigten Dithiocarbamat-Derivate der Formel (XI) lassen sich mittels bekannter Methoden herstellen (z.B. Organic Preparations and Procedures (1991), 23(5), 611-616). Ausgehend von den korrespondierenden Aminen, Schwefelkohlenstoff, zwei Äquivalenten einer geeigneten Base und anschließender Alkylierung können die Verbindungen der Formel (XI) hergestellt werden.

Das Verfahren H beschreibt die Herstellung von Verbindungen der Strukturen (IV-b) durch Reaktion von Aminen (IX) mit Dithiocarbamat-Derivaten der Struktur (XI).

Das Verfahren H wird analog dem Verfahren G durchgeführt.

### Verfahren I

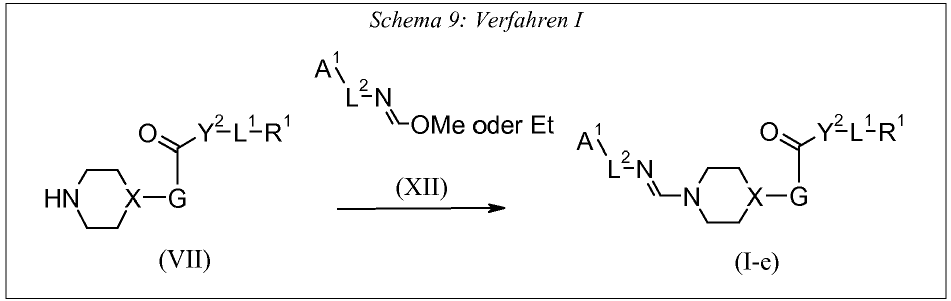

Eine Methode zur Herstellung von Verbindungen der Struktur (I-e) ist bereits in dem erfindungsgemäßen Verfahren C beschrieben.

Die bei der Durchführung des erfindungsgemäßen Verfahrens I als Ausgangsstoffe benötigten Methyl- und Ethyl-Imidoformiat-Derivate der Formel (XII) lassen sich mittels bekannter Methoden herstellen (Journal of Organic Chemistry (1988), 53(22), 5309-5315; Journal of Medicinal Chemistry (2006), 49(3), 955-970). Ausgehend von den korrespondierenden Aminen und Trimethyl- oder Triethylorthoformiat werden die Verbindungen (XII) erhalten. Bei der Durchführung der Synthese von (XII) kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin. Die Reaktionstemperaturen können bei der Durchführung der Synthese von (XII) in einem größeren Bereich variiert werden. Im Allgemeinen arbeitet man bei Temperaturen von 0 °C bis +150 °C, vorzugsweise bei Temperaturen von 50 °C bis +150 °C, ganz besonders bevorzugt bei 100 °C bis 150 °C.

Als geeignete Katalysatoren zur Synthese der Imidoformiat-Derivate (XII) kommen Säuren, wie beispielsweise p-Toluolsulfonsäure und Trifluoressigsäure infrage.

Das Verfahren I beschreibt die Herstellung von Verbindungen der Strukturen (I-e) durch Reaktion von Aminen (VII) mit Methyl- oder Ethyl-Imidoformiat-Derivaten der Struktur (XII).

Das erfindungsgemäße Verfahren I wird vorzugsweise unter Verwendung eines oder mehrerer Verdünnungsmittel durchgeführt. Als Verdünnungsmittel kommen praktisch alle inerten organischen Lösungsmittel in Frage. Bei der Durchführung des erfindungsgemäßen Verfahrens I kommen vorzugsweise folgende Lösungsmittel infrage: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens I in einem größeren Bereich variiert werden. Im Allgemeinen arbeitet man bei Temperaturen von 0 °C bis +150 °C, vorzugsweise bei Temperaturen von +50 °C bis +150 °C, ganz besonders bevorzugt bei +60 °C bis +120 °C.

Zur Durchführung der Reaktion des erfindungsgemäßen Verfahrens I setzt man pro Mol an Verbindung der Formel (VII) im Allgemeinen 0,5 bis 20 Mol, vorzugsweise 1 bis 5 Mol an Verbindung (XII) ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren J

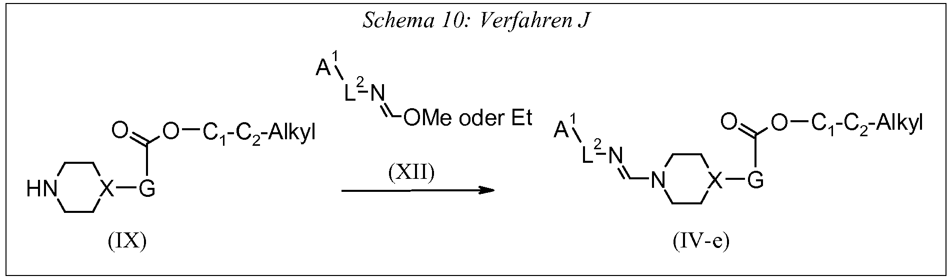

Eine Methode zur Herstellung von Verbindungen der Struktur (IV-e) ist bereits in dem erfindungsgemäßen Verfahren D beschrieben.

Die Synthese der als Ausgangsstoffe benötigten Methyl- und Ethyl-Imidoformiat-Derivate der Formel (XII) ist bereits in dem erfindungsgemäßen Verfahren I beschrieben.

Das Verfahren J beschreibt die Herstellung von Verbindungen der Strukturen (IV-e) durch Reaktion von Aminen (IX) mit Methyl- oder Ethyl-Imidoformiat-Derivaten der Struktur (XII).

Das Verfahren J wird analog dem Verfahren I durchgeführt.

### Verfahren K

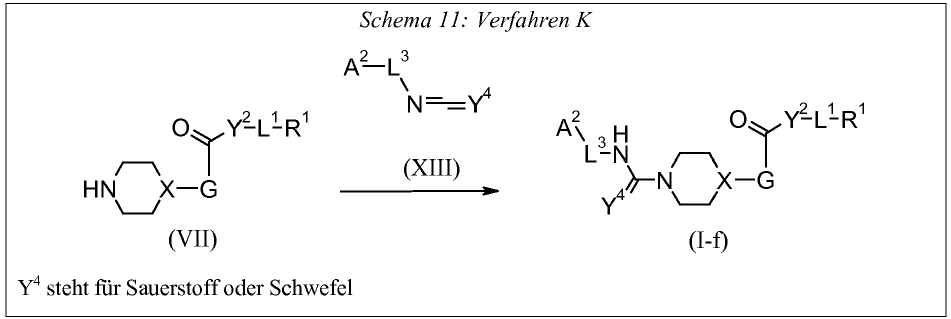

Das Verfahren K beschreibt die Herstellung von Verbindungen der Struktur (I-f) durch Reaktion von Aminen (VII) mit Isocyanaten oder Isothiocyanaten der Struktur (XIII).

Das erfindungsgemäße Verfahren K wird vorzugsweise unter Verwendung eines oder mehrerer Verdünnungsmittel durchgeführt. Bei der Durchführung des erfindungsgemäßen Verfahrens K kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens K in einem größeren Bereich variiert werden. Im Allgemeinen arbeitet man bei Temperaturen von -78 °C bis +150 °C, vorzugsweise bei Temperaturen von -20 °C bis +100 °C, ganz besonders bevorzugt bei 0 °C bis 50 °C.

Zur Durchführung der Reaktion des erfindungsgemäßen Verfahrens K setzt man pro Mol an Verbindung der Formel (VII) im Allgemeinen 0,5 bis 20 Mol, vorzugsweise 1 bis 5 Mol an Verbindung (XIII) ein. Die Reaktionsdauer beträgt 0,1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren L

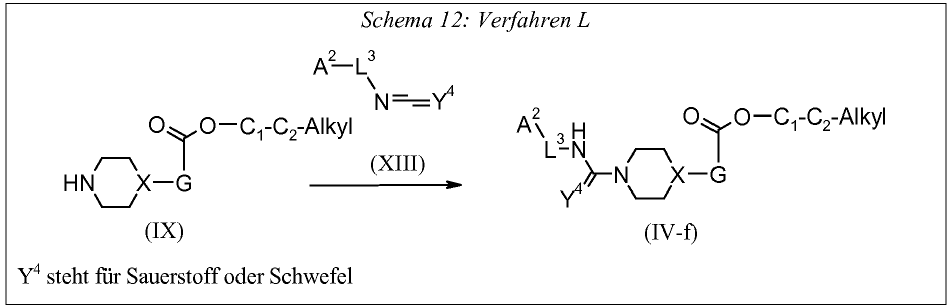

Das Verfahren L beschreibt die Herstellung von Verbindungen der Struktur (IV-f) durch Reaktion von Aminen (IX) mit Isocyanaten oder Isothiocyanaten der Struktur (XIII).

Das Verfahren L wird analog dem Verfahren K durchgeführt.

### Verfahren M

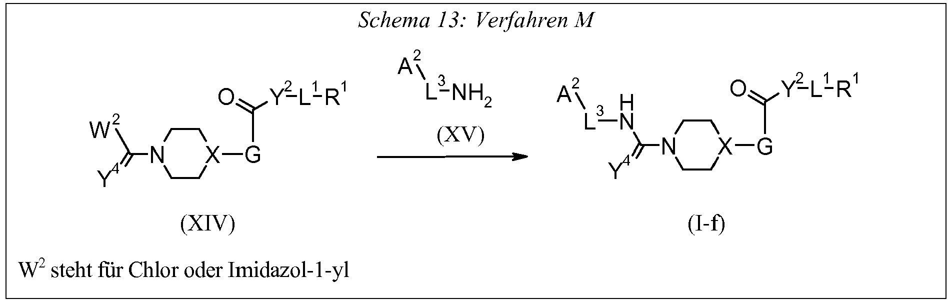

Die bei der Durchführung des erfindungsgemäßen Verfahrens M als Ausgangstoffe benötigten Carbamoyl- und Thiocarbamoylchloride der Formel (XIV, W² = Chlor) lassen sich mittels literaturbeschriebener Methoden herstellen (z.B. Tetrahedron (2008), 64(32), 7605-7610; Journal of Organic Chemistry (2004), 69(11), 3787-3793; Journal of Organic Chemistry (1983), 48(24), 4750-4761; European Journal of Organic Chemistry (2006), 5, 1177-1184). Typischerweise werden die Verbindungen der Formel (XIV, W² = Chlor) ausgehend von Aminen der Formel (VII) und Phosgen, Thiophosgen oder deren Äquivalenten hergestellt.

Die bei der Durchführung des erfindungsgemäßen Verfahrens M als Ausgangstoffe benötigten Carbamoyl- und Thiocarbamoylimidazole der Formel (XIV, W² = imidazol-1-yl) lassen sich mittels literaturbeschriebener Methoden herstellen (z.B. Tetrahedron Letters (2008), 49(36), 5279-5282; Tetrahedron (2005), 61(30), 7153-7175). Typischerweise werden die Verbindungen der Formel (XIV, W² = imidazol-1-yl) ausgehend von Aminen der Formel (VII) und 1,1'-Carbonyldiimidazole oder 1,1'-Thiocarbonyldimidazole hergestellt.

Das Verfahren M beschreibt die Herstellung von Verbindungen der Struktur (I-f) durch Reaktion von Verbindungen der Struktur (XIV, W² = Chlor oder imidazol-1-yl) und Aminen (XV).

Das Verfahren M wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU).

Die bei der Durchführung des erfindungsgemäßen Verfahrens M erhaltenen Verbindungen (I-f) können alternativ teilweise auch ohne Verwendung eines Säureakzeptors als korrespondierende Säurechloride [(I-f)-HCl] (Edukt: W² = Cl) erhalten werden. Bei Bedarf erfolgt die Freisetzung der Verbindungen (I-f) nach üblichen Methoden.

Das erfindungsgemäße Verfahren M wird vorzugsweise unter Verwendung eines oder mehrerer Verdünnungsmittel durchgeführt. Bei der Durchführung des erfindungsgemäßen Verfahrens M kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens M in einem größeren Bereich variiert werden. Im Allgemeinen arbeitet man bei Temperaturen von -78 °C bis +150 °C, vorzugsweise bei Temperaturen von -20 °C bis +100 °C, ganz besonders bevorzugt bei 0 °C bis 60 °C.

Zur Durchführung der Reaktion des erfindungsgemäßen Verfahrens M setzt man pro Mol an Verbindung der Formel (XIV) im Allgemeinen 0,5 bis 20 Mol, vorzugsweise 1 bis 5 Mol an Verbindung (XV) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

Die bei der Durchführung des erfindungsgemäßen Verfahrens A als Ausgangstoffe benötigten Imidoylchloride der Formel (II) lassen sich mittels literaturbeschriebener Methoden herstellen (z.B. Heterocycles (1998), 48, 319-327; Referenzen der Verfahren A und C). Typischerweise werden die Verbindungen der Formel (II) ausgehend von Harnstoffen oder Thioharnstoffen der Formel (I-f) und Thionylchlorid, Phosphoroxychlorid oder Phosphorpentachlorid hergestellt. Hierbei kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

### Verfahren N

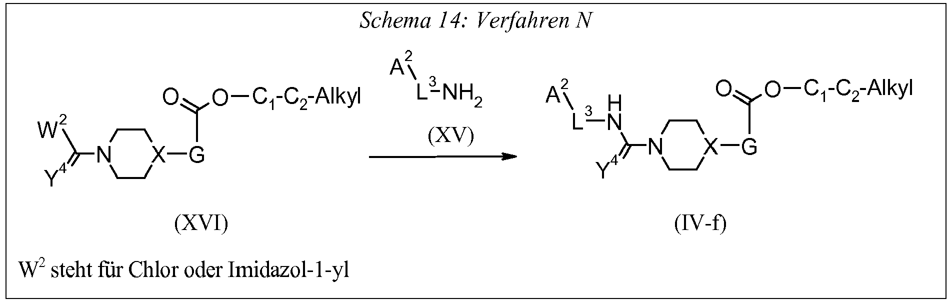

Die bei der Durchführung des erfindungsgemäßen Verfahrens N als Ausgangstoffe benötigten Carbamoyl- und Thiocarbamoylchloride der Formel (XVI, W² = Chlor) lassen sich mittels literaturbeschriebener Methoden herstellen (z.B. Tetrahedron (2008), 64(32), 7605-7610; Journal of Organic Chemistry (2004), 69(11), 3787-3793; Journal of Organic Chemistry (1983), 48(24), 4750-4761; European Journal of Organic Chemistry (2006), 5, 1177-1184). Typischerweise werden die Verbindungen der Formel (XVI, W²=Chlor) ausgehend von Aminen der Formel (IX) und Phosgen, Thiophosgen oder deren Äquivalenten hergestellt.

Die bei der Durchführung des erfindungsgemäßen Verfahrens N als Ausgangstoffe benötigten Carbamoyl- und Thiocarbamoylimidazole der Formel (XVI, W² = imidazol-1-yl) lassen sich mittels literaturbeschriebener Methoden herstellen (z.B. Tetrahedron Letters (2008), 49(36), 5279-5282; Tetrahedron (2005), 61(30), 7153-7175). Typischerweise werden die Verbindungen der Formel (XVI, W² = imidazol-1-yl) ausgehend von Aminen der Formel (IX) und 1,1'-Carbonyldiimidazole oder 1,1'-Thiocarbonyldimidazole hergestellt.

Das Verfahren N beschreibt die Herstellung von Verbindungen der Struktur (IV-f) durch Reaktion von Verbindungen der Struktur (XVI, W² = Chlor oder imidazol-1-yl) und Aminen (XV).

Das Verfahren N wird analog dem Verfahren M durchgeführt.

### Verfahren O

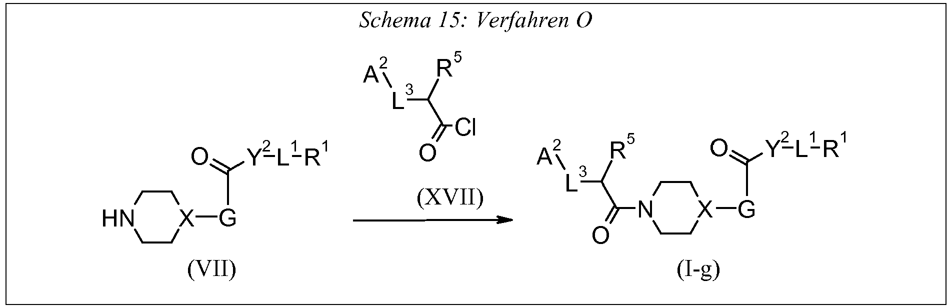

Die bei der Durchführung des erfindungsgemäßen Verfahrens O erhaltenen Amide (I-g) lassen sich mittels literaturbeschriebener Methoden in die korrespondierenden Thioamide überführen (z.B. Bioorganic & Medicinal Chemistry Letters (2009), 19(2), 462-468). Hierbei werden die Verbindungen der Formel (I-g) typischerweise mit Phosphorpentasulfid oder 2,4-Bis(4-Methoxyphenyl)-1,3-dithia-2,4-diphosphetane-2,4-disulfid (Lawesson Reagenz) umgesetzt.

Die Herstellung der bei der Durchführung des erfindungsgemäßen Verfahrens P eingesetzten Carbonsäurechloride (XVII) erfolgt nach üblichen Methoden aus den korrespondierenden Carbonsäuren (XVIII). Die Umsetzung der Carbonsäuren der Formel (XVIII) erfolgt mit einem Chlorierungsmittel (z.B. Thionylchlorid/Oxalylchlorid) in Gegenwart eines Verdünnungsmittels (z.B. Toluol oder Methylenchlorid). Die Reaktionstemperaturen können bei der Durchführung dieses Schrittes in einem größeren Bereich variiert werden. Im Allgemeinen arbeitet man bei Temperaturen von 0 °C bis +150 °C, vorzugsweise bei Temperaturen von 20 °C bis zu der Siedetemperatur des entsprechenden Lösungsmittels. Zur Durchführung der Chlorierung setzt man pro Mol an Verbindung der Formel (XVIII) im Allgemeinen 0,5 bis 20 Mol, vorzugsweise 1 bis 1,5 Mol an Chlorierungsmittel ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

Das Verfahren O beschreibt die Herstellung von Verbindungen der Struktur (I-g) durch Reaktion von Säurechloriden (XVII) mit Aminen der Struktur (VII). Ebenso können Salze der Amine (VII) als Startmaterialien verwendet werden, typischerweise die korrespondierenden Hydrochloride, Oxalate oder Trifluoracetate.

Das Verfahren O wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren O wird vorzugsweise unter Verwendung eines oder mehrerer Ver-dünnungsmittel durchgeführt. Bei der Durchführung des erfindungsgemäßen Verfahrens O kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens O in einem größeren Bereich variiert werden. Im Allgemeinen arbeitet man bei Temperaturen von -78 °C bis +150 °C, vorzugsweise bei Temperaturen von -20 °C bis +120 °C, ganz besonders bevorzugt bei 0 °C bis 80 °C.

Zur Durchführung der Reaktion des erfindungsgemäßen Verfahrens O setzt man pro Mol an Verbindung der Formel (XVII) im Allgemeinen 0,5 bis 20 Mol, vorzugsweise 1 bis 5 Mol an Verbindung (VII) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren P

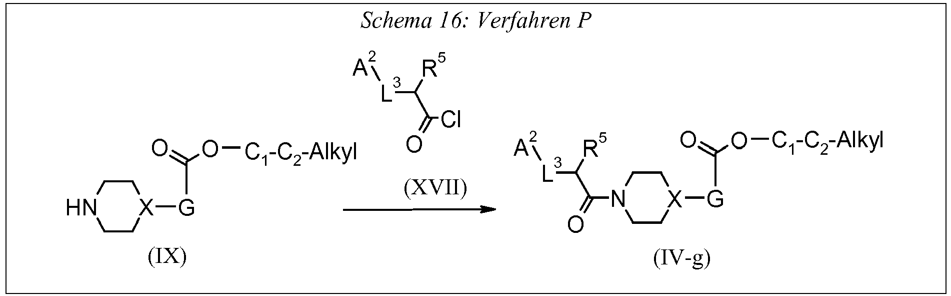

Das Verfahren P beschreibt die Herstellung von Verbindungen der Struktur (IV-g) durch Reaktion von Säurechloriden (XVII) mit Aminen der Struktur (IX). Ebenso können Salze der Amine (IX) als Startmaterialien verwendet werden, typischerweise die korrespondierenden Hydrochloride, Oxalate oder Trifluoracetate.

Das Verfahren P wird analog dem Verfahren O durchgeführt.

### Verfahren Q

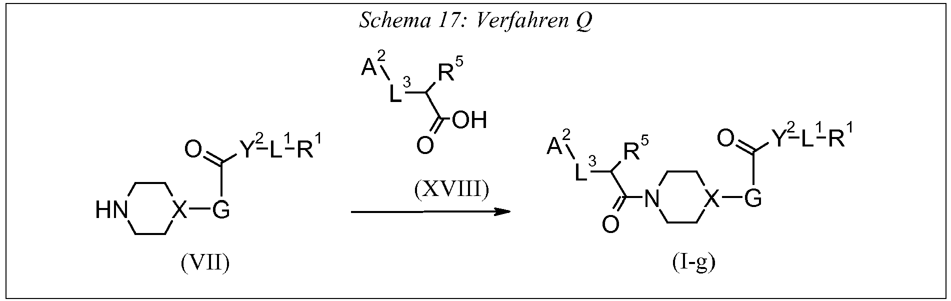

Die bei der Durchführung des erfindungsgemäßen Verfahrens Q erhaltenen Amide (I-g) lassen sich mittels literaturbeschriebener Methoden in die korrespondierenden Thioamide überführen (z.B. Bioorganic & Medicinal Chemistry Letters (2009), 19(2), 462-468; European Journal of Organic Chemistry (200), 19, 3273-3278). Hierbei werden die Verbindungen der Formel (I-g) typischerweise mit Phosphorpentasulfid oder 2,4-Bis(4-Methoxyphenyl)-1,3-dithia-2,4-diphosphetane-2,4-disulfid (Lawesson Reagenz) umgesetzt.

Die bei der Durchführung des erfindungsgemäßen Verfahrens Q eingesetzten Carbonsäuren (XVIII) lassen sich mittels literaturbeschriebener Methoden herstellen.

Das Verfahren Q beschreibt die Herstellung von Verbindungen der Struktur (I-g) durch Reaktion von Carbonsäuren (XVIII) mit Aminen der Struktur (VII). Ebenso können Salze der Amine (VII) als Startmaterialien verwendet werden, typischerweise die korrespondierenden Hydrochloride, Oxalate oder Trifluoracetate.

Als geeignete Kupplungsreagenzien kommen alle üblichen Kupplungsreagenzien, wie beispielsweise Dicyclohexylcarbodiimid (DCC), 1-(3-Dimethylaminopropyl)-3-ethylcarbodimid Hydrochlorid (EDC), O-Benzotriazol-1-yl-N,N,N',N'-tetramethyluronium hexafluorophosphat (HBTU) oder polymergebundene Reagenzien, wie beispielsweise polymergebundenes Cyclohexylcarbodiimid infrage.

Das Verfahren Q wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren Q wird vorzugsweise unter Verwendung eines oder mehrerer Verdünnungsmittel durchgeführt. Bei der Durchführung des erfindungsgemäßen Verfahrens Q kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens Q in einem größeren Bereich variiert werden. Im Allgemeinen arbeitet man bei Temperaturen von -78 °C bis +150 °C, vorzugsweise bei Temperaturen von -20 °C bis +100 °C, ganz besonders bevorzugt bei 0 °C bis 40 °C.

Zur Durchführung der Reaktion des erfindungsgemäßen Verfahrens Q setzt man pro Mol an Verbindung der Formel (XVIII) im Allgemeinen 0,5 bis 20 Mol, vorzugsweise 1 bis 2 Mol an Verbindung (VII) sowie 0 bis 20 Mol, vorzugsweise 1 bis 5 Mol an Säureakzeptor, sowie 1-10 Mol, vorzugsweise 1 bis 2 Mol an Kupplungsreagenz ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren R

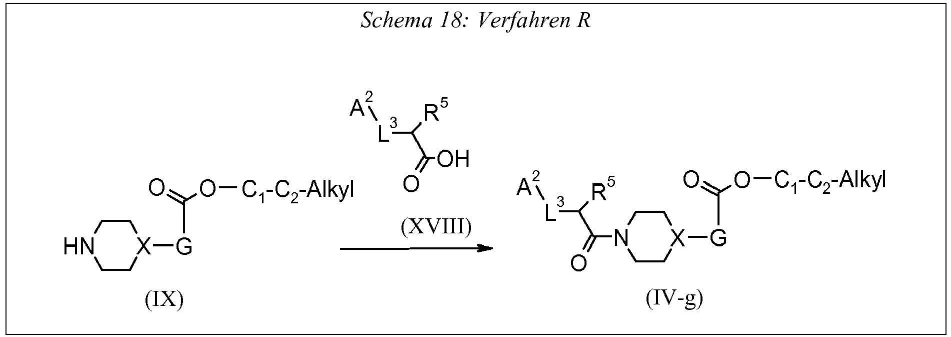

Das Verfahren R beschreibt die Herstellung von Verbindungen der Struktur (IV-g) durch Reaktion von Carbonsäuren (XVIII) mit Aminen der Struktur (IX). Ebenso können Salze der Amine (IX) als Startmaterialien verwendet werden, typischerweise die korrespondierenden Hydrochloride, Oxalate oder Trifluoracetate.

Das Verfahren R wird analog dem Verfahren Q durchgeführt.

### Verfahren S

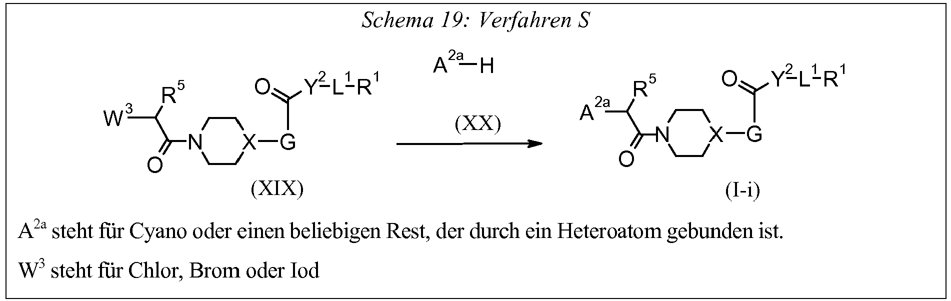

Die bei der Durchführung des erfindungsgemäßen Verfahrens S als Ausgangstoffe benötigten α-Halogen-acetamide der Formel (XIX, W³ = Chlor, Brom oder Iod) lassen sich mittels literaturbeschriebener Methoden herstellen (z.B. Journal of Organic Chemistry (2008), 73(12), 4452-4457, Journal of the American Chemical Society (2007), 129(29), 8928-8929, Heterocycles (2005), 65(8), 1857-1869, Bioorganic & Medicinal Chemistry Letters (2002), 12(18), 2519-2522). Typischerweise werden die Verbindungen der Formel (XIX, W² = Chlor, Brom oder Iod) ausgehend von Aminen der Formel (VII) und α-Halogen-carbonsäurehalogeniden, α-Halogen-carbonsäuren oder α-Halogen-carbonsäureanhydriden in Analogie zu den Verfahren O und Q hergestellt.

Das Verfahren S beschreibt die Herstellung von Verbindungen der Struktur (I-i) durch Reaktion von α-Halogenacetamiden der Formel (XIX, W³ = Chlor, Brom oder Iod) mit Verbindungen der Struktur (XX).

Das Verfahren S wird gegebenenfalls in Gegenwart einer geeigneten Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren S wird vorzugsweise unter Verwendung eines oder mehrerer Verdünnungsmittel durchgeführt. Bei der Durchführung des erfindungsgemäßen Verfahrens S kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens S in einem größeren Bereich variiert werden. Im Allgemeinen arbeitet man bei Temperaturen von -78 °C bis +150 °C, vorzugsweise bei Temperaturen von -20 °C bis +100 °C, ganz besonders bevorzugt bei 0 °C bis 80 °C.

Zur Durchführung der Reaktion des erfindungsgemäßen Verfahrens S setzt man pro Mol an Verbindung der Formel (XIX) im Allgemeinen 0,5 bis 20 Mol, vorzugsweise 1 bis 2 Mol an Verbindung (XX) sowie 0 bis 20 Mol, vorzugsweise 1 bis 5 Mol an Base ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren T

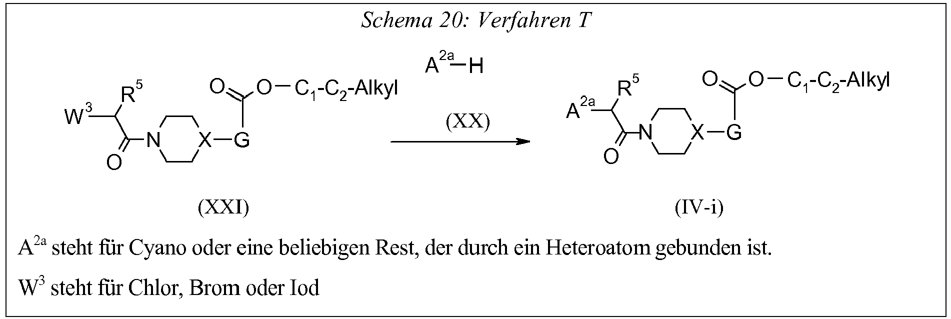

Das Verfahren T beschreibt die Herstellung von Verbindungen der Struktur (IV-i) durch Reaktion von α-Halogenacetamiden der Formel (XXI, W³ = Chlor, Brom oder Iod) mit Verbindungen der Struktur (XX).

Das Verfahren T wird analog dem Verfahren S durchgeführt.

### Verfahren U

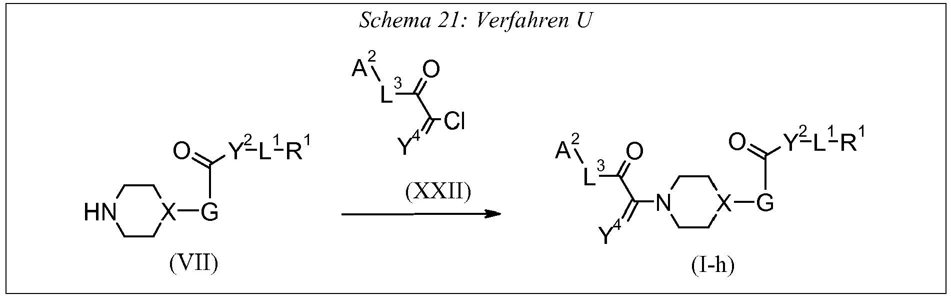

Die bei der Durchführung des erfindungsgemäßen Verfahrens U als Ausgangstoffe benötigten α-Ketocarbonsäurechloride der Formel (XXII) lassen sich mittels literaturbeschriebener Methoden aus den korrspondierenden Carbonsäuren herstellen (z.B. Bioorganic & Medicinal Chemistry Letters (2008), 18(20), 5456-5459; Synlett (1999), 11, 1763-1765; Journal of Heterocyclic Chemistry (1981), 18(5), 953-956). Die Chlorierung erfolgt nach üblichen Methoden. Die Umsetzung der korrespondierenden Carbonsäuren der Formel (XXIII) erfolgt mit einem Chlorierungsmittel (z.B. Thionylchlorid/Oxalylchlorid) in Gegenwart eines Verdünnungsmittels (z.B. Toluol oder Methylenchlorid). Die Reaktionstemperaturen können bei der Durchführung der Chlorierung von Verbindungen der Formel (XXIII) in einem größeren Bereich variiert werden. Im Allgemeinen arbeitet man bei Temperaturen von 0°C bis +150°C, vorzugsweise bei Temperaturen von 20°C bis zu der Siedetemperatur des entsprechenden Lösungsmittels. Zur Durchführung der Chlorierung setzt man pro Mol an Verbindung der Formel (XXIII) im Allgemeinen 0,5 bis 20 Mol, vorzugsweise 1 bis 1,5 Mol an Chlorierungsmittel ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

Das Verfahren U beschreibt die Herstellung von Verbindungen der Struktur (I-h) durch Reaktion von α-Ketocarbonsäurechloriden der Formel (XXII) mit Aminen der Struktur (VII).

Die Durchführung der Kupplungsreaktion (VII und XXII) des erfindungsgemäßen Verfahrens U erfolgt analog zum bereits beschriebenen Verfahren O.

### Verfahren V

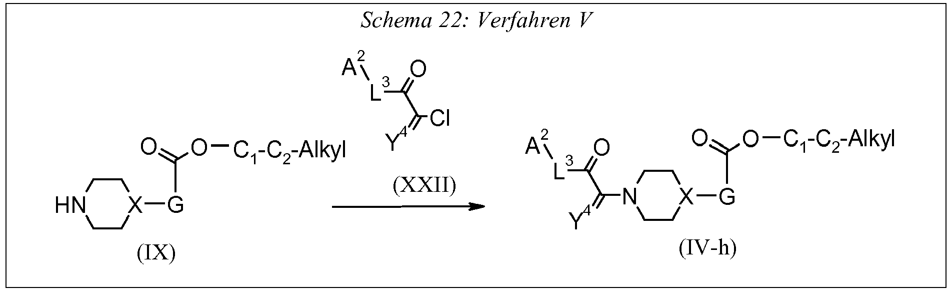

Das Verfahren V beschreibt die Herstellung von Verbindungen der Struktur (IV-h) durch Reaktion von α-Ketocarbonsäurechloriden der Formel (XXII) mit Aminen der Struktur (IX).

Die Durchführung der Kupplungsreaktion (IX und XXII) des erfindungsgemäßen Verfahrens V erfolgt analog zum bereits beschriebenen Verfahren O.

### Verfahren W

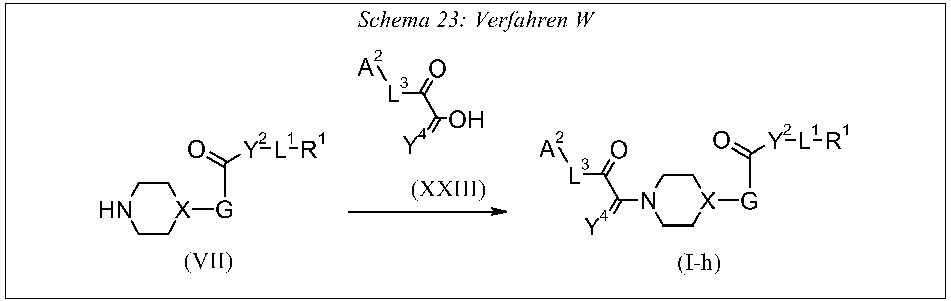

Die bei der Durchführung des erfindungsgemäßen Verfahrens W als Ausgangstoffe benötigten α-Ketocarbonsäuren der Formel (XXIII) lassen sich mittels literaturbeschriebener Methoden herstellen.

Das Verfahren W beschreibt die Herstellung von Verbindungen der Struktur (I-h) durch Reaktion von α-Ketocarbonsäuren der Formel (XXIII) mit Aminen der Struktur (VII).

Als geeignete Kupplungsreagenzien kommen alle üblichen Kupplungsreagenzien, wie beispielsweise Dicyclohexylcarbodiimid (DCC), 1-(3-Dimethylaminopropyl)-3-ethylcarbodimid Hydrochlorid (EDC), O-Benzotriazol-1-yl-N,N,N',N'-tetramethyluronium hexafluorophosphat (HBTU) oder polymergebundene Reagenzien, wie beispielsweise polymergebundenes Cyclohexylcarbodiimid infrage.

Das Verfahren W wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren W wird vorzugsweise unter Verwendung eines oder mehrerer Verdünnungsmittel durchgeführt. Bei der Durchführung des erfindungsgemäßen Verfahrens W kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens W in einem größeren Bereich variiert werden. Im Allgemeinen arbeitet man bei Temperaturen von -78°C bis +150°C, vorzugsweise bei Temperaturen von -20°C bis +100°C, ganz besonders bevorzugt bei 0°C bis 40°C.

Zur Durchführung der Reaktion des erfindungsgemäßen Verfahrens W setzt man pro Mol an Verbindung der Formel (XXIII) im Allgemeinen 0,5 bis 20 Mol, vorzugsweise 1 bis 2 Mol an Verbindung (VII) sowie 0 bis 20 Mol, vorzugsweise 1 bis 5 Mol an Säureakzeptor, sowie 1-10 Mol, vorzugsweise 1 bis 2 Mol an Kupplungsreagenz ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren X

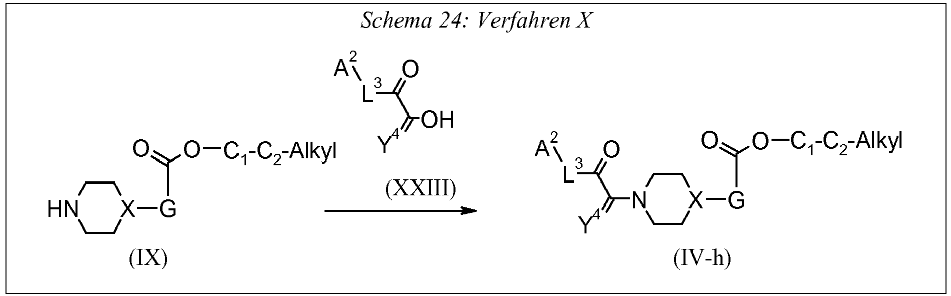

Die bei der Durchführung des erfindungsgemäßen Verfahrens X als Ausgangstoffe benötigten α-Ketocarbonsäuren der Formel (XXIII) lassen sich mittels literaturbeschriebener Methoden herstellen.

Das Verfahren X beschreibt die Herstellung von Verbindungen der Struktur (IV-h) durch Reaktion von α-Ketocarbonsäuren der Formel (XXIII) mit Aminen der Struktur (IX).

Das Verfahren X wird analog dem Verfahren W durchgeführt.

### Verfahren Y

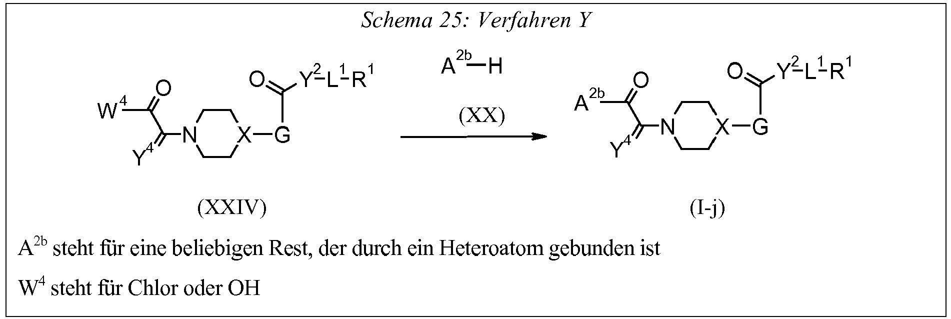

Die bei der Durchführung des erfindungsgemäßen Verfahrens Y als Ausgangstoffe benötigten Verbindungen der Formel (XXIV, W⁴ = Cl) lassen sich mittels literaturbeschriebener Methoden aus den korrspondierenden Aminen (VII) herstellen (z.B. European Journal of Medicinal Chemistry (2006), 41(6), 786-792; Journal of Medicinal Chemistry (2007), 50(5), 901-914). Die Umsetzung der korrspondierenden Amine der Formel (VII) erfolgt mit einem Carbonylierungsmittel (z.B. Oxalylchlorid; Ethylchloroxoacetat) in Gegenwart eines Verdünnungsmittels (z.B. Toluol oder Methylenchlorid). Die Reaktionstemperaturen können bei der Durchführung der Carbonylierung von Verbindungen der Formel (VII) in einem größeren Bereich variiert werden. Im Allgemeinen arbeitet man bei Temperaturen von 0°C bis +150°C, vorzugsweise bei Temperaturen von 0°C bis zu der Siedetemperatur des entsprechenden Lösungsmittels. Zur Durchführung der Carbonylierung setzt man pro Mol an Verbindung der Formel (VII) im Allgemeinen 0,5 bis 20 Mol, vorzugsweise 1 bis 1,5 Mol an Carbonylierungsmittel ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden. Die hierbei erhaltenen Produkte müssen teilweise weiter umgesetzt werden, um Verbindungen der Formel (XXIV, W⁴= C1) zu erhalten. So erhält man z.B. bei Verwendung von Ethylchloroacetat zunächst die korrespondiereden Ester, die anschließend zu den korrspondierenden Carbonsäuren mittels literaturbeschriebener Methoden hydrolysiert werden und dann mittels literaturbeschriebener Methoden in die benötigten Verbindungen der Formel (XXIV, W⁴ = Cl) überführt werden können (z.B. Journal of Medicinal Chemistry (2007), 50(5), 901-914). Die Umsetzung der korrspondierenden Carbonsäuren erfolgt mit einem Chlorierungsmittel (z.B. Thionylchlorid/Oxalylchlorid) in Gegenwart eines Verdünnungsmittels (z.B. Toluol oder Methylenchlorid).

Die bei der Durchführung des erfindungsgemäßen Verfahrens Y als Ausgangstoffe benötigten Verbindungen der Formel (XXIV, W⁴ = OH) lassen sich mittels literaturbeschriebener Methoden aus den korrspondierenden Aminen (VII) herstellen (z.B. European Journal of Medicinal Chemistry (2006), 41(6), 786-792; Journal of Medicinal Chemistry (2007), 50(5), 901-914). Die Umsetzung der korrspondierenden Amine der Formel (VII) erfolgt mit einem Carbonylierungsmittel (z.B. Oxalylchlorid; Ethylchloroxoacetat) in Gegenwart eines Verdünnungsmittels (z.B. Toluol oder Methylenchlorid). Die hierbei erhaltenen Produkte müssen teilweise weiter umgesetzt werden, um Verbindungen der Formel (XXIV, W⁴ = OH) zu erhalten. So erhält man z.B. bei Verwendung von Ethylchloroacetat zunächst die korrespondiereden Ester, die anschließend zu den korrspondierenden Carbonsäuren mittels literaturbeschriebener Methoden hydrolysiert werden können. Ebenso lassen sich die Verbindungen der Formel (XXIV, W⁴ = OH) durch Hydrolyse der oben beschriebenen Verbindungen der Formel (XXIV, W⁴= Cl) herstellen. Die Hydrolyse erfolgt nach üblichen Methoden.

Das Verfahren Y beschreibt die Herstellung von Verbindungen der Struktur (I-j) durch Reaktion von Verbindungen der Formel (XXIV) mit Verbindungen der Struktur (XX).

Die Durchführung der Reaktion (XX und XXIV; W⁴ = Cl) des erfindungsgemäßen Verfahrens Y erfolgt analog zum bereits beschriebenen Verfahren O.

Die Durchführung der Reaktion (XX und XXIV; W⁴= OH) des erfindungsgemäßen Verfahrens Y erfolgt analog zum bereits beschriebenen Verfahren Q.

### Verfahren Z

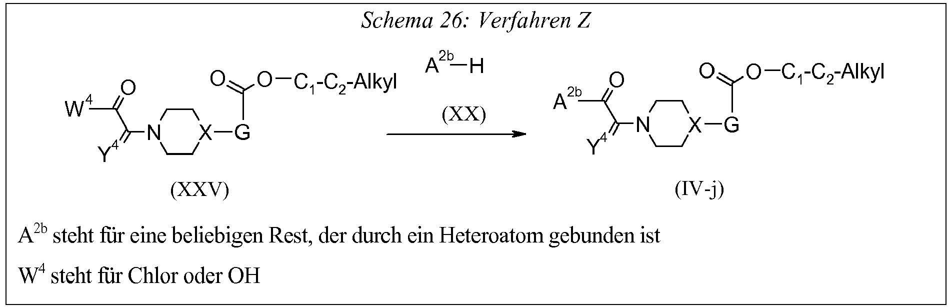

Das Verfahren Z beschreibt die Herstellung von Verbindungen der Struktur (IV-j) durch Reaktion von Verbindungen der Formel (XXV) mit Verbindungen der Struktur (XX).

Die Ausgansstoffe (XXV) lassen sich analog der Ausgangsstoffe (XXIV) herstellen (s. Verfahren Y).

Die Durchführung der Reaktion (XX und XXV; W⁴ = Cl) des erfindungsgemäßen Verfahrens Z erfolgt analog zum bereits beschriebenen Verfahren P.

Die Durchführung der Reaktion (XX und XXV; W⁴ = OH) des erfindungsgemäßen Verfahrens Z erfolgt analog zum bereits beschriebenen Verfahren R.

### Verfahren AA

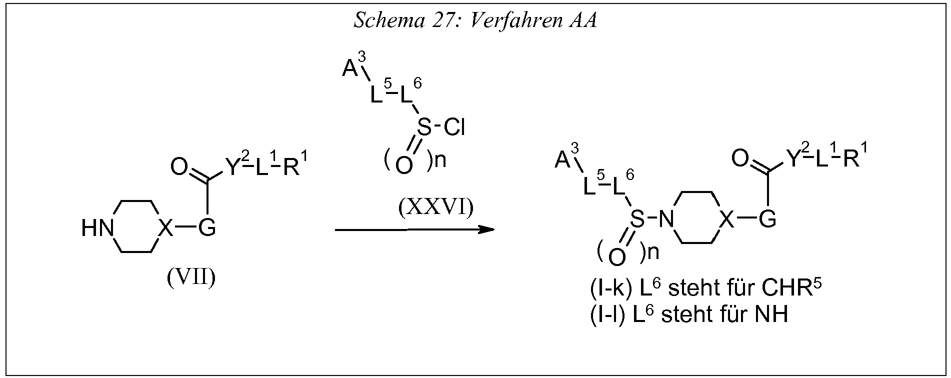

Die Herstellung der bei der Durchführung des erfindungsgemäßen Verfahrens AA eingesetzten Sulfinyl-, Sulfonyl-, Sulfanyl- und Sulfamylchloriden der Formel (XXVI) erfolgt nach üblichen Methoden. Für die Herstellung von Sulfinyl-, Sulfonyl-, Sulfanyl- und Sulfamylchloriden und deren Umsetzung zu den korrespondierenden Sulfinyl-, Sulfonyl-, Sulfanylamiden und Schwefelsäurediamiden siehe z.B. The Chemistry of Sulfinic Acids, Esters and their Derivatives (1990), S. Patai, John Wiley & Sons; Tetrahedron Letters (1986), 27(13), 1493-1494 und darin zitierte Referenzen; Comprehensive Organic Chemistry (1979), vol. 3, N. Jones, Pergamon Press.

Das Verfahren AA beschreibt die Herstellung von Verbindungen der Struktur (I-k und I-1) durch Reaktion von Verbindungen der Formel (XXVI) mit Aminen der Struktur (VII). Ebenso können Salze der Amine (VII) als Startmaterialien verwendet werden, z.B. die korrespondierenden Hydrochloride, Oxalate oder Trifluoracetate.

Das Verfahren AA wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren AA wird vorzugsweise unter Verwendung eines oder mehrerer Verdünnungsmittel durchgeführt. Bei der Durchführung des erfindungsgemäßen Verfahrens AA kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens AA in einem größeren Bereich variiert werden. Im Allgemeinen arbeitet man bei Temperaturen von -78 °C bis +150 °C, vorzugsweise bei Temperaturen von -20 °C bis +150 °C, ganz besonders bevorzugt bei - 20 °C bis +100 °C.

Zur Durchführung der Reaktion des erfindungsgemäßen Verfahrens AA setzt man pro Mol an Verbindung der Formel (XXVI) im Allgemeinen 0,5 bis 20 Mol, vorzugsweise 1 bis 2 Mol an Verbindung (VII) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren AB

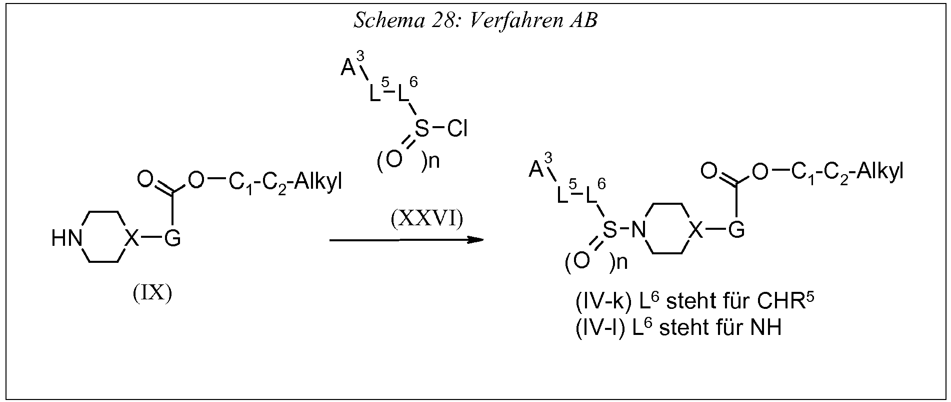

Das Verfahren AB wird analog dem Verfahren AA durchgeführt.

### Verfahren AC

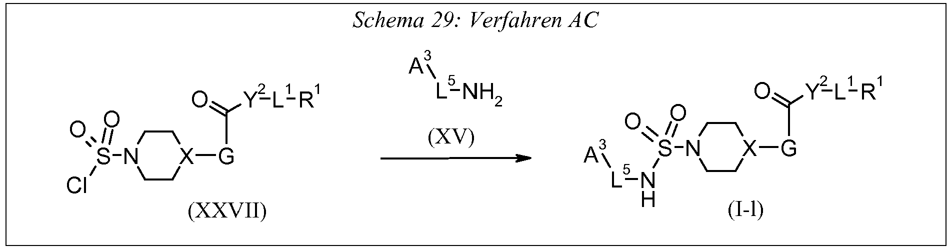

Die Herstellung der bei der Durchführung des erfindungsgemäßen Verfahrens AC eingesetzten Sulfamylchloriden der Formel (XXVII) erfolgt nach üblichen Methoden (z.B. Biorganic & Medicinal Chemistry Letters (2005), 15(4), 983-987, Referenzen des Verfahrens AB). Typischerweise werden die Verbindungen der Formel (XXVII) ausgehend von Aminen der Formel (VII) und Sulfurylchlorid hergestellt. Alternativ können zunächst die korrespondierenden Sulfonsäuren hergestellt werden, typischerweise ausgehend von Aminen der Formel (VII) und Chlorschwefelsäure oder Schwefeltrioxid, die anschließend nach literaturbekannten Methoden chloriert werden (z.B. Biorganic & Medicinal Chemistry Letters (2006), 16(13), 3367-3370).

Das Verfahren AC beschreibt die Herstellung von Verbindungen der Struktur (I-1) durch Reaktion von Verbindungen der Formel (XXVII) mit Aminen der Struktur (XV). Ebenso können Salze der Amine als Startmaterialien verwendet werden, z.B. die korrespondierenden Hydrochloride, Oxalate oder Trifluoracetate.

Die Durchführung der Reaktion (XXVII und XV) des erfindungsgemäßen Verfahrens AC erfolgt analog zum bereits beschriebenen Verfahren AA.

### Verfahren AD

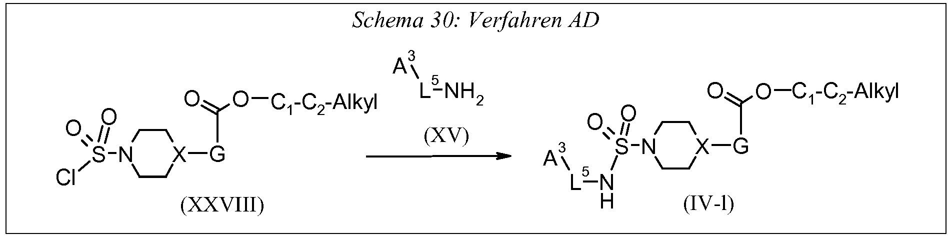

Die Herstellung der bei der Durchführung des erfindungsgemäßen Verfahrens AD eingesetzten Sulfamylchloriden der Formel (XXVIII) erfolgt nach üblichen Methoden (z.B. Biorganic & Medicinal Chemistry Letters (2005), 15(4), 983-987, Referenzen des Verfahrens AB). Typischerweise werden die Verbindungen der Formel (XXVIII) ausgehend von Aminen der Formel (IX) und Sulfurylchlorid hergestellt. Alternativ können zunächst die korrespondierenden Sulfonsäuren hergestellt werden, typischerweise ausgehend von Aminen der Formel (IX) und Chlorschwefelsäure oder Schwefeltrioxid, die anschließend nach literaturbekannten Methoden chloriert werden (z.B. Biorganic & Medicinal Chemistry Letters (2006), 16(13), 3367-3370).

Das Verfahren AD beschreibt die Herstellung von Verbindungen der Struktur (IV-1) durch Reaktion von Verbindungen der Formel (XXVIII) mit Aminen der Struktur (XV). Ebenso können Salze der Amine als Startmaterialien verwendet werden, z.B. die korrespondierenden Hydrochloride, Oxalate oder Trifluoracetate.

Die Durchführung des Verfahrens AD erfolgt analog zum bereits beschriebenen Verfahren AA.

### Verfahren AE

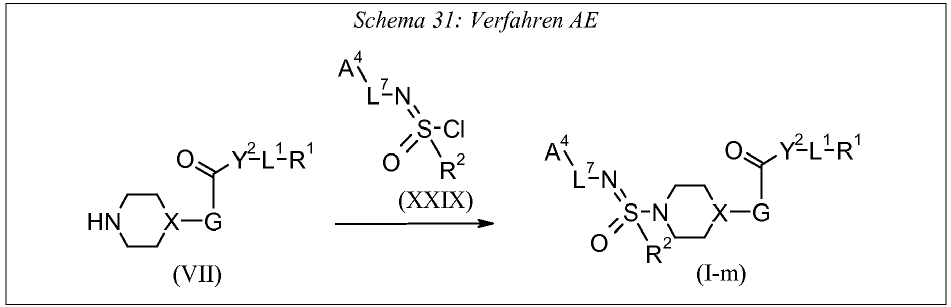

Die Herstellung der bei der Durchführung des erfindungsgemäßen Verfahrens AE eingesetzten Sulfonimidoylchloriden der Formel (XXIX) erfolgt nach üblichen Methoden. Für die Herstellung von Sulfonimidoylchloriden der Formel (XXIX) und deren Umsetzung zu den korrespondierenden Sulfonimidoamiden siehe z.B. Journal of Organic Chermistry (1979), 44, 2055-2061, Journal of Organic Chemistry (1988), 53, 4190-4193, Comprehensive Organic Chemistry (1979), vol. 3, part 11, Pergamon Press).

Das Verfahren AE beschreibt die Herstellung von Verbindungen der Struktur (I-m) durch Reaktion von Verbindungen der Formel (XXIX) mit Aminen der Struktur (VII). Ebenso können Salze der Amine (VII) als Startmaterialien verwendet werden, z.B. die korrespondierenden Hydrochloride, Oxalate oder Trifluoracetate.

Das Verfahren AE wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Di-methylanilin, N,N-Dimethyl-benzylamin, Pyridin, N-Methylpiperidin, N-Methylmorpholin, N,N-Di-methylaminopyridin, Diazabicyclooctan (DABCO), Diazabicyclononen (DBN) oder Diazabicycloundecen (DBU), sowie polymergebundene Säureakzeptoren, wie polymergebundenes N,N-Diisopropylethylamin oder polymergebundenes N,N-Dimethylaminopyridin.

Das erfindungsgemäße Verfahren AE wird vorzugsweise unter Verwendung eines oder mehrerer Verdünnungsmittel durchgeführt. Bei der Durchführung des erfindungsgemäßen Verfahrens AE kommen vorzugsweise alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens AE in einem größeren Bereich variiert werden. Im Allgemeinen arbeitet man bei Temperaturen von -78 °C bis +150 °C, vorzugsweise bei Temperaturen von -20 °C bis +150 °C, ganz besonders bevorzugt bei - 20 °C bis +100 °C.

Zur Durchführung der Reaktion des erfindungsgemäßen Verfahrens AE setzt man pro Mol an Verbindung der Formel (XXIX) im Allgemeinen 0,5 bis 20 Mol, vorzugsweise 1 bis 2 Mol an Verbindung (VII) sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren AF

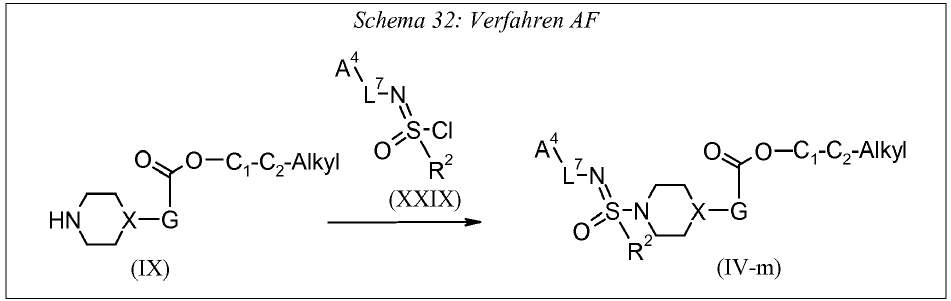

Die Herstellung der bei der Durchführung des erfindungsgemäßen Verfahrens AF eingesetzten Sulfonimidoylchloriden der Formel (XXIX) erfolgt nach üblichen Methoden. Für die Herstellung von Sulfonimidoylchloriden der Formel (XXIX) und deren Umsetzung zu den korrespondierenden Sulfonimidoamiden siehe z.B. Journal of Organic Chermistry (1979), 44, 2055-2061, Journal of Organic Chemistry (1988), 53, 4190-4193, Comprehensive Organic Chemistry (1979), vol. 3, part 11, Pergamon Press).

Das Verfahren AF beschreibt die Herstellung von Verbindungen der Struktur (IV-m) durch Reaktion von Verbindungen der Formel (XXIX) mit Aminen der Struktur (IX). Ebenso können Salze der Amine (IX) als Startmaterialien verwendet werden, z.B. die korrespondierenden Hydrochloride, Oxalate oder Trifluoracetate.

Das Verfahren AF wird analog dem Verfahren AE durchgeführt.

### Verfahren AG

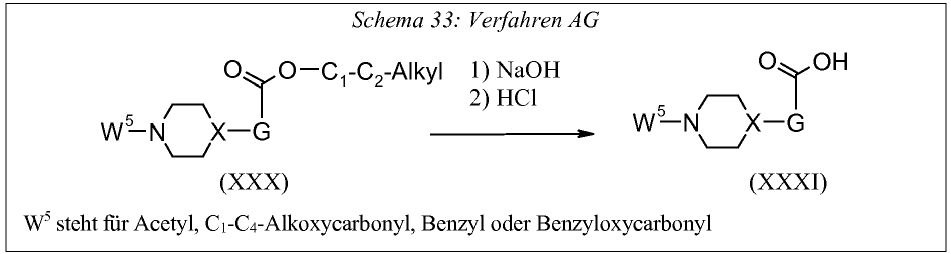

Eine Möglichkeit das Zwischenprodukt (XXXI) aus entsprechenden Verbindungen (XXX) darzustellen ist in Schema 33 gezeigt.

Die Carbonsäure der Formel (XXXI) kann durch Verseifung des entsprechenden C₁-C₂-Alkylesters der Formel (XXX) dargestellt werden. Zum Beispiel kann die Methode, die in WO2007/014290 beschrieben wird, verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff) und halogenierte aromatische Kohlenwasserstoffe (z.B. Chlorbenzol, Dichlorbenzol) verwendet werden oder die Reaktion kann in Mischungen von zwei oder mehreren dieser Lösungsmittel ausgeführt werden.

Geeignete Alkalimetallhydroxide sind beispielsweise LiOH, NaOH oder KOH, gewöhnlich in der Gegenwart von Wasser zusammen mit einem Cosolvens, bevorzugt THF und/oder Methanol, um das Lösen des Esters zu vereinfachen. Das Startmaterial und das Alkalimetallhydroxid werden in equimolaren Mengen verwendet, aber das Alkalimetallhydroxid kann gegebenenfalls auch im Überschuss verwendet werden. Das entstehende Carboxylatsalz wird in die freie Säure durch das Behandeln mit einem geringen Überschuss an Mineralsäuren, wie zum Beispiel Salzsäure oder Schwefelsäure überführt.

Die Reaktion wird normalerweise bei Temperaturen von 0 °C - 60 °C durchgeführt, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion werden die Verbindungen (XXXI) von der Reaktionsmischung durch eine der üblichen Trenntechniken getrennt. Falls notwendig werden die Verbindungen durch Umkristallisation, Destillation oder Chromatographie gereinigt.

### Verfahren AH

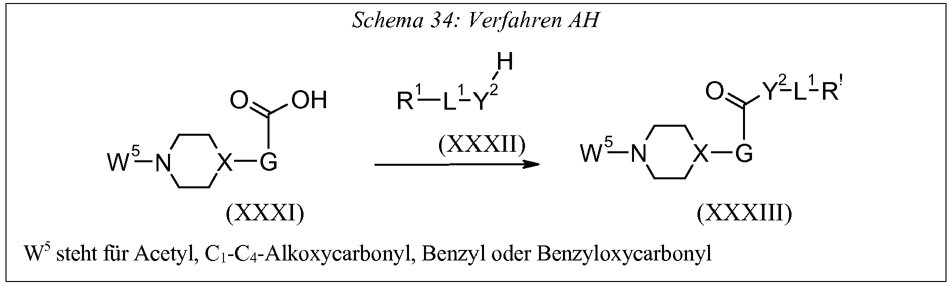

Eine Möglichkeit Verbindungen der Formel (XXXIII) aus entsprechenden Verbindungen (XXXI) darzustellen ist in Schema 34 gezeigt.

Eine Verbindung mit der Formel (XXXIII) kann aus der entsprechenden Verbindung der Formel (XXXI) mit einem Substrat der Formel (XXXII) in der Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (wie etwa N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Die bevorzugten Lösungsmittel sind *N,N*-Dimethylformamid und Dichlormethan.

Die Reaktion wird normalerweise bei Temperaturen von 0 °C - 100 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (XXXIII) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung der Formel (XXXIII) auch ausgehend von der Verbindung der Formel (XXXI) durch eine zweistufige Transformation synthetisiert werden, unter Verwendung literaturbekannter Prozesse (z.B. Tetrahedron 2005, 61, 10827-10852, und darin zitierte Literatur), gegebenenfalls in der Gegenwart eines Säurefängers / Base. Typischerweise wird eine Verbindung der Formel (XXXI) zunächst in das korrespondierende Säurehalogenid oder -sulfonat überführt, dann erfolgt eine Kupplungsreaktion mit einem Substrat der Formel (XXXII).

Substrate mit der allgemeinen Formel (XXXII) sind kommerziell erhältlich oder durch in der Literatur beschriebene Prozesse darstellbar (siehe z.B. "The Chemistry of Functional groups"; "The Chemistry of the Thiol Group"; John Wiley & Sons, 1974, 163-269, und darin zitierte Referenzen; "The Chemistry of Functional groups"; "Supplement F2: The Chemistry of amino, nitroso, nitro and related groups"; John Wiley & Sons, und darin zitierte Referenzen; "Science of Synthesis"; "Alcohols", Volume 36, Thieme, 2008 und darin zitierte Referenzen; "Science of Synthesis"; "Amines and Ammonium Salts", Volume 40a, Thieme, 2008, und darin zitierte Referenzen).

Bei der Durchführung des erfindungsgemäßen Verfahrens AH können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden, oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig-Base, Triethylamin oder kommerziell erhältliche polymere Säurefänger) im Verhältnis zum Startmaterial der allgemeinen Formel (XXXII) wird verwendet. Ist das Startmaterial ein Salz, werden wenigstens zwei Äquivalente des Säurefängers benötigt.

Die Reaktion wird normalerweise bei Temperaturen von 0 °C - 100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (XXXIII), von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren AI

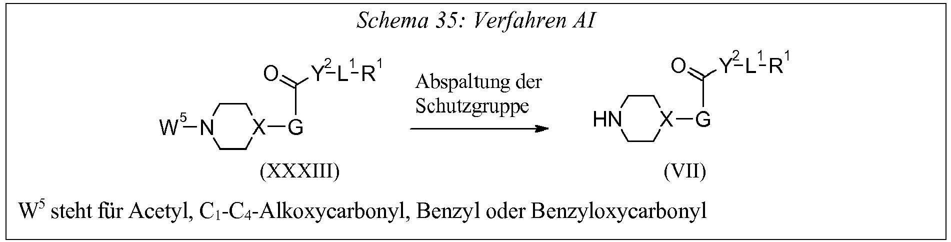

Eine Möglichkeit Verbindungen der Formel (VII) aus entsprechenden Verbindungen (XXXIII) darzustellen ist in Schema 35 gezeigt.

Eine Verbindung der Formel (XXXIII) wird in eine Verbindung der Formel (VII) durch geeignete Methoden zur Entfernung von Schutzgruppen, die in der Literatur beschrieben sind ("Protective Groups in Organic Synthesis"; Third Edition; 494-653, und dort zitierte Literatur), überführt.

*tert*-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis", Third Edition; S. 494-653).

Die Reaktion wird normalerweise bei Temperaturen von 0° C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion werden die Verbindungen (VII) von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel (VII) als Salz zu isolieren, z.B. als Salz der Salzsäure oder der Trifluoressigsäure.

### Verfahren AJ

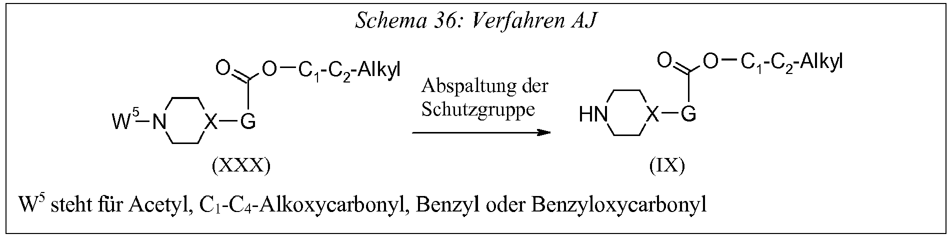

Eine Möglichkeit Verbindungen der Formel (IX) aus entsprechenden Verbindungen (XXX) herzustellen ist in Schema 36 gezeigt.

Es wird das gleiche Verfahren verwendet, dass bereits im Schema 35 (Verfahren AI) beschrieben wurde.

### Verfahren AK

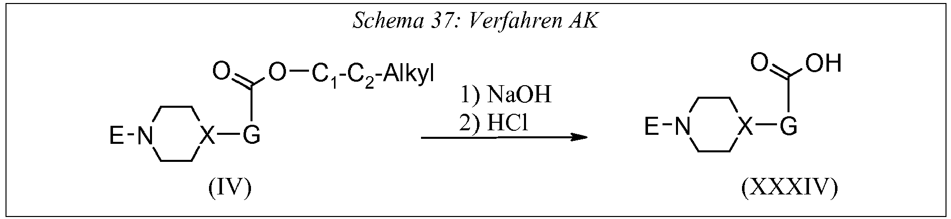

Eine Möglichkeit Verbindungen der Formel (XXXIV) aus entsprechenden Verbindungen (IV) herzustellen ist in Schema 37 gezeigt.

Es wird das gleiche Verfahren verwendet, dass bereits im Schema 33 (Verfahren AG) beschrieben wurde.

### Verfahren AL

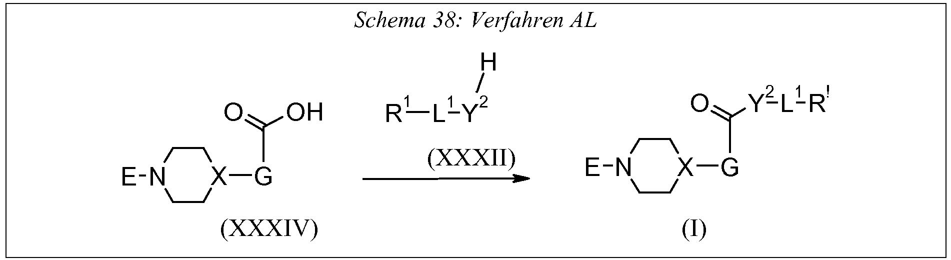

Eine Möglichkeit Verbindungen der Formel (I) aus entsprechenden Verbindungen (XXXIV) und (XXXII) herzustellen ist in Schema 38 gezeigt.

Es wird das gleiche Verfahren verwendet, dass bereits im Schema 34 (Verfahren AH) beschrieben wurde.

Bei allen erfindungsgemäßen Verfahren wird im Allgemeinen (wenn nicht anders angegeben) unter Normaldruck gearbeitet. Es ist jedoch auch möglich, unter erhöhtem oder vermindertem Druck - im Allgemeinen zwischen 0,1 bar und 10 bar - zu arbeiten.

Bevorzugte Restedefinitionen der vorstehenden und nachfolgend genannten Formeln und Schemata sind bereits oben angegeben. Diese Definitionen gelten nicht nur für die Endprodukte der Formel (I) sondern auch für alle Zwischenprodukte gleichermaßen.

Die vorliegende Erfindung betrifft weiterhin ein Mittel, zum Bekämpfen von unerwünschten Mikroorganismen, umfassend die erfindungsgemäßen Wirkstoffe. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man die erfindungsgemäßen Wirkstoffe auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zukkerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Com Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren BeizmittelFormulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Biound Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference] Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trokkenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffe auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter
   http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Veizweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffeauch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich, sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen produziert werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hof mannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und lekkend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

### Borstenschwänze wie Lepisma saccharina.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alf reddugesi, Neutrombicula autumnalis, Dermatophagoides pteronissimus, Dermatophagoides forinae. Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die aufgeführten Pflanzen können besonders vorteilhaft erfindungsgemäß mit den Verbindungen der allgemeinen Formel (I) den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Erfindung wird durch die folgenden Beispiele veranschaulicht. Die Erfindung ist jedoch nicht auf die Beispiele limitiert.

### Herstellung von Ausgangsstoffen der Formel (XXXI):

### 2-[1-(tert-Butoxycarbonyl)piperidin-4-yl]-1,3-thiazol-4-carbonsäure (XXXI-1)

Zu einer Lösung von *tert*-Butyl-4-[4-(ethoxycarbonyl)-1,3-thiazol-2-yl]piperidin-1-carboxylat (24.0 g) in Tetrahydrofuran (240 mL) und Wasser (60 mL) wird bei Raumtemperatur Lithiumhydroxid-Monohydrad (8.88 g) in einer Portion gegeben. Das Gemisch wird für 4 Stunden gerührt und dann mit verdünnter Salzsäure (IM) (100 mL) und Ethylacetat (100 mL) verrührt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Man erhält 2-[1-(*tert*-Butoxycarbonyl)piperidin-4-yl]-1,3-thiazol-4-carbonsäure (21 g, 94%).

logP (pH 2.7): 2.04

¹H NMR (DMSO-d₆, 400 MHz): δₚₚₘ : 1.41 (s, 9H), 1.59 (qd, 2H), 2.02 (dd, 2H), 2.91 (m, 2H), 3.23 (m, 1H), 3.97-4.02 (m, 2H), 8.27 (s, 1H)

MS (ESI): 256 ([M+H-C(CH₃)₃]⁺)

### Herstellung von Ausgangsstoffen der Formel (XXXIII):

### tert-Butyl-4-{4-[(cyclohexyloxy)carbonyl]-1,3-thiazol-2-yl}piperidin-1-carboxylat (XXXIII-1)

Zu einer Lösung von 2-[1-(*tert*-Butoxycarbonyl)piperidin-4-yl]-1,3-thiazol-4-carbonsäure (**XXXI-1,** 2.90 g) in Dichlormethan (30 mL) wird bei Raumtemperatur Cyclohexanol (1.21 g), Dimethylaminopyridin (113 mg) und 1-Ethyl-3-(3'-dimethylaminopropyl)carbodiimide (1.87 g) gegeben. Das Gemisch wird über Nacht bei Raumtemperatur gerührt und dann mit Wasser versetzt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Der Rückstand wird chromatographisch gereinigt. Man erhält *tert*-Butyl-4-{4-[(cyclohexyloxy)carbonyl]-1,3-thiazol-2-yl}piperidin-1-carboxylat (2.63 g, 72%).

logP (pH 2.7): 4.62

¹H NMR (DMSO-d₆, 400 MHz): δₚₚₘ : 1.13-1.81 (m + s, 21H), 2.02 (m, 2H), 2.90 (m, 2H), 3.40 (m, 1H), 3.98-4.01 (m, 2H), 4.90 (m, 1H), 8.32 (s, 1H)

MS (ESI): 339 ([M+2H-C(CH₃)₃]⁺)

### Herstellung von Ausgangsstoffen der Formel (VII):

### 4-{4-[(Cyclohexyloxy)carbonyl]-1,3-thiazol-2-yl}piperidiniumchlorid (VII-1)

Zu einer Lösung von *tert*-Butyl-4-{4-[(cyclohexyloxy)carbonyl]-1,3-thiazol-2-yl}piperidin-1-carboxylat (**XXXIII-1**, 2.63 g) in Dioxan (20 mL) wird unter Argon bei 0 °C eine 2-molare Lösung von Chlorwasserstoff in Diethylether (50 mL) getropft. Das Reaktionsgemisch wird bei 0 °C gerührt und dann langsam auf Raumtemperatur erwärmt. Nach Rühren über Nacht werden das Lösungsmittel und überschüssiger Chlorwasserstoff entfernt. Man erhält 4-{4-[(Cyclohexyloxy)carbonyl]-1,3-thiazol-2-yl}piperidiniumchlorid (2.19 g, 99%).

logP (pH 2.7): 1.25

¹H NMR (DMSO-d₆, 400 MHz): δₚₚₘ: 1.15-1.55 (m, 6H), 1.71-1.75 (m, 2H), 1.85-1.90 (m, 2H), 1.98-2.04 (m, 2H), 2.20 (dd, 2H), 3.01-3.03 (m, 2H), 3.14-3.34 (m, 2H), 3.40 (m, 1H), 4.90 (m, 1H), 8.36 (s, 1H), 9.05 (bs, 1H), 9.25 (bs, 1H)

MS (ESI): 295 ([M-Cl]⁺)

### Herstellung von Verbindungen der Formel (I):

### Cyclohexyl-2-{1-[(2,5-dibromphenyl)acetyl]piperidin-4-yl}-1,3-thiazol-4-carboxylat (1-15)

(2,5-Dibromphenyl)essigsäure (323 mg) und Hünigbase (323 mg) werden in Dichlormethan (10 mL) gelöst und für 30 min bei Raumtemperatur gerührt. 4-{4-[(Cyclohexyloxy)carbonyl]-1,3-thiazol-2-yl}piperidiniumchlorid (**VII-1,** 330 mg) wird zugegeben und das Gemisch weitere 5 min gerührt, bevor Brom-tris-pyrrolidino-phosphoniumhexafluorophosphat (559 mg) zugegeben wird. Die Reaktionsmischung wird über Nacht bei Raumtemperatur gerührt. Nach Entfernen des Lösungsmittels unter vermindertem Druck wird der Rückstand chromatographisch gereinigt. Man erhält Cyclohexyl-2-{1-[(2,5-dibromphenyl)acetyl]piperidin-4-yl}-1,3-thiazol-4-carboxylat (250 mg, 43%).

logP (pH 2.7): 4.72

¹H NMR (DMSO-d₆, 400 MHz): δₚₚₘ : 1.25-1.57 (m, 6H), 1.68-1.80 (m, 4H), 1.88 (m, 2H), 2.09 (m, 2H), 2.83 (bs, 1H), 3.29 (bs, 1H), 3.37 (m ,1H), 3.86 (s, 2H), 4.06 (bs, 1H), 4.38 (bs, 1H), 4.90 (m, 1H), 7.36 (dd, 1H), 7.50-7.53 (m, 2H), 8.33 (s, 1H)

### Cyclohexyl-2-{1-[(2,5-difluorbenzyl)sulfonyl]piperidin-4-yl}-1,3-thiazol-4-carboxylat (1-30)

Zu einer Lösung von 4-{4-[(Cyclohexyloxy)carbonyl]-1,3-thiazol-2-yl}piperidiniumchlorid (**VII**-1, 330 mg) und Triethylamin (303 mg) in Dichlormethan (5 mL) wird bei Raumtemperatur (2,5-difluorophenyl)methanesulfonyl chloride (226 mg) gegeben. Das Gemisch wird über Nacht bei Raumtemperatur gerührt und dann mit Wasser versetzt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Der Rückstand wird chromatographisch gereinigt. Man erhält Cyclohexyl-2-{1-[(2,5-difluorbenzyl)sulfonyl]piperidin-4-yl}-1,3-thiazol-4-carboxylat (156 mg, 32%).

logP(pH 2.7): 4.05

¹H NMR (DMSO-d₆, 400 MHz): δₚₚₘ : 1.25-1.60 (m, 6H), 1.63-1.78 (m, 4H), 1.85-1.89 (m, 2H), 2.09-2.13 (m, 2H), 2.95-3.01 (m, 2H), 3.22 (m, 1H), 3.63-3.66 (m, 2H), 4.44 (s, 2H), 4.90 (m, 1H), 7.21-7.34 (m, 3H), 8.34 (s, 1H)

MS (ESI): 485 ([M+H]⁺)

### 2-Methyl-5-(trifluormethyl)phenyl-4-{4-[(cyclohexyloxy)carbonyl]-1,3-thiazol-2-yl}piperidin-1-carboxylat (I-38)

Zu einer Lösung von 4-{4-[(Cyclohexyloxy)carbonyl]-1,3-thiazol-2-yl}piperidiniumchlorid **(VII-1,** 324 mg) und Diisopropylethylamin (323 mg) in Dichlormethan (8 mL) wird bei Raumtemperatur 2-Methyl-5-(trifluormethyl)phenylchlorocarbonat (239 mg) gegeben. Das Gemisch wird über Nacht bei Raumtemperatur gerührt und dann mit Wasser versetzt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Der Rückstand wird chromatographisch gereinigt. Man erhält 2-Methyl-5-(trifluormethyl)phenyl-4-{4-[(cyclohexyloxy)carbonyl]-1,3-thiazol-2-yl}piperidin-1-carboxylat (193 mg, 39%).

logP(pH 2.7): 5.28

¹H NMR (DMSO-d₆, 400 MHz): δₚₚₘ : 1.28-1.58 (m, 6H), 1.72-1.89 (m, 6H), 2.15 (m, 2H), 2.24 (s, 3H), 3.20 (m, 2H), 3.34-3.41 (m, 1H), 4.18 (m, 2H), 4.91 (m, 1H), 7.46-7.50 (m, 3H), 8.35 (s, 1H)

MS (ESI): 497 ([M+H]⁺)

### Cyclohexyl-2-{1-[(2,5-dimethylphenyl)carbamoyl]piperidin-4-yl}-1,3-thiazol-4-carboxylat (I-59)

4-{4-[(Cyclohexyloxy)carbonyl]-1,3-thiazol-2-yl}piperidiniumchlorid (**VII-1**, 330 mg) wird mit Dichloromethan und einer gesättigen Bicarbonatlösung 10 Minuten gerührt. Die organische Phase wird abgetrennt, getrocknet und eingedampft. Der Rückstand wird in Dichloromethan (5 mL) gelöst. Zu dieser Lösung wird nacheinander bei Raumtemperatur 2-Isocyanato-1,4-dimethylbenzol (162 mg) und ein Tropfen 2,3,4,6,7,8,9,10-Octahydropyrimido[1,2-a]azepin (DBU) gegeben. Das Gemisch wird über Nacht bei Raumtemperatur gerührt und dann mit Wasser versetzt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Der Rückstand wird chromatographisch gereinigt. Man erhält Cyclohexyl-2-{1-[(2,5-dimethylphenyl)carbamoyl]piperidin-4-yl}-1,3-thiazol-4-carboxylat (95 mg, 19%).

logP (pH 2.7): 3.82

¹H NMR (DMSO-d₆, 400 MHz): δₚₚₘ : 1.22-1.58 (m, 6H), 1.59-1.75 (m, 4H), 1.88 (m, 2H), 2.08 (m, 2H), 2.11 (s, 3H), 2.23 (s, 3H), 2.94-2.99 (m, 2H), 3.04-3.34 (m, 1H), 4.14-4.17 (m, 2H), 4.89 (m, 1H), 6.84 (d, 1H), 7.00 (s, 1H), 7.03 (d, 1H), 7.99 (s, 1H), 8.40 (s, 1H)

MS (ESI): 442 ([M+H]⁺)

### Cyclohexyl-2-(4-{[5-methyl-3-(trifluormethyl)-1H-pyrazol-1-yl]acetyl}piperazin-1-yl)-1,3-thiazol-4-carboxylat (I-46)

Zu einer Lösung von 2-(4-{[5-Methyl-3-(trifluormethyl)-1H-pyrazol-1-yl]acetyl}piperazin-1-yl)-1,3-thiazol-4-carbonsäure **(XXXIV-1,** 300 mg) in Dichlormethan (5 mL) wird bei Raumtemperatur Cyclohexanol (82 mg), Dimethylaminopyridin (10 mg) und 1-Ethyl-3-(3'-dimethylaminopropyl)carbodiimide (150 mg) gegeben. Das Gemisch wird über Nacht gerührt und dann mit Wasser versetzt. Die wässrige Phase wird abgetrennt und mit Ethylacetat extrahiert. Die vereinigten organischen Phasen werden mit Natriumsulfat getrocknet. Der Feststoff wird abfiltriert und das Lösungsmittel abdestilliert. Der Rückstand wird chromatographisch gereinigt. Man erhält Cyclohexyl-2-(4-{[5-methyl-3-(trifluormethyl)-1H-pyrazol-1-yl]acetyl}piperazin-1-yl)-1,3-thiazol-4-carboxylat (45 mg, 12%).

logP (pH 2.7): 3.69

¹H NMR (DMSO-d₆, 400 MHz): δₚₚₘ : 1.23-1.58 (m, 6H), 1.65-1.78 (m, 2H), 1.80-1.90 (m, 2H), 2.22 (s, 3H), 3.40-3.70 (m, 8H), 4.80-4.89 (m, 1H), 5.25 (s, 2H), 6.46 (s, 1H), 7.67 (s, 1H)

MS (ESI): 486 ([M+H]⁺)

Analog den vorangehenden Beispielen sowie entsprechend den allgemeinen Beschreibungen der erfindungsgemäßen Verfahren können die in der folgenden Tabelle 1 genannten Verbindungen der Formel (I) erhalten werden.

**Tabelle I**

| | | | | | | | | | |
|---|---|---|---|---|---|---|---|---|---|
| | | | 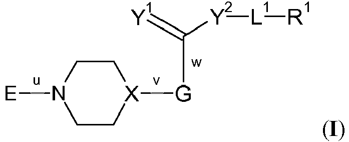 | | | | | | |
| 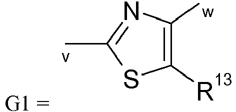 | | 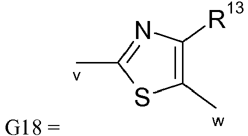 | | | | | | | |
| Y¹ = O für alle Beispiele aus Tabelle I. | | | | | | | | | |
| R¹³ =H für alle Beispiele aus Tabelle I. | | | | | | | | | |

| Beispiel | E | | | X | G | Y² | L¹ | R¹ | logp |
|---|---|---|---|---|---|---|---|---|---|
| 1 | [3-(Trifluormethyl)phenyl]acetyl | | | CH | G1 | O | Bindung | Naphthalen-1-yl | 4,16^{[b]} |
| 2 | [3-(Trifluormethyl)phenyl]acetyl | | | CH | G1 | O | Bindung | Cyclohexyl | 4,21^{[b]} |
| 3 | Pentanoyl | | | CH | G1 | O | Bindung | Naphthalen-1-yl | 3,66^{[b]} |
| 4 | Pentanoyl | | | CH | G1 | O | Bindung | Cyclohexyl | 3,64^{[b]} |
| 5 | (5-Chlor-1-methyl-1H-pyrazol-4-yl)acetyl | | | CH | G1 | O | Bindung | Naphthalen-1-yl | 3,00^{[b]} |
| 6 | (5-Chlor-1-methyl-1H-pyrazol-4-yl)acetyl | | | CH | G1 | O | Bindung | Cyclohexyl | 2,93^{[b]} |
| 7 | Thiophen-3-ylacetyl | | | CH | G1 | O | Bindung | Naphthalen-1-yl | 3,46^{[b]} |
| 8 | Thiophen-3-ylacetyl | | | CH | G1 | O | Bindung | Cyclohexyl | 3,42^{[b]} |
| 9 | (3-Chlorphenyl)acetyl | | | CH | G1 | O | Bindung | Cyclohexyl | 4,04^{[b]} |
| 10 | (3-Chlorphenyl)acetyl | | | CH | G1 | O | Bindung | Naphthalen-1-yl | 4,01^{[b]} |
| 11 | [2-Chlor-5-(trifluormethyl)phenyl]acetyl | | | CH | G1 | O | Bindung | Naphthalen-1-yl | 4,56^{[b]} |
| 12 | [2-Chlor-5-(trifluormethyl)phenyl]acetyl | | | CH | G1 | O | Bindung | Cyclohexyl | 4,64^{[b]} |
| 13 | [2-Brom-5-(trifluormethyl)phenyl]acetyl | | | CH | G1 | O | Bindung | Cyclohexyl | 4,75^{[b]} |
| 14 | (2,5-Dichlorphenyl)acetyl | | | CH | G1 | O | Bindung | Cyclohexyl | 4,49^{[b]} |
| 15 | (2,5-Dibromphenyl)acetyl | | | CH | G1 | O | Bindung | Cyclohexyl | 4,72^{[b]} |
| 16 | [2,5-Bis(trifluormethyl)phenyl]acetyl | | | CH | G1 | O | Bindung | Cyclohexyl | 4,81^{[b]} |
| 17 | (5-Brom-2-methylphenyl)acetyl | | | CH | G1 | O | Bindung | Cyclohexyl | 4,47^{[b]} |
| 18 | [5-Chlor-2-(trifluormethyl)phenyl]acetyl | | | CH | G1 | O | Bindung | Cyclohexyl | 4,68^{[b]} |
| 19 | (5-Iod-2-methylphenyl)acetyl | | | CH | G1 | O | Bindung | Cyclohexyl | 4,66^{[b]} |
| 20 | (2,5-Dimethylthiophen-3-yl)acetyl | | | CH | G1 | O | Bindung | Cyclohexyl | 4,24^{[b]} |
| 21 | (5-Methyl-2-phenyl-1,3-thiazol-4-yl)acetyl | | | CH | G1 | O | Bindung | Cyclohexyl | 4,57^{[b]} |
| 22 | (5-Methyl-2-phenyl-1,3-oxazol-4-yl)acetyl | | | CH | G1 | O | Bindung | Cyclohexyl | 4,15^{[b]} |
| 23 | (5-Methyl-2-phenyl-1,3-thiazol-4-yl)acetyl | | | CH | G1 | O | Bindung | Naphthalen-1-yl | 4,43^{[b]} |
| 24 | (5-Methyl-2-phenyl-1,3-oxazol-4-yl)acetyl | | | CH | G1 | O | Bindung | Naphthalen-1-yl | 4,04^{[b]} |
| 25 | (2,5-Dimethylthiophen-3-yl)acetyl | | | CH | G1 | O | Bindung | Naphthalen-1-yl | 4,12^{[b]} |
| 26 | (2,5-Dimethyl-1,3-thiazol-4-yl)acetyl | | | CH | G1 | O | Bindung | Naphthalen-1-yl | 3,10^{[b]} |
| 27 | (2,5-Dimethyl-1,3-thiazol-4-yl)acetyl | | | CH | G1 | O | Bindung | Cyclohexyl | 3,02^{[b]} |
| 28 | (3,5-Dimethyl-1H-1,2,4-triazol-1-yl)acetyl | | | CH | G1 | O | Bindung | Naphthalen-1-yl | 2,17^{[b]} |
| 29 | (3,5-Dimethyl-1H-1,2,4-triazol-1-yl)acetyl | | | CH | G1 | O | Bindung | Cyclohexyl | 2,08^{[b]} |
| 30 | (2,5-Difluorbenzyl)sulfonyl | | | CH | G1 | O | Bindung | Cyclohexyl | 4,05^{[b]} |
| 31 | [5-Methyl-3-(trifluormethyl)-1H-pyrazol-1-yl]acetyl | | | N | G1 | O | CHCH₃ | 2,5-Dichlorphenyl | 4,28^{[b]} |
| 32 | [5-Methyl-3-(trifluormethyl)-1H-pyrazol-1-yl]acetyl | | | N | G1 | O | Bindung | 3-(Trifluormethyl)cyclohexyl | 3,81^{[c]} |
| 33 | [5-Methyl-3-(trifluormethyl)-1H-pyrazol-1-yl]acetyl | | | N | G1 | S | Bindung | 2-Chlor-5-(trifluormethyl)phenyl | 4,51^{[b]} |
| 34 | [5-Methyl-3-(trifluormethyl)-1H-pyrazol-1-yl]acetyl | | | N | G1 | S | Bindung | Cyclohexyl | 4,43^{[c]} |
| 35 | [5-Methyl-3-(trifluormethyl)-1H-pyrazol-1-yl]acetyl | | | N | G1 | O | CH₂ | 4-Methoxyphenyl | 3,19^{[c]} |
| 36 | [5-Methyl-3-(trifluormethyl)-1H-pyrazol-1-yl]acetyl | | | N | G1 | O | Bindung | Chinolin-8-yl | 2,83^{[c]}; 2.85^{[b]} |
| 37 | [5-Methyl-3-(trifluormethyl)-1H-pyrazol-1-yl]acetyl | | | N | G1 | O | Bindung | 2,4-Dichlorphenyl | 3,87^{[c]}; 3,96^{[b]} |
| 38 | [2-Methyl-5-(trifluormethyl)phenoxy]carbonyl | | | CH | G1 | O | Bindung | Cyclohexyl | 5,28^{[b]} |
| 39 | [5-Methyl-3-(trifluormethyl)-1H-pyrazol-1-yl]acetyl | | | N | G1 | O | Bindung | 2-Chlorphenyl | 3,41^{[c]}; 3,46^{[b]} |
| 40 | [5-Methyl-3-(trifluormethyl)-1H-pyrazol-1-yl]acetyl | | | N | G1 | O | Bindung | Naphthalen-2-yl | 3,76^{[c]}; 3,81^{[b]} |
| 41 | [5-Methyl-3-(trifluormethyl)-1H-pyrazol-1-yl]acetyl | | | N | G1 | O | Bindung | 2,3-Dihydro-1H-inden-2-yl | 3,56^{[c]}; 3,64^{[b]} |
| 42 | [5-Methyl-3-(trifluormethyl)-1H-pyrazol-1-yl]acetyl | | | N | G1 | O | Bindung | 1,2,3,4-Tetrahydronaphthalen-2-yl | 3,82^{[c]}; 3,87^{[b]} |
| 43 | [5-Methyl-3-(trifluormethyl)-1H-pyrazol-1-yl]acetyl | | | N | G1 | O | Bindung | 1,2,3,4-Tetrahydronaphthalen-1-yl | 3,91^{[c]}; 3,98^{[b]} |
| 44 | [5-Methyl-3-(trifluormethyl)-1H-pyrazol-1-yl]acetyl | | | N | G1 | O | CH₂ | 2,4-Dichlorphenyl | 4,12^{[c]}; 4,12^{[b]} |
| 45 | [5-Methyl-3-(trifluormethyl)-1H-pyrazol-1-yl]acetyl | | | N | G1 | S | Bindung | Naphthalen-2-yl | 4,33^{[c]}; 4,33^{[b]} |
| 46 | [5-Methyl-3-(trifluormethyl)-1H-pyrazol-1-yl]acetyl | | | N | G1 | O | Bindung | Cyclohexyl | 3,68^{[c]}; 3,69^{[b]} |
| 47 | [5-Methyl-3-(trifluormethyl)-1H-pyrazol-1-yl]acetyl | | | N | G1 | S | Bindung | 2,4-Dichlorphenyl | 4,5^{[c]}; 4,49^{[b]} |
| 48 | [5-Methyl-3-(trifluormethyl)-1H-pyrazol-1-yl]acetyl | | | N | G1 | O | CH₂ | 2-Chlorphenyl | 3,64^{[c]}; 3,64^{[b]} |
| 49 | [5-Methyl-3-(trifluormethyl)-1H-pyrazol-1-yl]acetyl | | | N | G1 | S | CH₂ | 2-Chlorphenyl | 4,32^{[c]}; 4,29^{[b]} |
| 50 | [5-Methyl-3-(trifluormethyl)-1H-pyrazol-1-yl]acetyl | | | N | G1 | S | CH₂ | 2,4-Dichlorphenyl | 4,87^{[c]}; 4,79^{[b]} |
| 51 | [5-Methyl-3-(trifluormethyl)-1H-pyrazol-1-yl]acetyl | | | N | G1 | O | CH₂ | 2,6-Difluorphenyl | 3,33^{[c]}; 3,28^{[b]} |
| 52 | [3-Chlor-6-(trifluormethyl)pyridin-2-yl]acetyl | | | CH | G1 | O | Bindung | Cyclohexyl | 4,08^{[b]} |
| 53 | [5-Methyl-3-(trifluormethyl)-1H-pyrazol-1-yl]acetyl | | | N | G1 | O | CH₂ | 2,4-Difluorphenyl | 3,45^{[c]}; 3,42^{[b]} |
| 54 | [5-Methyl-3-(trifluormethyl)-1H-pyrazol-1-yl]acetyl | | | N | G1 | O | CHCH₃ | Cyclohexyl | 4,46^{[c]}; 4,4^{[b]} |
| 55 | Oxo(thiophen-2-yl)acetyl | | | CH | G1 | O | Bindung | Cyclohexyl | 3,46^{[b]} |
| 56 | [3-(Trifluormethyl)cyclohexyl]acetyl | | | CH | G1 | O | Bindung | Cyclohexyl | 4,58^{[b]} |
| 57 | 5,5,6,6,6-Pentafluorhexanoyl | | | CH | G1 | O | Bindung | Cyclohexyl | 4,22^{[b]} |
| 58 | Heptanoyl | | | CH | G1 | O | Bindung | Cyclohexyl | 4,51^{[b]} |
| 59 | (2,5-Dimethylphenyl)carbamoyl | | | CH | G1 | O | Bindung | Cyclohexyl | 3,82^{[b]} |
| 60 | Furan-2-yl(oxo)acetyl | | | CH | G1 | O | Bindung | Cyclohexyl | 3,07^{[b]} |
| 61 | [2-Chlor-5-(trifluormethyl)phenyl]carbamoyl | | | CH | G1 | O | Bindung | Cyclohexyl | 4,76^{[b]} |
| 62 | (2-Chlor-5-methylphenyl)carbamoyl | | | CH | G1 | O | Bindung | Cyclohexyl | 4,30^{[b]} |
| 63 | (2,5-Dimethylphenyl)carbamothioyl | | | CH | G1 | O | Bindung | Cyclohexyl | 4,17^{[b]} |
| 64 | (2-Fluor-5-methylphenyl)carbamoyl | | | CH | G1 | O | Bindung | Cyclohexyl | 3,81^{[b]} |
| 65 | (3,5-Dimethylphenyl)acetyl | | | CH | G1 | O | Bindung | Cyclohexyl | 4,34^{[c]}; 4,32^{[b]} |
| 66 | (2,4-Dimethylphenyl)acetyl | | | CH | G1 | O | Bindung | Cyclohexyl | 4,33^{[c]}; 4,3^{[b]} |
| 67 | (2,5-Difluorphenyl)carbamothioyl | | | CH | G1 | O | Bindung | Cyclohexyl | 3,86^{[b]} |
| 68 | [3,5-Bis(difluormethyl)-1H-pyrazol-1-yl]acetyl | | | CF | G1 | O | Bindung | (1R)-1,2,3,4-Tetrahydronaphthalen-1-yl | 4,12^{[b]} |
| 69 | [3,5-Bis(difluormethyl)-1H-pyrazol-1-yl]acetyl | | | CF | G1 | O | Bindung | Cyclohexyl | 3,91^{[b]} |
| 70 | (Z)-{[2-Chlor-5-(trifluormethyl)phenyl]imino}(methoxy)methyl | | | CH | G1 | O | Bindung | Cyclohexyl | |
| 71 | (2-Methoxy-5-methylphenyl)carbamoyl | | | CH | G1 | O | Bindung | Cyclohexyl | 4,18^{[b]} |
| 72 | (5-Chlor-2-methylphenyl)carbamoyl | | | CH | G1 | O | Bindung | Cyclohexyl | 4,10^{[b]} |
| 73 | (3,5-Dimethyl-1,2-oxazol-4-yl)carbamothioyl | | | CH | G1 | O | Bindung | Cyclohexyl | 3,18^{[b]} |
| 74 | (5-Fluor-2-methylphenyl)carbamoyl | | | CH | G1 | O | Bindung | Cyclohexyl | 3,74^{[b]} |
| 75 | (5-Chlor-2-methoxyphenyl)carbamoyl | | | CH | G1 | O | Bindung | Cyclohexyl | 4,35^{[b]} |
| 76 | (3,5-Dimethyl-1,2-oxazol-4-yl)carbamoyl | | | CH | G1 | O | Bindung | Cyclohexyl | 2,66^{[b]} |
| 77 | (2,5-Dimethoxyphenyl)carbamoyl | | | CH | G1 | O | Bindung | 1,2,3,4-Tetrahydronaphthalen-1-yl | 4,05^{[b]} |
| 78 | (2,5-Dimethylphenyl)carbamoyl | | | CH | G1 | O | Bindung | 1,2,3,4-Tetrahydronaphthalen-1-yl | 4,09^{[b]} |
| 79 | (5-Fluor-2-methylphenyl)carbamoyl | | | CH | G1 | O | Bindung | 1,2,3,4-Tetrahydronaphthalen-1-yl | 4,00^{[b]} |
| 80 | (2-Fluor-5-methylphenyl)carbamoyl | | | CH | G1 | O | Bindung | 1,2,3,4-Tetrahydronaphthalen-1-yl | 4,08^{[b]} |
| 81 | (2,5-Dimethylphenyl)carbamothioyl | | | CH | G1 | O | Bindung | 1,2,3,4-Tetrahydronaphthalen-1-yl | 4,39^{[b]} |
| 82 | (2-Methoxy-5-methylphenyl)carbamoyl | | | CH | G1 | O | Bindung | 1,2,3,4-Tetrahydronaphthalen-1-yl | 4,33^{[b]} |
| 83 | Cyclopentylacetyl | | | CH | G1 | O | Bindung | Cyclohexyl | 4,12^{[c]}; 4,15^{[b]} |
| 84 | (2,5-Difluorphenyl)carbamoyl | | | CH | G1 | O | CH₂ | 2-Bromphenyl | 3,79^{[c]}; 3,79^{[b]} |
| 85 | (5-Chlor-2-methylphenyl)carbamoyl | | | CH | G1 | O | CH₂ | 2-Bromphenyl | 4,06^{[c]}; 4,04^{[b]} |
| 86 | (1,3-Dimethyl-1H-pyrazol-5-yl)acetyl | | | CH | G1 | O | Bindung | Cyclohexyl | 2,67^{[c]}; 2,6^{[b]} |
| 87 | (2,5-Dichlorphenyl)acetyl | | | CH | G1 | O | CH₂ | 2-Bromphenyl | 4,44^{[c]}; 4,41^{[b]} |
| 88 | (2,5-Dimethylphenyl)carbamothioyl | | | CH | G1 | O | CH₂ | 2-Bromphenyl | 3,71^{[c]}; 3,68^{[b]} |
| 89 | (1,5-Dimethyl-1H-pyrazol-3-yl)acetyl | | | CH | G1 | O | Bindung | Cyclohexyl | 2,68^{[c]}; 2,66^{[b]} |
| 90 | (5-Fluor-2-methylphenyl)carbamoyl | | | CH | G1 | O | CH₂ | 2-Bromphenyl | 3,75^{[c]}; 3,77^{[b]} |
| 91 | (2,5-Dimethylphenyl)carbamoyl | | | CH | G1 | O | CH₂ | 2-Bromphenyl | 3,84^{[c]}; 3,86^{[b]} |
| 92 | (2-Fluor-5-methylphenyl)carbamoyl | | | CH | G1 | O | CH₂ | 2-Bromphenyl | 3,83^{[c]}; 3,85^{[b]} |
| 93 | (2,5-Dimethoxyphenyl)carbamoyl | | | CH | G1 | O | Bindung | Cyclohexyl | 3,76^{[b]} |
| 94 | (2-Methyl-5-nitrophenyl)carbamoyl | | | CH | G1 | O | Bindung | Cyclohexyl | 3,62^{[b]} |
| 95 | (2,5-Dichlorphenyl)carbamoyl | | | CH | G1 | O | Bindung | 1,2,3,4-Tetrahydronaphthalen-1--yl | 4,92^{[b]} |
| 96 | (2,5-Dimethylphenyl)acetyl | | | CH | G1 | O | Bindung | 1,2,3,4-Tetrahydronaphthalen-1-yl | 4,54^{[b]} |
| 97 | (5-Chlor-2-methoxyphenyl)carbamoyl | | | CH | G1 | O | Bindung | 1,2,3,4-Tetrahydronaphthalen-1-yl | 4,64^{[b]} |
| 98 | (3,5-Dimethyl-1,2-oxazol-4-yl)carbamoyl | | | CH | G1 | O | Bindung | 1,2,3,4-Tetrahydronaphthalen-1-yl | 2,97^{[b]} |
| 99 | (2,5-Dichlorphenyl)acetyl | | | CH | G1 | O | Bindung | 1,2,3,4-Tetrahydronaphthalen-1-yl | 4,73^{[b]} |
| 100 | (2-Chlor-5-methylphenyl)carbamoyl | | | CH | G1 | O | Bindung | 1,2,3,4-Tetrahydronaphthalen-1-yl | 4,61^{[b]} |
| 101 | (2,5-Dibromphenyl)acetyl | | | CH | G1 | O | CH₂ | 2-Bromphenyl | 4,64^{[c]}; 4,68^{[b]} |

| Beispiel | E | | | X | G | Y² | L¹ | R¹ | logp |
|---|---|---|---|---|---|---|---|---|---|
| 102 | (2,5-Dimethylphenyl)acetyl | | | CH | G1 | O | CH₂ | 2-Bromphenyl | 4,27^{[c]}; 4,23^{[b]} |
| 103 | (2,5-Dimethoxyphenyl)carbamoyl | | | CH | G1 | O | CH₂ | 2-Bromphenyl | 3,81^{[c]}; 3,83^{[b]} |
| 104 | (2-Methoxy-5-methylphenyl)carbamoyl | | | CH | G1 | O | CH₂ | 2-Bromphenyl | 4,12^{[c]}; 4,08^{[b]} |
| 105 | (5-Chlor-2-methoxyphenyl)carbamoyl | | | CH | G1 | O | CH₂ | 2-Bromphenyl | 4,36^{[c]}; 4,35^{[b]} |
| 106 | [2-Chlor-5-(trifluormethyl)phenyl]carbamoyl | | | CH | G1 | O | CH₂ | 2-Bromphenyl | 4,75^{[c]}; 4,7^{[b]} |
| 107 | (2-Chlor-5-methylphenyl)carbamoyl | | | CH | G1 | O | CH₂ | 2-Bromphenyl | 4,33^{[c]}; 4,28^{[b]} |
| 108 | (2,5-Dichlorphenyl)carbamoyl | | | CH | G1 | O | CH₂ | 2-Bromphenyl | 4,61^{[c]}; 4,56^{[b]} |
| 109 | {[1-Methyl-3-(trifluormethyl)-1H-pyrazol-5-yl]oxy}carbonyl | | | CH | G1 | O | Bindung | Cyclohexyl | 4,37^{[cl}; 4,36^{[b]} |
| 110 | (2-Methyl-5-nitrophenyl)carbamoyl | | | CH | G1 | O | CH₂ | 2-Bromphenyl | 3,70^{[c]}; 3,69^{[b]} |
| 111 | [2-Fluor-5-(trifluormethyl)phenyl]carbamoyl | | | CH | G1 | O | CH₂ | 2-Bromphenyl | 4,29^{[c]}; 4,29^{[b]} |
| 112 | (5-Chlor-2-methylphenyl)carbamothioyl | | | CH | G1 | O | CH₂ | 2-Bromphenyl | 4,27^{[c]}; 4,3^{[b]} |
| 113 | (2,5-Dichlorphenyl)carbamothioyl | | | CH | G1 | O | CH₂ | 2-Bromphenyl | 4,39^{[c]}; 4,4^{[b]} |
| 114 | (2-Brom-5-fluorphenyl)carbamothioyl | | | CH | G1 | O | CH₂ | 2-Bromphenyl | 4,02^{[c]}; 4,04^{[b]} |
| 115 | (5-Chlor-2-fluorphenyl)carbamothioyl | | | CH | G1 | O | CH₂ | 2-Bromphenyl | 4,14^{[c]}; 4,15^{[b]} |
| 116 | (2,5-Difluorphenyl)carbamothioyl | | | CH | G1 | O | CH₂ | 2-Bromphenyl | 3,87^{[c]}; 3,83^{[b]} |
| 117 | (5-Fluor-2-methylphenyl)carbamothioyl | | | CH | G1 | O | CH₂ | 2-Bromphenyl | 3,98^{[c]}; 3,98^{[b]} |
| 118 | [2-Fluor-5-(trifluormethyl)phenyl]carbamothioyl | | | CH | G1 | O | CH₂ | 2-Bromphenyl | 4,37^{[c]}; 4,34^{[b]} |
| 119 | (3,5-Dimethyl-1,2-oxazol-4-yl)carbamothioyl | | | CH | G1 | O | CH₂ | 2-Bromphenyl | 3,21^{[c]}; 3,2^{[b]} |
| 120 | (2,5-Dimethylphenyl)carbamoyl | | | CH | G1 | O | CH₂ | 2-Chlor-6-fluorphenyl | 3,66^{[b]} |
| 121 | (2,5-Dimethylphenyl)carbamoyl | | | CH | G1 | O | CH₂ | 2,6-Difluorphenyl | 3,38^{[b]} |
| 122 | (2,5-Dimethylphenyl)carbamoyl | | | CH | G1 | O | Bindung | (1R,2S)-2-Phenylcyclohexyl | 4,43^{[b]} |
| 123 | (2,5-Dimethylphenyl)carbamoyl | | | CH | G1 | O | Bindung | (1S,2R)-2-Phenylcyclohexyl | 4,42^{[b]} |
| 124 | (2,5-Difluorphenyl)carbamothioyl | | | CH | G1 | O | Bindung | 1,2,3,4-Tetrahydronaphthalen-1-yl | 3,94^{[c]}; 4,06^{[b]} |
| 125 | (5-Chlor-2-methylphenyl)carbamoyl | | | CH | G1 | O | Bindung | 1,2,3,4-Tetrahydronaphthalen-1-yl | 4,14^{[c]}; 4,27^{[b]} |
| 126 | (2,5-Dimethylphenyl)carbamoyl | | | CF | G1 | O | Bindung | (1R)-1,2,3,4-Tetrahydronaphthalen-1-yl | 4,30^{[b]} |
| 127 | [5-Methyl-3-(trifluormethyl)-1H-pyrazol-1-yl]acetyl | | | CF | G1 | O | Bindung | (1R)-1,2,3,4-Tetrahydronaphthalen-1-yl | 4,25^{[b]} |
| 128 | (2,5-Dichlorphenyl)acetyl | | | CF | G1 | O | Bindung | (1R)-1,2,3,4-Tetrahydronaphthalen-1-yl | |
| 129 | (3,5-Dimethyl-1,2-oxazol-4-yl)carbamoyl | | | CH | G1 | O | CH₂ | 2-Bromphenyl | |
| 130 | (2-Fluor-5-methylphenyl)carbamoyl | | | CH | G1 | O | Bindung | (1R,2S)-2-Phenylcyclohexyl | 4,45^{[b]} |
| 131 | [2-Chlor-5-(trifluormethyl)phenyl]carbamoyl | | | CH | G1 | O | Bindung | (1R,2S)-2-Phenylcyclohexyl | 5,32^{[b]} |
| 132 | (2-Methoxy-5-methylphenyl)carbamoyl | | | CH | G1 | O | Bindung | (1R,2S)-2-Phenylcyclohexyl | 4,74^{[b]} |
| 133 | (5-Chlor-2-methylphenyl)carbamoyl | | | CH | G1 | O | Bindung | (1R,2S)-2-Phenylcyclohexyl | 4,65^{[b]} |
| 134 | (2-Chlor-5-methylphenyl)carbamoyl | | | CH | G1 | O | Bindung | (1R,2S)-2-Phenylcyclohexyl | 4,96^{[b]} |
| 135 | (2,5-Dimethylphenyl)carbamoyl | | | CH | G1 | O | CH₂ | 2-Chlorphenyl | 3,73^{[b]} |
| 136 | (2,5-Dimethylphenyl)carbamoyl | | | CH | G1 | O | CH₂ | 2-Iodphenyl | 3,96^{[b]} |
| 137 | (2,5-Dimethylphenyl)carbamoyl | | | CH | G1 | O | CH₂ | 2,6-Dichlorphenyl | 3,97^{[b]} |
| 138 | (2,5-Dimethylphenyl)carbamoyl | | | CH | G1 | O | CHCH₃ | 2-Fluor-6-methoxyphenyl | 3,79^{[b]} |
| 139 | (2,5-Dimethylphenyl)carbamoyl | | | CH | G1 | O | CH₂ | 3-Chlorpyridin-4-yl | 2,77^{[b]} |
| 140 | (2,5-Dimethylphenyl)carbamoyl | | | CH | G1 | O | CH₂ | 2-Fluorphenyl | 3,39^{[b]} |
| 141 | (2,5-Dimethylphenyl)carbamoyl | | | CH | G1 | O | Bindung | 2-Brom-6-methylphenyl | 3,82^{[b]} |
| 142 | (2,5-Dimethylphenyl)carbamoyl | | | CH | G1 | O | Bindung | 2-Brom-6-methoxyphenyl | 3,56^{[b]} |
| 143 | (2,5-Dimethylphenyl)carbamoyl | | | CH | G1 | O | Bindung | 2-Chlor-6-methylphenyl | 3,76^{[b]} |
| 144 | (2,5-Dimethylphenyl)carbamoyl | | | CH | G1 | O | Bindung | 2,6-Dibromphenyl | 3,93^{[b]} |
| 145 | (2,5-Dimethylphenyl)carbamoyl | | | CH | G1 | O | Bindung | 2-Fluor-6-methoxyphenyl | 3,28^{[b]} |
| 146 | (2,5-Dimethylphenyl)carbamoyl | | | CH | G1 | O | Bindung | 2-Bromphenyl | 3,58^{[b]} |
| 147 | (2,5-Dimethylphenyl)carbamoyl | | | CH | G1 | O | Bindung | 2-Fluorphenyl | 3,27^{[b]} |
| 148 | (2,5-Dimethylphenyl)carbamoyl | | | CH | G1 | O | Bindung | 2-Chlorphenyl | 3,51^{[b]} |
| 149 | (2,5-Dimethylphenyl)carbamoyl | | | CH | G1 | O | Bindung | 2-Chlor-6-fluorphenyl | 3,62^{[b]} |
| 150 | (2,5-Dimethylphenyl)carbamoyl | | | CH | G1 | O | Bindung | 2,6-Difluorphenyl | 3,39^{[b]} |
| 151 | (2,5-Dimethylphenyl)carbamoyl | | | CH | G1 | O | Bindung | 2,6-Dichlorphenyl | 3,82^{[b]} |
| 152 | (2,5-Dimethylphenyl)carbamoyl | | | CH | G1 | O | Bindung | 2-Brom-6-fluorphenyl | 3,86^{[b]} |
| 153 | (2,5-Dimethylphenyl)carbamoyl | | | CH | G1 | O | CHCH₃ | 2-Fluorphenyl | 3,71^{[b]} |
| 154 | (2,5-Dimethylphenyl)carbamoyl | | | CH | G1 | O | Bindung | 2-Iodphenyl | 3,69^{[b]} |
| 155 | (2,5-Dimethylphenyl)carbamoyl | | | CH | G1 | O | CH₂ | 2-Brom-6-fluorphenyl | 3,79^{[b]} |
| 156 | (2,5-Dimethylphenyl)carbamoyl | | | CH | G1 | O | CHCH₃ | 2,6-Dichlorphenyl | 4,35^{[b]} |
| 157 | (2,5-Dimethylphenyl)carbamoyl | | | CH | G1 | O | CH₂CH₂ | 2,6-Dichlorphenyl | 4,29^{[b]} |
| 158 | (2,5-Dimethylphenyl)carbamothioyl | | | CH | G1 | O | Bindung | (1R,2S)-2-Phenylcyclohexyl | 4,73^{[b]} |

| Beispiel | E | | | X | G | Y² | L¹ | R¹ | logp |
|---|---|---|---|---|---|---|---|---|---|
| 159 | (5-Chlor-2-methylphenyl)carbamothioyl | | | CH | G1 | O | Bindung | (1R,2S)-2-Phenylcyclohexyl | 4,83^{[b]} |
| 160 | (2,5-Dimethylphenyl)carbamoyl | | | CH | G1 | O | Bindung | (1R,2S)-2-(3,4-Difluorphenyl)cyclohexyl | 4,52^{[b]} |
| 161 | (5-Chlor-2-methylphenyl)carbamoyl | | | CH | G1 | O | CH₂ | 2-Chlor-6-fluorphenyl | 3,89^{[b]} |
| 162 | (2-Methoxy-5-methylphenyl)carbamoyl | | | CH | G1 | O | CH₂ | 2-Chlor-6-fluorphenyl | 3,92^{[b]} |
| 163 | (5-Chlor-2-methylphenyl)carbamoyl | | | CH | G1 | O | CH₂ | 2,6-Difluorphenyl | 3,62^{[b]} |
| 164 | (2-Methoxy-5-methylphenyl)carbamoyl | | | CH | G1 | O | CH₂ | 2,6-Difluorphenyl | 3,66^{[b]} |
| 165 | [5-Methyl-3-(trifluormethyl)-1H-pyrazol-1-yl]acetyl | | | CF | G1 | O | CH₂ | 2,6-Difluorphenyl | 3,67^{[c]}; 3,65^{[b]} |
| 166 | (5-Chlor-2-methylphenyl)carbamoyl | | | CF | G1 | O | CH₂ | 2,6-Difluorphenyl | 3,86^{[c]}; 3,87^{[b]} |
| 167 | (5-Chlor-2-methylphenyl)carbamoyl | | | CF | G1 | O | Bindung | Cyclohexyl | 4,32^{[c]}; 4,33^{[b]} |
| 168 | [3,5-Bis(difluormethyl)-1H-pyrazol-1-yl]acetyl | | | CF | G1 | O | CH₂ | 2,6-Difluorphenyl | 3,56^{[c]}; 3,53^{[b]} |
| 169 | (2-Methoxy-5-methylphenyl)carbamoyl | | | CF | G1 | O | Bindung | Cyclohexyl | 4,42^{[c]}; 4,4^{[b]} |
| 170 | [5-Methyl-3-(trifluormethyl)-1H-pyrazol-1-yl]acetyl | | | CF | G1 | O | Bindung | Cyclohexyl | 4,09^{[c]}; 4,07^{[b]} |
| 171 | (2,5-Dimethylphenyl)carbamoyl | | | CF | G1 | O | CH₂ | 2,6-Difluorphenyl | 3,71^{[c]}; 3,69^{[b]} |
| 172 | (2,5-Dimethylphenyl)carbamoyl | | | CF | G1 | O | Bindung | Cyclohexyl | 4,15^{[c]}; 4,15^{[b]} |
| 173 | (2-Methoxy-5-methylphenyl)carbamoyl | | | CF | G1 | O | CH₂ | 2,6-Difluorphenyl | 3,92^{[c]}; 3,89^{[b]} |
| 174 | (2-Methoxyethoxy)acetyl | | | CH | G1 | O | Bindung | Cyclohexyl | 2,37^{[a]} |
| 175 | [3,5-Bis(difluormethyl)-1H-pyrazol-1-yl]acetyl | | | CH | G18 | O | CH₂ | 2,6-Difluorphenyl | 3,45^{[b]} |
| 176 | [3,5-Bis(difluormethyl)-1H-pyrazol-1-yl]acetyl | | | CH | G18 | O | Bindung | 2-Chlor-6-fluorphenyl | 3,67^{[b]} |
| 177 | [3,5-Bis(difluormethyl)-1H-pyrazol-1-yl]acetyl | | | CH | G18 | O | CH₂ | 2,3-Dimethylphenyl | 4,00^{[b]} |
| 178 | [3,5-Bis(difluormethyl)-1H-pyrazol-1-yl]acetyl | | | CH | G18 | O | Bindung | Cyclohexyl | 3,97^{[b]} |

| | | | | | | | | | |
|---|---|---|---|---|---|---|---|---|---|
| R¹ besitzt in den Beispielen 68, 122, 123, 126, 127, 128, 130, 131, 132, 133, 134, 158, 159 und 160 jene Spezifizierung der Chiralität, die nach Einbindung von R¹ ins Produkt resultiert. Die Messung der logP Werte erfolgte gemäß EEC Directive 79/831 Annex V.A8 durch HPLC (High Performance Liquid Chromatography) ^{[a]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril. ^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril ^{[c]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).Die lambda-maX-Werte wurden an Hand der UV-Spektren von 200 nm bis 400 nm in den Maxima der chromatographischen Signale ermittelt. | | | | | | | | | |

### NMR-Daten ausgewählter Beispiele

| **Bsp** | **NMR-Daten** |
|---|---|
| I-15 | ¹HNMR δ= 1.25-1.57 (m, 6H), 1.68-1.80 (m, 4H), 1.88 (m, 2H), 2.09 (m, 2H), 2.83 (bs, 1H), 3.29 (bs, 1H), 3.37 (m ,1H), 3.86 (s, 2H), 4.06 (bs, 1H), 4.38 (bs, 1H), 4.90 (m, 1H), 7.36 (dd, 1H), 7.50-7.53 (m, 2H), 8.33 (s, 1H) ppm |
| I-30 | ¹HNMR δ= 1.25-1.60 (m, 6H), 1.63-1.78 (m, 4H), 1.85-1.89 (m, 2H), 2.09-2.13 (m, 2H), 2.95-3.01 (m, 2H), 3.22 (m, 1H), 3.63-3.66 (m, 2H), 4.44 (s, 2H), 4.90 (m, 1H), 7.21-7.34 (m, 3H), 8.34 (s, 1H) ppm |
| I-31 | ¹HNMR δ= 1.59 (d, 3H), 1.94 (s, 1H), 2.21 (s, 3H), 3.46-3.61 (m, 4H), 3.61-3.70 (m, 4H), 5.22 (s, 2H), 6.48 (s, 1H), 7.41 (dd, 1H), 7.49 (d, 1H), 7.60 (s, 1H), 7.89 (s, 1H) ppm |
| I-32 | ¹HNMR δ= 1.21-1.30 (m, 2H), 1.32-1.49 (m, 2H), 1.58-1.72 (m, 2H), 1.80-1.97 (m, 2H), 1.98-2.03 (m, 1H), 2.22 (s, 3H), 2.32 (s, 1H), 3.41-3.60 (m, 4H), 3.60-3.69 (m, 4H), 5.24 (s, 2H), 6.43 (s, 1H), 7.69 (s, 1H) ppm |
| I-33 | ¹HNMR δ= 2.21 (s, 3H), 3.52-3.65 (m, 4H), 3.65-3.72 (m, 4H), 5.25 (s, 2H), 6.49 (s, 1H), 7.81-7.92 (m, 3H), 7.99 (s, 1H) ppm |
| I-34 | ¹HNMR δ= 1,21-1.32 (m, 1H), 1.45-1.51 (m, 4H), 1.51-1.59 (m, 1H), 1.63-1.72 (m, 2H), 1.88-1.97 (m, 2H), 2.22 (s, 3H), 2.50 (s, 1H), 3.45-3.58 (m, 4H), 3.61-3.70 (m, 4H), 5.24 (s, 2H), 6.47 (s, 1H), 7.62 (s, 1H) ppm |
| I-35 | ¹HNMR δ= 1.18 (t, 2H), 1.99 (s, 2H), 2.21 (s, 3H), 3.52 (s, 2H), 3.65 (s, 2H), 3.76 (s, 3H), 4.12 (q, 1H), 5.22 (d, 3H), 6.46 (s, 1H), 6.93 (d, 2H), 7.38 (d, 2H), 7.72 (s, 1H) ppm |
| I-38 | ¹HNMR δ= 1.28-1.58 (m, 6H), 1.72-1.89 (m, 6H), 2.15 (m, 2H), 2.24 (s, 3H), 3.20 (m, 2H), 3.34-3.41 (m, 1H), 4.18 (m, 2H), 4.91 (m, 1H), 7.46-7.50 (m, 3H), 8.35 (s, 1H) ppm |
| I-41 | ¹HNMR δ= 2.22 (s, 3H), 3.03 (dd, 2H), 3.38 (dd, 2H), 3.49 (bs, 4H), 3.63 (bs, 4H), 2.23 (s, 2H), 5.61 (tt, 1H), 6.45 (s, 1H), 7.14-7.20 (m, 2H), 7.22-7.29 (m, 2H), 7.63 ( s, 1H) ppm |
| I-42 | ¹HNMR δ= 1.90-2.12 (m, 2H), 2.23 (s, 3H), 2.75-3.00 (m, 4H), 3.51 (bs, 4H), 3.65 (bs, 4H), 5.24 (s, 2H), 5.21-5.23 (m, 1H), 6.45 (s, 1H), 7.07-7.13 (m, 4H), 7.62 (s, 1H) ppm |
| I-43 | ¹HNMR δ= 1.75-1.89 (m, 1H), 1.90-2.10 (m, 3H), 2.22 (s, 3H), 2.68-2.91 (m, 2H), 3.51 (bs, 4H), 3.64 (bs, 4H), 5.26 (s, 2H), 6.07 (t, 1H), 6.45 (s, 1H), 7.16 (t, 2H), 7.19-7.28 (m, 2H), 7.67 (s, 1H) ppm |
| I-44 | ¹HNMR δ= 2.23 (s, 3H), 3.58 (bs, 4H), 3.68 (bs, 4H), 5.26 (s, 2H), 6.46 (s, 1H), 7.41-7.53 (m, 2H), 7.75 (d, 1H), 8.08 (s, 1H) ppm |
| I-45 | ¹HNMR δ= 2.23 (s, 3H), 3.59 (bs, 4H), 3.69 (bs, 4H), 5.27 (s, 2H), 6.46 (s, 1H), 7.39 (dd, 1H), 7.49-7.58 (m, 2H), 7.76 (d, 1H), 7.89-7.99 (m, 2H), 7.99 (d, 1H), 8.06 (s, 1H) ppm |
| I-46 | ¹HNMR δ= 1.23-1.58 (m, 6H), 1.65-1.78 (m, 2H), 1.80-1.90 (m, 2H), 2.22 (s, 3H), 3.40-3.70 (m, 8H), 4.80-4.89 (m, 1H), 5.25 (s, 2H), 6.46 (s, 1H), 7.67 (s, 1H) ppm |
| I-48 | ¹HNMR δ= 2.23 (s, 3H), 3.58 (bs, 4H), 3.68 (bs, 4H), 5.26 (s, 2H), 6.46 (s, 1H), 7.30-7.48 (m, 3H), 7.59 (dd, 1H), 8.06 (s, 1H) ppm |
| I-49 | ¹HNMR δ= 2.22 (s, 3H), 3.53 (bs, 4H), 3.65 (bs, 4H), 4.29 (s, 2H), 5.24 (s, 2H), 6.54 (s, 1H), 7.28-7.33 (m, 2H), 7.41-7.48 (m, 1H), 7.49-7.55 (m, 1H), 7.72 (s, 1H) ppm |
| I-51 | ¹HNMR δ= 2.21 (s, 3H), 3.40-3.70 (m, 8H), 5.29 (s, 2H), 5.34 (s, 2H), 6.50 (s, 1H), 7.17 (t, 2H), 7.47-7.58 (m, 1H), 7.75 (s, 1H) ppm |
| I-52 | ¹HNMR δ= 1.23-1.59 (m, 6H), 1.68-1.79 (m, 4H), 1.88 (m, 2H), 2.09 (m, 2H), 2.80 (m, 1H), 3.20-3.40 (m, 2H), 4.05 (m, 1H), 4.12 (d, 1H), 4.21 (d, 1H), 4.42 (m, 1H), 4.89 (m, 1H), 7.85 (d, 1H), 8.20 (d, 1H), 8.40 (s, 1H) ppm |
| I-53 | ¹HNMR δ= 2.21 (s, 3H), 3.40-3.70 (m, 8H), 5.30 (s, 4H), 6.50 (s, 1H), 7.13 (td, 1H), 7.30 (td, 1H), 7.60 (q, 1H), 7.80 (s, 1H) ppm |
| I-59 | ¹HNMR δ= 1.22-1.58 (m, 6H), 1.59-1.75 (m, 4H), 1.88 (m, 2H), 2.08 (m, 2H), 2.11 (s, 3H), 2.23 (s, 3H), 2.94-2.99 (m, 2H), 3.04-3.34 (m, 1H), 4.14-4.17 (m, 2H), 4.89 (m, 1H), 6.84 (d, 1H), 7.00 (s, 1H), 7.03 (d, 1H), 7.99 (s, 1H), 8.40 (s, 1H) ppm |

Die chemischen NMR-Verschiebungen in ppm wurden bei 400 MHz falls nicht anders angegeben im Lösungsmittel DMSO-d₆ mit Tetramethylsilan als internem Standard gemessen.

Folgende Abbkürzungen beschreiben die Signalaufspaltung:
b = Breite, s = Singulett, d = Dublett, t = Triplett, q = Quadruplett, m = Multiplett
NMR-Peak Listverfahren

Die 1H-NMR-Daten der Beispiele 1-3 bis 1-178 sind in Form von 1H-NMR-Peaklisten notiert. Zu jedem Signalpeak sind der d-Wert in ppm und die Signalintensität aufgeführt:

| **Bsp** | **NMR-Daten** |
|---|---|
| I-3 | 8.826 (2.01); 8.025 (0.41); 8.019 (0.43); 8.001 (0.46); 7.902 (0.93); 7.880 (0.91); 7.595 (0.63); 7.594 (0.64); 7.589 (0.52); 7.584 (0.60); 7.577 (1.80); 7.569 (0.58); 7.564 (0.50); 7.560 (0.54); 7.556 (0.62); 7.459 (0.71); 7.457 (0.70); 7.441 (0.53); 7.439 (0.53); 5.686 (16.00); 3.418 (0.37); 3.112 (49.27); 2.498 (3.35); 2.493 (6.63); 2.488 (9.18); 2.484 (6.63); 2.479 (3.39); 2.351 (0.75); 2.332 (1.16); 2.314 (0.86); 2.134 (0.35); 1.532 (0.55); 1.513 (0.79); 1.495 (0.65); 1.476 (0.34); 1.351 (0.46); 1.332 (0.71); 1.313 (0.73); 1.295 (0.43); 0.904 (1.58); 0.886 (3.15); 0.872 (0.54); 0.868 (1.36); -0.000 (0.33); |
| I-4 | 8.323 (10.22); 4.922 (0.99); 4.910 (1.15); 4.900 (1.91); 4.890 (1.23); 4.878 (0.97); 4.868 (0.51); 3.356 (0.51); 3.346 (1.00); 3.337 (0.68); 3.328 (1.15); 3.318 (2.09); 3.308 (1.31); 3.305 (1.14); 3.300 (0.88); 3.290 (1.27); 3.280 (0.72); 3.125 (113.32); 3.086 (0.67); 3.075 (0.54); 2.697 (2.08); 2.501 (4.55); 2.496 (8.73); 2.491 (11.92); 2.487 (8.48); 2.482 (4.24); 2.335 (3.89); 2.317 (5.98); 2.298 (4.37); 2.282 (0.78); 2.263 (0.54); 2.082 (1.56); 2.053 (1.74); 1.906 (0.98); 1.903 (1.27); 1.891 (1.75); 1.888 (1.69); 1.881 (1.78); 1.870 (1.80); 1.860 (1.87); 1.851 (1.91); 1.768 (0.57); 1.760 (1.00); 1.753 (1.23); 1.746 (1.52); 1.738 (1.63); 1.729 (1.73); 1.722 (1.84); 1.714 (1.84); 1.572 (1.70); 1.563 (1.74); 1.547 (2.89); 1.540 (4.31); 1.522 (5.01); 1.517 (4.78); 1.504 (5.30); 1.486 (4.34); 1.466 (1.53); 1.445 (0.87); 1.437 (1.11); 1.421 (1.35); 1.413 (2.28); 1.405 (1.51); 1.397 (1.08); 1.389 (2.02); 1.380 (2.00); 1.373 (0.99); 1.363 (1.45); 1.356 (1.33); 1.344 (3.36); 1.325 (4.24); 1.306 (4.22); 1.289 (3.03); 1.271 (0.87); 0.901 (8.16); 0.891 (1.67); 0.883 (16.00); 0.873 (2.20); 0.864 (6.70); 0.855 (0.81); |
| I-5 | 8.830 (4.68); 8.026 (1.00); 8.020 (0.99); 8.011 (0.63); 8.002 (1.11); 7.949 (0.54); 7.903 (2.25); 7.881 (2.20); 7.607 (0.59); 7.595 (1.70); 7.590 (1.35); 7.586 (1.53); 7.578 (4.14); 7.570 (1.45); 7.567 (1.23); 7.562 (1.24); 7.557 (1.44); 7.549 (0.43); 7.461 (1.72); 7.459 (1.70); 7.443 (1.29); 7.441 (1.26); 7.409 (3.24); 5.688 (1.03); 4.065 (0.70); 4.047 (1.79); 4.029 (1.79); 4.011 (0.65); 3.758 (16.00); 3.510 (5.62); 3.460 (1.14); 3.450 (0.35); 3.441 (0.51); 3.431 (0.89); 3.422 (0.56); 3.413 (0.34); 3.403 (0.48); 3.188 (0.36); 3.170 (0.44); 3.153 (0.58); 3.109 (98.29); 2.887 (4.97); 2.751 (0.53); 2.733 (4.15); 2.526 (0.73); 2.497 (11.23); 2.492 (21.48); 2.488 (29.17); 2.483 (20.61); 2.478 (10.14); 2.170 (0.94); 2.161 (0.87); 2.134 (1.00); 2.040 (0.77); 1.974 (7.34); 1.901 (0.40); 1.693 (0.54); 1.675 (0.54); 1.657 (0.52); 1.389 (1.62); 1.195 (1.98); 1.177 (3.88); 1.159 (1.93); -0.000 (1.12); |
| I-6 | 8.326 (4.86); 7.398 (3.25); 5.688 (2.51); 4.922 (0.46); 4.910 (0.55); 4.900 (0.90); 4.890 (0.57); 4.878 (0.45); 4.065 (0.33); 4.047 (0.77); 4.029 (0.79); 4.012 (0.36); 3.759 (16.00); 3.493 (5.90); 3.368 (0.34); 3.359 (0.55); 3.348 (0.46); 3.340 (0.65); 3.330 (1.08); 3.320 (0.69); 3.312 (0.52); 3.302 (0.70); 3.292 (0.50); 3.146 (1.78); 2.526 (0.43); 2.497 (7.21); 2.493 (13.97); 2.488 (19.11); 2.483 (13.64); 2.479 (6.83); 2.086 (0.86); 2.079 (0.87); 2.053 (0.98); 2.047 (0.96); 1.974 (2.85); 1.901 (9.04); 1.881 (0.83); 1.860 (0.84); 1.852 (0.85); 1.746 (0.70); 1.738 (0.79); 1.730 (0.79); 1.723 (0.86); 1.715 (0.85); 1.572 (0.84); 1.563 (0.83); 1.548 (1.29); 1.540 (1.56); 1.532 (1.07); 1.525 (1.13); 1.517 (1.60); 1.509 (1.12); 1.495 (0.72); 1.486 (0.64); 1.446 (0.37); 1.439 (0.48); 1.423 (0.61); 1.414 (1.04); 1.406 (0.73); 1.398 (0.52); 1.390 (0.95); 1.382 (0.92); 1.375 (0.45); 1.366 (0.38); 1.357 (0.54); 1.351 (0.53); 1.322 (0.46); 1.315 (0.46); 1.291 (0.39); 1.195 (0.78); 1.178 (1.51); 1.160 (0.75); -0.000 (0.86); |
| I-7 | 8.818 (12.52); 8.025 (2.64); 8.020 (2.77); 8.010 (1.66); 8.002 (2.86); 7.900 (4.94); 7.881 (6.58); 7.607 (1.40); 7.595 (5.51); 7.590 (3.49); 7.585 (3.84); 7.577 (10.45); 7.570 (3.77); 7.565 (3.08); 7.561 (3.38); 7.556 (3.90); 7.548 (1.16); 7.458 (4.64); 7.456 (4.69); 7.444 (3.56); 7.437 (6.68); 7.432 (3.85); 7.425 (3.50); 7.253 (3.14); 7.250 (3.35); 7.246 (3.16); 7.243 (2.82); 7.019 (3.78); 7.016 (3.73); 7.007 (3.64); 7.004 (3.64); 4.065 (1.66); 4.047 (4.11); 4.030 (4.03 ); 4.012 (1.53); 3.746 (12.85); 3.698 (1.94); 3.599 (2.34); 3.570 (1.50); 3.441 (1.47); 3.434 (1.61); 3.424 (1.49); 3.416 (1.63); 3.406 (2.62); 3.396 (1.70); 3.388 (1.10); 3.378 (1.55); 3.368 (0.99); 3.317 (1.12); 3.299 (1.59); 3.283 (1.41); 3.131 (210.09); 3.043 (1.41); 3.033 (1.16); 2.888 (4.59); 2.734 (3.76); 2.528 (1.56); 2.499 (26.76); 2.494 (51.16); 2.489 (69.18); 2.485 (48.91); 2.480 (24.21); 2.276 (2.69); 2.126 (1.96); 2.098 (2.16); 1.974 (16.00); 1.677 (1.40); 1.659 (1.50); 1.615 (2.02); 1.606 (2.16); 1.584 (2.05); 1.577 (1.99); 1.400 (1.39); 1.281 (3.09); 1.263 (3.01); 1.244 (1.01); 1.195 (5.78); 1.178 (9.12); 1.160 (4.17); -0.000 (1.63); |
| I-8 | 8.316 (15.93); 7.439 (3.58); 7.432 (4.36); 7.427 (4.09); 7.419 (4.10); 7.240 (3.74); 7.238 (4.10); 7.235 (3.48); 7.233 (3.96); 7.231 (3.79); 7.008 (4.55); 7.005 (4.80); 6.996 (4.42); 6.993 (4.48); 4.906 (1.82); 4.896 (2.96); 4.886 (1.90); 4.048 (2.65); 4.030 (2.71); 3.730 (16.00); 3.699 (2.74); 3.600 (1.97); 3.333 (1.75); 3.314 (2.17); 3.304 (3.67); 3.295 (2.31); 3.286 (1.73); 3.276 (2.12); 3.135 (108.67); 2.889 (6.65); 2.735 (5.69); 2.499 (13.57); 2.495 (26.31); 2.490 (36.01); 2.485 (25.70); 2.481 (12.85); 2.040 (2.71); 2.015 (2.70); 1.974 (9.89); 1.903 (2.79); 1.893 (2.21); 1.878 (2.80); 1.865 (3.08); 1.857 (2.90); 1.848 (3.23); 1.759 (1.78); 1.751 (2.35); 1.744 (2.77); 1.735 (3.17); 1.727 (2.95); 1.719 (3.04); 1.712 (2.88); 1.569 (1.99); 1.561 (2.71); 1.546 (4.47); 1.537 (6.03); 1.530 (5.41); 1.523 (5.89); 1.515 (6.44); 1.506 (5.10); 1.492 (4.42); 1.484 (2.93); 1.436 (1.65); 1.420 (2.05); 1.412 (3.50); 1.403 (2.39); 1.395 (1.66); 1.387 (3.12); 1.379 (3.09); 1.354 (1.92); 1.349 (1.87); 1.314 (1.66); 1.295 (4.27); 1.285 (2.37); 1.280 (6.44); 1.267 (3.28); 1.264 (4.00); 1.196 (2.69); 1.178 (5.23); 1.160 (2.59); |
| I-9 | 8.322 (5.69); 7.340 (0.74); 7.321 (1.97); 7.302 (3.75); 7.275 (1.34); 7.272 (1.56); 7.267 (0.90); 7.255 (0.66); 7.252 (0.63); 7.247 (0.45); 7.209 (1.30);7.190 (0.96); 5.687 (0.41); 4.920 (0.44); 4.908 (0.55); 4.898 (0.89); 4.888 (0.57); 4.876 (0.46); 4.047 (0.85); 4.029 (0.89); 4.011 (0.39); 3.761 (6.14); 3.727 (1.11); 3.352 (0.49); 3.333 (0.52); 3.323 (0.94); 3.313 (0.58); 3.295 (0.56); 3.181 (0.38); 3.114 (184.58); 3.064 (0.57); 3.053 (0.41); 2.526 (1.32); 2.510 (0.98); 2.497 (16.42); 2.493 (32.78); 2.488 (45.47); 2.484 (32.30); 2.479 (16.00); 2.068 (0.84); 2.063 (0.85); 2.040 (1.72); 1.974 (3.34); 1.878 (0.75); 1.867 (0.81); 1.857 (0.87); 1.849 (0.91); 1.760 (0.44); 1.751 (0.54); 1.745 (0.66); 1.737 (0.70); 1.729 (0.76); 1.721 (0.82); 1.714 (0.82); 1.600 (0.47); 1.590 (0.52); 1.570 (1.08); 1.561 (1.20); 1.545 (1.49); 1.539 (1.99); 1.530 (1.50); 1.515 (1.67); 1.493 (0.74); 1.484 (0.63); 1.438 (0.47); 1.422 (0.59); 1.413 (1.03); 1.404 (0.83); 1.398 (0.48); 1.389 (0.92); 1.381 (0.91); 1.356 (0.53); 1.351 (0.54); 1.322 (0.46); 1.315 (0.46); 1.290 (0.38); 1.195 (0.92); 1.177 (1.81); 1.160 (0.91); -0.000 (3.86); |
| I-10 | 8.825 (3.09); 8.026 (0.58); 8.021 (0.64); 8.010 (0.35); 8.003 (0.61); 7.901 (1.15); 7.882 (1.50); 7.608 (0.32); 7.595 (1.11); 7.590 (0.74); 7.585 (0.82); 7.578 (2.53); 7.571 (0.78); 7.565 (0.64); 7.561 (0.77); 7.557 (0.90); 7.459 (1.05); 7.457 (1.09); 7.440 (0.77); 7.438 (0.73); 7.345 (0.50); 7.325 (1.25); 7.317 (0.77); 7.312 (1.13); 7.306 (1.66); 7.277 (0.85); 7.274 (1.01); 7.270 (0.66); 7.253 (0.44); 7.220 (0.76); 7.201 (0.55); 4.065 (0.35); 4.047 (0.90); 4.029 (0.85); 4.011 (0.33); 3.778 (3.47); 3.727 (0.81); 3.425 (0.54); 3.398 (0.34); 3.116 (198.58); 2.526 (1.61); 2.510 (1.25); 2.497 (17.37); 2.493 (34.14); 2.488 (46.95); 2.483 (32.98); 2.479 (16.00); 2.151 (0.48); 2.117 (0.56); 1.974 (3.51); 1.658 (0.44); 1.650 (0.44); 1.633 (0.34); 1.624 (0.48); 1.195 (1.17); 1.177 (2.04); 1.160 (0.95); -0.000 (3.66); |
| I-13 | 8.340 (16.00); 7.828 (3.30); 7.807 (3.60); 7.691 (4.25); 7.686 (4.54); 7.532 (2.35); 7.527 (2.54); 7.511 (2.01); 7.506 (2.14); 5.688 (15.00); 4.929 (1.17); 4.917 (1.29); 4.907 (2.31); 4.897 (1.41); 4.885 (1.14); 3.983 (3.97); 3.860 (2.85); 3.847 (1.33); 3.549 (0.93); 3.410 (1.06); 3.391 (1.19); 3.381 (2.22); 3.371 (1.27); 3.363 (0.84); 3.353 (1.27); 3.343 (1.04); 3.324 (1.09); 3.112 (169.11); 2.527 (1.49); 2.506 (1.10); 2.498 (18.57); 2.493 (38.66); 2.489 (54.80); 2.484 (38.20); 2.479 (18.19); 2.126 (1.32); 2.102 (1.47); 1.974 (1.67); 1.901 (2.27); 1.896 (1.58); 1.893 (1.57); 1.886 (1.76); 1.880 (1.63); 1.874 (1.73); 1.870 (1.71); 1.865 (1.81); 1.857 (1.96); 1.848 (1.13); 1.766 (1.24); 1.760 (1.51); 1.751 (1.84); 1.743 (1.97); 1.734 (2.06); 1.727 (2.23); 1.719 (2.26); 1.578 (1.18); 1.569 (1.16); 1.554 (2.35); 1.546 (3.07); 1.531 (2.19); 1.522 (3.38); 1.513 (2.46); 1.500 (1.60); 1.492 (1.42); 1.442 (1.12); 1.427 (1.36); 1.418 (2.53); 1.413 (2.25); 1.402 (1.03); 1.394 (2.20); 1.386 (2.18); 1.378 (0.92); 1.361 (1.20); 1.355 (1.15); 1.349 (1.06); 1.327 (1.00); 1.320 (1.04); 1.296 (0.91); 1.178 (0.95); -0.000 (3.63); |
| I-14 | 8.408 (0.05); 8.406 (0.04); 7.470 (0.02); 7.449 (0.03); 7.437 (0.02); 7.431 (0.02); 7.371 (0.02); 7.364 (0.01); 7.349 (0.01); 7.343 (0.01); 5.732 (0.03); 5.729 (0.03); 3.885 (0.02); 3.864 (0.02); 3.641 (0.04); 3.636 (0.03); 3.540 (16.00); 3.537 (13.32); 3.484 (0.05); 3.475 (0.05); 3.436 (0.02); 3.420 (0.01); 3.410 (0.01); 3.401 (0.01); 3.391 (0.01); 3.381 (0.01); 3.372 (0.01); 2.538 (0.01); 2.525 (0.20); 2.521 (0.43); 2.516 (0.59); 2.512 (0.44); 2.508 (0.21); 1.885 (0.01); 1.738 (0.01); 1.730 (0.01); 1.712 (0.01); 1.548 (0.01); 1.531 (0.01); 1.523 (0.01); 1.508 (0.01); 1.500 (0.01); -0.000 (0.09); |
| I-16 | 8.345 (14.29); 7.925 (2.07); 7.904 (2.84); 7.825 (2.33); 7.807 (5.74); 5.688 (16.00); 4.933 (0.98); 4.920 (1.08); 4.911 (1.91); 4.901 (1.15); 4.889 (0.94); 4.066 (0.94); 4.048 (4.34); 4.030 (1.10); 3.924 (0.94); 3.509 (0.60); 3.412 (0.84); 3.402 (0.56); 3.393 (0.94); 3.383 (1.75); 3.374 (0.99); 3.365 (0.64); 3.355 (0.99); 3.345 (0.57); 3.314 (0.71); 3.117 (77.91); 2.500 (8.77); 2.495 (18.33); 2.490 (26.00); 2.485 (18.09); 2.481 (8.59); 2.124 (1.01); 2.101 (1.11); 1.975 (2.22); 1.913 (0.63); 1.903 (1.37); 1.898 (1.25); 1.888 (1.41); 1.883 (1.31); 1.873 (1.38); 1.868 (1.48); 1.859 (1.57); 1.850 (0.89); 1.768 (0.96); 1.761 (1.15); 1.753 (1.43); 1.745 (1.56); 1.737 (1.64); 1.729 (1.78); 1.721 (1.81); 1.581 (1.06); 1.572 (1.03); 1.557 (2.11); 1.549 (2.59); 1.535 (1.96); 1.526 (2.75); 1.517 (1.91); 1.504 (1.50); 1.495 (1.21); 1.452 (0.68); 1.444 (0.90); 1.429 (1.12); 1.420 (2.08); 1.413 (1.46); 1.403 (2.99); 1.396 (1.83); 1.387 (1.82); 1.380 (0.75); 1.372 (0.65); 1.363 (0.99); 1.357 (0.94); 1.351 (0.87); 1.343 (0.55); 1.328 (0.83); 1.321 (0.85); 1.297 (0.73); 1.196 (0.68); 1.179 (1.27); 1.161 (0.67); -0.000 (1.77); |
| I-17 | 8.335 (8.17); 7.303 (0.78); 7.298 (1.97); 7.290 (2.46); 7.284 (3.23); 7.276 (1.01); 7.115 (1.81); 7.104 (0.71); 7.093 (1.47); 5.687 (2.09); 4.925 (0.58); 4.913 (0.64); 4.903 (1.14); 4.894 (0.67); 4.881 (0.55); 4.047 (0.81); 4.029 (0.80); 3.728 (4.35); 3.606 (1.96); 3.486 (0.66); 3.382 (0.53); 3.363 (0.60); 3.353 (1.11); 3.343 (0.64); 3.334 (0.68); 3.325 (0.70); 3.316 (0.86); 3.118 (107.81); 2.527 (0.72); 2.506 (0.69); 2.498 (10.83); 2.493 (22.54); 2.489 (31.96); 2.484 (22.20); 2.479 (10.51); 2.162 (16.00); 2.143 (0.56); 2.104 (0.95); 2.098 (0.97); 2.072 (1.12); 2.066 (1.06); 1.974 (3.12); 1.901 (1.10); 1.889 (1.08); 1.872 (0.93); 1.862 (0.90); 1.854 (0.94); 1.762 (0.53); 1.757 (0.64); 1.749 (0.75); 1.741 (0.82); 1.732 (0.85); 1.724 (0.93); 1.717 (0.94);1.575 (0.72); 1.566 (0.68); 1.551 (1.21); 1.543 (1.58); 1.533 (0.96); 1.528 (1.10); 1.519 (1.73); 1.511 (1.22); 1.497 (0.76); 1.489 (0.70); 1.440 (0.53); 1.424 (0.65); 1.416 (1.22); 1.408 (0.76); 1.404 (0.77); 1.392 (1.08); 1.383 (1.06); 1.359 (0.57); 1.352 (0.54); 1.292 (0.91); 1.277 (0.91); 1.263 (0.90); 1.195 (0.91); 1.178 (1.84); 1.160 (0.90); -0.000 (2.28); |
| I-18 | 8.342 (12.37); 7.698 (2.53); 7.678 (3.08); 7.526 (2.04); 7.524 (1.91); 7.505 (6.07); 4.930 (0.92); 4.918 (1.02); 4.908 (1.79); 4.898 (1.08); 4.886 (0.89); 4.065 (0.83); 4.047 (2.13); 4.029 (2.14); 4.011 (0.84); 3.928 (4.41); 3.806 (0.52); 3.403 (0.80); 3.393 (0.52); 3.385 (0.90); 3.375 (1.68); 3.365 (0.95); 3.356 (0.61); 3.346 (0.94); 3.111 (247.22); 2.657 (0.52); 2.511 (1.11); 2.506 (1.52); 2.498 (26.57); 2.493 (55.40); 2.488 (78.58); 2.484 (54.88); 2.479 (26.25); 2.117 (1.07); 2.089 (1.18); 1.974 (8.77); 1.901 (1.30); 1.887 (1.33); 1.880 (1.25); 1.874 (1.30); 1.871 (1.29); 1.866 (1.40); 1.857 (1.49); 1.849 (0.81); 1.766 (0.84); 1.760 (1.03); 1.751 (1.29); 1.744 (1.39); 1.735 (1.50); 1.727 (1.65); 1.719 (1.68); 1.579 (1.03); 1.570 (1.00); 1.556 (1.99); 1.547 (2.44); 1.533 (1.82); 1.524 (2.55); 1.515 (1.82); 1.502 (1.37); 1.493 (1.11); 1.451 (0.67); 1.443 (0.86); 1.427 (1.08); 1.419 (1.99); 1.410 (1.45); 1.404 (16.00); 1.395 (1.97); 1.386 (1.72); 1.379 (0.73); 1.370 (0.61); 1.362 (0.94); 1.356 (0.90); 1.350 (0.84); 1.327 (0.79); 1.320 (0.81); 1.296 (0.68); 1.195 (2.57); 1.178 (5.18); 1.160 (2.53); -0.000 (1.65); |
| I-19 | 8.335 (8.65); 7.472 (1.02); 7.467 (2.03); 7.462 (1.84); 7.458 (2.52); 7.452 (3.81); 7.446 (1.01); 6.969 (1.81); 6.968 (1.81); 6.960 (0.74); 6.947 (1.72); 5.688 (5.81); 4.925 (0.64); 4.913 (0.71); 4.903 (1.23); 4.893 (0.75); 4.881 (0.62); 4.047 (0.96); 4.029 (0.99); 3.698 (3.47); 3.576 (2.31); 3.480 (0.81); 3.361 (0.62); 3.351 (1.15); 3.341 (0.67); 3.332 (0.64); 3.323 (0.76); 3.312 (1.03); 3.112 (177.24); 2.526 (1.04); 2.510 (0.74); 2.506 (1.02); 2.497 (15.73); 2.493 (32.74); 2.488 (46.45); 2.483 (32.35); 2.479 (15.39); 2.183 (0.60); 2.161 (1.29); 2.151 (16.00); 2.101 (1.05); 2.095 (1.07); 2.069 (1.24); 2.062 (1.19); 1.974 (3.86); 1.901 (1.53); 1.886 (1.29); 1.869 (1.05); 1.863 (1.00); 1.854 (1.05); 1.846 (0.58); 1.788 (0.57); 1.756 (0.69); 1.749 (0.82); 1.740 (0.88); 1.732 (0.93); 1.725 (0.99); 1.717 (1.01); 1.575 (0.84); 1.566 (0.80); 1.551 (1.37); 1.543 (1.76); 1.528 (1.22); 1.520 (1.90); 1.511 (1.34); 1.497 (0.85); 1.488 (0.77); 1.440 (0.60); 1.424 (0.73); 1.416 (1.35); 1.408 (0.86); 1.404 (0.64); 1.391 (1.16); 1.383 (1.17); 1.359 (0.64); 1.353 (0.62); 1.195 (1.17); 1.177 (2.26); 1.160 (1.13); -0.000 (3.04); |
| I-20 | 8.320 (11.08); 6.493 (4.42); 6.491 (4.51); 5.687 (4.37); 4.919 (0.84); 4.907 (0.94); 4.898 (1.64); 4.888 (0.99); 4.876 (0.80); 3.517 (6.35); 3.486 (0.49); 3.436 (0.67); 3.419 (1.28); 3.401 (0.87); 3.394 (3.91); 3.338 (0.81); 3.327 (0.53); 3.319 (0.89); 3.309 (1.78); 3.299 (1.16); 3.286 (1.38); 3.281 (1.24); 3.270 (1.00); 3.117 (179.61); 2.511 (0.44); 2.506 (0.63); 2.498 (10.25); 2.493 (21.22); 2.489 (30.04); 2.484 (21.00); 2.479 (10.08); 2.316 (14.91); 2.253 (16.00); 2.240 (5.26); 2.040 (1.38); 2.011 (1.40); 1.974 (0.52); 1.894 (1.04); 1.885 (1.51); 1.867 (1.97); 1.857 (1.47); 1.850 (1.99); 1.840 (0.85); 1.776 (0.74); 1.759 (1.44); 1.743 (1.37); 1.739 (1.40); 1.729 (1.24); 1.721 (1.36); 1.713 (1.31); 1.569 (0.76); 1.559 (1.43); 1.547 (2.19); 1.538 (2.40); 1.530 (2.89); 1.515 (3.40); 1.506 (1.83); 1.497 (2.13); 1.490 (2.03); 1.487 (1.92); 1.468 (0.70); 1.457 (0.60); 1.445 (0.60); 1.437 (0.79); 1.421 (0.98); 1.413 (1.80); 1.405 (1.10); 1.396 (0.71); 1.388 (1.55); 1.380 (1.54); 1.372 (0.64); 1.364 (0.56); 1.355 (0.84); 1.349 (0.79); 1.335 (0.45); 1.321 (0.69); 1.314 (0.74); 1.298 (0.44); 1.289 (0.61); -0.000 (1.24); |
| I-22 | 8.320 (7.94); 7.900 (3.02); 7.895 (2.20); 7.893 (2.12); 7.890 (1.30); 7.886 (1.23); 7.884 (1.47); 7.880 (3.55); 7.876 (3.45); 7.495 (1.05); 7.491 (0.82); 7.483 (2.86); 7.482 (2.96); 7.478 (1.80); 7.471 (2.61); 7.469 (3.16); 7.466 (4.68); 7.463 (4.70); 7.461 (2.57); 7.457 (0.72); 7.453 (0.92); 7.449 (0.74); 5.687 (6.02); 4.918 (0.62); 4.906 (0.68); 4.896 (1.18); 4.886 (0.69); 3.637 (1.87); 3.512 (0.98); 3.352 (0.62); 3.347 (1.17); 3.338 (0.68); 3.329 (0.62); 3.319 (0.73); 3.310 (0.71); 3.145 (0.82); 3.116 (280.09); 2.511 (0.74); 2.506 (1.07); 2.498 (18.61); 2.493 (38.58); 2.489 (54.93); 2.484 (38.45); 2.479 (18.40); 2.340 (16.00); 2.324 (2.22); 2.099 (0.84); 2.069 (0.96); 1.974 (2.20); 1.888 (0.90); 1.876 (0.90); 1.871 (0.90); 1.855 (0.95); 1.846 (0.98); 1.752 (0.76); 1.744 (0.92); 1.735 (0.99); 1.727 (1.04); 1.719 (1.13); 1.712 (1.15); 1.565 (0.70); 1.556 (0.78); 1.541 (1.37); 1.533 (1.60); 1.510 (1.84); 1.501 (1.15); 1.487 (0.73); 1.479 (0.67); 1.419 (0.67); 1.411 (1.27); 1.404 (0.84); 1.387 (1.11); 1.379 (1.13); 1.292 (1.04); 1.277 (1.33); 1.262 (1.13); 1.195 (0.67); 1.178 (1.30); 1.160 (0.64); -0.000 (0.94); |
| I-23 | 8.818 (7.72); 8.026 (1.17); 8.022 (1.56); 8.009 (0.84); 8.003 (1.33); 7.903 (1.71); 7.894 (1.17); 7.892 (1.21); 7.882 (1.99); 7.876 (1.42); 7.874 (1.22); 7.872 (1.08); 7.870 (1.22); 7.832 (2.60); 7.828 (3.27); 7.823 (1.12); 7.816 (1.80); 7.812 (3.47); 7.808 (2.93); 7.802 (0.53); 7.610 (0.51); 7.606 (0.70); 7.597 (1.79); 7.593 (1.74); 7.589 (1.46); 7.578 (3.99); 7.575 (3.25); 7.573 (2.49); 7.569 (1.66); 7.561 (0.85); 7.557 (2.96); 7.555 (1.61); 7.541 (0.59);7.456 (2.98); 7.453 (3.37); 7.447 (0.86); 7.441 (1.14); 7.437 (4.51); 7.435 (5.43); 7.431 (1.82); 7.430 (1.55); 7.420 (1.60); 7.417 (3.11); 7.415 (2.35); 7.409 (1.29); 7.405 (2.33); 7.401 (1.30); 7.394 (0.63); 7.387 (1.51); 7.369 (0.47); 5.690 (10.05); 4.047 (0.72); 4.029 (0.73); 3.868 (0.65); 3.849 (0.67); 3.731 (0.51); 3.701 (0.52); 3.453 (0.52); 3.444 (0.98); 3.434 (0.55); 3.415 (0.53); 3.115 (65.03); 2.506 (0.58); 2.497 (9.26); 2.493 (19.16); 2.488 (27.00); 2.483 (18.86); 2.479 (9.02); 2.424 (16.00); 2.407 (1.09); 2.399 (1.08); 2.175 (0.93); 2.169 (0.96); 2.143 (1.11); 2.137 (1.06); 1.974 (3.35); 1.195 (0.93); 1.177 (1.90); 1.159 (0.93); -0.000 (1.94); |
| I-24 | 8.826 (8.23); 8.026 (1.25); 8.022 (1.68); 8.010 (0.86); 8.003 (1.43); 7.904 (4.89); 7.899 (4.22); 7.894 (2.02); 7.891 (1.19); 7.883 (5.70); 7.880 (5.34); 7.875 (1.63); 7.610 (0.52); 7.606 (0.73); 7.597 (1.89); 7.593 (1.82); 7.589 (1.56); 7.578 (3.61); 7.576 (3.49); 7.569 (1.78); 7.562 (0.90); 7.558 (2.91); 7.542 (0.62); 7.538 (0.46); 7.494 (0.95); 7.490 (0.77); 7.487 (1.20); 7.483 (0.70); 7.479 (0.73); 7.472 (3.52); 7.469 (1.73); 7.464 (1.14); 7.458 (4.52); 7.456 (5.21); 7.454 (4.86); 7.453 (4.45); 7.447 (2.21); 7.443 (0.99); 7.439 (2.14); 7.437 (2.88); 7.435 (1.40); 7.417 (0.37); 5.690 (10.09); 4.047 (0.87); 4.029 (0.88); 3.652 (1.83); 3.487 (0.70); 3.476 (0.52); 3.467 (0.35); 3.458 (0.57); 3.448 (1.06); 3.438 (0.59); 3.430 (0.37); 3.420 (0.57); 3.118 (63.22); 2.887 (0.61); 2.733 (0.39); 2.511 (0.42); 2.506 (0.64); 2.498 (9.29); 2.493 (19.23); 2.489 (27.08); 2.484 (18.89); 2.479 (9.02); 2.347 (16.00); 2.341 (1.58); 2.327 (0.56); 2.183 (0.88); 2.146 (0.97); 1.974 (4.03); 1.195 (1.15); 1.177 (2.30); 1.159 (1.11); -0.000 (2.84); |
| I-25 | 8.824 (10.79); 8.026 (1.80); 8.020 (2.02); 8.010 (1.06); 8.002 (1.94); 7.901 (3.88); 7.894 (0.98); 7.882 (4.36); 7.879 (3.03); 7.611 (0.55); 7.606 (0.84); 7.595 (2.90); 7.594 (2.83); 7.589 (2.03); 7.584 (2.36); 7.577 (8.26); 7.570 (2.24); 7.567 (1.28); 7.564 (1.89); 7.560 (2.42); 7.556 (2.88); 7.547 (0.79); 7.543 (0.54); 7.459 (3.17); 7.457 (3.21); 7.440 (2.38); 7.438 (2.25); 6.507 (3.60); 6.505 (3.60); 6.493 (0.42); 5.689 (12.66); 4.047 (0.48); 4.029 (0.51); 3.534 (6.52); 3.448 (0.38); 3.438 (0.83); 3.428 (0.53); 3.419 (1.12); 3.410 (1.63); 3.400 (1.17); 3.393 (1.63); 3.382 (0.84); 3.372 (0.45); 3.286 (0.47); 3.269 (0.33); 3.118 (55.30); 2.886 (0.42); 2.510 (0.32); 2.505 (0.48); 2.497 (7.69); 2.493 (15.95); 2.488 (22.55); 2.483 (15.84); 2.478 (7.65); 2.316 (14.53); 2.262 (16.00); 2.240 (1.97); 2.120 (1.25); 2.093 (1.39); 1.974 (1.73); 1.645 (0.73); 1.634 (0.87); 1.615 (1.59); 1.605 (1.71); 1.583 (1.60); 1.572 (1.43); 1.554 (0.73); 1.543 (0.62); 1.195 (0.51); 1.177 (0.98); 1.159 (0.49); -0.000 (1.62); |
| I-26 | 8.826 (6.65); 8.106 (0.60); 8.027 (1.15); 8.021 (1.13); 8.011 (0.69); 8.003 (1.28); 7.903 (2.45); 7.897 (0.63); 7.882 (2.46); 7.881 (2.47); 7.607 (0.52); 7.597 (1.69); 7.595 (1.66); 7.590 (1.36); 7.586 (1.56); 7.578 (5.15); 7.571 (1.50); 7.566 (1.32); 7.562 (1.36); 7.558 (1.65); 7.549 (0.46); 7.545 (0.33); 7.462 (1.98); 7.460 (1.98); 7.444 (1.54); 7.441 (1.49); 5.689 (11.82); 3.726 (1.06); 3.668 (0.36); 3.659 (0.36); 3.652 (0.84); 3.642 (0.83); 3.635 (1.13); 3.625 (1.09); 3.619 (0.87); 3.609 (0.81); 3.602 (0.35); 3.452 (0.44); 3.433 (0.51); 3.423 (0.94); 3.413 (0.51); 3.405 (0.33); 3.395 (0.51); 3.171 (6.93); 3.153 (2.88); 3.142 (2.14); 3.135 (1.95); 3.124 (1.76); 3.116 (0.74); 3.105 (0.65); 3.034 (0.33); 3.024 (0.33); 2.526 (12.18); 2.526 (12.18); 2.506 (0.36); 2.498 (5.19); 2.493 (10.72); 2.488 (15.10); 2.484 (10.56); 2.479 (5.04); 2.307 (10.78); 2.287 (0.48); 2.223 (1.06); 2.221 (1.03); 2.133 (0.89); 2.126 (0.91); 2.101 (1.04); 2.095 (0.98); 1.974 (0.95); 1.734 (0.44); 1.650 (0.41); 1.643 (0.42); 1.291 (10.06); 1.282 (4.29); 1.276 (16.00); 1.263 (7.89); 1.260 (10.66); 1.245 (2.99); 1.177 (0.54); -0.000 (1.76); |
| I-27 | 8.328 (3.99); 5.689 (5.11); 4.900 (0.56); 3.758 (0.66); 3.665 (0.89); 3.656 (1.31); 3.649 (2.82); 3.633 (11.06); 3.623 (7.35); 3.606 (1.64); 3.599 (0.76); 3.590 (0.54); 3.334 (0.58); 3.325 (0.36); 3.168 (0.65); 3.157 (0.66); 3.149 (1.95); 3.138 (1.91); 3.131 (1.99); 3.120 (1.91); 3.112 (0.66); 3.102 (0.62); 3.065 (1.47); 3.062 (0.62); 3.059 (0.92); 3.055 (2.27); 3.049 (3.93); 3.045 (1.72); 3.043 (1.73); 3.039 (3.91); 3.032 (2.26); 3.029 (0.84); 3.022 (1.46); 2.579 (7.00); 2.507 (0.44); 2.499 (6.28); 2.494 (12.91); 2.489 (18.14); 2.484 (12.62); 2.480 (6.00); 2.318 (6.29); 2.065 (0.50); 2.058 (0.51); 2.041 (0.42); 2.033 (0.59); 2.026 (0.56); 1.891 (0.38); 1.882 (0.42); 1.860 (0.45); 1.854 (0.46); 1.764 (2.17); 1.756 (2.26); 1.747 (5.50); 1.740 (2.36); 1.731 (2.34); 1.715 (0.64); 1.707 (0.39); 1.572 (0.42); 1.563 (0.45); 1.548 (0.76); 1.540 (0.90); 1.532 (0.62); 1.525 (0.65); 1.516 (0.96); 1.495 (0.40); 1.485 (0.37); 1.423 (0.39); 1.414 (0.63); 1.407 (0.39); 1.390 (0.54); 1.382 (0.54); 1.314 (0.39); 1.302 (12.41); 1.292 (4.58); 1.284 (15.46); 1.283 (16.00); 1.274 (7.88); 1.266 (12.80); 1.255 (3.60); -0.000 (1.24); |
| I-28 | 8.846 (2.60); 8.029 (0.49); 8.023 (0.42); 8.005 (0.56); 7.906 (1.06); 7.884 (1.18); 7.600 (0.71); 7.597 (0.72); 7.592 (0.67); 7.590 (0.79); 7.581 (2.15); 7.573 (0.73); 7.570 (0.61); 7.565 (0.59); 7.561 (0.71); 7.466 (0.84); 7.464 (0.86); 7.447 (0.65); 7.445 (0.64); 5.690 (6.95); 5.115 (0.41); 3.651 (0.66); 3.641 (0.65); 3.635 (0.91); 3.625 (0.86); 3.618 (0.70); 3.608 (0.65); 3.473 (0.42); 3.315 (0.40); 3.311 (0.47); 3.307 (0.42); 3.294 (0.43); 3.254 (3.37); 3.171 (0.44); 3.160 (0.45); 3.152 (1.21); 3.141 (1.21); 3.134 (1.23); 3.123 (1.21); 3.115 (0.45); 3.105 (0.41); 3.051 (3.08); 3.048 (1.37); 3.044 (2.00); 3.041 (4.90); 3.034 (8.46); 3.031 (3.92); 3.028 (3.95); 3.024 (8.48); 3.018 (5.08); 3.014 (2.02); 3.008 (3.23); 2.999 (0.35); 2.497 (3.17); 2.493 (6.52); 2.488 (9.19); 2.483 (6.50); 2.479 (3.16); 2.275 (7.72); 2.200 (0.33); 2.174 (7.65); 2.166 (1.03); 2.160 (0.53); 1.958 (0.35); 1.766 (0.47); 1.751 (4.53); 1.742 (4.47); 1.734 (11.49); 1.726 (4.49); 1.717 (4.37); 1.702 (0.49); 1.295 (8.19); 1.285 (3.35); 1.278 (16.00); 1.267 (5.86); 1.262 (8.57); 1.248 (2.43); -0.000 (1.52); |
| I-36 | 8.916 (2.04); 8.912 (2.13); 8.906 (2.25); 8.902 (2.16); 8.875 (1.69); 8.871 (1.91); 8.865 (1.99); 8.861 (1.92); 8.570 (2.38); 8.566 (3.28); 8.555 (3.15); 8.552 (2.39); 8.444 (2.05); 8.441 (2.77); 8.438 (1.79); 8.424 (2.24); 8.420 (2.91); 8.417 (1.72); 8.065 (5.68); 7.941 (2.38); 7.936 (2.91); 7.922 (2.16); 7.917 (2.79); 7.778 (1.54); 7.763 (1.38); 7.759 (3.14); 7.754 (1.27); 7.740 (1.87); 7.656 (1.73); 7.652 (2.71); 7.637 (4.13); 7.633 (2.69); 7.627 (2.84); 7.622 (2.98); 7.618 (7.13); 7.613 (6.21); 7.608 (1.38); 7.603 (3.03); 7.599 (1.40); 7.593 (3.89); 7.582 (3.50); 7.572 (1.73); 7.562 (1.63); 7.414 (1.28); 7.387 (1.15); 7.373 (4.14); 7.369 (2.70); 7.362 (2.47); 7.359 (4.16); 7.354 (3.76); 7.350 (2.21); 7.343 (2.21); 7.340 (3.64); 6.541 (3.06); 6.462 (2.55); 5.688 (6.36); 5.596 (11.79); 5.267 (6.01); 3.692 (2.00); 3.117 (361.56); 2.888 (1.84); 2.733 (1.60); 2.657 (1.33); 2.527 (2.12); 2.511 (3.69); 2.506 (5.05); 2.498 (72.66); 2.493 (149.60); 2.488 (210.69); 2.484 (146.89); 2.479 (70.03); 2.467 (16.00); 2.466 (15.65); 2.315 (1.30); 2.258 (1.68); 2.256 (1.72); 2.236 (10.12); 2.234 (10.07); 2.040 (5.37); -0.000 (17.78); |
| I-37 | 8.076 (9.48); 7.753 (3.46); 7.747 (3.62); 7.518 (1.96); 7.512 (1.79); 7.497 (4.00); 7.491 (3.96); 7.459 (5.85); 7.438 (2.67); 6.459 (3.62); 5.688 (12.82); 5.258 (9.74); 5.222 (0.36); 4.064 (0.45); 4.047 (1.36); 4.029 (1.40); 4.011 (0.46); 3.675 (2.84); 3.578 (1.57); 3.112 (162.99); 2.657 (0.34); 2.526 (0.64); 2.510 (0.82); 2.505 (1.22); 2.497 (19.87); 2.493 (41.04); 2.488 (57.99); 2.483 (40.44); 2.478 (19.28); 2.315 (0.33); 2.230 (16.00); 2.229 (15.89); 2.198 (0.93); 2.040 (1.07); 1.974 (6.34); 1.195 (1.86); 1.177 (3.74); 1.160 (1.79); 0.008 (0.33); -0.000 (10.58); -0.008 (0.33); |
| I-39 | 8.059 (8.89); 7.604 (1.88); 7.600 (1.96); 7.584 (2.19); 7.580 (2.26); 7.455 (0.78); 7.451 (0.81); 7.437 (1.19); 7.434 (1.78); 7.431 (1.76); 7.417 (2.53); 7.413 (2.36); 7.396 (2.49); 7.392 (3.48); 7.376 (1.47); 7.372 (1.16); 7.359 (2.03); 7.354 (1.61); 7.341 (1.57); 7.339 (1.86); 7.336 (1.42); 7.334 (1.53); 7.321 (1.17); 7.316 (1.01); 6.460 (3.56); 5.686 (4.33); 5.259 (9.73); 4.065 (0.85); 4.047 (2.58); 4.029 (2.60); 4.011 (0.89); 3.679 (2.83); 3.578 (1.51); 3.127 (357.80); 3.104 (3.10); 2.511 (0.58); 2.507 (0.84); 2.499 (13.08); 2.494 (27.02); 2.489 (38.10); 2.484 (26.59); 2.480 (12.69); 2.232 (15.92); 2.230 (16.00); 2.200 (0.68); 1.974 (11.80); 1.404 (1.33); 1.195 (3.42); 1.178 (6.93); 1.160 (3.39); -0.000 (1.11); |
| I-40 | 8.055 (9.05); 8.005 (2.47); 7.983 (2.71); 7.971 (1.46); 7.953 (1.49); 7.949 (1.42); 7.937 (1.34); 7.933 (1.40); 7.914 (1.57); 7.766 (2.56); 7.761 (2.63); 7.575 (0.58); 7.571 (0.78); 7.558 (1.87); 7.554 (1.55); 7.544 (2.13); 7.540 (2.59); 7.538 (2.70); 7.534 (1.76); 7.524 (1.61); 7.520 (1.85); 7.507 (0.75); 7.503 (0.54); 7.407 (2.23); 7.401 (2.12); 7.385 (2.10); 7.379 (2.05); 6.463 (3.58); 5.688 (10.03); 5.265 (9.68); 4.047 (0.92); 4.029 (0.91); 3.689 (2.89); 3.594 (1.56); 3.119 (403.30); 2.662 (0.38); 2.658 (0.48); 2.653 (0.34); 2.511 (1.18); 2.506 (1.73); 2.498 (26.27); 2.493 (54.16); 2.488 (76.31); 2.484 (53.30); 2.479 (25.44); 2.320 (0.33); 2.315 (0.48); 2.236 (15.84); 2.234 (16.00); 2.201 (0.52); 2.040 (1.31); 1.974 (4.10); 1.195 (1.24); 1.177 (2.40); 1.160 (1.21); -0.000 (0.92); |
| I-47 | 7.797 (2.97); 7.793 (7.88); 7.792 (7.88); 7.660 (2.88); 7.639 (3.85); 7.529 (2.11); 7.528 (2.15); 7.524 (2.03); 7.523 (2.01); 7.509 (1.57); 7.507 (1.60); 7.503 (1.51); 7.502 (1.47); 6.464 (3.59); 5.690 (5.99); 5.689 (5.80); 5.269 (8.95); 5.233 (0.37); 4.047 (0.64); 4.029 (0.64); 3.697 (3.18); 3.594 (1.65); 3.112 (130.20); 2.526 (0.66); 2.497 (16.88); 2.493 (31.87); 2.489 (42.91); 2.484 (31.22); 2.480 (15.81); 2.316 (0.37); 2.236 (16.00); 2.203 (0.76); 1.974 (2.71); 1.195 (0.74); 1.178 (1.41); 1.177 (1.47); 1.160 (0.73); 0.001 (1.51); -0.000 (1.56); |
| I-50 | 7.728 (8.04); 7.581 (3.01); 7.576 (3.16); 7.547 (3.05); 7.527 (3.86); 7.391 (2.45); 7.385 (2.32); 7.370 (1.91); 7.364 (1.80); 6.453 (3.71); 5.689 (8.61); 5.244 (9.88); 5.208 (0.32); 4.269 (11.32); 4.065 (0.53); 4.047 (1.58); 4.029 (1.62); 4.011 (0.55); 3.654 (3.17); 3.530 (1.72); 3.109 (93.11); 2.526 (0.66); 2.510 (0.90); 2.497 (14.93); 2.493 (29.66); 2.488 (41.08); 2.483 (28.89); 2.479 (14.03); 2.220 (16.00); 2.219 (15.92); 2.193 (0.77); 1.974 (7.26); 1.403 (0.52); 1.195 (2.01); 1.178 (4.05); 1.160 (1.99); -0.000 (6.20); |
| I-54 | 7.715 (8.71); 6.504 (3.89); 5.744 (5.54); 5.305 (7.80); 5.263 (0.37); 4.828 (0.31); 4.812 (1.30); 4.796 (2.01); 4.780 (1.33); 4.765 (0.34); 4.040 (0.87); 4.022 (0.88); 4.004 (0.31); 3.668 (2.12); 3.656 (2.05); 3.623 (2.26); 3.609 (1.77); 3.573 (1.83); 3.561 (2.10); 3.473 (1.85); 3.460 (2.25); 3.329 (596.33); 3.306 (6.50); 2.891 (0.45); 2.733 (0.39); 2.676 (0.33); 2.671 (0.41); 2.667 (0.31); 2.524 (2.00); 2.511 (22.75); 2.506 (40.65); 2.502 (51.31); 2.498 (34.94); 2.493 (16.59); 2.333 (0.31); 2.329 (0.38); 2.215 (15.00); 2.197 (0.96); 2.069 (0.34); 1.987 (3.83); 1.778 (0.81); 1.729 (1.47); 1.721 (1.34); 1.696 (2.41); 1.670 (0.92); 1.641 (0.89); 1.613 (0.75); 1.552 (0.36); 1.544 (0.38); 1.524 (0.70); 1.516 (0.69); 1.508 (0.68); 1.495 (0.48); 1.487 (0.41); 1.480 (0.37); 1.398 (0.43); 1.270 (0.31); 1.242 (0.97); 1.214 (10.18); 1.198 (9.45); 1.194 (2.79); 1.181 (1.46); 1.176 (3.05); 1.158 (2.24); 1.134 (0.82); 1.102 (0.54); 1.088 (0.38); 1.075 (0.55); 1.047 (1.07); 1.016 (1.28); 0.987 (0.80); 0.008 (0.65); -0.000 (11.51); -0.008 (0.39); |
| I-55 | 8.410 (7.80); 8.248 (2.73); 8.245 (2.48); 8.235 (2.82); 8.233 (2.42); 7.904 (2.95); 7.901 (2.66); 7.894 (2.85); 7.891 (2.54); 7.345 (2.71); 7.335 (3.23); 7.333 (3.20); 7.323 (2.79); 4.899 (0.87); 4.889 (1.32); 4.879 (0.87); 4.468 (0.92); 4.435 (0.95); 4.058 (1.17); 4.040 (3.47); 4.023 (3.49); 4.005 (1.20); 3.615 (0.85); 3.580 (1.09); 3.432 (0.87); 3.423 (1.40); 3.414 (0.91); 3.395 (0.99); 3.347 (1.11); 3.340 (1.40); 3.307 (73.61); 3.284 (1.60); 3.276 (1.13); 3.062 (1.11); 3.056 (1.18); 2.994 (3.40); 2.916 (3.44); 2.541 (0.95); 2.524 (0.88); 2.511 (9.70); 2.507 (17.17); 2.502 (21.55); 2.498 (14.94); 2.213 (0.96); 2.181 (1.15); 2.069 (0.93); 1.988 (15.00); 1.910 (1.23); 1.882 (1.41); 1.861 (1.56); 1.854 (1.55); 1.756 (1.32); 1.748 (1.80); 1.740 (1.55); 1.727 (2.10); 1.717 (2.19); 1.709 (1.70); 1.698 (1.48); 1.621 (0.99); 1.611 (1.00); 1.589 (0.87); 1.550 (1.26); 1.542 (1.25); 1.526 (1.96); 1.519 (2.16); 1.495 (2.24); 1.472 (0.90); 1.464 (0.87); 1.411 (1.18); 1.403 (1.73); 1.397 (1.44); 1.385 (0.94); 1.378 (1.47); 1.371 (1.49); 1.298 (0.84); 1.293 (0.88); 1.194 (4.18); 1.176 (8.23); 1.158 (4.21); -0.000 (2.21); |
| I-57 | 8.395 (15.00); 5.747 (1.47); 4.919 (0.81); 4.909 (1.47); 4.896 (1.81); 4.886 (2.78); 4.877 (1.83); 4.864 (1.41); 4.473 (1.57); 4.439 (1.65); 3.940 (1.58); 3.905 (1.74); 3.362 (2.85); 3.333 (7.27); 3.303 (830.63); 3.184 (1.61); 3.154 (2.35); 3.125 (1.31); 2.936 (0.87); 2.815 (0.85); 2.754 (1.18); 2.723 (2.12); 2.696 (1.43); 2.674 (1.24); 2.669 (1.63); 2.665 (1.22); 2.565 (1.67); 2.539 (8.69); 2.509 (91.69); 2.505 (160.54); 2.500 (200.93); 2.496 (141.68); 2.470 (4.95); 2.451 (2.22); 2.434 (1.30); 2.424 (0.95); 2.331 (1.22); 2.327 (1.52); 2.322 (1.21); 2.311 (1.08); 2.289 (1.33); 2.262 (2.17); 2.242 (2.51); 2.221 (2.44); 2.194 (1.45); 2.172 (1.29); 2.089 (1.74); 2.058 (3.35); 2.032 (1.89); 1.987 (1.21); 1.856 (3.20); 1.783 (1.67); 1.765 (4.30); 1.745 (6.53); 1.725 (6.39); 1.707 (4.57); 1.672 (1.70); 1.664 (1.63); 1.641 (1.53); 1.633 (1.42); 1.540 (3.06); 1.523 (5.28); 1.516 (5.95); 1.492 (5.49); 1.468 (2.06); 1.461 (2.23); 1.436 (1.36); 1.428 (1.80); 1.403 (3.63); 1.399 (3.44); 1.378 (2.99); 1.370 (3.07); 1.355 (1.28); 1.346 (1.58); 1.322 (1.41); 1.293 (2.49); 1.273 (1.20); 1.267 (1.38); 1.243 (1.05); -0.000 (20.79); |
| I-58 | 8.392 (13.08); 5.747 (0.63); 4.918 (0.58); 4.908 (1.13); 4.896 (1.35); 4.886 (2.11); 4.876 (1.39); 4.864 (1.06); 4.468 (1.19); 4.435 (1.20); 3.964 (1.15); 3.929 (1.22); 3.359 (1.16); 3.350 (1.84); 3.330 (3.25); 3.302 (296.70); 3.180 (0.95); 3.149 (1.52); 3.118 (0.84); 2.941 (0.75); 2.793 (0.63); 2.723 (0.86); 2.695 (1.67); 2.674 (0.80); 2.670 (1.04); 2.665 (1.17); 2.540 (3.28); 2.509 (32.20); 2.505 (56.80); 2.501 (71.51); 2.496 (49.20); 2.336 (2.82); 2.320 (4.97); 2.316 (4.60); 2.300 (3.24); 2.203 (1.00); 2.185 (1.95); 2.167 (1.11); 2.086 (1.19); 2.050 (2.42); 2.021 (1.30); 1.881 (2.14); 1.864 (2.28); 1.861 (2.28); 1.855 (2.30); 1.741 (1.90); 1.732 (1.99); 1.717 (2.20); 1.710 (2.24); 1.664 (0.61); 1.642 (1.11); 1.635 (1.13); 1.613 (1.05); 1.605 (1.02); 1.583 (0.60); 1.574 (0.59); 1.547 (1.96); 1.540 (2.35); 1.523 (4.26); 1.515 (4.98); 1.508 (5.20); 1.500 (5.65); 1.492 (7.55); 1.471 (4.80); 1.435 (1.45); 1.428 (1.68); 1.411 (1.71); 1.403 (2.96); 1.398 (3.72); 1.378 (2.31); 1.370 (2.38); 1.353 (1.07); 1.346 (1.33); 1.321 (1.70); 1.266 (15.00); 0.879 (4.30); 0.875 (4.38); 0.863 (13.35); 0.845 (4.83); -0.000 (9.42); |
| I-61 | 8.454 (2.70); 8.407 (5.50); 7.944 (2.11); 7.939 (2.08); 7.709 (1.51); 7.688 (1.78); 7.484 (1.23); 7.480 (1.22); 7.464 (1.02); 7.459 (0.98); 4.917 (0.51); 4.904 (0.65); 4.894 (0.98); 4.885 (0.65); 4.872 (0.49); 4.190 (1.39); 4.156 (1.44); 3.374 (1.01); 3.345 (2.43); 3.308 (324.98); 3.075 (0.98); 3.046 (1.74); 3.017 (0.94); 2.674 (0.35); 2.670 (0.45); 2.665 (0.35); 2.540 (1.89); 2.505 (46.20); 2.501 (58.54); 2.497 (41.93); 2.332 (0.34); 2.327 (0.42); 2.323 (0.33); 2.105 (1.19); 2.079 (1.38); 2.070 (1.62); 1.888 (0.99); 1.867 (1.07); 1.860 (1.07); 1.739 (1.30); 1.730 (1.44); 1.710 (1.96); 1.701 (1.80); 1.679 (1.13); 1.670 (1.05); 1.648 (0.45); 1.638 (0.38); 1.556 (0.75); 1.548 (0.86); 1.525 (1.61); 1.501 (1.68); 1.477 (0.63); 1.470 (0.61); 1.440 (0.49); 1.432 (0.66); 1.398 (15.00); 1.383 (1.32); 1.375 (1.21); 1.357 (0.50); 1.350 (0.59); 1.327 (0.50); 1.303 (0.60); 1.298 (0.61); 1.272 (0.47); -0.000 (0.44); |
| I-62 | 8.403 (7.83); 8.141 (3.34); 7.323 (2.86); 7.315 (4.00); 7.294 (3.56); 6.963 (1.57); 6.959 (1.58); 6.943 (1.43); 6.939 (1.41); 4.925 (0.33); 4.916 (0.66); 4.904 (0.82); 4.894 (1.27); 4.884 (0.84); 4.872 (0.65); 4.862 (0.33); 4.174 (1.79); 4.141 (1.87); 3.311 (564.80); 3.030 (1.31); 3.001 (2.28); 2.972 (1.28); 2.674 (0.46); 2.670 (0.59); 2.665 (0.46); 2.540 (2.56); 2.509 (32.65); 2.505 (57.69); 2.501 (72.56); 2.496 (50.29); 2.332 (0.47); 2.328 (0.58); 2.323 (0.46); 2.271 (15.00); 2.081 (1.52); 2.069 (1.08); 2.056 (1.77); 2.050 (1.75); 1.867 (1.35); 1.860 (1.35); 1.737 (1.25); 1.712 (1.80); 1.680 (1.45); 1.672 (1.54); 1.650 (1.41); 1.641 (1.31); 1.620 (0.57); 1.611 (0.48); 1.557 (0.95); 1.549 (1.08); 1.533 (1.86); 1.526 (2.07); 1.510 (1.64); 1.502 (2.15); 1.479 (0.79); 1.471 (0.75); 1.432 (0.77); 1.408 (1.77); 1.398 (5.26); 1.383 (1.39); 1.375 (1.37); 1.358 (0.57); 1.350 (0.70); 1.329 (0.58); 1.304 (0.72); 1.273 (0.55); -0.000 (0.60); |
| I-63 | 9.044 (3.29); 8.412 (7.37); 7.092 (2.22); 7.073 (2.85); 6.961 (1.98); 6.941 (1.58); 6.871 (3.33); 4.930 (0.44); 4.921 (0.80); 4.909 (0.98); 4.898 (1.44); 4.889 (0.97); 4.875 (0.80); 4.866 (0.44); 4.807 (1.88); 4.775 (1.93); 3.603 (0.32); 3.562 (0.31); 3.470 (1.07); 3.452 (1.33); 3.442 (1.79); 3.433 (1.47); 3.413 (1.60); 3.404 (1.49); 3.308 (1013.44); 3.260 (4.27); 3.230 (2.04); 3.184 (0.40); 3.157 (0.31); 2.669 (1.25); 2.665 (1.05); 2.539 (6.90); 2.504 (136.77); 2.500 (166.47); 2.496 (116.50); 2.327 (1.24); 2.323 (0.93); 2.290 (0.38); 2.256 (13.27); 2.118 (15.00); 2.092 (2.09); 2.069 (1.07); 2.049 (0.35); 1.890 (1.49); 1.870 (1.58); 1.754 (1.78); 1.725 (3.15); 1.703 (2.40); 1.672 (0.66); 1.552 (1.16); 1.536 (2.13); 1.531 (2.36); 1.512 (2.01); 1.507 (2.46); 1.484 (0.88); 1.475 (0.87); 1.465 (0.31); 1.435 (0.84); 1.411 (1.91); 1.398 (6.71); 1.386 (1.65); 1.378 (1.55); 1.354 (0.81); 1.332 (0.67); 1.305 (0.89); 1.292 (0.89); 1.277 (0.69); 1.244 (0.35); -0.000 (2.37); |
| I-64 | 8.404 (7.30); 8.228 (3.64); 7.255 (1.66); 7.251 (1.69); 7.236 (1.71); 7.232 (1.65); 7.062 (1.54); 7.041 (2.19); 7.035 (1.75); 7.014 (1.96); 6.910 (1.07); 6.905 (1.13); 6.898 (1.21); 6.892 (1.32); 6.884 (0.97); 6.878 (0.88); 5.747 (3.73); 4.926 (0.42); 4.916 (0.80); 4.905 (0.98); 4.894 (1.48); 4.885 (1.03); 4.872 (0.80); 4.177 (2.18); 4.144 (2.28); 4.039 (0.94); 4.022 (0.94); 3.340 (1.86); 3.301 (234.82); 3.004 (1.54); 2.975 (2.77); 2.946 (1.53); 2.669 (0.57); 2.665 (0.45); 2.539 (2.71); 2.504 (58.42); 2.500 (70.13); 2.496 (49.25); 2.327 (0.55); 2.249 (15.00); 2.076 (1.92); 2.049 (2.29); 1.987 (3.89); 1.908 (2.92); 1.869 (1.73); 1.745 (1.50); 1.737 (1.57); 1.722 (1.72); 1.715 (1.73); 1.691 (1.15); 1.682 (0.95); 1.660 (1.80); 1.651 (1.89); 1.629 (1.76); 1.621 (1.61); 1.599 (0.75); 1.590 (0.62); 1.557 (1.21); 1.549 (1.36); 1.526 (2.59); 1.502 (2.65); 1.479 (1.00); 1.472 (0.94); 1.432 (0.92); 1.408 (1.87); 1.401 (1.47); 1.383 (1.67); 1.375 (1.66); 1.357 (0.71); 1.350 (0.88); 1.328 (0.81); 1.303 (0.98); 1.280 (0.69); 1.273 (0.78); 1.247 (0.43); 1.237 (0.67); 1.193 (1.12); 1.175 (2.04); 1.158 (1.05); -0.000 (3.75); |
| I-65 | 8.384 (3.19); 6.838 (3.53); 4.882 (0.63); 4.873 (0.46); 4.475 (0.47); 4.442 (0.45); 4.002 (0.52); 3.994 (0.45); 3.973 (0.54); 3.958 (0.42); 3.651 (2.73); 3.611 (0.40); 3.574 (0.44); 3.552 (0.41); 3.526 (0.47); 3.500 (0.52); 3.484 (0.61); 3.475 (0.62); 3.460 (0.79); 3.452 (1.21); 3.395 (1.50); 3.304 (1232.16); 3.217 (0.78); 3.205 (0.60); 3.196 (0.57); 3.180 (0.51); 3.164 (0.54); 3.155 (0.63); 3.124 (0.68); 3.098 (0.50); 2.890 (0.49); 2.763 (0.50); 2.732 (1.07); 2.694 (0.70); 2.673 (1.69); 2.669 (2.12); 2.665 (1.71); 2.633 (0.51); 2.630 (0.51); 2.612 (0.66); 2.608 (0.79); 2.539 (13.94); 2.504 (229.40); 2.500 (283.39); 2.496 (198.65); 2.402 (0.57); 2.375 (0.43); 2.331 (1.59); 2.327 (2.10); 2.322 (1.56); 2.236 (15.00); 2.069 (0.41); 2.049 (1.19); 2.019 (0.50); 2.012 (0.49); 1.987 (0.69); 1.976 (0.43); 1.956 (0.53); 1.939 (0.49); 1.908 (0.51); 1.894 (0.66); 1.874 (0.73); 1.860 (0.77); 1.739 (0.68); 1.724 (0.69); 1.720 (0.76); 1.695 (0.54); 1.511 (1.18); 1.487 (1.22); 1.466 (0.84); 1.437 (0.59); 1.418 (0.65); 1.402 (0.99); 1.386 (0.65); 1.377 (0.75); 1.368 (0.75); 1.350 (0.40); 1.292 (0.99); 1.237 (0.75); -0.000 (21.13); |
| I-66 | 8.396 (6.51); 7.028 (0.44); 6.967 (4.51); 6.948 (3.70); 6.927 (2.44); 6.907 (1.27); 4.920 (0.43); 4.911 (0.72); 4.900 (0.79); 4.888 (1.25); 4.878 (0.82); 4.866 (0.60); 4.488 (0.69); 4.459 (0.74); 4.022 (0.43); 3.990 (0.70); 3.958 (0.74); 3.678 (0.45); 3.654 (6.07); 3.588 (0.90); 3.506 (1.31); 3.304 (754.62); 3.201 (0.74); 3.173 (0.98); 3.139 (0.58); 2.995 (1.21); 2.842 (1.19); 2.793 (0.51); 2.766 (0.89); 2.731 (0.71); 2.673 (1.02); 2.669 (1.31); 2.664 (1.07); 2.573 (0.74); 2.539 (8.82); 2.504 (143.74); 2.500 (178.45); 2.496 (124.83); 2.430 (0.60); 2.331 (1.05); 2.326 (1.35); 2.322 (1.03); 2.272 (0.53); 2.234 (15.00); 2.199 (0.59); 2.186 (1.94); 2.161 (14.02); 2.137 (1.42); 2.069 (0.97); 2.049 (1.93); 2.025 (0.99); 2.016 (0.97); 1.987 (1.77); 1.907 (2.25); 1.862 (1.38); 1.857 (1.37); 1.718 (1.33); 1.711 (1.33); 1.595 (0.69); 1.589 (0.70); 1.541 (1.77); 1.527 (2.45); 1.518 (2.42); 1.496 (2.36); 1.473 (0.97); 1.429 (0.82); 1.413 (0.98); 1.405 (1.51); 1.399 (1.53); 1.381 (1.34); 1.373 (1.35); 1.349 (0.80); 1.325 (0.65); 1.292 (0.93); 1.269 (0.63); 1.237 (0.65); 1.193 (0.51); 1.175 (0.85); 1.157 (0.48);-0.000 (17.27); |
| I-68 | 8.276 (1.46); 7.348 (0.30); 7.335 (0.34); 7.260 (0.38); 7.250 (0.28); 7.198 (0.74); 7.186 (0.67); 6.995 (0.30); 6.906 (0.62); 6.882 (0.32); 6.839 (0.59); 6.816 (0.32); 6.791 (0.68); 6.700 (0.34); 6.202 (0.42); 5.264 (0.50); 5.235 (0.50); 4.064 (0.76); 4.052 (0.77); 2.151 (13.50); 2.109 (0.27); 2.100 (0.29); 1.972 (3.43); 1.949 (3.20); 1.945 (6.14); 1.941 (8.69); 1.937 (6.02); 1.933 (3.05); 1.215 (0.91); 1.204 (1.91); 1.192 (0.90); -0.000 (4.63); |
| I-69 | 8.307 (2.02); 7.004 (0.38); 6.914 (0.77); 6.886 (0.41); 6.842 (0.67); 6.825 (0.39); 6.795 (0.88); 6.704 (0.43); 5.277 (0.64); 5.242 (0.64); 2.550 (0.39); 2.543 (0.38); 2.501 (1.12); 2.148 (8.67); 1.973 (1.17); 1.957 (0.31); 1.953 (0.41); 1.949 (5.03); 1.945 (9.53); 1.941 (13.50); 1.937 (9.27); 1.933 (4.79); 1.928 (0.32); 1.793 (0.32); 1.785 (0.31); 1.782 (0.32); 1.776 (0.35); 1.770 (0.30); 1.571 (0.31); 1.566 (0.38); 1.549 (0.31); 1.451 (0.34); 1.434 (0.28); 1.429 (0.31); 1.216 (0.32); 1.204 (0.72); 1.192 (0.33); -0.000 (7.24); |
| I-70 | 8.454 (5.80); 7.944 (1.93); 7.939 (1.89); 7.709 (1.42); 7.688 (1.72); 7.485 (1.15); 7.480 (1.14); 7.464 (0.95); 7.459 (0.91); 4.184 (1.33); 4.151 (1.39); 4.039 (0.46); 4.022 (0.47); 3.826 (15.00); 3.717 (0.55); 3.688 (0.56); 3.372 (0.87); 3.362 (0.89); 3.352 (1.31); 3.343 (2.00); 3.305 (239.93); 3.282 (4.16); 3.079 (0.93); 3.051 (1.66); 3.022 (0.92); 2.674 (0.31); 2.669 (0.40); 2.665 (0.31); 2.539 (0.85); 2.505 (41.57); 2.501 (51.47); 2.497 (36.19); 2.332 (0.30); 2.327 (0.38); 2.114 (1.10); 2.109 (1.12); 2.082 (1.31); 1.987 (1.87); 1.745 (0.45); 1.735 (0.49); 1.715 (1.03); 1.706 (1.11); 1.684 (1.02); 1.676 (0.95); 1.654 (0.42); 1.644 (0.35); 1.398 (1.22); 1.193 (0.54); 1.175 (1.00); 1.157 (0.51); 0.008 (0.39); -0.000 (5.66); |
| I-71 | 8.784 (0.36); 8.401 (5.13); 7.947 (0.30); 7.621 (2.33); 7.483 (2.00); 7.478 (2.00); 6.878 (1.85); 6.858 (3.13); 6.811 (1.37); 6.807 (1.33); 6.791 (0.77); 6.786 (0.75); 5.747 (5.23); 4.914 (0.49); 4.902 (0.60); 4.892 (0.91); 4.882 (0.61); 4.869 (0.46); 4.134 (1.29); 4.101 (1.35); 3.818 (2.29); 3.772 (15.00); 3.334 (1.21); 3.301 (156.89); 3.011 (0.93); 2.982 (1.64); 2.953 (0.91); 2.669 (0.31); 2.539 (1.29); 2.508 (19.68); 2.504 (33.50); 2.500 (41.15); 2.496 (28.48); 2.326 (0.32); 2.221 (2.28); 2.211 (10.77); 2.077 (1.13); 2.069 (0.91); 2.051 (1.34); 1.987 (0.41); 1.908 (1.17); 1.870 (1.02); 1.859 (1.00); 1.721 (1.02); 1.712 (1.06); 1.703 (1.10); 1.671 (1.07); 1.663 (1.11); 1.640 (1.04); 1.632 (0.97); 1.610 (0.42); 1.601 (0.37); 1.554 (0.72); 1.546 (0.82); 1.522 (1.49); 1.499 (1.55); 1.476 (0.57); 1.468 (0.55); 1.431 (0.55); 1.406 (1.12); 1.399 (0.85); 1.381 (0.99); 1.373 (0.99); 1.357 (0.42); 1.348 (0.53); 1.325 (0.45); 1.295 (0.54); 1.270 (0.42); -0.000 (3.35); |
| I-72 | 8.405 (6.84); 8.124 (3.14); 7.337 (3.01); 7.332 (3.02); 7.200 (2.12); 7.180 (2.78); 7.083 (2.06); 7.078 (1.97); 7.063 (1.49); 7.057 (1.37); 5.746 (2.89); 4.926 (0.35); 4.917 (0.66); 4.905 (0.82); 4.895 (1.25); 4.885 (0.82); 4.872 (0.63); 4.863 (0.32); 4.177 (1.79); 4.144 (1.87); 3.668 (0.55); 3.355 (1.12); 3.308 (165.80); 3.028 (1.26); 2.999 (2.25); 2.970 (1.24); 2.540 (0.96); 2.505 (24.88); 2.501 (30.53); 2.497 (21.33); 2.328 (0.30); 2.253 (0.81); 2.180 (0.77); 2.158 (15.00); 2.089 (1.56); 2.069 (1.58); 2.063 (1.79); 2.004 (0.53); 1.888 (1.28); 1.868 (1.39); 1.861 (1.36); 1.745 (1.18); 1.737 (1.26); 1.722 (1.44); 1.711 (1.68); 1.700 (1.44); 1.677 (1.46); 1.669 (1.56); 1.646 (1.46); 1.638 (1.34); 1.617 (0.59); 1.607 (0.49); 1.557 (0.95); 1.549 (1.11); 1.533 (1.90); 1.526 (2.11); 1.510 (1.69); 1.502 (2.21); 1.479 (0.83); 1.471 (0.78); 1.440 (0.59); 1.433 (0.76); 1.408 (1.60); 1.398 (1.44); 1.383 (1.43); 1.375 (1.44); 1.357 (0.76); 1.351 (0.83); 1.328 (0.69); 1.321 (0.55); 1.303 (0.90); 1.299 (0.90); 1.280 (0.70); 1.273 (0.73); 1.248 (0.37); 1.238 (0.39); 0.890 (0.34); 0.872 (0.58); 0.854 (0.32); -0.000 (1.78); |
| I-74 | 8.405 (8.57); 8.078 (3.67); 7.194 (1.43); 7.174 (2.11); 7.164 (2.09); 7.157 (3.23); 7.136 (1.82); 7.130 (1.75); 6.865 (1.10); 6.859 (1.04); 6.844 (2.00); 6.837 (1.76); 6.824 (0.97); 6.817 (0.88); 5.746 (4.60); 4.916 (0.78); 4.904 (0.99); 4.894 (1.52); 4.884 (1.00); 4.871 (0.74); 4.179 (2.16); 4.147 (2.23); 4.039 (0.66); 4.021 (0.65); 3.305 (930.93); 3.028 (1.60); 2.997 (2.80); 2.969 (1.56); 2.674 (0.94); 2.669 (1.18); 2.664 (0.90); 2.539 (3.41); 2.509 (72.46); 2.504 (122.62); 2.500 (149.80); 2.496 (103.35); 2.331 (0.92); 2.327 (1.14); 2.322 (0.86); 2.245 (0.79); 2.151 (15.00); 2.089 (1.90); 2.063 (2.18); 1.987 (2.71); 1.888 (1.56); 1.866 (1.68); 1.860 (1.67); 1.744 (1.41); 1.735 (1.50); 1.711 (2.02); 1.678 (1.78); 1.669 (1.86); 1.646 (1.73); 1.638 (1.61); 1.616 (0.70); 1.556 (1.20); 1.548 (1.36); 1.532 (2.28); 1.525 (2.52); 1.501 (2.61); 1.477 (0.97); 1.470 (0.92); 1.433 (0.90); 1.415 (1.17); 1.408 (1.86); 1.401 (1.44); 1.383 (1.63); 1.375 (1.65); 1.358 (0.76); 1.350 (0.88); 1.344 (0.72); 1.328 (0.81); 1.303 (0.93); 1.298 (0.94); 1.273 (0.76); 1.237 (0.83); 1.193 (0.81); 1.175 (1.52); 1.157 (0.75); -0.000 (2.35); |
| I-76 | 8.402 (5.02); 7.926 (2.23); 7.812 (1.27); 5.746 (1.03); 4.916 (0.49); 4.903 (0.59); 4.893 (0.91); 4.883 (0.60); 4.871 (0.46); 4.141 (1.29); 4.108 (1.37); 4.039 (0.36); 4.022 (0.35); 3.305 (207.08); 3.005 (0.93); 2.975 (1.64); 2.947 (0.89); 2.669 (0.32); 2.539 (0.80); 2.509 (18.33); 2.505 (31.25); 2.500 (38.41); 2.496 (26.55); 2.327 (0.33); 2.261 (9.69); 2.226 (12.69); 2.102 (11.59); 2.078 (1.43); 2.057 (15.00); 1.987 (1.43); 1.893 (0.95); 1.887 (0.93); 1.866 (0.99); 1.859 (0.97); 1.744 (0.86); 1.736 (0.90); 1.720 (1.00); 1.713 (1.00); 1.680 (0.48); 1.670 (0.53); 1.649 (1.03); 1.640 (1.09); 1.618 (1.02); 1.610 (0.94); 1.588 (0.42); 1.579 (0.38); 1.555 (0.72); 1.547 (0.82); 1.531 (1.34); 1.524 (1.48); 1.500 (1.52); 1.477 (0.57); 1.469 (0.53); 1.440 (0.40); 1.433 (0.54); 1.408 (1.13); 1.400 (0.84); 1.383 (0.96); 1.376 (0.97); 1.359 (0.40); 1.351 (0.51); 1.329 (0.43); 1.320 (0.32); 1.305 (0.52); 1.298 (0.51); 1.273 (0.40); 1.193 (0.39); 1.175 (0.75); 1.157 (0.37); -0.000 (1.02); |
| I-77 | 8.416 (4.67); 7.681 (2.21); 7.401 (2.11); 7.396 (2.14); 7.294 (0.93); 7.281 (1.32); 7.273 (0.46); 7.271 (0.45); 7.260 (1.22); 7.248 (0.86); 7.246 (0.75); 7.196 (0.86); 7.186 (2.09); 7.173 (1.37); 6.903 (1.94); 6.889 (2.09); 6.552 (1.19); 6.547 (1.18); 6.537 (1.11); 6.532 (1.09); 6.130 (0.67); 6.123 (1.36); 6.115 (0.66); 5.762 (5.27); 4.117 (1.01); 4.095 (1.02); 3.768 (0.52); 3.756 (12.28); 3.672 (13.50); 3.344 (79.72); 3.312 (0.37); 3.298 (0.40); 3.292 (0.69); 3.286 (0.39); 3.273 (0.35); 2.992 (0.67); 2.973 (1.26); 2.953 (0.65); 2.877 (0.44); 2.857 (0.37); 2.849 (0.71); 2.840 (0.34); 2.777 (0.33); 2.768 (0.43); 2.762 (0.40); 2.752 (0.42); 2.542 (0.59); 2.524 (0.61); 2.521 (0.79); 2.518 (0.93); 2.506 (27.38); 2.503 (36.28); 2.500 (26.62); 2.067 (0.94); 2.050 (1.15); 2.044 (1.41); 2.035 (0.88); 2.028 (1.13); 2.022 (0.95); 2.015 (0.45); 1.990 (0.83); 1.949 (0.33); 1.940 (0.37); 1.933 (0.41); 1.925 (0.37); 1.918 (0.29); 1.839 (0.35); 1.830 (0.44); 1.823 (0.39); 1.817 (0.30); 1.674 (0.30); 1.667 (0.35); 1.653 (0.77); 1.647 (0.84); 1.633 (0.80); 1.627 (0.74); 1.612 (0.31); 1.396 (0.30); 1.174 (0.43); -0.000 (5.26); |
| I-78 | 8.400 (7.71); 7.982 (3.59); 7.299 (1.69); 7.291 (0.71); 7.281 (2.93); 7.260 (2.19); 7.242 (1.99); 7.238 (1.61); 7.200 (2.11); 7.187 (3.45); 7.168 (1.93); 7.044 (2.62); 7.025 (3.12); 6.999 (3.47); 6.856 (2.07); 6.837 (1.72); 6.137 (1.28); 6.126 (2.57); 6.115 (1.27); 5.746 (6.04); 4.167 (2.11); 4.134 (2.12); 3.305 (258.83); 2.985 (1.46); 2.955 (2.59); 2.926 (1.43); 2.903 (0.49); 2.890 (0.83); 2.876 (0.51); 2.860 (0.86); 2.847 (1.56); 2.834 (0.79); 2.793 (0.76); 2.778 (1.04); 2.771 (1.02); 2.756 (0.95); 2.735 (0.49); 2.728 (0.53); 2.714 (0.46); 2.673 (0.38); 2.669 (0.44); 2.664 (0.35); 2.539 (1.04); 2.504 (41.27); 2.500 (50.77); 2.496 (35.41); 2.327 (0.41); 2.231 (14.92); 2.105 (15.00); 2.068 (2.47); 2.065 (2.56); 2.055 (2.47); 2.031 (4.31); 2.004 (0.64); 1.990 (0.61); 1.975 (0.60); 1.965 (0.75); 1.953 (0.84); 1.942 (0.94); 1.931 (0.83); 1.919 (0.62); 1.908 (0.51); 1.895 (0.34); 1.865 (0.41); 1.852 (0.86); 1.839 (1.10); 1.827 (0.91); 1.811 (0.61); 1.795 (0.41); 1.712 (0.41); 1.706 (0.34); 1.681 (0.80); 1.671 (0.91); 1.649 (1.68); 1.641 (1.74); 1.619 (1.60); 1.611 (1.46); 1.589 (0.61); 1.579 (0.50); -0.000 (1.35); |
| I-79 | 8.403 (7.10); 8.069 (3.66); 7.298 (1.74); 7.279 (3.30); 7.260 (2.26); 7.241 (2.08); 7.199 (2.37); 7.189 (4.42); 7.169 (4.10); 7.158 (2.62); 7.152 (3.39); 7.130 (1.89); 7.124 (1.79); 6.863 (1.08); 6.856 (1.04); 6.842 (1.98); 6.835 (1.78); 6.820 (1.00); 6.814 (0.85); 6.136 (1.35); 6.125 (2.68); 6.114 (1.35); 5.746 (4.52); 4.170 (2.23); 4.137 (2.26); 3.340 (2.29); 3.303 (278.67); 3.014 (1.55); 2.984 (2.78); 2.954 (1.53); 2.902 (0.47); 2.889 (0.88); 2.876 (0.53); 2.859 (0.93); 2.846 (1.64); 2.833 (0.85); 2.792 (0.83); 2.777 (1.11); 2.770 (1.07); 2.755 (1.01); 2.734 (0.53); 2.727 (0.56); 2.713 (0.46); 2.669 (0.56); 2.665 (0.46); 2.539 (1.69); 2.504 (57.20); 2.500 (68.16); 2.496 (47.83); 2.327 (0.51); 2.143 (15.00); 2.077 (2.15); 2.064 (1.78); 2.052 (3.39); 2.041 (3.79); 2.030 (2.87); 2.001 (0.60); 1.987 (0.69); 1.973 (0.63); 1.963 (0.77); 1.951 (0.87); 1.940 (0.92); 1.928 (0.88); 1.917 (0.67); 1.907 (0.53); 1.851 (0.87); 1.839 (1.13); 1.826 (0.94); 1.811 (0.63); 1.805 (0.59); 1.712 (0.44); 1.697 (0.84); 1.688 (0.90); 1.666 (1.72); 1.658 (1.81); 1.636 (1.69); 1.628 (1.52); 1.606 (0.64); 1.596 (0.52); -0.000 (1.34); |
| I-80 | 8.401 (8.55); 8.219 (3.56); 7.299 (1.85); 7.280 (3.10); 7.259 (2.65); 7.254 (2.24); 7.251 (2.24); 7.241 (2.92); 7.237 (2.29); 7.231 (1.98); 7.227 (1.80); 7.200 (2.24); 7.186 (3.87); 7.167 (2.11); 7.133 (1.20); 7.123 (1.44); 7.115 (1.21); 7.110 (1.07); 7.058 (1.72); 7.048 (1.11); 7.037 (2.68); 7.031 (2.20); 7.010 (2.06); 6.906 (1.10); 6.901 (1.17); 6.896 (1.24); 6.889 (1.34); 6.881 (0.95); 6.875 (0.87); 6.137 (1.37); 6.126 (2.66); 6.114 (1.31); 5.746 (2.24); 5.047 (0.99); 5.032 (1.04); 4.167 (2.20); 4.133 (2.23); 3.302 (231.17); 3.270 (1.67); 2.990 (1.53); 2.961 (2.71); 2.932 (1.50); 2.889 (0.85); 2.860 (0.90); 2.847 (1.62); 2.834 (0.83); 2.792 (0.82); 2.777 (1.10); 2.770 (1.09); 2.754 (1.06); 2.539 (1.40); 2.504 (47.68); 2.500 (58.54); 2.496 (40.93); 2.246 (15.00); 2.065 (2.61); 2.054 (2.30); 2.040 (4.15); 2.031 (4.32); 1.987 (0.99); 1.952 (0.87); 1.941 (0.93); 1.930 (0.90); 1.908 (1.04); 1.899 (0.87); 1.894 (0.90); 1.880 (0.97); 1.874 (1.00); 1.870 (1.00); 1.857 (0.79); 1.852 (1.03); 1.839 (1.22); 1.826 (0.96); 1.678 (1.36); 1.671 (1.58); 1.649 (2.15); 1.641 (2.00); 1.619 (1.71); 1.610 (1.60); -0.000 (2.27); |
| I-81 | 9.034 (3.32); 8.407 (6.28); 7.390 (0.94); 7.381 (1.00); 7.367 (1.11); 7.301 (1.58); 7.283 (2.79); 7.261 (2.08); 7.243 (1.90); 7.201 (2.03); 7.188 (3.52); 7.169 (2.06); 7.132 (1.95); 7.122 (2.22); 7.114 (2.09); 7.088 (2.40); 7.068 (3.03); 7.048 (1.58); 7.033 (1.10); 6.956 (2.17); 6.937 (1.69); 6.864 (3.39); 6.139 (1.21); 6.128 (2.46); 6.117 (1.25); 5.746 (0.90); 5.046 (2.02); 5.031 (2.14); 4.798 (1.93); 4.766 (1.93); 4.548 (0.96); 3.440 (0.97); 3.431 (1.40); 3.420 (1.02); 3.402 (1.04); 3.392 (0.91); 3.301 (556.78); 3.246 (3.94); 3.216 (2.08); 2.862 (0.91); 2.849 (1.58); 2.779 (1.21); 2.772 (1.25); 2.757 (1.19); 2.717 (1.01); 2.705 (1.12); 2.673 (1.56); 2.668 (1.63); 2.663 (1.44); 2.538 (2.89); 2.503 (125.14); 2.499 (149.04); 2.326 (1.18); 2.322 (0.96); 2.252 (13.90); 2.110 (15.00); 2.080 (2.41); 2.069 (2.54); 2.057 (1.69); 2.043 (2.19); 2.033 (2.56); 1.955 (0.95); 1.944 (1.01); 1.933 (0.98); 1.923 (0.90); 1.906 (1.14); 1.898 (1.39); 1.880 (1.63); 1.870 (1.70); 1.857 (1.31); 1.842 (1.25); 1.830 (1.07); 1.744 (0.97); 1.715 (2.45); 1.693 (2.49); 1.685 (2.42); 1.670 (1.60); 1.662 (1.41); 1.653 (1.61); -0.000 (5.09); |
| I-82 | 8.399 (5.09); 7.613 (2.35); 7.474 (2.09); 7.469 (2.04); 7.296 (1.11); 7.278 (2.13); 7.259 (1.42); 7.240 (1.29); 7.237 (1.03); 7.198 (1.39); 7.185 (2.11); 7.168 (1.18); 6.871 (1.59); 6.851 (2.91); 6.808 (1.47); 6.805 (1.40); 6.788 (0.78); 6.784 (0.75); 6.135 (0.85); 6.124 (1.65); 6.112 (0.82); 5.746 (0.52); 4.124 (1.36); 4.090 (1.37); 3.773 (1.10); 3.761 (15.00); 3.305 (180.80); 3.264 (1.07); 2.996 (0.95); 2.967 (1.68); 2.938 (0.92); 2.888 (0.53); 2.874 (0.33); 2.858 (0.56); 2.845 (1.00); 2.832 (0.52); 2.790 (0.51); 2.776 (0.66); 2.769 (0.65); 2.753 (0.60); 2.726 (0.36); 2.712 (0.30); 2.674 (0.34); 2.669 (0.41); 2.664 (0.32); 2.539 (0.98); 2.504 (38.99); 2.500 (48.02); 2.496 (33.62); 2.327 (0.36); 2.208 (11.00); 2.069 (1.68); 2.063 (1.56); 2.050 (1.49); 2.039 (2.63); 2.029 (2.54); 2.001 (0.39); 1.987 (0.48); 1.971 (0.37); 1.962 (0.46); 1.948 (0.53); 1.938 (0.57); 1.926 (0.54); 1.915 (0.41); 1.908 (0.35); 1.849 (0.56); 1.836 (0.71); 1.824 (0.58); 1.691 (0.51); 1.681 (0.58); 1.659 (1.08); 1.651 (1.15); 1.629 (1.05); 1.621 (0.97); 1.599 (0.40); 1.589 (0.32); - 0.000 (2.57); |
| I-83 | 8.391 (15.00); 5.747 (0.93); 4.909 (1.40); 4.896 (1.74); 4.886 (2.67); 4.876 (1.79); 4.864 (1.37); 4.473 (1.49); 4.442 (1.53); 3.984 (1.45); 3.949 (1.54); 3.358 (1.57); 3.348 (2.51); 3.303 (368.64); 3.279 (7.02); 3.182 (1.24); 3.151 (1.99); 3.121 (1.10); 2.722 (1.11); 2.693 (1.95); 2.669 (1.31); 2.665 (1.48); 2.540 (1.54); 2.509 (35.82); 2.505 (61.89); 2.501 (76.77); 2.496 (53.39); 2.359 (7.31); 2.340 (10.12); 2.328 (1.17); 2.174 (0.97); 2.154 (2.09); 2.135 (2.71); 2.116 (2.15); 2.096 (2.06); 2.070 (2.66); 2.055 (3.11); 2.025 (1.73); 1.882 (2.74); 1.860 (2.94); 1.854 (2.93); 1.754 (4.31); 1.739 (5.34); 1.710 (4.06); 1.625 (1.87); 1.617 (1.56); 1.598 (3.30); 1.591 (3.64); 1.577 (4.31); 1.571 (3.37); 1.566 (3.46); 1.561 (3.98); 1.550 (3.97); 1.539 (4.28); 1.525 (6.51); 1.516 (6.12); 1.501 (7.33); 1.492 (8.27); 1.485 (6.43); 1.471 (5.42); 1.464 (4.57); 1.435 (1.97); 1.428 (2.11); 1.419 (1.34); 1.411 (2.12); 1.403 (3.44); 1.396 (2.58); 1.386 (1.83); 1.378 (2.91); 1.371 (2.98); 1.354 (1.30); 1.346 (1.56); 1.338 (1.16); 1.323 (1.34); 1.315 (1.01); 1.298 (1.64); 1.293 (1.60); 1.268 (1.28); 1.122 (2.65);-0.000 (2.88); |
| I-84 | 8.521 (9.42); 8.442 (4.29); 7.704 (2.98); 7.685 (3.34); 7.682 (3.17); 7.601 (2.37); 7.597 (2.39); 7.581 (3.07); 7.468 (1.82); 7.446 (3.88); 7.436 (1.86); 7.428 (2.95); 7.420 (2.16); 7.410 (1.47); 7.403 (1.41); 7.395 (1.19); 7.356 (1.86); 7.352 (1.88); 7.337 (2.70); 7.333 (2.59); 7.318 (1.27); 7.314 (1.18); 7.252 (1.09); 7.239 (1.29); 7.229 (1.73); 7.216 (1.72); 7.203 (1.43); 7.190 (1.27); 6.923 (0.83); 6.914 (1.33); 6.904 (1.28); 6.893 (1.85); 6.886 (1.16); 6.872 (1.05); 6.865 (0.61); 5.373 (15.00); 4.177 (2.81); 4.142 (3.01); 4.039 (0.40); 4.022 (0.39); 3.506 (0.42); 3.502 (0.41); 3.456 (0.55); 3.427 (0.79); 3.415 (0.93); 3.305 (965.91); 3.177 (0.54); 3.141 (0.38); 3.136 (0.37); 3.098 (0.37); 3.025 (2.05); 2.995 (3.60); 2.967 (1.98); 2.674 (1.25); 2.669 (1.49); 2.664 (1.21); 2.539 (3.92); 2.504 (157.47); 2.500 (192.11); 2.496 (136.39); 2.417 (0.58); 2.327 (1.37); 2.322 (1.10); 2.093 (2.46); 2.069 (4.05); 1.987 (1.63); 1.711 (0.89); 1.702 (1.09); 1.680 (2.20); 1.671 (2.33); 1.649 (2.20); 1.641 (2.03); 1.619 (0.90); 1.610 (0.67); 1.398 (7.53); 1.236 (0.51); 1.193 (0.43); 1.175 (0.75); 1.157 (0.42); -0.000 (4.76); |
| I-85 | 8.539 (8.81); 8.160 (2.87); 7.708 (1.82); 7.706 (1.88); 7.695 (2.01); 7.693 (2.00); 7.602 (1.29); 7.599 (1.36); 7.589 (1.58); 7.587 (1.59); 7.466 (0.96); 7.464 (0.97); 7.454 (1.95); 7.452 (1.93); 7.441 (1.05); 7.439 (1.01); 7.356 (0.98); 7.353 (1.01); 7.343 (1.49); 7.340 (1.49); 7.330 (0.92); 7.326 (3.02); 7.322 (2.93); 7.198 (1.92); 7.184 (2.36); 7.086 (1.86); 7.082 (1.80); 7.072 (1.48); 7.069 (1.45); 5.372 (8.71); 4.170 (1.28); 4.147 (1.33); 4.034 (0.42); 4.022 (0.43); 3.347 (294.05); 3.336 (1.74); 3.329 (0.85); 3.323 (2.59); 3.316 (0.56); 3.310 (0.28); 3.012 (0.74); 3.009 (0.88); 2.989 (1.64); 2.970 (0.89); 2.966 (0.75); 2.926 (0.39); 2.618 (0.38); 2.615 (0.51); 2.612 (0.37); 2.524 (0.95); 2.521 (1.16); 2.518 (1.13); 2.509 (28.91); 2.506 (63.09); 2.503 (88.13); 2.500 (63.50); 2.497 (28.51); 2.390 (0.35); 2.387 (0.49); 2.384 (0.35); 2.150 (13.50); 2.139 (0.29); 2.091 (1.01); 2.087 (1.07); 2.077 (1.48); 2.070 (1.16); 1.990 (1.86); 1.689 (0.39); 1.682 (0.44); 1.668 (0.95); 1.662 (1.06); 1.648 (1.03); 1.642 (0.95); 1.628 (0.40); 1.621 (0.33); 1.397 (4.18); 1.186 (0.50); 1.174 (0.99); 1.163 (0.47); -0.000 (2.83); |
| I-86 | 8.399 (5.19); 5.833 (3.83); 5.747 (0.50); 4.911 (0.51); 4.898 (0.60); 4.889 (0.94); 4.878 (0.65); 4.866 (0.49); 4.448 (0.58); 4.416 (0.61); 4.057 (0.40); 4.039 (1.24); 4.021 (1.52); 4.003 (0.70); 3.992 (0.62); 3.788 (6.82); 3.651 (0.63); 3.619 (1.54); 3.607 (15.00); 3.369 (0.66); 3.350 (0.97); 3.341 (1.49); 3.299 (165.06); 3.230 (0.71); 3.200 (0.89); 3.170 (0.52); 2.804 (0.42); 2.798 (0.44); 2.769 (0.78); 2.741 (0.46); 2.669 (0.41); 2.539 (0.96); 2.504 (41.97); 2.500 (51.83); 2.496 (36.46); 2.326 (0.40); 2.322 (0.32); 2.074 (14.51); 2.050 (1.43); 1.987 (4.42); 1.908 (2.72); 1.899 (0.94); 1.864 (1.09); 1.741 (0.92); 1.734 (0.97); 1.712 (1.23); 1.688 (0.62); 1.680 (0.61); 1.643 (0.63); 1.542 (1.38); 1.525 (1.66); 1.517 (2.02); 1.502 (1.61); 1.494 (1.65); 1.471 (0.74); 1.463 (0.65); 1.437 (0.48); 1.429 (0.61); 1.405 (1.20); 1.397 (0.92); 1.380 (1.03); 1.372 (1.05); 1.355 (0.46); 1.348 (0.57); 1.324 (0.50); 1.298 (0.71); 1.283 (0.50); 1.268 (0.49); 1.237 (0.34); 1.193 (1.25); 1.175 (2.36); 1.157 (1.18); -0.000 (3.67); |
| I-87 | 8.523 (9.84); 7.707 (2.98); 7.687 (3.32); 7.684 (3.21); 7.601 (2.40); 7.597 (2.39); 7.582 (3.04); 7.578 (3.00); 7.567 (0.56); 7.467 (2.18); 7.460 (4.93); 7.451 (4.19); 7.449 (4.02); 7.438 (10.79); 7.432 (7.40); 7.393 (0.65); 7.360 (5.34); 7.354 (4.72); 7.339 (5.02); 7.321 (1.41); 5.374 (15.00); 4.511 (0.66); 4.434 (1.15); 4.403 (1.19); 4.096 (1.18); 4.064 (1.25); 3.915 (0.74); 3.876 (4.98); 3.860 (4.93); 3.835 (0.67); 3.825 (0.79); 3.804 (1.28); 3.603 (0.66); 3.591 (0.71); 3.575 (0.74); 3.546 (0.88); 3.493 (1.43); 3.414 (4.36); 3.386 (8.22); 3.313 (3660.05); 3.171 (1.55); 3.154 (1.30); 3.136 (1.07); 3.125 (1.03); 3.118 (1.04); 3.084 (0.81); 3.070 (2.28); 3.049 (0.67); 3.037 (0.59); 3.012 (0.64); 2.847 (1.83); 2.825 (1.08); 2.788 (1.73); 2.761 (1.21); 2.704 (0.59); 2.674 (2.87); 2.670 (3.61); 2.665 (2.80); 2.539 (8.98); 2.505 (367.09); 2.501 (452.69); 2.497 (316.87); 2.369 (0.69); 2.332 (2.74); 2.328 (3.34); 2.323 (2.63); 2.293 (0.55); 2.138 (1.31); 2.085 (10.31); 2.069 (9.49); 1.753 (1.07); 1.744 (1.05); 1.722 (0.96); 1.714 (0.95); 1.557 (0.97); 1.547 (1.05); 1.526 (1.00); 1.398 (5.04); 1.237 (1.12); -0.000 (18.59); |
| I-88 | 9.041 (3.08); 8.528 (7.31); 7.707 (2.19); 7.705 (2.15); 7.688 (2.46); 7.685 (2.31); 7.603 (1.69); 7.601 (1.74); 7.585 (2.21); 7.582 (2.09); 7.470 (1.26); 7.451 (2.44); 7.449 (2.32); 7.433 (1.35); 7.359 (1.39); 7.354 (1.40); 7.339 (1.97); 7.335 (1.89); 7.320 (0.92); 7.316 (0.82); 7.088 (2.46); 7.069 (3.17); 6.958 (2.26); 6.939 (1.68); 6.866 (3.55); 5.378 (10.89); 4.805 (1.88); 4.772 (1.97); 4.057 (0.35); 4.039 (1.07); 4.021 (1.07); 4.003 (0.38); 3.830 (0.47); 3.481 (0.75); 3.461 (0.92); 3.452 (1.39); 3.442 (0.95); 3.433 (0.74); 3.423 (0.89); 3.414 (0.64); 3.300 (304.43); 3.277 (15.00); 3.260 (3.50); 3.230 (1.71); 2.674 (0.59); 2.669 (0.73); 2.538 (1.63); 2.504 (76.49); 2.500 (94.36); 2.496 (66.21); 2.409 (0.32); 2.331 (0.60); 2.326 (0.75); 2.289 (0.38); 2.252 (14.68); 2.113 (15.00); 2.069 (0.72); 1.987 (4.54); 1.761 (0.88); 1.731 (1.73); 1.707 (1.54); 1.702 (1.49); 1.678 (0.62); 1.669 (0.53); 1.398 (8.81); 1.237 (0.34); 1.193 (1.25); 1.175 (2.46); 1.157 (1.24); 0.890 (0.32); -0.000 (1.99); |
| I-89 | 8.387 (4.79); 5.891 (0.31); 5.849 (3.05); 4.907 (0.54); 4.894 (0.65); 4.885 (0.94); 4.875 (0.60); 4.862 (0.47); 4.443 (0.61); 4.409 (0.60); 4.065 (0.61); 4.028 (0.72); 3.636 (15.00); 3.558 (3.44); 3.551 (3.39); 3.512 (0.60); 3.450 (0.74); 3.385 (2.46); 3.305 (938.53); 3.165 (0.70); 3.133 (0.90); 3.096 (0.56); 2.737 (0.50); 2.710 (0.82); 2.674 (1.48); 2.669 (1.60); 2.583 (0.68); 2.539 (3.04); 2.504 (165.53); 2.500 (203.24); 2.496 (142.33); 2.409 (0.76); 2.327 (1.66); 2.276 (0.35); 2.262 (0.31); 2.192 (12.48); 2.069 (1.49); 2.035 (0.68); 2.004 (1.28); 1.986 (1.06); 1.972 (0.83); 1.908 (2.22); 1.865 (1.13); 1.718 (1.09); 1.520 (1.97); 1.492 (2.07); 1.467 (1.14); 1.427 (0.81); 1.402 (1.26); 1.378 (1.07); 1.371 (1.09); 1.347 (0.57); 1.321 (0.54); 1.294 (0.71); 1.269 (0.59); 1.236 (0.55); 1.175 (0.39); 0.890 (0.37); -0.000 (3.24); |
| I-90 | 8.522 (8.15); 8.075 (3.66); 7.706 (2.43); 7.703 (2.33); 7.686 (2.68); 7.602 (1.92); 7.598 (1.86); 7.582 (2.35); 7.579 (2.14); 7.469 (1.41); 7.465 (1.39); 7.450 (2.69); 7.447 (2.44); 7.431 (1.41); 7.357 (1.58); 7.353 (1.51); 7.338 (2.12); 7.334 (2.02); 7.319 (0.96); 7.314 (0.83); 7.191 (1.51); 7.172 (2.16); 7.160 (2.17); 7.153 (3.11); 7.132 (1.77); 7.125 (1.65); 6.864 (1.19); 6.857 (1.04); 6.843 (2.02); 6.836 (1.79); 6.822 (0.96); 6.815 (0.79); 5.374 (11.68); 4.177 (2.17); 4.142 (2.29); 4.039 (0.52); 4.021 (0.54); 3.562 (0.32); 3.480 (0.50); 3.305 (826.38); 3.283 (9.10); 3.025 (1.50); 2.995 (2.74); 2.966 (1.48); 2.669 (1.13); 2.665 (0.86); 2.504 (119.25); 2.500 (147.12); 2.496 (103.21); 2.327 (1.06); 2.245 (0.71); 2.145 (15.00); 2.095 (1.91); 2.069 (3.08); 1.987 (2.06); 1.907 (0.30); 1.703 (0.82); 1.681 (1.62); 1.674 (1.79); 1.651 (1.68); 1.644 (1.63); 1.621 (0.72); 1.611 (0.58); 1.398 (7.85); 1.234 (0.31); 1.192 (0.57); 1.175 (1.11); 1.157 (0.59); -0.000 (1.29); |
| I-91 | 8.521 (8.12); 7.986 (3.24); 7.706 (2.18); 7.703 (2.18); 7.686 (2.61); 7.684 (2.51); 7.659 (0.41); 7.603 (1.75); 7.599 (1.75); 7.584 (2.29); 7.580 (2.14); 7.469 (1.30); 7.466 (1.35); 7.450 (2.60); 7.448 (2.44); 7.432 (1.43); 7.429 (1.35); 7.357 (1.45); 7.353 (1.39); 7.338 (2.03); 7.334 (1.97); 7.319 (1.01); 7.315 (0.97); 7.166 (0.40); 7.071 (0.40); 7.045 (2.54); 7.026 (3.02); 7.002 (3.15); 6.888 (0.36); 6.856 (2.02); 6.838 (1.70); 5.747 (0.41); 5.375 (11.05); 5.172 (1.52); 4.173 (2.02); 4.140 (2.09); 4.057 (0.38); 4.039 (1.04); 4.021 (1.04); 4.003 (0.39); 3.354 (1.54); 3.300 (400.47); 3.276 (17.79); 2.995 (1.45); 2.967 (2.65); 2.938 (1.47); 2.909 (0.62); 2.673 (0.75); 2.669 (0.89); 2.664 (0.68); 2.539 (1.87); 2.504 (96.48); 2.500 (117.29); 2.496 (81.20); 2.387 (0.42); 2.331 (0.81); 2.327 (0.93); 2.232 (15.00); 2.154 (2.31); 2.107 (14.89); 2.087 (2.14); 2.069 (1.65); 2.053 (2.17); 1.987 (4.59); 1.908 (0.40); 1.696 (0.68); 1.687 (0.79); 1.666 (1.67); 1.658 (1.74); 1.635 (1.64); 1.627 (1.53); 1.605 (0.66); 1.595 (0.57); 1.398 (3.72); 1.236 (0.45); 1.193 (1.31); 1.175 (2.43); 1.157 (1.27); 0.891 (0.34); -0.000 (5.11); |
| I-92 | 8.521 (7.30); 8.224 (3.16); 7.705 (2.27); 7.703 (2.26); 7.685 (2.52); 7.602 (1.80); 7.598 (1.83); 7.583 (2.32); 7.579 (2.20); 7.469 (1.29); 7.450 (2.63); 7.431 (1.43); 7.356 (1.38); 7.352 (1.43); 7.337 (2.03); 7.333 (1.97); 7.318 (0.97); 7.314 (0.93); 7.251 (1.54); 7.232 (1.59); 7.059 (1.52); 7.039 (2.18); 7.033 (1.77); 7.012 (1.96); 6.903 (1.13); 6.897 (1.24); 6.890 (1.34); 5.374 (11.63); 4.173 (2.15); 4.139 (2.28); 4.039 (0.63); 4.021 (0.56); 3.443 (0.32); 3.397 (0.51); 3.349 (2.17); 3.300 (432.74); 3.277 (20.18); 3.002 (1.56); 2.973 (2.76); 2.943 (1.58); 2.669 (1.08); 2.607 (0.42); 2.594 (0.53); 2.539 (2.40); 2.504 (112.41); 2.500 (137.57); 2.496 (97.19); 2.330 (0.87); 2.327 (1.10); 2.247 (15.00); 2.217 (0.33); 2.083 (2.11); 2.069 (1.66); 2.057 (2.16); 1.987 (2.34); 1.696 (0.70); 1.686 (0.83); 1.664 (1.70); 1.656 (1.83); 1.634 (1.69); 1.626 (1.60); 1.604 (0.70); 1.596 (0.58); 1.398 (4.72); 1.236 (0.40); 1.193 (0.70); 1.175 (1.28); 1.157 (0.62); 0.890 (0.36); -0.000 (3.42); |
| I-93 | 8.401 (4.58); 7.656 (2.34); 7.426 (2.35); 7.419 (2.37); 6.911 (2.14); 6.889 (2.39); 6.555 (1.37); 6.547 (1.34); 6.533 (1.21); 6.525 (1.19); 5.747 (1.15); 4.913 (0.46); 4.901 (0.58); 4.891 (0.87); 4.881 (0.59); 4.868 (0.45); 4.128 (1.26); 4.095 (1.33); 4.040 (0.60); 4.022 (0.60); 3.769 (13.81); 3.676 (15.00); 3.306 (12.00); 3.027 (0.90); 2.997 (1.61); 2.969 (0.91); 2.539 (0.32); 2.505 (15.53); 2.500 (19.17); 2.496 (13.59); 2.088 (1.09); 2.062 (1.26); 1.987 (2.46); 1.886 (0.92); 1.868 (1.00); 1.859 (0.99); 1.743 (0.85); 1.732 (0.90); 1.711 (1.34); 1.701 (1.14); 1.679 (1.07); 1.670 (1.11); 1.648 (1.03); 1.640 (0.95); 1.618 (0.42); 1.609 (0.35); 1.553 (0.70); 1.544 (0.81); 1.521 (1.47); 1.497 (1.50); 1.475 (0.58); 1.467 (0.55); 1.437 (0.41); 1.430 (0.54); 1.405 (1.11); 1.397 (0.83); 1.380 (0.96); 1.372 (0.97); 1.355 (0.42); 1.347 (0.51); 1.324 (0.43); 1.316 (0.33); 1.299 (0.53); 1.275 (0.40); 1.269 (0.43); 1.193 (0.70); 1.175 (1.36); 1.158 (0.69); -0.000 (1.62); |
| I-94 | 8.410 (6.62); 8.361 (3.32); 8.203 (2.94); 8.197 (3.03); 7.900 (1.65); 7.894 (1.68); 7.879 (1.83); 7.873 (1.80); 7.470 (2.62); 7.449 (2.70); 4.918 (0.68); 4.905 (0.84); 4.896 (1.27); 4.886 (0.86); 4.873 (0.65); 4.206 (1.81); 4.173 (1.94); 3.438 (0.42); 3.373 (2.02); 3.304 (638.85); 3.186 (1.11); 3.169 (0.69); 3.066 (1.50); 3.037 (2.45); 3.008 (1.39); 2.673 (1.17); 2.669 (1.42); 2.664 (1.15); 2.597 (0.56); 2.539 (2.98); 2.504 (150.80); 2.500 (187.87); 2.496 (134.00); 2.420 (0.97); 2.363 (0.47); 2.336 (1.18); 2.326 (1.99); 2.321 (2.03); 2.310 (15.00); 2.294 (1.07); 2.212 (0.49); 2.140 (1.69); 2.110 (1.73); 2.084 (1.98); 2.069 (4.85); 1.987 (0.52); 1.890 (1.48); 1.870 (1.61); 1.736 (2.16); 1.727 (2.33); 1.707 (2.76); 1.698 (2.47); 1.674 (1.65); 1.667 (1.57); 1.645 (0.89); 1.635 (0.79); 1.620 (0.50); 1.558 (1.13); 1.549 (1.27); 1.527 (2.28); 1.502 (2.32); 1.478 (1.03); 1.471 (1.00); 1.434 (0.94); 1.417 (1.11); 1.409 (1.68); 1.401 (1.34); 1.383 (1.50); 1.376 (1.54); 1.360 (0.73); 1.352 (0.88); 1.328 (0.74); 1.299 (0.93); 1.273 (0.83); 1.236 (0.99); 1.210 (0.39); 1.175 (0.55); 1.158 (0.62); 1.139 (0.48); -0.000 (15.44); |
| I-95 | 8.402 (15.00); 8.293 (6.73); 7.663 (6.76); 7.657 (6.74); 7.485 (7.37); 7.464 (8.40); 7.297 (3.17); 7.278 (6.01); 7.259 (4.14); 7.240 (3.69); 7.237 (2.99); 7.199 (8.61); 7.192 (7.65); 7.186 (6.73); 7.178 (6.86); 7.171 (7.12); 7.133 (1.52); 7.123 (1.72); 7.114 (1.44); 7.110 (1.29); 6.135 (2.37); 6.124 (4.72); 6.113 (2.34); 5.747 (10.38); 5.047 (1.84); 5.032 (1.92); 4.162 (3.88); 4.129 (3.95); 3.352 (3.16); 3.302 (618.90); 3.279 (25.37); 3.043 (2.75); 3.013 (4.87); 2.984 (2.64); 2.888 (1.50); 2.859 (1.65); 2.846 (2.89); 2.832 (1.53); 2.791 (1.47); 2.776 (1.94); 2.770 (1.94); 2.753 (1.88); 2.728 (1.18); 2.714 (1.16); 2.673 (1.45); 2.669 (1.62); 2.664 (1.36); 2.660 (1.23); 2.539 (2.97); 2.504 (130.48); 2.500 (161.64); 2.496 (113.53); 2.088 (3.58); 2.083 (3.57); 2.069 (2.87); 2.053 (5.94); 2.040 (5.09); 2.029 (4.70); 1.987 (2.08); 1.962 (1.38); 1.951 (1.54); 1.940 (1.70); 1.928 (1.66); 1.916 (1.33); 1.907 (1.33); 1.892 (1.29); 1.879 (1.21); 1.868 (1.24); 1.850 (1.77); 1.837 (2.15); 1.825 (1.76); 1.819 (1.32); 1.714 (1.86); 1.705 (1.94); 1.684 (3.59); 1.675 (3.91); 1.653 (3.68); 1.644 (3.17); 1.623 (1.31); -0.000 (3.03); |
| I-96 | 8.394 (7.76); 7.291 (1.67); 7.274 (3.32); 7.259 (2.20); 7.241 (2.00); 7.197 (2.26); 7.187 (2.93); 7.181 (2.86); 7.171 (1.92); 7.031 (2.49); 7.012 (3.43); 6.931 (2.16); 6.911 (1.57); 6.884 (3.52); 6.132 (1.31); 6.121 (2.59); 6.110 (1.29); 5.747 (9.05); 4.493 (0.91); 4.459 (0.89); 3.971 (0.84); 3.942 (0.87); 3.703 (0.53); 3.664 (3.66); 3.646 (3.50); 3.628 (1.65); 3.606 (0.55); 3.356 (2.13); 3.301 (417.50); 3.280 (12.22); 3.223 (0.67); 3.200 (0.94); 3.172 (1.22); 3.142 (0.72); 2.900 (0.51); 2.887 (0.91); 2.874 (0.58); 2.856 (0.93); 2.844 (1.67); 2.831 (0.94); 2.790 (1.32); 2.776 (1.51); 2.767 (1.47); 2.754 (1.96); 2.726 (1.15); 2.673 (0.85); 2.669 (1.01); 2.538 (2.30); 2.504 (99.12); 2.500 (122.16); 2.496 (85.39); 2.331 (0.76); 2.326 (0.92); 2.322 (0.74); 2.220 (14.66); 2.140 (15.00); 2.128 (2.99); 2.069 (2.01); 2.060 (1.73); 2.026 (3.78); 1.987 (2.95); 1.968 (0.78); 1.958 (0.83); 1.946 (0.87); 1.935 (0.88); 1.924 (0.86); 1.911 (0.69); 1.849 (0.98); 1.835 (1.20); 1.824 (1.08); 1.599 (0.53); 1.568 (0.93); 1.548 (1.02); 1.535 (1.10); 1.506 (0.75); 1.500 (0.83); 1.398 (1.03); 1.235 (0.55); 1.175 (0.96); -0.000 (4.41); |
| I-97 | 8.401 (5.44); 7.802 (2.26); 7.797 (2.34); 7.786 (2.44); 7.295 (1.10); 7.277 (2.19); 7.258 (1.44); 7.240 (1.32); 7.236 (1.07); 7.197 (1.40); 7.185 (2.11); 7.166 (1.23); 7.038 (0.52); 7.032 (0.41); 7.016 (3.16); 7.010 (4.11); 7.008 (4.43); 6.986 (0.60); 6.135 (0.85); 6.123 (1.69); 6.111 (0.84); 5.747 (4.61); 4.123 (1.32); 4.090 (1.37); 3.877 (0.60); 3.817 (15.00); 3.301 (141.53); 3.278 (6.58); 3.019 (0.96); 2.990 (1.71); 2.961 (0.95); 2.887 (0.54); 2.873 (0.33); 2.858 (0.56); 2.845 (1.02); 2.832 (0.53); 2.789 (0.51); 2.775 (0.65); 2.768 (0.65); 2.752 (0.61); 2.732 (0.32); 2.725 (0.34); 2.669 (0.33); 2.539 (0.65); 2.509 (18.74); 2.504 (32.63); 2.500 (40.84); 2.496 (28.76); 2.327 (0.33); 2.076 (1.32); 2.061 (1.10); 2.050 (2.13); 2.039 (2.28); 2.028 (1.81); 2.011 (0.85); 1.999 (0.39); 1.987 (0.79); 1.970 (0.39); 1.960 (0.49); 1.947 (0.56); 1.938 (0.59); 1.925 (0.57); 1.913 (0.43); 1.848 (0.58); 1.836 (0.75); 1.823 (0.61); 1.807 (0.42); 1.801 (0.40); 1.702 (0.56); 1.692 (0.59); 1.670 (1.08); 1.662 (1.16); 1.639 (1.07); 1.631 (1.00); 1.610 (0.45); 1.600 (0.35); 1.398 (0.55); 1.046 (0.33); 1.031 (0.34); -0.000 (1.79); |
| I-98 | 8.400 (5.10); 8.120 (0.47); 8.109 (0.48); 7.919 (2.17); 7.296 (1.08); 7.278 (2.13); 7.260 (1.39); 7.241 (1.30); 7.238 (1.03); 7.199 (1.43); 7.187 (1.98); 7.170 (1.19); 6.666 (0.66); 6.650 (0.65); 6.135 (0.84); 6.124 (1.65); 6.112 (0.82); 5.747 (3.82); 4.132 (1.37); 4.099 (1.39); 3.306 (124.63); 2.990 (10.16); 2.961 (1.73); 2.932 (0.95); 2.900 (0.32); 2.888 (0.55); 2.875 (0.35); 2.858 (0.59); 2.845 (1.03); 2.832 (0.54); 2.793 (0.53); 2.777 (0.70); 2.771 (0.68); 2.755 (0.62); 2.738 (0.40); 2.726 (0.37); 2.712 (0.31); 2.674 (0.38); 2.669 (0.44); 2.539 (1.03); 2.504 (44.14); 2.500 (54.13); 2.496 (37.93); 2.327 (0.43); 2.261 (0.30); 2.219 (12.57); 2.102 (0.43); 2.069 (1.81); 2.061 (2.40); 2.049 (15.00); 2.040 (3.16); 2.030 (2.76); 1.994 (0.39); 1.987 (0.62); 1.971 (0.41); 1.963 (0.49); 1.949 (0.55); 1.939 (0.58); 1.927 (0.53); 1.915 (0.44); 1.908 (0.46); 1.850 (0.56); 1.838 (0.69); 1.826 (0.57); 1.809 (0.39); 1.668 (0.46); 1.658 (0.56); 1.636 (1.06); 1.629 (1.13); 1.607 (1.03); 1.598 (0.95); 1.577 (0.40); 1.569 (0.33); -0.000 (0.94); |
| I-99 | 8.405 (14.59); 7.458 (6.08); 7.436 (15.00); 7.430 (8.22); 7.358 (5.26); 7.352 (4.44); 7.337 (3.51); 7.330 (3.16); 7.296 (3.33); 7.279 (6.54); 7.260 (4.34); 7.242 (3.94); 7.239 (3.19); 7.200 (4.21); 7.187 (6.28); 7.169 (3.71); 6.137 (2.52); 6.125 (5.02); 6.114 (2.58); 5.747 (12.59); 4.431 (1.68); 4.399 (1.73); 4.089 (1.60); 4.056 (1.72); 3.871 (7.14); 3.856 (6.97); 3.403 (0.96); 3.393 (1.63); 3.383 (1.39); 3.374 (2.12); 3.365 (3.28); 3.355 (2.62); 3.301 (515.55); 3.278 (25.09); 3.253 (3.91); 3.224 (2.02); 2.889 (1.60); 2.876 (1.02); 2.859 (1.77); 2.846 (3.11); 2.834 (1.67); 2.805 (1.49); 2.792 (2.14); 2.777 (3.93); 2.755 (2.43); 2.714 (0.92); 2.669 (1.17); 2.538 (2.38); 2.504 (121.13); 2.500 (149.59); 2.496 (106.17); 2.326 (1.24); 2.116 (1.75); 2.085 (2.90); 2.079 (2.96); 2.070 (3.06); 2.063 (3.16); 2.053 (3.72); 2.039 (5.63); 2.029 (5.79); 2.001 (1.23); 1.987 (1.76); 1.972 (1.22); 1.962 (1.53); 1.950 (1.68); 1.939 (1.82); 1.929 (1.69); 1.916 (1.32); 1.850 (1.69); 1.838 (2.19); 1.826 (1.88); 1.809 (1.33); 1.794 (1.01); 1.727 (1.66); 1.706 (1.66); 1.540 (1.46); 1.531 (1.49); 1.510 (1.36); 1.501 (1.29);-0.000 (6.73); |
| I-100 | 8.400 (7.05); 8.131 (3.14); 7.311 (5.05); 7.298 (1.97); 7.290 (4.05); 7.280 (2.93); 7.259 (1.98); 7.241 (1.77); 7.238 (1.40); 7.199 (1.86); 7.186 (3.00); 7.167 (1.68); 6.960 (1.70); 6.956 (1.63); 6.940 (1.53); 6.936 (1.44); 6.136 (1.12); 6.125 (2.24); 6.113 (1.12); 5.746 (7.80); 4.165 (1.83); 4.132 (1.89); 3.306 (392.68); 3.282 (12.84); 3.016 (1.35); 2.987 (2.34); 2.958 (1.28); 2.941 (1.95); 2.902 (0.39); 2.889 (0.74); 2.876 (0.46); 2.860 (0.82); 2.847 (1.37); 2.835 (0.73); 2.792 (0.71); 2.776 (0.91); 2.769 (0.91); 2.755 (0.85); 2.727 (0.52); 2.713 (0.45); 2.674 (0.56); 2.669 (0.65); 2.539 (1.41); 2.504 (62.43); 2.500 (76.46); 2.496 (53.76); 2.430 (0.35); 2.331 (0.55); 2.327 (0.64); 2.282 (2.06); 2.268 (15.00); 2.069 (2.07); 2.040 (3.49); 2.001 (0.57); 1.987 (0.74); 1.974 (0.56); 1.965 (0.66); 1.953 (0.73); 1.941 (0.80); 1.930 (0.75); 1.918 (0.60); 1.908 (0.43); 1.895 (0.34); 1.851 (0.73); 1.838 (0.97); 1.826 (0.82); 1.811 (0.55); 1.795 (0.37); 1.700 (0.76); 1.691 (0.84); 1.670 (1.55); 1.661 (1.60); 1.639 (1.44); 1.630 (1.35); 1.610 (0.57); 1.398 (0.38); 1.236 (0.30); -0.000 (3.93); |
| I-101 | 8.523 (8.20); 7.706 (2.61); 7.684 (2.97); 7.597 (2.26); 7.581 (3.12); 7.558 (4.54); 7.552 (4.86); 7.544 (4.52); 7.523 (5.16); 7.467 (1.80); 7.451 (3.07); 7.432 (2.00); 7.398 (2.96); 7.392 (2.91); 7.377 (2.37); 7.371 (2.17); 7.359 (1.82); 7.355 (1.89); 7.335 (2.59); 7.320 (1.30); 7.316 (1.35); 5.373 (12.70); 5.352 (0.81); 4.437 (0.96); 4.407 (0.94); 4.094 (0.88); 4.057 (1.06); 3.881 (3.40); 3.858 (3.23); 3.417 (1.33); 3.389 (2.87); 3.307 (1756.43); 3.180 (1.87); 3.171 (1.77); 3.155 (1.55); 3.110 (1.14); 3.092 (0.97); 3.075 (0.96); 3.061 (0.93); 3.038 (0.92); 3.005 (0.77); 2.989 (0.73); 2.959 (0.74); 2.818 (1.08); 2.791 (1.50); 2.759 (1.01); 2.674 (1.89); 2.669 (2.34); 2.617 (0.64); 2.590 (0.95); 2.539 (6.45); 2.504 (242.43); 2.500 (301.45); 2.496 (215.38); 2.393 (1.29); 2.341 (1.11); 2.327 (2.71); 2.323 (2.15); 2.292 (0.75); 2.274 (0.69); 2.257 (0.72); 2.217 (0.67); 2.171 (0.63); 2.141 (1.36); 2.104 (1.83); 2.069 (2.34); 2.049 (1.14); 1.987 (1.80); 1.908 (0.61); 1.781 (0.80); 1.758 (1.14); 1.730 (1.15); 1.583 (0.70); 1.552 (1.12); 1.527 (1.06); 1.398 (15.00); 1.235 (0.88); 1.175 (1.08); 1.158 (0.70); -0.000 (8.21); |
| I-102 | 8.512 (7.99); 7.705 (2.26); 7.702 (2.20); 7.685 (2.48); 7.596 (1.74); 7.592 (1.80); 7.577 (2.30); 7.573 (2.25); 7.467 (1.29); 7.464 (1.24); 7.449 (2.56); 7.446 (2.41); 7.430 (1.48); 7.427 (1.29); 7.357 (1.40); 7.353 (1.42); 7.338 (2.03); 7.334 (1.93); 7.319 (1.06); 7.314 (0.87); 7.033 (2.23); 7.014 (3.14); 6.932 (2.20); 6.912 (1.65); 6.887 (3.58); 5.369 (11.27); 4.496 (0.85); 4.463 (0.91); 4.022 (0.40); 3.980 (0.85); 3.950 (0.90); 3.707 (0.50); 3.668 (3.53); 3.651 (3.41); 3.610 (0.52); 3.451 (0.44); 3.446 (0.43); 3.436 (0.50); 3.421 (0.60); 3.410 (0.77); 3.401 (0.86); 3.388 (1.30); 3.379 (1.80); 3.349 (3.97); 3.303 (759.68); 3.217 (1.36); 3.183 (1.49); 3.162 (0.73); 3.155 (0.88); 2.796 (0.77); 2.767 (1.14); 2.735 (0.73); 2.669 (1.24); 2.621 (0.45); 2.539 (3.97); 2.504 (127.08); 2.500 (154.05); 2.496 (106.97); 2.331 (1.02); 2.327 (1.21); 2.298 (0.37); 2.222 (14.76); 2.186 (0.43); 2.143 (15.00); 2.085 (1.13); 2.069 (1.67); 2.048 (1.72); 2.015 (1.07); 1.987 (1.62); 1.618 (0.45); 1.587 (0.93); 1.565 (1.10); 1.556 (1.07); 1.550 (1.03); 1.518 (0.82); 1.398 (3.65); 1.193 (0.46); 1.175 (0.79); 1.157 (0.45); -0.000 (2.46); |
| I-103 | 8.519 (4.28); 7.703 (1.46); 7.684 (1.60); 7.653 (2.37); 7.595 (1.16); 7.575 (1.41); 7.466 (0.82); 7.448 (1.66); 7.428 (1.02); 7.416 (2.37); 7.409 (2.23); 7.355 (0.95); 7.335 (1.21); 7.317 (0.59); 6.906 (2.10); 6.884 (2.35); 6.554 (1.35); 6.546 (1.29); 6.531 (1.27); 6.523 (1.20); 5.371 (6.62); 4.123 (1.40); 4.089 (1.57); 4.067 (0.48); 4.039 (0.69); 4.022 (0.72); 3.922 (0.38); 3.852 (0.44); 3.808 (0.64); 3.805 (0.86); 3.760 (13.78); 3.730 (0.58); 3.673 (15.00); 3.641 (0.59); 3.627 (0.53); 3.614 (0.56); 3.604 (0.56); 3.578 (0.62); 3.567 (0.87); 3.488 (0.96); 3.460 (1.23); 3.442 (1.31); 3.303 (1289.43); 3.226 (0.76); 3.216 (0.70); 3.184 (0.42); 3.173 (0.39); 3.026 (0.98); 2.993 (1.61); 2.966 (0.98); 2.755 (0.41); 2.737 (0.41); 2.729 (0.45); 2.695 (0.66); 2.673 (1.90); 2.669 (2.29); 2.653 (0.87); 2.646 (0.82); 2.539 (12.25); 2.504 (224.27); 2.500 (268.36); 2.496 (186.30); 2.326 (1.73); 2.099 (1.08); 2.069 (4.18); 1.987 (1.23); 1.714 (0.47); 1.704 (0.52); 1.682 (1.06); 1.674 (1.07); 1.654 (1.01); 1.642 (0.99); 1.612 (0.37); 1.398 (1.88); 1.292 (0.75); 1.237 (0.54); 1.192 (0.58); 1.175 (1.00); 1.158 (0.91); -0.000 (29.58); |
| I-104 | 8.535 (6.09); 7.707 (1.18); 7.705 (1.22); 7.693 (1.32); 7.691 (1.31); 7.656 (1.95); 7.599 (0.83); 7.597 (0.86); 7.587 (1.05); 7.584 (1.02); 7.465 (0.66); 7.463 (0.70); 7.456 (1.57); 7.453 (2.75); 7.440 (0.72); 7.438 (0.69); 7.354 (0.65); 7.351 (0.67); 7.341 (0.94); 7.338 (0.94); 7.328 (0.52); 7.326 (0.51); 6.871 (1.61); 6.857 (2.52); 6.813 (0.92); 6.812 (0.94); 6.810 (0.96); 6.799 (0.58); 6.799 (0.62); 6.796 (0.60); 5.370 (5.58); 4.130 (0.82); 4.108 (0.87); 3.766 (0.89); 3.760 (13.50); 3.388 (0.55); 3.376 (0.56); 3.367 (0.49); 3.364 (0.60); 3.348 (408.25); 3.325 (0.59); 3.321 (0.76); 3.314 (0.78); 2.992 (0.50); 2.989 (0.61); 2.969 (1.05); 2.950 (0.59); 2.946 (0.50); 2.618 (0.45); 2.615 (0.64); 2.612 (0.46); 2.542 (3.59); 2.524 (1.21); 2.521 (1.52); 2.518 (1.40); 2.509 (34.73); 2.506 (76.23); 2.503 (106.87); 2.500 (77.30); 2.497 (35.42); 2.390 (0.46); 2.387 (0.64); 2.384 (0.45); 2.208 (9.05); 2.077 (1.23); 2.074 (0.73); 2.057 (0.74); 2.053 (0.78); 1.990 (1.62); 1.661 (0.63); 1.655 (0.73); 1.640 (0.69); 1.635 (0.63); 1.186 (0.46); 1.174 (0.94); 1.163 (0.45); 1.102 (0.45); 1.091 (0.88); 1.079 (0.42); -0.000 (4.14); |
| I-105 | 9.154 (0.40); 8.520 (4.83); 8.204 (0.43); 7.803 (2.58); 7.798 (3.00); 7.791 (2.83); 7.704 (1.48); 7.683 (1.79); 7.598 (1.22); 7.580 (1.55); 7.467 (0.96); 7.448 (1.73); 7.430 (0.90); 7.426 (0.88); 7.354 (0.87); 7.351 (0.90); 7.336 (1.40); 7.316 (0.67); 7.039 (0.70); 7.034 (0.46); 7.017 (3.57); 7.010 (4.82); 6.990 (0.69); 6.569 (0.38); 5.371 (7.43); 4.131 (1.47); 4.095 (1.50); 4.038 (0.50); 4.021 (0.44); 3.877 (2.22); 3.819 (15.00); 3.500 (0.43); 3.483 (0.39); 3.454 (0.48); 3.437 (0.64); 3.412 (0.83); 3.379 (1.46); 3.299 (711.73); 3.249 (1.91); 3.227 (1.04); 3.205 (0.79); 3.187 (0.64); 3.168 (0.52); 3.136 (0.41); 3.098 (0.37); 3.031 (1.19); 3.001 (1.97); 2.972 (1.14); 2.931 (0.53); 2.713 (0.41); 2.696 (0.50); 2.673 (1.56); 2.669 (1.83); 2.664 (1.55); 2.653 (0.62); 2.630 (0.60); 2.539 (10.28); 2.504 (197.41); 2.500 (236.72); 2.496 (165.86); 2.331 (1.44); 2.327 (1.81); 2.096 (1.28); 2.069 (2.97); 1.987 (1.76); 1.715 (0.51); 1.710 (0.60); 1.686 (1.17); 1.677 (1.20); 1.655 (1.15); 1.647 (1.13); 1.626 (0.50); 1.617 (0.43); 1.398 (0.90); 1.292 (0.66); 1.236 (0.53); 1.193 (0.62); 1.175 (1.05); 1.159 (0.65); -0.000 (26.78); |
| I-106 | 9.465 (0.54); 8.524 (10.45); 8.448 (5.33); 8.426 (0.46); 7.939 (4.48); 7.934 (4.38); 7.758 (0.38); 7.737 (0.51); 7.705 (6.12); 7.685 (7.15); 7.601 (2.29); 7.598 (2.47); 7.582 (3.03); 7.579 (3.02); 7.482 (2.72); 7.478 (2.85); 7.465 (3.00); 7.457 (2.70); 7.449 (3.94); 7.447 (3.67); 7.431 (2.10); 7.428 (1.98); 7.357 (1.88); 7.353 (1.90); 7.338 (2.75); 7.333 (2.61); 7.318 (1.32); 7.314 (1.21); 5.374 (15.00); 4.185 (2.75); 4.151 (2.94); 3.489 (0.44); 3.482 (0.47); 3.451 (0.59); 3.383 (2.70); 3.353 (6.21); 3.305 (1392.67); 3.112 (0.82); 3.077 (2.40); 3.072 (2.40); 3.041 (3.83); 3.013 (2.22); 2.971 (0.39); 2.936 (0.38); 2.696 (0.54); 2.674 (1.80); 2.669 (2.17); 2.665 (1.70); 2.634 (0.56); 2.539 (10.84); 2.504 (222.38); 2.500 (275.02); 2.496 (194.12); 2.362 (0.62); 2.331 (1.81); 2.327 (2.19); 2.322 (1.71); 2.303 (0.47); 2.296 (0.42); 2.200 (0.35); 2.175 (0.34); 2.111 (2.63); 2.085 (3.05); 2.069 (2.74); 1.987 (0.61); 1.766 (0.34); 1.745 (1.04); 1.735 (1.24); 1.713 (2.31); 1.704 (2.49); 1.683 (2.35); 1.674 (2.13); 1.653 (1.01); 1.643 (0.87); 1.398 (12.78); 1.292 (0.77); 1.236 (0.70); 1.175 (0.40); 1.159 (0.39); -0.000 (52.96); |
| I-107 | 8.520 (5.77); 8.137 (3.46); 7.704 (2.01); 7.685 (2.25); 7.598 (1.63); 7.582 (2.00); 7.468 (1.11); 7.449 (2.25); 7.429 (1.20); 7.357 (1.23); 7.353 (1.27); 7.337 (2.05); 7.313 (5.45); 7.291 (3.32); 6.961 (1.70); 6.941 (1.57); 6.937 (1.45); 5.373 (9.91); 4.171 (1.93); 4.137 (2.05); 3.519 (0.35); 3.496 (0.42); 3.475 (0.48); 3.457 (0.58); 3.305 (863.19); 3.174 (0.58); 3.163 (0.49); 3.110 (0.33); 3.089 (0.32); 3.028 (1.52); 2.998 (2.52); 2.968 (1.44); 2.695 (0.38); 2.669 (1.50); 2.539 (7.14); 2.504 (155.89); 2.500 (184.35); 2.327 (1.44); 2.295 (0.48); 2.269 (15.00); 2.088 (1.73); 2.069 (2.38); 2.061 (2.00); 1.987 (0.34); 1.715 (0.65); 1.705 (0.76); 1.685 (1.53); 1.676 (1.62); 1.654 (1.54); 1.645 (1.45); 1.625 (0.63); 1.398 (8.39); 1.292 (0.45); 1.236 (0.38); - 0.000 (34.88); |
| I-108 | 9.287 (0.98); 8.522 (10.14); 8.299 (5.23); 8.223 (1.12); 8.216 (1.12); 7.703 (3.22); 7.685 (3.40); 7.665 (5.34); 7.658 (5.03); 7.599 (2.47); 7.582 (2.98); 7.529 (1.42); 7.507 (1.40); 7.487 (5.63); 7.465 (7.80); 7.449 (3.73); 7.431 (2.08); 7.357 (1.98); 7.352 (1.89); 7.336 (2.83); 7.333 (2.73); 7.318 (1.48); 7.200 (3.53); 7.194 (3.75); 7.179 (3.05); 7.173 (3.00); 5.372 (15.00); 4.168 (2.80); 4.135 (3.10); 3.302 (4496.06); 3.147 (2.26); 3.053 (2.98); 3.025 (4.51); 3.011 (1.81); 2.996 (2.80); 2.939 (1.05); 2.910 (0.94); 2.838 (1.06); 2.820 (0.94); 2.801 (0.97); 2.780 (0.94); 2.775 (0.94); 2.765 (1.02); 2.706 (1.38); 2.695 (1.98); 2.673 (6.65); 2.669 (8.48); 2.664 (6.55); 2.641 (2.17); 2.539 (42.84); 2.504 (856.94); 2.500 (1055.52); 2.496 (741.08); 2.331 (6.33); 2.326 (8.20); 2.322 (6.29); 2.281 (1.24); 2.261 (1.14); 2.250 (1.01); 2.237 (1.05); 2.212 (0.93); 2.202 (0.96); 2.102 (2.87); 2.069 (8.51); 1.720 (1.21); 1.698 (2.53); 1.688 (2.50); 1.668 (2.68); 1.660 (2.42); 1.642 (1.19); 1.638 (1.19); 1.398 (6.16); 1.326 (0.99); 1.292 (2.77); 1.237 (1.89); 1.158 (0.94); 0.146 (1.44); -0.000 (295.19); -0.008 (17.22); -0.150 (1.17); |
| I-109 | 11.642 (0.57); 8.418 (7.18); 6.547 (4.82); 5.713 (0.65); 4.926 (0.37); 4.917 (0.67); 4.904 (0.86); 4.894 (1.29); 4.884 (0.86); 4.872 (0.65); 4.862 (0.35); 4.265 (0.67); 4.231 (0.75); 4.096 (0.72); 4.058 (0.80); 4.040 (0.81); 4.022 (0.74); 3.743 (15.00); 3.584 (1.94); 3.409 (0.78); 3.400 (0.66); 3.390 (0.99); 3.381 (1.54); 3.372 (1.14); 3.362 (1.06); 3.352 (1.44); 3.342 (1.44); 3.304 (209.83); 3.251 (0.94); 3.163 (0.62); 3.133 (0.93); 3.103 (0.53); 2.670 (0.36); 2.540 (1.82); 2.505 (37.38); 2.501 (45.14); 2.497 (31.39); 2.327 (0.36); 2.158 (1.59); 2.132 (1.85); 2.070 (1.12); 1.987 (3.02); 1.908 (1.19); 1.867 (1.70); 1.861 (1.72); 1.807 (0.69); 1.745 (1.85); 1.723 (1.99); 1.715 (1.97); 1.553 (1.09); 1.544 (1.26); 1.528 (1.99); 1.521 (2.26); 1.497 (2.11); 1.474 (0.87); 1.466 (0.84); 1.432 (0.81); 1.407 (1.69); 1.398 (2.25); 1.382 (1.43); 1.375 (1.43); 1.358 (0.62); 1.350 (0.76); 1.326 (0.65); 1.301 (0.82); 1.296 (0.81); 1.272 (0.65); 1.237 (1.09); 1.193 (0.86); 1.175 (1.67); 1.158 (0.86); -0.000 (9.84); -0.008 (0.57); |
| I-110 | 8.527 (6.56); 8.359 (3.34); 8.198 (2.94); 8.192 (2.99); 7.899 (1.72); 7.893 (1.61); 7.877 (1.81); 7.872 (1.75); 7.707 (2.04); 7.687 (2.20); 7.684 (2.17); 7.603 (1.65); 7.584 (2.01); 7.581 (2.07); 7.469 (3.67); 7.448 (4.12); 7.432 (1.36); 7.358 (1.24); 7.354 (1.24); 7.338 (1.82); 7.335 (1.76); 7.327 (0.49); 7.320 (0.89); 5.746 (0.42); 5.375 (9.77); 5.251 (0.59); 4.202 (1.86); 4.168 (1.97); 4.057 (0.40); 4.039 (1.16); 4.021 (1.19); 4.003 (0.48); 3.556 (0.46); 3.542 (0.55); 3.510 (0.58); 3.420 (1.60); 3.309 (1624.50); 3.207 (1.48); 3.138 (0.72); 3.129 (0.67); 3.065 (1.64); 3.034 (2.59); 3.006 (1.52); 2.960 (0.42); 2.934 (0.40); 2.669 (2.09); 2.665 (1.62); 2.634 (0.65); 2.609 (0.87); 2.539 (6.78); 2.505 (209.34); 2.500 (255.92); 2.496 (180.31); 2.419 (1.58); 2.356 (1.34); 2.331 (1.81); 2.327 (2.22); 2.322 (2.01); 2.305 (15.00); 2.280 (0.62); 2.262 (0.44); 2.157 (0.38); 2.116 (1.69); 2.088 (1.95); 2.084 (1.96); 2.069 (3.04); 1.987 (4.36); 1.907 (0.41); 1.730 (0.74); 1.708 (1.50); 1.701 (1.54); 1.679 (1.48); 1.674 (1.55); 1.650 (0.67); 1.398 (1.18); 1.235 (0.82); 1.193 (1.24); 1.175 (2.43); 1.157 (1.18); -0.000 (8.19); |
| I-111 | 8.641 (3.61); 8.540 (13.50); 7.916 (1.42); 7.912 (1.55); 7.904 (1.51); 7.900 (1.48); 7.708 (2.69); 7.706 (2.77); 7.694 (2.99); 7.692 (2.96); 7.601 (1.94); 7.599 (2.04); 7.589 (2.35); 7.586 (2.34); 7.484 (0.54); 7.481 (0.64); 7.474 (1.21); 7.470 (1.43); 7.466 (2.62); 7.464 (2.48); 7.454 (4.60); 7.452 (3.52); 7.441 (2.54); 7.439 (3.24); 7.423 (0.86); 7.355 (1.53); 7.352 (1.57); 7.342 (2.21); 7.339 (2.16); 7.329 (1.23); 7.327 (1.19); 5.761 (1.34); 5.372 (12.92); 4.183 (1.87); 4.160 (1.94); 4.034 (0.68); 4.022 (0.70); 3.351 (1150.97); 3.333 (2.35); 3.328 (1.92); 3.319 (1.34); 3.313 (0.75); 3.029 (1.21); 3.009 (2.30); 2.990 (1.24); 2.618 (0.90); 2.615 (1.27); 2.612 (0.91); 2.543 (7.71); 2.524 (2.35); 2.521 (2.99); 2.518 (2.84); 2.510 (66.55); 2.507 (145.74); 2.503 (201.18); 2.500 (144.14); 2.497 (66.42); 2.391 (0.94); 2.388 (1.29); 2.385 (0.93); 2.096 (1.62); 2.087 (0.76); 2.077 (3.12); 2.053 (0.52); 1.990 (3.12); 1.702 (0.57); 1.695 (0.67); 1.681 (1.40); 1.675 (1.59); 1.661 (1.53); 1.655 (1.41); 1.641 (0.62); 1.634 (0.52); 1.397 (4.93); 1.234 (0.56); 1.186 (0.89); 1.174 (1.82); 1.163 (0.88); 1.158 (0.59); -0.000 (3.30); |
| I-112 | 9.131 (2.19); 8.526 (6.62); 7.706 (1.44); 7.704 (1.46); 7.687 (1.64); 7.684 (1.65); 7.604 (1.03); 7.600 (1.11); 7.584 (1.38); 7.580 (1.38); 7.470 (0.83); 7.466 (0.84); 7.451 (1.70); 7.448 (1.64); 7.432 (0.92); 7.429 (0.88); 7.358 (0.97); 7.354 (0.97); 7.339 (1.26); 7.335 (1.26); 7.319 (0.65); 7.315 (0.62); 7.247 (1.00); 7.227 (2.73); 7.208 (1.96); 7.203 (2.09); 7.188 (0.64); 7.182 (0.82); 7.134 (2.28); 7.128 (1.96); 5.378 (7.61); 4.804 (1.03); 4.769 (1.09); 4.057 (1.08); 4.040 (3.34); 4.022 (3.38); 4.004 (1.12); 3.618 (1.17); 3.616 (0.82); 3.612 (0.76); 3.608 (1.05); 3.601 (2.77); 3.595 (1.07); 3.591 (0.72); 3.585 (1.17); 3.464 (0.79); 3.291 (1046.47); 3.268 (2.67); 3.257 (1.35); 2.673 (0.84); 2.668 (1.18); 2.664 (0.86); 2.538 (5.57); 2.521 (3.34); 2.517 (5.27); 2.508 (63.56); 2.504 (130.33); 2.499 (177.81); 2.494 (126.20); 2.490 (58.70); 2.330 (0.84); 2.326 (1.16); 2.322 (1.00); 2.143 (11.77); 2.118 (1.11); 2.067 (1.28); 1.986 (15.00); 1.781 (0.59); 1.777 (1.39); 1.769 (1.53); 1.760 (4.19); 1.752 (1.92); 1.744 (1.37); 1.731 (0.89); 1.725 (0.86); 1.399 (4.04); 1.193 (4.18); 1.175 (8.27); 1.157 (4.18); -0.000 (8.44); |
| I-113 | 9.290 (1.24); 8.526 (7.27); 7.706 (1.61); 7.703 (1.59); 7.686 (1.80); 7.683 (1.71); 7.603 (1.12); 7.599 (1.23); 7.584 (1.45); 7.580 (1.57); 7.546 (0.76); 7.543 (0.63); 7.516 (3.00); 7.495 (3.80); 7.469 (0.94); 7.467 (0.97); 7.451 (1.76); 7.448 (1.84); 7.432 (1.12); 7.429 (1.08); 7.420 (3.07); 7.413 (3.70); 7.358 (1.00); 7.353 (1.17); 7.347 (2.13); 7.340 (2.28); 7.334 (1.54); 7.326 (1.64); 7.319 (2.07); 5.393 (0.60); 5.378 (8.34); 4.806 (1.06); 4.773 (1.14); 4.518 (0.90); 4.504 (0.86); 4.040 (1.31); 4.022 (1.20); 3.608 (0.59); 3.601 (1.37); 3.475 (0.91); 3.465 (0.59); 3.447 (0.58); 3.352 (1.19); 3.346 (1.48); 3.331 (1.22); 3.291 (1334.77); 3.273 (4.30); 3.269 (3.11); 3.254 (1.00); 2.677 (0.65); 2.673 (1.24); 2.668 (1.62); 2.664 (1.13); 2.538 (7.70); 2.521 (5.01); 2.517 (8.04); 2.508 (89.15); 2.504 (180.11); 2.499 (243.26); 2.494 (169.78); 2.490 (77.33); 2.330 (1.08); 2.326 (1.54); 2.321 (1.09); 2.153 (0.98); 2.126 (1.16); 2.067 (1.16); 1.986 (5.35); 1.777 (1.47); 1.770 (1.00); 1.760 (1.83); 1.752 (1.40); 1.744 (1.38); 1.399 (15.00); 1.193 (1.49); 1.175 (3.04); 1.157 (1.63); 0.955 (0.67); 0.937 (1.36); -0.000 (12.67); |
| I-114 | 9.228 (1.45); 8.524 (4.38); 7.706 (1.00); 7.703 (0.97); 7.686 (1.14); 7.683 (1.11); 7.600 (1.46); 7.593 (1.03); 7.579 (1.85); 7.572 (0.95); 7.469 (0.52); 7.467 (0.56); 7.451 (1.13); 7.448 (1.11); 7.432 (0.64); 7.429 (0.58); 7.358 (0.67); 7.353 (1.13); 7.338 (1.48); 7.334 (1.11); 7.331 (1.12); 7.316 (1.25); 7.260 (0.62); 7.253 (0.56); 7.239 (0.82); 7.232 (0.77); 7.218 (0.40); 7.211 (0.36); 5.377 (5.16); 4.821 (0.70); 4.788 (0.76); 4.040 (0.55); 4.022 (0.53); 3.485 (0.35); 3.475 (0.54); 3.446 (0.34); 3.356 (0.39); 3.337 (1.05); 3.292 (692.36); 3.259 (0.74); 2.677 (0.36); 2.673 (0.66); 2.668 (0.91); 2.664 (0.67); 2.659 (0.33); 2.538 (4.21); 2.521 (2.53); 2.516 (4.04); 2.508 (51.00); 2.504 (104.47); 2.499 (142.57); 2.494 (101.12); 2.490 (47.07); 2.330 (0.72); 2.326 (0.88); 2.321 (0.66); 2.317 (0.33); 2.149 (0.65); 2.123 (0.74); 2.067 (0.47); 1.986 (2.46); 1.806 (0.30); 1.783 (0.61); 1.776 (0.66); 1.753 (0.59); 1.745 (0.55); 1.399 (15.00); 1.193 (0.71); 1.175 (1.32); 1.157 (0.72); -0.000 (4.93); |
| I-115 | 9.211 (1.71); 8.532 (9.82); 7.707 (3.09); 7.705 (3.11); 7.687 (4.17); 7.685 (4.25); 7.665 (1.26); 7.604 (2.55); 7.601 (2.61); 7.585 (3.30); 7.581 (3.37); 7.568 (1.01); 7.547 (1.16); 7.530 (0.82); 7.489 (0.92); 7.485 (0.70); 7.469 (2.39); 7.452 (3.70); 7.449 (3.67); 7.433 (2.62); 7.415 (1.09); 7.397 (1.09); 7.378 (2.93); 7.371 (3.39); 7.359 (4.67); 7.354 (5.54); 7.339 (4.21); 7.335 (4.46); 7.320 (3.90); 7.315 (3.25); 7.309 (3.11); 7.302 (3.11); 7.298 (3.02); 7.292 (2.43); 7.281 (3.55); 7.258 (3.81); 7.235 (1.62); 5.529 (4.12); 5.393 (1.47); 5.379 (15.00); 5.115 (0.70); 4.799 (2.20); 4.765 (2.27); 4.519 (1.00); 4.504 (0.99); 4.039 (0.78); 4.022 (0.84); 3.494 (1.24); 3.485 (1.10); 3.476 (1.52); 3.467 (2.10); 3.457 (1.57); 3.449 (1.37); 3.438 (1.60); 3.429 (1.24); 3.307 (888.84); 2.674 (1.06); 2.669 (1.26); 2.539 (6.54); 2.505 (128.72); 2.500 (157.21); 2.496 (111.14); 2.331 (0.96); 2.327 (1.18); 2.152 (2.32); 2.125 (2.66); 2.095 (1.61); 1.987 (3.29); 1.800 (0.87); 1.792 (1.06); 1.762 (2.24); 1.739 (2.09); 1.733 (2.00); 1.709 (0.90); 1.398 (1.69); 1.237 (0.88); 1.193 (1.09); 1.175 (1.84); 1.157 (1.12); -0.000 (6.05); |
| I-116 | 9.196 (4.59); 8.527 (12.63); 7.706 (2.75); 7.703 (2.95); 7.686 (3.21); 7.683 (3.25); 7.603 (2.05); 7.599 (2.25); 7.584 (2.72); 7.580 (2.81); 7.470 (1.63); 7.467 (1.72); 7.451 (3.20); 7.448 (3.10); 7.432 (1.90); 7.429 (1.75); 7.357 (1.70); 7.353 (1.81); 7.338 (2.52); 7.333 (2.50); 7.319 (1.25); 7.314 (1.18); 7.274 (0.94); 7.261 (1.09); 7.250 (1.93); 7.238 (1.97); 7.227 (1.31); 7.214 (1.27); 7.194 (0.95); 7.187 (1.20); 7.179 (1.12); 7.171 (2.06); 7.163 (1.19); 7.155 (1.11); 7.148 (1.12); 7.119 (0.84); 7.110 (1.30); 7.098 (1.11); 7.089 (1.66); 7.082 (0.89); 7.067 (0.89); 5.742 (9.77); 5.379 (15.00); 4.802 (1.83); 4.768 (1.96); 3.475 (0.85); 3.465 (1.52); 3.456 (0.84); 3.437 (0.83); 3.334 (2.42); 3.295 (1174.36); 3.257 (1.73); 3.236 (0.86); 2.673 (1.09); 2.669 (1.43); 2.664 (1.04); 2.538 (2.28); 2.522 (4.29); 2.517 (7.02); 2.509 (82.92); 2.504 (168.98); 2.499 (230.04); 2.495 (163.28); 2.490 (76.10); 2.331 (1.23); 2.326 (1.54); 2.321 (1.15); 2.151 (1.79); 2.125 (2.16); 2.067 (3.02); 1.986 (1.32); 1.790 (0.80); 1.760 (1.83); 1.737 (1.78); 1.728 (1.60); 1.425 (1.22); 1.399 (5.61); 1.237 (1.51); 1.175 (0.81); -0.000 (9.51); |
| I-117 | 9.116 (3.53); 8.525 (10.01); 7.706 (2.31); 7.703 (2.45); 7.686 (2.75); 7.683 (2.79); 7.603 (1.77); 7.599 (1.88); 7.584 (2.36); 7.580 (2.38); 7.469 (1.35); 7.466 (1.45); 7.450 (2.73); 7.448 (2.70); 7.431 (1.55); 7.429 (1.53); 7.358 (1.69); 7.354 (1.58); 7.338 (2.21); 7.334 (2.23); 7.319 (1.14); 7.315 (1.13); 7.243 (1.29); 7.223 (1.80); 7.205 (1.52); 7.007 (0.94); 7.000 (1.12); 6.986 (1.74); 6.979 (1.93); 6.965 (0.82); 6.957 (1.01); 6.943 (1.88); 6.936 (1.51); 6.918 (1.88); 6.911 (1.51); 5.532 (0.65); 5.378 (12.81); 4.804 (1.80); 4.771 (1.89); 4.519 (0.80); 4.504 (0.74); 3.490 (0.72); 3.471 (0.82); 3.461 (1.33); 3.451 (0.83); 3.434 (0.77); 3.345 (0.78); 3.337 (0.78); 3.317 (3.52); 3.291 (1058.53); 3.267 (3.93); 3.258 (2.36); 2.673 (1.01); 2.668 (1.35); 2.664 (1.08); 2.538 (11.28); 2.521 (4.58); 2.508 (76.57); 2.503 (154.74); 2.499 (210.42); 2.494 (150.84); 2.490 (71.23); 2.330 (0.90); 2.326 (1.30); 2.321 (0.99); 2.316 (1.04); 2.159 (2.08); 2.130 (15.00); 2.067 (0.69); 1.986 (2.91); 1.781 (0.73); 1.751 (1.58); 1.728 (1.54); 1.722 (1.35); 1.399 (3.33); 1.236 (0.70); 1.193 (0.90); 1.175 (1.75); 1.157 (0.96); -0.000 (9.18); |
| I-118 | 9.297 (2.06); 8.531 (5.62); 7.706 (1.24); 7.704 (1.24); 7.686 (1.83); 7.684 (2.01); 7.678 (0.99); 7.661 (1.31); 7.650 (0.61); 7.640 (0.55); 7.629 (0.61); 7.605 (0.92); 7.600 (0.96); 7.585 (1.18); 7.582 (1.19); 7.470 (1.13); 7.467 (0.83); 7.451 (2.37); 7.449 (2.33); 7.432 (0.94); 7.429 (1.16); 7.358 (0.78); 7.353 (0.76); 7.338 (1.07); 7.334 (1.05); 7.319 (0.54); 7.315 (0.50); 5.380 (6.40); 4.816 (0.78); 4.783 (0.77); 3.505 (0.31); 3.486 (0.41); 3.476 (0.65); 3.466 (0.44); 3.448 (0.38); 3.358 (0.81); 3.328 (1.67); 3.291 (459.03); 3.271 (1.16); 3.261 (0.57); 2.673 (0.45); 2.668 (0.59); 2.664 (0.44); 2.538 (5.25); 2.522 (1.96); 2.517 (3.21); 2.508 (34.42); 2.504 (69.51); 2.499 (93.73); 2.495 (66.13); 2.490 (30.52); 2.331 (0.44); 2.326 (0.59); 2.321 (0.40); 2.172 (0.76); 2.167 (0.76); 2.139 (0.89); 1.986 (0.75); 1.824 (0.31); 1.814 (0.34); 1.790 (0.72); 1.785 (0.79); 1.761 (0.73); 1.755 (0.68); 1.399 (15.00); 1.175 (0.41); -0.000 (3.74); |
| I-119 | 8.770 (2.11); 8.523 (6.32); 7.705 (1.52); 7.702 (1.49); 7.685 (1.69); 7.682 (1.60); 7.600 (1.06); 7.596 (1.14); 7.580 (1.41); 7.577 (1.41); 7.566 (0.53); 7.563 (0.51); 7.546 (0.95); 7.529 (0.45); 7.466 (0.83); 7.464 (0.87); 7.448 (1.71); 7.445 (1.62); 7.429 (0.92); 7.426 (0.85); 7.395 (0.56); 7.357 (0.96); 7.352 (0.91); 7.338 (1.33); 7.333 (1.27); 7.318 (0.72); 7.314 (0.62); 5.545 (0.47); 5.392 (0.67); 5.375 (7.58); 5.365 (0.79); 4.787 (0.99); 4.753 (1.02); 4.518 (1.29); 4.504 (1.25); 3.470 (0.42); 3.460 (0.77); 3.450 (0.46); 3.432 (0.46); 3.333 (1.07); 3.289 (620.66); 3.269 (4.45); 3.239 (0.41); 2.673 (0.67); 2.668 (0.87); 2.664 (0.66); 2.538 (7.70); 2.521 (2.81); 2.516 (4.66); 2.508 (51.62); 2.504 (104.09); 2.499 (140.56); 2.494 (99.54); 2.490 (46.28); 2.434 (2.53); 2.330 (0.68); 2.326 (0.92); 2.321 (0.66); 2.265 (2.57); 2.259 (0.89); 2.203 (12.91); 2.155 (0.95); 2.151 (1.00); 2.118 (1.14); 2.107 (1.40); 2.094 (0.71); 2.063 (15.00); 2.049 (0.65); 1.986 (0.65); 1.770 (0.43); 1.747 (0.89); 1.741 (0.96); 1.717 (0.90); 1.709 (0.83); 1.688 (0.37); 1.399 (0.47); 1.237 (0.41); 1.175 (0.38); 1.159 (0.42); -0.000 (5.64); |
| I-120 | 8.428 (9.21); 7.968 (3.40); 7.549 (0.81); 7.534 (0.93); 7.529 (2.01); 7.513 (2.08); 7.508 (1.50); 7.493 (1.40); 7.439 (2.87); 7.419 (1.79); 7.351 (1.30); 7.349 (1.25); 7.328 (2.24); 7.307 (1.06); 7.304 (0.98); 7.042 (2.42); 7.023 (2.92); 6.998 (3.16); 6.854 (1.88); 6.835 (1.57); 5.742 (9.65); 5.448 (7.40); 5.444 (7.34); 4.163 (1.83); 4.129 (1.92); 4.040 (0.81); 4.022 (0.81); 3.341 (0.42); 3.332 (0.78); 3.322 (0.76); 3.292 (223.01); 2.985 (1.12); 2.980 (1.29); 2.951 (2.35); 2.922 (1.31); 2.669 (0.34); 2.539 (0.94); 2.522 (1.21); 2.509 (19.78); 2.504 (38.85); 2.500 (51.83); 2.495 (36.52); 2.490 (17.07); 2.326 (0.35); 2.230 (15.00); 2.102 (14.84); 2.067 (1.80); 2.058 (1.56); 2.032 (1.79); 1.986 (3.57); 1.908 (1.00); 1.676 (0.58); 1.666 (0.69); 1.645 (1.42); 1.636 (1.54); 1.614 (1.42); 1.606 (1.30); 1.584 (0.54); 1.575 (0.45); 1.193 (0.99); 1.175 (1.93); 1.158 (0.97); -0.000 (3.34); |
| I-121 | 8.439 (9.38); 7.968 (3.40); 7.573 (0.47); 7.556 (1.00); 7.552 (0.92); 7.539 (0.72); 7.535 (1.91); 7.531 (0.72); 7.518 (0.95); 7.514 (1.13); 7.497 (0.51); 7.216 (0.35); 7.212 (0.47); 7.203 (2.85); 7.191 (0.64); 7.183 (4.34); 7.174 (0.65); 7.170 (0.50); 7.162 (2.44); 7.153 (0.39); 7.042 (2.42); 7.023 (2.92); 6.998 (3.17); 6.854 (1.86); 6.835 (1.56); 5.742 (0.62); 5.397 (8.36); 4.162 (1.83); 4.129 (1.91); 4.058 (0.42); 4.040 (1.21); 4.022 (1.21); 4.004 (0.41); 3.341 (0.60); 3.332 (1.03); 3.322 (1.17); 3.293 (382.66); 3.269 (2.79); 2.981 (1.27); 2.952 (2.34); 2.923 (1.31); 2.891 (0.38); 2.673 (0.37); 2.669 (0.50); 2.664 (0.36); 2.538 (1.51); 2.522 (1.80); 2.509 (28.22); 2.504 (55.78); 2.499 (74.64); 2.495 (52.46); 2.490 (24.47); 2.331 (0.36); 2.326 (0.49); 2.322 (0.36); 2.230 (15.00); 2.102 (14.86); 2.067 (2.25); 2.058 (1.53); 2.032 (1.76); 1.986 (5.40); 1.908 (1.26); 1.676 (0.58); 1.666 (0.68); 1.645 (1.41); 1.636 (1.53); 1.614 (1.41); 1.605 (1.30); 1.584 (0.54); 1.575 (0.43); 1.193 (1.52); 1.175 (2.98); 1.158 (1.49); -0.000 (4.09); |
| I-122 | 8.122 (9.67); 7.959 (3.40); 7.307 (2.35); 7.304 (3.14); 7.286 (4.76); 7.236 (2.79); 7.218 (4.85); 7.198 (2.42); 7.134 (1.01); 7.131 (1.68); 7.128 (0.98); 7.113 (2.32); 7.098 (0.58); 7.095 (0.88); 7.041 (2.35); 7.022 (2.84); 6.995 (3.14); 6.853 (1.80); 6.834 (1.49); 5.742 (9.55); 5.100 (0.51); 5.085 (0.99); 5.074 (0.86); 5.058 (0.63); 4.139 (1.61); 4.106 (1.70); 4.040 (1.23); 4.022 (1.23); 3.293 (278.64); 3.269 (1.73); 3.260 (1.03); 3.249 (1.01); 3.240 (1.42); 3.230 (0.87); 3.221 (0.57); 3.211 (0.71); 2.970 (1.23); 2.941 (2.26); 2.912 (1.26); 2.871 (0.57); 2.863 (0.55); 2.842 (1.03); 2.833 (0.91); 2.814 (0.66); 2.805 (0.56); 2.538 (1.01); 2.521 (1.27); 2.508 (21.20); 2.503 (42.12); 2.499 (56.59); 2.494 (40.09); 2.490 (19.01); 2.228 (14.49); 2.101 (15.00); 2.066 (0.68); 2.012 (1.49); 1.986 (7.05); 1.907 (1.02); 1.865 (0.84); 1.858 (0.86); 1.827 (1.68); 1.764 (0.74); 1.734 (0.85); 1.720 (0.80); 1.689 (0.96); 1.681 (0.78); 1.657 (0.92); 1.649 (0.77); 1.627 (0.78); 1.598 (1.41); 1.568 (1.48); 1.538 (1.22); 1.520 (1.79); 1.492 (1.06); 1.398 (0.69); 1.367 (0.52); 1.193 (1.51); 1.175 (2.96); 1.157 (1.48); -0.000 (3.14); |
| I-123 | 8.123 (9.43); 7.958 (3.36); 7.307 (2.35); 7.304 (3.10); 7.286 (4.70); 7.236 (2.78); 7.218 (4.80); 7.198 (2.40); 7.131 (1.63); 7.128 (0.99); 7.113 (2.28); 7.095 (0.86); 7.041 (2.36); 7.022 (2.86); 6.994 (3.11); 6.852 (1.80); 6.833 (1.50); 5.742 (8.26); 5.084 (0.97); 5.073 (0.85); 5.058 (0.64); 4.138 (1.61); 4.105 (1.69); 4.040 (0.94); 4.022 (0.94); 3.337 (0.59); 3.292 (641.54); 3.250 (1.25); 3.240 (1.53); 3.231 (0.96); 3.222 (0.65); 3.211 (0.75); 2.971 (1.26); 2.941 (2.26); 2.912 (1.25); 2.871 (0.56); 2.841 (1.05); 2.834 (0.93); 2.814 (0.64); 2.672 (0.66); 2.668 (0.86); 2.664 (0.62); 2.538 (2.42); 2.521 (3.18); 2.508 (49.05); 2.503 (96.66); 2.499 (129.40); 2.494 (91.33); 2.490 (42.99); 2.330 (0.64); 2.326 (0.85); 2.321 (0.62); 2.228 (14.61); 2.101 (15.00); 2.067 (1.31); 2.017 (1.44); 2.011 (1.49); 1.986 (5.79); 1.907 (1.53); 1.864 (0.87); 1.858 (0.87); 1.827 (1.66); 1.767 (0.74); 1.734 (0.84); 1.689 (0.98); 1.681 (0.80); 1.656 (0.92); 1.648 (0.75); 1.627 (0.81); 1.618 (0.65); 1.597 (1.42); 1.567 (1.46); 1.536 (1.22); 1.519 (1.77); 1.492 (1.04); 1.398 (0.80); 1.193 (1.15); 1.175 (2.32); 1.157 (1.14); -0.000 (6.78); |
| I-124 | 8.142 (15.00); 7.677 (2.49); 7.345 (2.01); 7.341 (2.51); 7.323 (3.68); 7.255 (2.63); 7.239 (3.19); 7.235 (2.97); 7.226 (1.61); 7.218 (1.70); 7.211 (1.77); 7.202 (4.09); 7.196 (6.40); 7.187 (2.69); 7.179 (5.49); 7.166 (2.41); 7.154 (1.76); 7.143 (2.64); 7.131 (2.58); 7.119 (1.90); 7.107 (1.83); 7.002 (1.63); 6.991 (1.40); 6.982 (2.03); 6.200 (1.83); 6.189 (3.84); 6.177 (1.99); 5.444 (9.38); 4.779 (2.62); 4.745 (2.68); 4.069 (2.15); 4.051 (2.18); 3.431 (1.39); 3.420 (2.55); 3.410 (1.41); 3.392 (1.50); 3.353 (2.44); 3.347 (2.43); 3.323 (3.29); 3.318 (4.14); 3.289 (2.49); 3.283 (2.38); 2.889 (2.77); 2.815 (1.61); 2.807 (1.54); 2.205 (2.39); 2.198 (2.43); 2.173 (3.11); 2.165 (2.99); 2.127 (143.78); 2.106 (5.22); 2.095 (5.33); 2.085 (3.39); 2.075 (2.42); 2.015 (1.52); 2.006 (1.57); 1.993 (1.84); 1.970 (11.21); 1.962 (3.86); 1.955 (6.55); 1.950 (49.40); 1.944 (93.24); 1.937 (129.60); 1.931 (88.28); 1.925 (44.59); 1.893 (2.55); 1.882 (2.61); 1.872 (1.79); 1.863 (3.51); 1.859 (2.91); 1.853 (3.84); 1.830 (2.53); 1.824 (2.32); 1.820 (2.15); 1.277 (2.68); 1.221 (2.77); 1.204 (5.43); 1.186 (2.69); 0.008 (1.56); -0.000 (33.31); |
| I-125 | 8.134 (15.00); 7.498 (5.10); 7.493 (5.12); 7.342 (2.08); 7.338 (2.77); 7.320 (3.84); 7.253 (2.73); 7.237 (3.13); 7.234 (2.72); 7.194 (6.10); 7.177 (4.65); 7.164 (4.54); 7.144 (5.00); 7.037 (3.76); 7.031 (3.46); 7.016 (2.70); 7.011 (2.54); 6.812 (2.52); 6.195 (1.96); 6.184 (4.15); 6.173 (2.11); 4.155 (3.49); 4.122 (3.63); 4.069 (2.40); 4.051 (2.44); 3.323 (1.32); 3.304 (1.45); 3.294 (2.73); 3.284 (1.59); 3.265 (1.43); 3.073 (2.52); 3.067 (2.54); 3.038 (4.46); 3.009 (2.45); 3.003 (2.30); 2.930 (1.37); 2.900 (1.48); 2.887 (3.01); 2.828 (1.37); 2.813 (1.61); 2.805 (1.57); 2.791 (1.43); 2.203 (1.90); 2.190 (32.05); 2.145 (3.30); 2.125 (166.53); 2.105 (6.60); 2.092 (5.81); 2.082 (3.44); 2.072 (2.41); 2.024 (1.28); 2.011 (1.45); 2.002 (1.49); 1.989 (1.81); 1.970 (11.70); 1.962 (3.65); 1.955 (7.09); 1.950 (46.23); 1.944 (88.28); 1.937 (126.10); 1.931 (86.45); 1.925 (43.80); 1.890 (1.48); 1.883 (1.67); 1.878 (1.60); 1.869 (1.55); 1.861 (1.28); 1.850 (1.36); 1.806 (1.49); 1.796 (1.53); 1.776 (3.22); 1.773 (3.20); 1.766 (4.22); 1.743 (2.87); 1.733 (2.54); 1.221 (3.05); 1.204 (5.95); 1.186 (2.96); 0.008 (1.60); -0.000 (35.11); |
| I-126 | 8.568 (7.50); 8.113 (2.92); 7.319 (1.11); 7.315 (1.32); 7.297 (2.09); 7.280 (0.59); 7.277 (0.60); 7.262 (1.71); 7.243 (1.61); 7.240 (1.33); 7.201 (1.66); 7.189 (2.29); 7.185 (2.14); 7.171 (1.29); 7.054 (2.05); 7.035 (2.57); 7.019 (2.63); 6.871 (1.56); 6.852 (1.32); 6.154 (1.00); 6.142 (2.09); 6.131 (1.01); 5.742 (15.00); 4.071 (1.35); 4.040 (1.88); 4.022 (1.06); 3.289 (114.88); 3.265 (3.76); 3.233 (1.69); 3.205 (0.89); 3.198 (0.79); 2.894 (0.60); 2.880 (0.32); 2.864 (0.61); 2.851 (1.19); 2.838 (0.55); 2.796 (0.52); 2.782 (0.65); 2.774 (0.65); 2.759 (0.65); 2.739 (0.32); 2.732 (0.37); 2.717 (0.31); 2.521 (0.86); 2.508 (14.93); 2.503 (30.10); 2.499 (40.71); 2.494 (28.98); 2.490 (13.70); 2.285 (0.37); 2.274 (0.38); 2.239 (12.70); 2.218 (0.70); 2.209 (0.52); 2.149 (1.68); 2.109 (13.41); 2.076 (0.73); 2.065 (1.19); 2.053 (1.81); 2.041 (2.08); 2.031 (1.06); 2.015 (0.32); 1.993 (0.35); 1.986 (3.14); 1.975 (0.57); 1.962 (0.64); 1.952 (0.65); 1.940 (0.64); 1.929 (0.40); 1.901 (0.77); 1.858 (0.63); 1.845 (0.85); 1.832 (0.66); 1.825 (0.46); 1.816 (0.42); 1.810 (0.41); 1.193 (0.76); 1.175 (1.51); 1.157 (0.74); -0.000 (4.10); |
| I-127 | 8.584 (6.52); 8.577 (0.52); 7.311 (1.18); 7.307 (1.37); 7.299 (0.47); 7.290 (2.17); 7.281 (0.71); 7.277 (0.70); 7.262 (1.91); 7.244 (1.76); 7.240 (1.42); 7.201 (1.68); 7.189 (2.70); 7.169 (1.49); 6.491 (3.89); 6.156 (0.96); 6.145 (1.91); 6.134 (0.93); 5.743 (9.24); 5.415 (0.72); 5.372 (1.79); 5.288 (1.67); 5.246 (0.67); 4.311 (0.57); 4.279 (0.64); 4.040 (1.24); 4.022 (1.24); 3.931 (0.55); 3.898 (0.62); 3.479 (0.45); 3.449 (0.77); 3.420 (0.47); 3.321 (0.52); 3.289 (329.26); 3.265 (7.96); 3.060 (0.65); 2.891 (0.62); 2.862 (0.64); 2.849 (1.21); 2.836 (0.57); 2.794 (0.56); 2.780 (0.73); 2.772 (0.68); 2.757 (0.72); 2.673 (0.46); 2.668 (0.63); 2.664 (0.44); 2.538 (0.67); 2.521 (2.22); 2.516 (3.45); 2.508 (34.02); 2.503 (67.93); 2.499 (91.69); 2.494 (64.68); 2.490 (30.04); 2.330 (0.47); 2.326 (0.61); 2.321 (0.46); 2.216 (15.00); 2.197 (1.54); 2.169 (1.31); 2.073 (0.75); 2.067 (1.08); 2.062 (1.31); 2.050 (2.13); 2.039 (2.28); 1.986 (5.69); 1.977 (0.49); 1.968 (0.57); 1.955 (0.65); 1.944 (0.65); 1.932 (0.67); 1.855 (0.66); 1.842 (0.90); 1.829 (0.68); 1.822 (0.54); 1.193 (1.54); 1.175 (3.06); 1.157 (1.48); -0.000 (4.58); |
| I-128 | 8.578 (9.40); 7.459 (7.40); 7.453 (4.53); 7.437 (5.99); 7.391 (1.12); 7.382 (1.16); 7.368 (1.58); 7.362 (2.95); 7.356 (2.51); 7.341 (1.86); 7.334 (1.68); 7.314 (1.62); 7.310 (1.97); 7.292 (3.01); 7.282 (0.97); 7.264 (2.50); 7.245 (2.35); 7.242 (1.96); 7.203 (2.38); 7.191 (3.30); 7.187 (3.13); 7.173 (1.93); 7.135 (1.58); 7.131 (2.13); 7.122 (2.87); 7.114 (2.15); 7.109 (2.05); 7.047 (1.34); 7.034 (0.97); 6.157 (1.32); 6.146 (2.73); 6.135 (1.32); 5.742 (15.00); 5.034 (3.70); 5.020 (3.95); 4.549 (0.98); 4.040 (1.27); 4.022 (1.58); 3.933 (3.06); 3.905 (2.83); 3.290 (275.53); 3.266 (6.59); 2.852 (1.63); 2.782 (0.99); 2.760 (1.12); 2.673 (0.98); 2.659 (0.97); 2.522 (1.71); 2.517 (2.68); 2.508 (28.34); 2.504 (57.02); 2.499 (77.21); 2.494 (54.56); 2.490 (25.55); 2.185 (0.98); 2.146 (1.60); 2.112 (0.99); 2.064 (1.73); 2.051 (2.62); 2.040 (2.91); 2.025 (1.62); 1.986 (4.34); 1.906 (1.01); 1.900 (1.22); 1.895 (1.15); 1.893 (1.14); 1.880 (1.51); 1.874 (1.54); 1.870 (1.67); 1.864 (0.98); 1.858 (1.52); 1.846 (1.33); 1.833 (1.13); 1.678 (1.18); 1.671 (1.22); 1.654 (1.11); 1.193 (1.09); 1.175 (2.15); 1.157 (1.03); -0.000 (3.31); |
| I-129 | 8.515 (5.39); 7.912 (2.05); 7.803 (1.02); 7.704 (1.29); 7.701 (1.34); 7.684 (1.46); 7.681 (1.46); 7.599 (0.96); 7.595 (1.02); 7.580 (1.29); 7.577 (1.31); 7.467 (0.73); 7.464 (0.75); 7.448 (1.50); 7.445 (1.47); 7.429 (0.81); 7.426 (0.78); 7.356 (0.81); 7.352 (0.84); 7.337 (1.15); 7.333 (1.17); 7.318 (0.55); 7.313 (0.53); 5.743 (3.41); 5.373 (6.89); 4.138 (1.09); 4.104 (1.15); 4.040 (0.65); 4.022 (0.65); 3.351 (0.37); 3.341 (0.30); 3.332 (0.50); 3.323 (0.88); 3.313 (0.77); 3.288 (119.84); 3.003 (0.76); 2.974 (1.41); 2.945 (0.77); 2.538 (1.88); 2.521 (0.89); 2.508 (15.29); 2.504 (30.69); 2.499 (41.49); 2.494 (29.64); 2.490 (14.09); 2.359 (0.44); 2.268 (1.20); 2.261 (8.33); 2.220 (12.31); 2.148 (0.43); 2.108 (1.48); 2.102 (10.51); 2.084 (1.02); 2.067 (0.85); 2.052 (15.00); 1.986 (2.84); 1.687 (0.34); 1.677 (0.39); 1.656 (0.85); 1.647 (0.91); 1.625 (0.86); 1.617 (0.80); 1.595 (0.32); 1.406 (0.39); 1.193 (0.81); 1.175 (1.56); 1.157 (0.78); 0.008 (0.31); -0.000 (7.87); |
| I-130 | 8.195 (0.82); 8.122 (2.48); 7.306 (0.60); 7.303 (0.80); 7.285 (1.21); 7.247 (0.39); 7.236 (0.81); 7.228 (0.49); 7.217 (1.29); 7.198 (0.62); 7.130 (0.42); 7.112 (0.59); 7.053 (0.39); 7.032 (0.52); 7.026 (0.42); 7.005 (0.50); 5.743 (0.36); 4.136 (0.42); 4.104 (0.44); 3.287 (86.69); 3.263 (0.35); 3.234 (0.34); 2.976 (0.32); 2.946 (0.58); 2.918 (0.32); 2.538 (0.91); 2.521 (0.58); 2.508 (9.07); 2.503 (17.98); 2.499 (24.14); 2.494 (17.17); 2.490 (8.05); 2.242 (3.44); 2.013 (0.37); 1.986 (1.05); 1.907 (0.40); 1.826 (0.43); 1.596 (0.35); 1.567 (0.37); 1.519 (0.45); 1.399 (15.00); 1.175 (0.36); -0.000 (1.38); |
| I-131 | 8.418 (4.55); 8.126 (11.45); 7.934 (3.43); 7.929 (3.43); 7.701 (2.50); 7.680 (3.02); 7.476 (1.99); 7.471 (1.99); 7.455 (1.67); 7.451 (1.65); 7.305 (2.83); 7.301 (3.79); 7.284 (5.75); 7.234 (3.38); 7.215 (5.84); 7.196 (2.95); 7.129 (2.00); 7.126 (1.21); 7.110 (2.85); 7.092 (1.04); 5.111 (0.53); 5.100 (0.62); 5.085 (1.17); 5.074 (1.05); 5.059 (0.79); 4.150 (1.93); 4.115 (2.05); 3.568 (15.00); 3.329 (0.71); 3.288 (914.49); 3.240 (1.49); 3.047 (1.52); 3.017 (2.75); 2.988 (1.55); 2.984 (1.32); 2.869 (0.71); 2.860 (0.70); 2.839 (1.29); 2.832 (1.14); 2.812 (0.81); 2.803 (0.72); 2.673 (0.98); 2.668 (1.35); 2.664 (0.94); 2.538 (7.36); 2.521 (4.33); 2.508 (75.42); 2.503 (150.61); 2.499 (202.57); 2.494 (144.57); 2.490 (68.71); 2.335 (0.63); 2.330 (1.12); 2.326 (1.49); 2.321 (1.04); 2.125 (1.15); 2.115 (1.20); 2.103 (1.00); 2.067 (0.67); 2.048 (2.62); 2.042 (1.86); 2.016 (2.13); 1.986 (0.67); 1.864 (1.01); 1.858 (1.02); 1.826 (2.10); 1.764 (1.01); 1.733 (1.07); 1.687 (1.37); 1.679 (1.44); 1.646 (2.39); 1.615 (1.92); 1.586 (0.71); 1.517 (2.13); 1.490 (1.29); 1.399 (7.35); 1.383 (0.59); 1.367 (0.69); 1.237 (0.58); -0.000 (6.91); |
| I-132 | 8.120 (5.39); 7.591 (2.03); 7.475 (1.71); 7.470 (1.70); 7.303 (1.35); 7.300 (1.78); 7.282 (2.72); 7.233 (1.62); 7.215 (2.78); 7.195 (1.38); 7.127 (0.96); 7.124 (0.58); 7.114 (0.52); 7.109 (1.33); 7.094 (0.33); 7.091 (0.50); 6.871 (1.46); 6.851 (2.59); 6.805 (1.15); 6.801 (1.12); 6.784 (0.63); 6.780 (0.62); 5.084 (0.56); 5.073 (0.49); 5.059 (0.35); 4.093 (0.93); 4.059 (0.98); 3.763 (14.12); 3.568 (15.00); 3.288 (239.12); 3.260 (0.80); 3.249 (0.55); 3.240 (0.63); 3.230 (0.85); 3.220 (0.51); 3.211 (0.35); 3.201 (0.44); 2.989 (0.63); 2.983 (0.71); 2.954 (1.29); 2.925 (0.73); 2.869 (0.33); 2.860 (0.31); 2.838 (0.60); 2.831 (0.54); 2.811 (0.39); 2.803 (0.33); 2.668 (0.37); 2.538 (2.01); 2.521 (1.37); 2.508 (20.95); 2.503 (41.27); 2.499 (55.16); 2.494 (39.14); 2.490 (18.44); 2.325 (0.38); 2.205 (9.57); 2.113 (0.55); 2.103 (0.46); 2.048 (0.31); 2.020 (0.83); 2.014 (0.85); 1.986 (1.02); 1.856 (0.48); 1.824 (0.96); 1.762 (0.44); 1.731 (0.49); 1.685 (0.52); 1.677 (0.42); 1.652 (0.53); 1.645 (0.61); 1.612 (0.85); 1.577 (0.78); 1.540 (0.63); 1.528 (0.58); 1.517 (1.01); 1.490 (0.61); 1.399 (2.88); 1.364 (0.31); -0.000 (2.38); |
| I-133 | 8.125 (8.60); 8.092 (3.07); 7.325 (2.93); 7.319 (3.07); 7.306 (2.08); 7.302 (2.79); 7.284 (4.17); 7.235 (2.44); 7.216 (4.28); 7.196 (2.64); 7.193 (2.47); 7.172 (2.71); 7.130 (1.49); 7.127 (0.93); 7.117 (0.78); 7.112 (2.04); 7.094 (0.79); 7.076 (2.07); 7.071 (1.96); 7.056 (1.47); 7.050 (1.41); 5.084 (0.89); 5.073 (0.76); 5.058 (0.56); 4.137 (1.40); 4.103 (1.49); 3.289 (637.08); 3.260 (1.98); 3.250 (1.91); 3.240 (1.18); 3.230 (0.78); 3.221 (0.90); 3.211 (0.56); 2.999 (1.16); 2.969 (2.02); 2.941 (1.12); 2.869 (0.51); 2.839 (0.92); 2.832 (0.83); 2.812 (0.59); 2.803 (0.50); 2.673 (0.62); 2.668 (0.86); 2.663 (0.62); 2.538 (4.70); 2.521 (2.58); 2.508 (47.72); 2.503 (96.15); 2.499 (130.08); 2.494 (93.40); 2.490 (44.63); 2.330 (0.70); 2.326 (0.92); 2.321 (0.67); 2.145 (15.00); 2.126 (0.92); 2.115 (0.92); 2.105 (0.75); 2.048 (0.78); 2.031 (1.29); 2.025 (1.31); 1.998 (1.54); 1.986 (1.96); 1.858 (0.73); 1.826 (1.50); 1.765 (0.68); 1.733 (0.77); 1.719 (0.71); 1.688 (0.80); 1.679 (0.64); 1.654 (0.91); 1.646 (0.96); 1.613 (1.39); 1.583 (1.28); 1.543 (0.86); 1.518 (1.54); 1.491 (0.93); 1.399 (4.23); 1.175 (0.60); -0.000 (5.15); |
| I-134 | 8.122 (6.98); 8.104 (2.73); 7.316 (2.32); 7.311 (2.62); 7.306 (4.50); 7.302 (2.72); 7.286 (5.87); 7.235 (2.11); 7.216 (3.63); 7.197 (1.82); 7.129 (1.26); 7.111 (1.74); 7.093 (0.66); 6.955 (1.25); 6.951 (1.24); 6.935 (1.11); 6.931 (1.09); 5.110 (0.34); 5.099 (0.38); 5.083 (0.76); 5.073 (0.64); 5.058 (0.47); 5.046 (0.32); 4.135 (1.20); 4.102 (1.29); 3.567 (15.00); 3.287 (490.18); 3.252 (1.64); 3.243 (0.99); 3.232 (0.67); 3.224 (0.78); 3.002 (0.96); 2.973 (1.74); 2.944 (1.00); 2.870 (0.43); 2.861 (0.41); 2.840 (0.80); 2.833 (0.70); 2.813 (0.51); 2.804 (0.43); 2.672 (0.59); 2.668 (0.78); 2.663 (0.56); 2.538 (4.23); 2.521 (2.55); 2.508 (44.52); 2.503 (88.96); 2.499 (120.03); 2.494 (85.92); 2.490 (41.22); 2.335 (0.37); 2.330 (0.63); 2.326 (0.83); 2.321 (0.61); 2.264 (12.39); 2.115 (0.74); 2.104 (0.66); 2.048 (0.63); 2.019 (1.14); 1.992 (1.32); 1.986 (1.52); 1.858 (0.63); 1.826 (1.29); 1.765 (0.58); 1.731 (0.66); 1.688 (0.70); 1.680 (0.57); 1.656 (0.89); 1.648 (0.92); 1.617 (1.25); 1.587 (1.06); 1.540 (0.66); 1.518 (1.33); 1.491 (0.81); 1.399 (2.75); 1.374 (0.35); 1.366 (0.43); -0.000 (4.23); |
| I-135 | 8.512 (9.04); 7.975 (3.47); 7.613 (1.54); 7.607 (1.06); 7.599 (1.59); 7.589 (1.97); 7.540 (1.55); 7.534 (1.19); 7.531 (1.19); 7.526 (0.97); 7.521 (1.29); 7.516 (2.35); 7.443 (0.34); 7.437 (0.71); 7.425 (2.68); 7.419 (3.97); 7.411 (4.75); 7.401 (2.76); 7.397 (2.30); 7.384 (0.50); 7.044 (2.43); 7.025 (2.95); 7.003 (3.32); 6.856 (1.92); 6.837 (1.60); 5.743 (5.00); 5.411 (12.22); 4.172 (1.89); 4.139 (1.96); 4.058 (0.56); 4.040 (1.68); 4.022 (1.69); 4.004 (0.56); 3.361 (0.39); 3.352 (0.68); 3.342 (0.52); 3.332 (0.89); 3.323 (1.50); 3.313 (1.14); 3.289 (125.26); 2.996 (1.31); 2.967 (2.42); 2.958 (1.91); 2.939 (1.32); 2.538 (1.35); 2.521 (0.92); 2.508 (13.89); 2.504 (27.45); 2.499 (36.64); 2.494 (26.07); 2.490 (12.26); 2.286 (0.44); 2.232 (15.00); 2.108 (14.99); 2.085 (1.58); 2.079 (1.62); 2.067 (1.05); 2.052 (1.81); 2.049 (1.83); 1.986 (7.46); 1.698 (0.56); 1.688 (0.68); 1.667 (1.42); 1.658 (1.56); 1.636 (1.44); 1.628 (1.30); 1.606 (0.53); 1.597 (0.43); 1.237 (0.32); 1.193 (2.03); 1.175 (4.06); 1.158 (1.96); -0.000 (3.11); |
| I-136 | 8.507 (9.48); 8.314 (0.32); 8.103 (0.32); 8.087 (0.33); 7.976 (3.52); 7.942 (2.37); 7.939 (2.38); 7.922 (2.51); 7.919 (2.41); 7.555 (1.50); 7.551 (1.60); 7.536 (2.40); 7.532 (2.31); 7.476 (1.51); 7.473 (1.50); 7.457 (2.49); 7.454 (2.40); 7.438 (1.14); 7.435 (1.08); 7.165 (1.22); 7.161 (1.25); 7.146 (2.00); 7.142 (1.97); 7.127 (1.08); 7.123 (1.01); 7.044 (2.44); 7.025 (2.94); 7.003 (3.31); 6.856 (1.90); 6.837 (1.59); 6.590 (0.37); 6.574 (0.36); 5.742 (12.57); 5.301 (11.40); 4.174 (1.86); 4.140 (1.95); 4.040 (0.43); 4.022 (0.44); 3.364 (0.34); 3.355 (0.67); 3.345 (0.51); 3.335 (0.82); 3.326 (1.45); 3.316 (1.09); 3.289 (166.79); 3.004 (1.13); 2.999 (1.31); 2.969 (2.38); 2.945 (5.26); 2.668 (0.31); 2.538 (1.71); 2.521 (1.14); 2.508 (17.72); 2.503 (35.03); 2.499 (46.82); 2.494 (33.21); 2.490 (15.62); 2.326 (0.32); 2.286 (0.51); 2.232 (15.00); 2.109 (15.00); 2.089 (1.63); 2.083 (1.63); 2.067 (1.45); 2.056 (1.80); 2.049 (1.80); 1.986 (1.95); 1.701 (0.57); 1.691 (0.68); 1.670 (1.41); 1.661 (1.54); 1.639 (1.43); 1.631 (1.31); 1.609 (0.55); 1.599 (0.44); 1.193 (0.53); 1.175 (1.06); 1.157 (0.51); -0.000 (6.32); |
| I-137 | 8.422 (8.73); 8.314 (0.42); 7.968 (3.42); 7.589 (3.76); 7.586 (3.99); 7.567 (7.88); 7.505 (3.57); 7.487 (2.62); 7.483 (2.21); 7.465 (1.58); 7.041 (2.35); 7.022 (2.85); 6.998 (3.21); 6.853 (1.84); 6.835 (1.55); 5.743 (12.06); 5.530 (12.99); 4.165 (1.87); 4.131 (1.91); 4.040 (0.59); 4.022 (0.59); 3.343 (0.38); 3.333 (0.65); 3.324 (0.59); 3.314 (1.07); 3.287 (167.35); 3.028 (0.94); 2.983 (1.15); 2.979 (1.30); 2.949 (2.34); 2.920 (1.27); 2.668 (0.35); 2.538 (1.81); 2.521 (1.16); 2.508 (19.55); 2.504 (39.08); 2.499 (52.80); 2.494 (37.83); 2.490 (18.14); 2.326 (0.37); 2.286 (0.71); 2.230 (15.00); 2.120 (1.00); 2.102 (14.59); 2.067 (1.84); 2.059 (1.56); 2.049 (0.92); 2.032 (1.76); 1.986 (2.62); 1.676 (0.56); 1.666 (0.65); 1.645 (1.38); 1.636 (1.51); 1.614 (1.40); 1.605 (1.30); 1.584 (0.55); 1.575 (0.44); 1.193 (0.71); 1.175 (1.41); 1.158 (0.71); -0.000 (5.41); |
| I-138 | 8.343 (5.64); 7.971 (2.05); 7.361 (0.57); 7.344 (0.70); 7.340 (1.25); 7.323 (1.25); 7.319 (0.80); 7.302 (0.66); 7.043 (1.43); 7.024 (1.73); 6.999 (1.92); 6.911 (1.60); 6.890 (1.45); 6.855 (1.12); 6.832 (1.57); 6.809 (0.86); 6.805 (0.93); 6.784 (0.74); 6.431 (0.35); 6.413 (1.07); 6.397 (1.07); 6.380 (0.34); 5.743 (3.05); 4.162 (1.11); 4.129 (1.14); 4.040 (0.85); 4.022 (0.80); 3.860 (15.00); 3.333 (0.65); 3.287 (276.11); 2.994 (0.77); 2.964 (1.39); 2.936 (0.77); 2.672 (0.36); 2.668 (0.48); 2.663 (0.35); 2.538 (2.67); 2.521 (1.82); 2.508 (26.61); 2.503 (52.24); 2.499 (69.52); 2.494 (49.14); 2.490 (22.93); 2.330 (0.34); 2.326 (0.45); 2.321 (0.31); 2.231 (8.68); 2.106 (8.77); 2.067 (1.35); 2.058 (0.90); 2.049 (0.67); 2.033 (1.03); 1.986 (3.43); 1.678 (0.35); 1.669 (0.42); 1.647 (5.47); 1.630 (4.98); 1.617 (0.88); 1.608 (0.76); 1.587 (0.30); 1.237 (0.52); 1.193 (0.96); 1.175 (1.90); 1.157 (0.94); -0.000 (5.49); |
| I-139 | 8.679 (5.60); 8.627 (9.22); 8.580 (3.98); 8.567 (4.06); 8.106 (0.37); 8.089 (0.39); 7.981 (3.33); 7.591 (2.81); 7.590 (2.76); 7.578 (2.72); 7.046 (2.33); 7.027 (2.84); 7.004 (3.13); 6.858 (1.77); 6.839 (1.48); 6.603 (0.38); 6.587 (0.36); 5.743 (4.12); 5.450 (10.67); 4.179 (1.83); 4.145 (1.90); 4.040 (0.70); 4.022 (0.72); 3.381 (0.46); 3.371 (0.76); 3.361 (0.64); 3.352 (0.98); 3.342 (1.62); 3.333 (1.13); 3.286 (466.24); 3.015 (1.11); 3.010 (1.28); 2.981 (2.30); 2.953 (6.23); 2.677 (0.35); 2.672 (0.65); 2.668 (0.87); 2.663 (0.63); 2.538 (4.96); 2.521 (3.49); 2.508 (48.75); 2.503 (95.63); 2.499 (127.26); 2.494 (89.58); 2.490 (41.56); 2.335 (0.31); 2.330 (0.61); 2.326 (0.82); 2.321 (0.59); 2.233 (14.08); 2.111 (15.00); 2.067 (3.13); 2.049 (0.76); 1.986 (3.13); 1.714 (0.54); 1.704 (0.65); 1.683 (1.36); 1.674 (1.47); 1.652 (1.39); 1.644 (1.27); 1.623 (0.51); 1.613 (0.42); 1.193 (0.88); 1.175 (1.71); 1.157 (0.87); 0.008 (0.50); -0.000 (11.00); -0.008 (0.35); |
| I-140 | 8.483 (9.47); 7.971 (3.44); 7.581 (0.87); 7.577 (0.93); 7.563 (1.73); 7.558 (1.84); 7.543 (0.98); 7.539 (1.00); 7.474 (0.47); 7.469 (0.48); 7.460 (0.55); 7.455 (1.12); 7.440 (1.00); 7.435 (1.26); 7.430 (0.71); 7.421 (0.69); 7.416 (0.60); 7.283 (1.45); 7.269 (1.63); 7.267 (1.95); 7.259 (1.77); 7.256 (1.67); 7.251 (2.89); 7.248 (2.33); 7.239 (1.16); 7.236 (1.26); 7.232 (1.43); 7.229 (1.03); 7.043 (2.45); 7.024 (2.95); 7.001 (3.24); 6.855 (1.88); 6.836 (1.58); 5.743 (4.60); 5.385 (9.04); 4.167 (1.88); 4.134 (1.96); 4.040 (0.62); 4.022 (0.62); 3.351 (0.40); 3.342 (0.72); 3.332 (0.61); 3.322 (1.03); 3.313 (1.86); 3.288 (237.25); 2.995 (1.15); 2.990 (1.32); 2.965 (3.60); 2.932 (1.34); 2.927 (1.12); 2.775 (0.65); 2.668 (0.43); 2.538 (2.45); 2.521 (1.59); 2.508 (24.66); 2.503 (48.75); 2.499 (65.21); 2.494 (46.16); 2.490 (21.61); 2.330 (0.36); 2.326 (0.45); 2.286 (0.37); 2.231 (14.95); 2.105 (15.00); 2.067 (2.16); 2.043 (1.80); 1.986 (2.79); 1.688 (0.58); 1.678 (0.68); 1.657 (1.43); 1.648 (1.55); 1.626 (1.44); 1.618 (1.31); 1.596 (0.55); 1.587 (0.45); 1.237 (0.50); 1.193 (0.77); 1.175 (1.54); 1.157 (0.77); -0.000 (7.31); |
| I-141 | 8.861 (8.05); 7.990 (3.16); 7.597 (1.60); 7.595 (1.66); 7.577 (1.82); 7.575 (1.78); 7.388 (1.58); 7.386 (1.56); 7.369 (1.97); 7.218 (2.12); 7.199 (3.27); 7.179 (1.60); 7.049 (2.17); 7.029 (2.66); 7.012 (2.95); 6.859 (1.68); 6.839 (1.39); 5.742 (3.25); 4.201 (1.65); 4.167 (1.74); 4.058 (0.76); 4.040 (2.27); 4.022 (2.31); 4.004 (0.78); 3.413 (0.53); 3.404 (0.37); 3.394 (0.63); 3.384 (1.11); 3.375 (0.66); 3.364 (0.45); 3.355 (0.63); 3.346 (0.40); 3.290 (238.65); 3.023 (1.17); 2.994 (2.12); 2.964 (1.17); 2.668 (0.35); 2.538 (1.86); 2.521 (1.22); 2.508 (19.58); 2.503 (38.78); 2.499 (51.97); 2.494 (37.00); 2.490 (17.52); 2.326 (0.36); 2.235 (13.17); 2.206 (15.00); 2.136 (1.58); 2.119 (14.23); 2.067 (0.54); 1.986 (10.10); 1.749 (0.49); 1.739 (0.59); 1.718 (1.24); 1.709 (1.37); 1.687 (1.29); 1.679 (1.20); 1.658 (0.51); 1.648 (0.40); 1.237 (0.38); 1.193 (2.73); 1.175 (5.46); 1.157 (2.65); -0.000 (5.43); |
| I-142 | 8.794 (5.58); 7.989 (2.21); 7.319 (0.78); 7.315 (0.99); 7.300 (2.21); 7.295 (2.27); 7.283 (1.58); 7.263 (2.85); 7.243 (1.59); 7.230 (2.03); 7.225 (1.92); 7.209 (0.92); 7.205 (0.73); 7.048 (1.53); 7.029 (1.87); 7.013 (2.05); 6.858 (1.17); 6.839 (0.95); 5.743 (5.57); 4.200 (1.17); 4.167 (1.22); 4.057 (0.35); 4.040 (1.01); 4.022 (1.04); 4.004 (0.35); 3.800 (15.00); 3.407 (0.38); 3.387 (0.46); 3.378 (0.81); 3.369 (0.49); 3.359 (0.35); 3.349 (0.50); 3.339 (0.32); 3.286 (201.83); 3.021 (0.82); 2.991 (1.50); 2.963 (0.83); 2.668 (0.38); 2.538 (1.64); 2.521 (1.42); 2.507 (21.49); 2.503 (42.51); 2.499 (56.90); 2.494 (40.48); 2.489 (19.08); 2.325 (0.37); 2.235 (9.09); 2.119 (9.73); 1.986 (4.47); 1.744 (0.35); 1.735 (0.41); 1.712 (0.88); 1.705 (0.96); 1.683 (0.89); 1.675 (0.81); 1.652 (0.33); 1.193 (1.23); 1.175 (2.44); 1.157 (1.21); 1.044 (0.40); 1.029 (0.38); -0.000 (3.22); |
| I-143 | 8.863 (7.89); 7.990 (3.08); 7.467 (1.41); 7.464 (1.50); 7.447 (1.76); 7.444 (1.79); 7.359 (1.26); 7.358 (1.28); 7.340 (2.11); 7.288 (2.50); 7.269 (3.28); 7.249 (1.31); 7.048 (2.11); 7.029 (2.60); 7.013 (2.88); 6.858 (1.63); 6.840 (1.36); 5.742 (1.92); 4.201 (1.64); 4.167 (1.72); 4.058 (0.45); 4.040 (1.34); 4.022 (1.34); 4.004 (0.46); 3.413 (0.54); 3.404 (0.38); 3.394 (0.65); 3.384 (1.11); 3.375 (0.66); 3.365 (0.45); 3.356 (0.63); 3.346 (0.38); 3.290 (157.62); 3.023 (1.14); 2.994 (2.08); 2.964 (1.15); 2.538 (1.07); 2.521 (0.92); 2.508 (14.06); 2.503 (27.78); 2.499 (37.08); 2.494 (26.42); 2.490 (12.49); 2.235 (12.81); 2.204 (15.00); 2.134 (1.60); 2.119 (13.82); 1.986 (5.97); 1.749 (0.49); 1.739 (0.57); 1.718 (1.22); 1.709 (1.35); 1.687 (1.26); 1.679 (1.17); 1.658 (0.48); 1.648 (0.39); 1.193 (1.61); 1.175 (3.22); 1.157 (1.56); -0.000 (1.18); |
| I-144 | 8.916 (8.28); 7.990 (3.29); 7.821 (8.83); 7.801 (9.56); 7.283 (2.59); 7.263 (4.24); 7.243 (2.28); 7.048 (2.24); 7.029 (2.77); 7.014 (3.03); 6.858 (1.71); 6.856 (1.69); 6.839 (1.44); 5.743 (1.38); 4.202 (1.72); 4.169 (1.81); 3.420 (0.55); 3.411 (0.43); 3.401 (0.67); 3.391 (1.16); 3.382 (0.70); 3.372 (0.46); 3.363 (0.66); 3.353 (0.37); 3.286 (185.24); 3.021 (1.22); 2.992 (2.20); 2.963 (1.22); 2.668 (0.39); 2.538 (1.67); 2.521 (1.49); 2.508 (21.68); 2.503 (42.79); 2.499 (57.19); 2.494 (40.70); 2.490 (19.17); 2.325 (0.38); 2.235 (13.46); 2.140 (1.60); 2.120 (15.00); 1.986 (0.33); 1.750 (0.50); 1.740 (0.60); 1.719 (1.30); 1.710 (1.40); 1.688 (1.32); 1.680 (1.22); 1.658 (0.50); 1.649 (0.40); -0.000 (5.08); |
| I-145 | 8.796 (5.52); 7.988 (2.14); 7.353 (0.55); 7.337 (0.67); 7.332 (1.21); 7.315 (1.22); 7.310 (0.75); 7.294 (0.67); 7.064 (1.46); 7.047 (1.97); 7.043 (1.56); 7.028 (2.48); 7.012 (2.14); 7.006 (1.34); 7.003 (1.55); 6.982 (0.73); 6.979 (0.66); 6.858 (1.14); 6.839 (0.94); 5.742 (0.55); 4.197 (1.13); 4.163 (1.19); 3.820 (15.00); 3.406 (0.37); 3.387 (0.45); 3.377 (0.76); 3.368 (0.45); 3.358 (0.32); 3.348 (0.48); 3.339 (0.30); 3.288 (263.50); 3.020 (0.79); 2.991 (1.45); 2.962 (0.81); 2.672 (0.30); 2.668 (0.41); 2.538 (1.77); 2.521 (1.41); 2.508 (23.71); 2.503 (47.25); 2.499 (63.43); 2.494 (45.27); 2.490 (21.44); 2.330 (0.33); 2.326 (0.41); 2.234 (8.84); 2.118 (9.58); 2.102 (1.22); 2.067 (0.32); 1.986 (0.71); 1.743 (0.35); 1.733 (0.39); 1.711 (0.84); 1.702 (0.93); 1.680 (0.87); 1.673 (0.79); 1.651 (0.35); 1.175 (0.37); -0.000 (2.36); |
| I-146 | 8.803 (8.64); 7.990 (3.39); 7.785 (2.23); 7.781 (2.30); 7.765 (2.43); 7.761 (2.38); 7.523 (0.85); 7.519 (0.83); 7.502 (2.03); 7.499 (1.80); 7.484 (2.02); 7.481 (1.92); 7.459 (2.91); 7.455 (3.31); 7.439 (1.59); 7.434 (1.31); 7.319 (1.50); 7.315 (1.40); 7.301 (1.78); 7.299 (1.85); 7.296 (1.76); 7.281 (1.23); 7.277 (1.08); 7.048 (2.34); 7.029 (2.86); 7.012 (3.14); 6.858 (1.81); 6.839 (1.50); 5.742 (10.21); 4.198 (1.80); 4.165 (1.87); 4.057 (0.32); 4.040 (0.95); 4.022 (0.97); 4.004 (0.33); 3.421 (0.32); 3.412 (0.59); 3.402 (0.45); 3.392 (0.71); 3.383 (1.24); 3.373 (0.74); 3.364 (0.50); 3.354 (0.72); 3.345 (0.42); 3.289 (127.02); 3.029 (1.09); 3.024 (1.24); 2.994 (2.28); 2.966 (1.25); 2.538 (1.49); 2.521 (0.99); 2.508 (14.70); 2.503 (28.86); 2.499 (38.41); 2.494 (27.13); 2.490 (12.63); 2.235 (14.04); 2.119 (15.00); 2.105 (1.91); 2.097 (1.95); 1.986 (4.18); 1.907 (0.30); 1.746 (0.54); 1.736 (0.63); 1.714 (1.34); 1.706 (1.48); 1.684 (1.38); 1.676 (1.26); 1.654 (0.53); 1.645 (0.42); 1.237 (0.74); 1.193 (1.16); 1.175 (2.29); 1.157 (1.14); -0.000 (2.85); |
| I-147 | 8.804 (8.81); 7.988 (3.32); 7.472 (0.92); 7.467 (1.01); 7.457 (0.97); 7.452 (2.35); 7.448 (2.14); 7.437 (1.67); 7.432 (2.78); 7.428 (1.87); 7.411 (1.48); 7.406 (1.90); 7.401 (0.82); 7.393 (0.83); 7.387 (1.40); 7.383 (1.01); 7.374 (1.21); 7.370 (1.06); 7.362 (0.51); 7.354 (0.54); 7.349 (0.42); 7.324 (1.30); 7.322 (1.29); 7.319 (1.41); 7.304 (1.68); 7.299 (1.67); 7.284 (0.65); 7.281 (0.63); 7.048 (2.31); 7.029 (2.84); 7.011 (3.07); 6.858 (1.77); 6.839 (1.46); 5.743 (6.54); 4.196 (1.79); 4.162 (1.87); 4.040 (1.00); 4.022 (1.00); 3.410 (0.68); 3.401 (0.52); 3.391 (0.83); 3.381 (1.33); 3.372 (0.84); 3.362 (0.66); 3.353 (0.87); 3.344 (0.64); 3.288 (406.51); 3.022 (1.24); 2.993 (2.25); 2.964 (1.24); 2.672 (0.49); 2.668 (0.66); 2.663 (0.49); 2.538 (3.74); 2.521 (2.68); 2.508 (36.53); 2.503 (70.91); 2.499 (93.62); 2.494 (65.50); 2.490 (30.13); 2.330 (0.44); 2.326 (0.59); 2.321 (0.42); 2.234 (13.93); 2.118 (15.00); 2.100 (1.85); 2.048 (0.44); 1.986 (4.38); 1.744 (0.54); 1.734 (0.63); 1.713 (1.36); 1.704 (1.45); 1.682 (1.38); 1.674 (1.24); 1.653 (0.52); 1.237 (0.61); 1.193 (1.20); 1.175 (2.42); 1.157 (1.19); -0.000 (3.75); |
| I-148 | 8.806 (8.68); 7.990 (3.37); 7.649 (2.49); 7.630 (2.45); 7.481 (0.46); 7.473 (4.66); 7.471 (4.97); 7.461 (5.50); 7.458 (2.99); 7.437 (0.31); 7.405 (0.31); 7.395 (1.67); 7.385 (1.72); 7.375 (1.59); 7.373 (1.25); 7.366 (1.27); 7.362 (1.11); 7.353 (0.91); 7.048 (2.33); 7.029 (2.85); 7.012 (3.12); 6.858 (1.79); 6.839 (1.48); 5.743 (4.09); 4.198 (1.78); 4.164 (1.87); 4.057 (0.73); 4.040 (2.19); 4.022 (2.22); 4.004 (0.73); 3.421 (0.31); 3.411 (0.60); 3.402 (0.43); 3.392 (0.71); 3.383 (1.23); 3.373 (0.72); 3.364 (0.50); 3.354 (0.71); 3.344 (0.43); 3.288 (199.49); 3.023 (1.25); 2.993 (2.28); 2.965 (1.26); 2.668 (0.34); 2.538 (1.92); 2.521 (1.26); 2.508 (19.08); 2.503 (37.77); 2.499 (50.48); 2.494 (35.81); 2.490 (16.76); 2.325 (0.34); 2.234 (13.99); 2.118 (15.00); 2.104 (1.91); 2.097 (1.86); 1.986 (9.73); 1.745 (0.53); 1.735 (0.64); 1.714 (1.35); 1.705 (1.46); 1.683 (1.37); 1.675 (1.25); 1.654 (0.53); 1.644 (0.42); 1.398 (0.46); 1.237 (0.37); 1.193 (2.68); 1.175 (5.33); 1.157 (2.62); -0.000 (2.11); |
| I-149 | 8.913 (8.35); 7.988 (3.26); 7.539 (0.98); 7.535 (1.44); 7.531 (1.01); 7.520 (1.44); 7.515 (2.31); 7.512 (2.27); 7.508 (1.01); 7.491 (1.97); 7.487 (1.39); 7.467 (2.01); 7.463 (3.04); 7.448 (2.04); 7.443 (1.45); 7.429 (1.48); 7.422 (0.59); 7.408 (0.47); 7.048 (2.29); 7.028 (2.81); 7.012 (3.07); 6.858 (1.76); 6.839 (1.46); 5.743 (6.50); 4.198 (1.78); 4.164 (1.86); 4.057 (0.46); 4.040 (1.27); 4.022 (1.30); 4.004 (0.45); 3.430 (0.37); 3.421 (0.65); 3.411 (0.49); 3.401 (0.75); 3.392 (1.30); 3.383 (0.81); 3.373 (0.61); 3.364 (0.82); 3.353 (0.55); 3.342 (0.43); 3.286 (415.52); 3.258 (0.40); 3.021 (1.27); 2.992 (2.24); 2.963 (1.23); 2.677 (0.33); 2.672 (0.61); 2.668 (0.79); 2.663 (0.58); 2.659 (0.32); 2.538 (4.51); 2.521 (3.11); 2.508 (44.37); 2.503 (87.00); 2.499 (116.18); 2.494 (82.14); 2.490 (38.32); 2.330 (0.56); 2.326 (0.74); 2.321 (0.53); 2.286 (0.43); 2.234 (13.95); 2.133 (1.72); 2.118 (15.00); 2.049 (0.54); 1.986 (5.55); 1.748 (0.55); 1.739 (0.65); 1.717 (1.35); 1.709 (1.43); 1.686 (1.33); 1.678 (1.21); 1.657 (0.49); 1.648 (0.42); 1.399 (1.96); 1.193 (1.57); 1.175 (3.04); 1.157 (1.56); -0.000 (6.26); |
| I-150 | 8.906 (8.67); 8.314 (0.42); 7.988 (3.24); 7.484 (0.32); 7.469 (0.72); 7.465 (0.53); 7.460 (0.57); 7.453 (0.52); 7.447 (1.39); 7.442 (0.74); 7.434 (0.54); 7.426 (1.37); 7.411 (0.62); 7.379 (0.41); 7.374 (0.78); 7.367 (3.00); 7.357 (0.55); 7.347 (4.19); 7.335 (0.49); 7.327 (1.61); 7.324 (1.29); 7.319 (0.45); 7.047 (2.31); 7.028 (2.86); 7.011 (3.09); 6.858 (1.78); 6.839 (1.48); 5.743 (7.03); 4.197 (1.79); 4.163 (1.87); 4.058 (0.34); 4.040 (1.00); 4.022 (1.02); 4.004 (0.36); 3.428 (0.34); 3.419 (0.62); 3.410 (0.45); 3.400 (0.73); 3.391 (1.24); 3.381 (0.75); 3.371 (0.51); 3.362 (0.74); 3.353 (0.48); 3.285 (293.64); 3.021 (1.29); 2.991 (2.26); 2.963 (1.23); 2.672 (0.48); 2.668 (0.66); 2.663 (0.46); 2.538 (3.76); 2.521 (2.55); 2.508 (36.11); 2.503 (70.91); 2.499 (94.74); 2.494 (66.89); 2.490 (31.23); 2.330 (0.44); 2.326 (0.62); 2.321 (0.44); 2.286 (0.69); 2.234 (14.24); 2.118 (15.00); 2.049 (0.46); 1.986 (4.44); 1.747 (0.57); 1.737 (0.69); 1.717 (1.36); 1.708 (1.46); 1.686 (1.34); 1.678 (1.23); 1.656 (0.52); 1.646 (0.43); 1.399 (2.01); 1.193 (1.24); 1.175 (2.45); 1.157 (1.25); 0.008 (0.32); -0.000 (6.98); |
| I-151 | 8.920 (8.70); 7.989 (3.27); 7.677 (7.48); 7.657 (9.48); 7.442 (3.03); 7.422 (3.43); 7.421 (3.12); 7.401 (2.20); 7.048 (2.26); 7.029 (2.78); 7.013 (3.04); 6.858 (1.73); 6.839 (1.42); 5.743 (6.63); 4.201 (1.72); 4.168 (1.82); 4.058 (0.46); 4.040 (1.34); 4.022 (1.38); 4.004 (0.47); 3.421 (0.55); 3.412 (0.41); 3.401 (0.66); 3.392 (1.19); 3.382 (0.67); 3.373 (0.44); 3.364 (0.65); 3.355 (0.35); 3.314 (0.41); 3.286 (170.97); 3.021 (1.22); 2.991 (2.21); 2.963 (1.22); 2.668 (0.39); 2.538 (2.28); 2.521 (1.36); 2.508 (22.16); 2.503 (43.96); 2.499 (58.73); 2.494 (41.56); 2.490 (19.42); 2.330 (0.31); 2.326 (0.39); 2.235 (13.60); 2.138 (1.59); 2.119 (15.00); 2.049 (0.30); 1.986 (6.07); 1.749 (0.52); 1.740 (0.61); 1.718 (1.30); 1.710 (1.42); 1.688 (1.33); 1.680 (1.21); 1.658 (0.50); 1.648 (0.41); 1.399 (1.86); 1.193 (1.68); 1.175 (3.34); 1.157 (1.64); -0.000 (4.05); |
| I-152 | 8.817 (8.39); 7.988 (3.34); 7.819 (1.85); 7.813 (1.85); 7.794 (1.87); 7.788 (1.87); 7.546 (1.04); 7.543 (1.08); 7.540 (1.05); 7.524 (1.76); 7.521 (1.96); 7.518 (1.86); 7.480 (2.37); 7.459 (2.96); 7.438 (1.37); 7.048 (2.31); 7.028 (2.81); 7.010 (3.08); 6.858 (1.78); 6.839 (1.47); 5.742 (6.17); 4.193 (1.77); 4.160 (1.84); 4.058 (0.48); 4.040 (1.42); 4.022 (1.45); 4.004 (0.49); 3.417 (0.37); 3.408 (0.63); 3.398 (0.47); 3.388 (0.78); 3.379 (1.26); 3.369 (0.79); 3.360 (0.60); 3.350 (0.82); 3.341 (0.63); 3.296 (236.06); 3.273 (0.48); 3.020 (1.22); 2.991 (2.24); 2.962 (1.23); 2.539 (1.37); 2.522 (0.98); 2.509 (14.01); 2.504 (27.22); 2.500 (35.97); 2.495 (25.26); 2.491 (11.69); 2.234 (13.85); 2.117 (15.00); 2.096 (1.87); 1.986 (6.33); 1.740 (0.54); 1.730 (0.61); 1.709 (1.32); 1.701 (1.41); 1.678 (1.35); 1.670 (1.20); 1.648 (0.52); 1.639 (0.39); 1.398 (0.72); 1.237 (0.64); 1.193 (1.72); 1.175 (3.40); 1.158 (1.67); -0.000 (1.38); |
| I-153 | 9.588 (0.60); 8.518 (7.54); 8.314 (1.62); 7.996 (0.76); 7.977 (2.92); 7.584 (0.96); 7.579 (0.81); 7.564 (1.52); 7.560 (1.57); 7.545 (0.85); 7.541 (0.83); 7.404 (0.47); 7.399 (1.02); 7.392 (0.62); 7.385 (0.80); 7.379 (1.10); 7.374 (0.63); 7.365 (0.63); 7.361 (0.56); 7.259 (1.49); 7.250 (1.31); 7.247 (1.47); 7.242 (2.16); 7.229 (1.09); 7.223 (2.24); 7.202 (0.96); 7.200 (0.85); 7.155 (0.46); 7.135 (0.56); 7.045 (2.12); 7.026 (2.54); 7.006 (3.27); 6.856 (1.86); 6.837 (1.55); 6.257 (0.54); 6.240 (1.77); 6.224 (1.79); 6.207 (0.54); 5.743 (3.31); 4.174 (1.62); 4.141 (1.64); 4.058 (0.43); 4.040 (1.27); 4.022 (1.28); 4.005 (0.44); 3.351 (0.65); 3.341 (0.50); 3.332 (0.75); 3.322 (1.25); 3.312 (1.02); 3.290 (128.58); 3.018 (0.52); 3.003 (0.99); 2.998 (1.14); 2.969 (1.99); 2.940 (1.11); 2.538 (1.15); 2.522 (0.83); 2.508 (12.76); 2.504 (24.96); 2.499 (33.15); 2.495 (23.45); 2.490 (11.00); 2.286 (2.70); 2.233 (15.00); 2.121 (3.72); 2.110 (12.65); 2.074 (1.39); 2.067 (1.02); 2.049 (1.60); 1.986 (5.64); 1.690 (0.53); 1.682 (0.55); 1.655 (1.21); 1.630 (8.79); 1.614 (7.98); 1.193 (1.54); 1.175 (3.03); 1.158 (1.49); -0.000 (2.56); |
| I-154 | 8.799 (9.09); 7.991 (3.42); 7.950 (2.36); 7.947 (2.40); 7.931 (2.52); 7.927 (2.39); 7.519 (1.09); 7.515 (1.08); 7.498 (2.00); 7.496 (1.86); 7.480 (1.64); 7.476 (1.55); 7.379 (2.69); 7.375 (2.83); 7.359 (2.14); 7.355 (1.96); 7.144 (1.47); 7.140 (1.44); 7.125 (2.16); 7.121 (2.07); 7.106 (1.31); 7.102 (1.21); 7.049 (2.36); 7.030 (2.88); 7.014 (3.10); 6.859 (1.81); 6.840 (1.51); 5.742 (0.97); 4.198 (1.78); 4.165 (1.86); 3.423 (0.32); 3.413 (0.58); 3.404 (0.44); 3.394 (0.73); 3.385 (1.21); 3.375 (0.74); 3.365 (0.51); 3.356 (0.73); 3.346 (0.45); 3.288 (251.80); 3.032 (1.06); 3.026 (1.24); 2.997 (2.25); 2.968 (1.25); 2.672 (0.31); 2.668 (0.40); 2.537 (2.33); 2.521 (1.63); 2.507 (22.89); 2.503 (44.58); 2.498 (59.09); 2.494 (41.30); 2.489 (19.01); 2.325 (0.39); 2.235 (13.99); 2.119 (15.00); 2.048 (0.30); 1.985 (0.87); 1.748 (0.53); 1.739 (0.64); 1.717 (1.34); 1.709 (1.47); 1.687 (1.38); 1.679 (1.28); 1.657 (0.54); 1.648 (0.43); 1.399 (1.26); 1.237 (0.69); 1.175 (0.49); -0.000 (2.77); |
| I-155 | 8.425 (9.08); 8.314 (0.36); 7.968 (3.46); 7.595 (2.09); 7.575 (2.71); 7.476 (1.00); 7.461 (1.17); 7.455 (1.93); 7.440 (1.98); 7.435 (1.27); 7.420 (1.12); 7.384 (1.44); 7.381 (1.44); 7.360 (2.24); 7.339 (0.94); 7.336 (0.87); 7.042 (2.38); 7.022 (2.89); 7.000 (3.22); 6.854 (1.87); 6.835 (1.57); 5.743 (8.83); 5.435 (7.14); 5.431 (7.07); 4.165 (1.88); 4.131 (1.93); 4.040 (0.43); 4.022 (0.44); 3.342 (0.39); 3.333 (0.70); 3.323 (0.63); 3.313 (1.15); 3.290 (114.47); 2.981 (1.31); 2.951 (4.15); 2.923 (1.30); 2.539 (1.16); 2.522 (0.79); 2.508 (12.58); 2.504 (24.72); 2.499 (32.93); 2.495 (23.37); 2.490 (11.01); 2.286 (0.60); 2.230 (15.00); 2.121 (0.89); 2.102 (14.73); 2.068 (1.71); 2.060 (1.56); 2.033 (1.77); 1.986 (1.91); 1.678 (0.56); 1.668 (0.66); 1.646 (1.40); 1.638 (1.51); 1.616 (1.42); 1.608 (1.30); 1.586 (0.54); 1.576 (0.43); 1.193 (0.53); 1.176 (1.03); 1.158 (0.52); -0.000 (3.91); |
| I-156 | 8.467 (8.72); 8.314 (0.69); 7.994 (0.36); 7.974 (3.36); 7.492 (4.88); 7.491 (4.84); 7.472 (8.28); 7.371 (3.20); 7.352 (2.73); 7.349 (2.43); 7.330 (1.82); 7.043 (2.34); 7.024 (2.78); 6.997 (3.14); 6.855 (1.87); 6.836 (1.57); 6.579 (0.75); 6.562 (2.76); 6.544 (2.76); 6.527 (0.76); 5.743 (4.32); 4.159 (1.80); 4.126 (1.76); 4.058 (0.64); 4.040 (1.90); 4.022 (1.93); 4.004 (0.64); 3.350 (0.47); 3.341 (0.78); 3.332 (0.73); 3.322 (1.18); 3.312 (2.25); 3.287 (385.62); 3.002 (1.30); 2.973 (2.33); 2.947 (1.82); 2.673 (0.51); 2.668 (0.68); 2.664 (0.50); 2.538 (3.75); 2.521 (2.56); 2.508 (39.36); 2.503 (77.85); 2.499 (104.15); 2.494 (74.30); 2.490 (35.30); 2.330 (0.52); 2.326 (0.71); 2.321 (0.51); 2.286 (1.18); 2.231 (15.00); 2.119 (1.72); 2.106 (14.36); 2.067 (2.07); 2.049 (1.14); 2.038 (1.70); 1.986 (8.44); 1.734 (10.96); 1.717 (10.95); 1.683 (0.62); 1.674 (0.50); 1.652 (1.41); 1.622 (1.33); 1.600 (0.40); 1.592 (0.48); 1.193 (2.28); 1.175 (4.58); 1.157 (2.24); -0.000 (5.84); |
| I-157 | 8.364 (9.15); 7.975 (3.49); 7.487 (5.98); 7.467 (8.51); 7.331 (2.95); 7.312 (2.94); 7.310 (2.71); 7.291 (1.90); 7.047 (2.44); 7.028 (2.94); 7.006 (3.37); 6.858 (1.90); 6.839 (1.58); 5.742 (6.86); 4.506 (2.79); 4.489 (6.42); 4.472 (2.96); 4.161 (1.91); 4.127 (1.97); 4.058 (0.31); 4.040 (0.92); 4.022 (0.93); 4.004 (0.32); 3.369 (2.83); 3.352 (5.94); 3.335 (3.38); 3.293 (377.10); 3.012 (1.16); 3.006 (1.31); 2.977 (2.41); 2.948 (1.32); 2.673 (0.35); 2.669 (0.48); 2.664 (0.35); 2.539 (2.52); 2.522 (1.61); 2.509 (26.57); 2.504 (52.84); 2.499 (70.91); 2.495 (50.64); 2.490 (24.01); 2.331 (0.35); 2.326 (0.49); 2.322 (0.35); 2.286 (0.48); 2.234 (15.00); 2.112 (14.99); 2.075 (1.60); 2.067 (2.10); 2.049 (1.46); 2.043 (1.81); 1.986 (4.06); 1.692 (0.58); 1.682 (0.68); 1.661 (1.40); 1.653 (1.54); 1.630 (1.44); 1.622 (1.30); 1.601 (0.55); 1.591 (0.45); 1.193 (1.13); 1.175 (2.25); 1.158 (1.11); -0.000 (3.76); |
| I-158 | 9.013 (2.58); 8.131 (7.64); 7.309 (1.84); 7.306 (2.46); 7.288 (3.75); 7.239 (2.22); 7.220 (3.79); 7.201 (1.89); 7.133 (1.35); 7.130 (0.81); 7.115 (1.83); 7.096 (0.73); 7.094 (0.51); 7.084 (1.75); 7.065 (2.24); 6.952 (1.46); 6.933 (1.11); 6.859 (2.48); 5.098 (0.41); 5.084 (0.77); 5.072 (0.68); 5.058 (0.50); 4.766 (1.26); 4.732 (1.33); 3.568 (0.50); 3.395 (0.43); 3.376 (0.57); 3.366 (0.95); 3.356 (0.66); 3.347 (0.54); 3.338 (0.81); 3.289 (625.35); 3.236 (2.21); 3.208 (1.23); 2.875 (0.46); 2.866 (0.44); 2.845 (0.85); 2.837 (0.76); 2.817 (0.55); 2.809 (0.46); 2.672 (0.62); 2.668 (0.87); 2.663 (0.63); 2.538 (3.30); 2.521 (3.08); 2.508 (50.11); 2.503 (99.13); 2.499 (132.67); 2.494 (94.37); 2.490 (44.64); 2.335 (0.39); 2.330 (0.71); 2.326 (0.93); 2.321 (0.69); 2.249 (10.25); 2.107 (11.05); 2.067 (1.39); 2.048 (1.49); 2.027 (1.34); 1.864 (0.68); 1.829 (1.37); 1.766 (0.62); 1.723 (0.76); 1.713 (0.48); 1.702 (0.61); 1.693 (1.05); 1.684 (1.10); 1.672 (1.22); 1.662 (1.27); 1.650 (1.17); 1.642 (1.11); 1.620 (0.49); 1.612 (0.43); 1.522 (1.45); 1.496 (0.88); 1.399 (15.00); 1.370 (0.45); 0.008 (0.50); -0.000 (10.70); -0.008 (0.41); |
| I-159 | 9.114 (1.61); 8.134 (4.68); 7.308 (1.16); 7.305 (1.54); 7.287 (2.33); 7.244 (0.94); 7.238 (1.50); 7.222 (2.86); 7.221 (2.85); 7.205 (1.97); 7.200 (2.50); 7.185 (0.50); 7.179 (0.62); 7.133 (0.97); 7.126 (1.78); 7.120 (1.71); 7.115 (1.26); 7.100 (0.32); 7.096 (0.45); 5.084 (0.48); 5.074 (0.44); 5.059 (0.33); 4.766 (0.74); 4.732 (0.76); 3.388 (0.33); 3.378 (0.56); 3.368 (0.36); 3.350 (0.36); 3.286 (235.97); 3.238 (0.81); 2.844 (0.51); 2.836 (0.45); 2.817 (0.33); 2.672 (0.34); 2.668 (0.46); 2.663 (0.32); 2.538 (1.75); 2.521 (1.68); 2.508 (26.34); 2.503 (51.96); 2.499 (69.35); 2.494 (49.16); 2.490 (23.15); 2.330 (0.37); 2.325 (0.52); 2.322 (0.48); 2.137 (7.82); 2.108 (0.50); 2.075 (0.75); 2.067 (0.61); 2.048 (1.14); 1.865 (0.42); 1.830 (0.88); 1.767 (0.41); 1.723 (0.69); 1.692 (1.03); 1.661 (0.93); 1.633 (0.32); 1.522 (0.90); 1.494 (0.54); 1.399 (15.00); 1.369 (0.31); 0.008 (0.32); -0.000 (6.74); |
| I-160 | 8.313 (1.02); 8.205 (8.88); 7.963 (3.24); 7.432 (0.81); 7.417 (0.94); 7.412 (0.95); 7.406 (0.91); 7.401 (0.95); 7.386 (0.83); 7.381 (0.81); 7.265 (1.50); 7.259 (0.78); 7.244 (1.11); 7.238 (1.45); 7.217 (0.90); 7.167 (0.96); 7.156 (1.24); 7.146 (0.78); 7.136 (0.99); 7.043 (2.24); 7.024 (2.66); 6.996 (2.94); 6.855 (1.80); 6.835 (1.48); 5.742 (7.47); 5.045 (0.99); 5.034 (0.78); 4.148 (1.81); 4.116 (1.71); 4.040 (1.07); 4.022 (1.08); 3.294 (1637.23); 3.255 (2.58); 3.247 (1.46); 3.237 (0.95); 3.227 (0.96); 2.979 (1.18); 2.948 (2.13); 2.920 (1.27); 2.872 (1.04); 2.673 (1.32); 2.668 (1.76); 2.664 (1.28); 2.538 (8.17); 2.522 (6.65); 2.508 (97.46); 2.504 (191.69); 2.499 (256.32); 2.495 (181.69); 2.490 (85.90); 2.331 (1.29); 2.326 (1.66); 2.322 (1.25); 2.286 (1.68); 2.229 (15.00); 2.120 (2.94); 2.103 (13.95); 2.067 (0.79); 2.049 (1.48); 2.027 (1.39); 2.000 (1.63); 1.994 (1.65); 1.986 (5.39); 1.846 (0.86); 1.819 (1.58); 1.754 (0.91); 1.747 (0.91); 1.720 (1.63); 1.689 (0.86); 1.616 (1.25); 1.608 (1.36); 1.586 (1.32); 1.579 (1.28); 1.546 (0.76); 1.519 (1.22); 1.507 (1.33); 1.193 (1.44); 1.175 (2.53); 1.157 (1.29);-0.000 (4.49); |
| I-161 | 8.436 (10.39); 8.114 (3.20); 7.551 (0.82); 7.535 (0.88); 7.530 (2.02); 7.515 (2.07); 7.510 (1.47); 7.494 (1.42); 7.442 (2.65); 7.421 (1.65); 7.355 (1.22); 7.352 (1.20); 7.328 (4.50); 7.323 (3.44); 7.310 (1.00); 7.307 (0.95); 7.194 (2.06); 7.173 (2.85); 7.080 (2.25); 7.074 (2.16); 7.060 (1.61); 7.054 (1.56); 5.747 (6.92); 5.446 (6.56); 5.442 (6.75); 4.164 (1.56); 4.130 (1.63); 4.040 (0.47); 4.022 (0.47); 3.352 (0.36); 3.342 (0.72); 3.332 (0.70); 3.306 (207.42); 3.284 (0.97); 3.275 (0.38); 3.013 (0.90); 3.007 (1.10); 2.977 (1.98); 2.949 (1.09); 2.944 (0.93); 2.540 (0.42); 2.523 (0.72); 2.519 (1.06); 2.510 (14.17); 2.506 (28.15); 2.501 (38.46); 2.497 (26.45); 2.492 (12.50); 2.146 (16.00); 2.077 (1.21); 2.070 (1.68); 2.046 (1.46); 2.040 (1.38); 1.987 (2.21); 1.909 (0.50); 1.690 (0.47); 1.680 (0.60); 1.658 (1.19); 1.650 (1.33); 1.628 (1.20); 1.620 (1.10); 1.598 (0.45); 1.589 (0.36); 1.236 (0.92); 1.193 (0.61); 1.175 (1.21); 1.158 (0.60); -0.000 (3.79); |
| I-162 | 8.432 (6.28); 7.613 (2.13); 7.550 (0.52); 7.534 (0.56); 7.529 (1.27); 7.514 (1.31); 7.509 (0.93); 7.493 (0.90); 7.469 (1.73); 7.464 (1.76); 7.440 (1.71); 7.420 (1.06); 7.354 (0.78); 7.351 (0.74); 7.330 (1.34); 7.309 (0.62); 7.307 (0.57); 6.869 (1.54); 6.848 (2.91); 6.809 (1.17); 6.808 (1.18); 6.804 (1.19); 6.787 (0.61); 6.783 (0.61); 5.747 (5.52); 5.444 (4.28); 5.439 (4.33); 4.121 (1.01); 4.087 (1.06); 3.758 (16.00); 3.321 (1.06); 3.302 (248.55); 3.264 (0.47); 2.995 (0.61); 2.989 (0.72); 2.960 (1.29); 2.931 (0.74); 2.925 (0.60); 2.669 (0.36); 2.539 (0.53); 2.523 (0.98); 2.518 (1.51); 2.509 (19.61); 2.505 (38.48); 2.500 (51.91); 2.496 (35.49); 2.491 (16.59); 2.327 (0.34); 2.207 (10.22); 2.070 (1.10); 2.060 (0.83); 2.050 (0.47); 2.034 (0.95); 1.987 (0.74); 1.673 (0.37); 1.652 (0.77); 1.643 (0.86); 1.621 (0.78); 1.613 (0.73); 1.175 (0.42); -0.000 (4.52); |
| I-163 | 8.447 (10.06); 8.115 (3.24); 7.575 (0.43); 7.558 (0.92); 7.553 (0.85); 7.541 (0.59); 7.537 (1.82); 7.532 (0.71); 7.520 (0.85); 7.516 (1.09); 7.499 (0.48); 7.330 (3.13); 7.324 (3.36); 7.215 (0.41); 7.206 (2.69); 7.194 (2.52); 7.185 (4.10); 7.174 (3.28); 7.165 (2.41); 7.156 (0.39); 7.081 (2.24); 7.075 (2.18); 7.060 (1.59); 7.055 (1.57); 5.748 (0.82); 5.397 (7.52); 4.165 (1.60); 4.131 (1.68); 3.352 (0.32); 3.342 (0.66); 3.332 (0.66); 3.310 (152.26); 3.285 (0.84); 3.276 (0.37); 3.014 (0.93); 3.009 (1.14); 2.979 (2.04); 2.951 (1.12); 2.945 (0.97); 2.524 (0.43); 2.520 (0.63); 2.511 (8.61); 2.506 (17.36); 2.502 (23.97); 2.497 (16.91); 2.493 (8.25); 2.147 (16.00); 2.078 (1.24); 2.071 (1.56); 2.046 (1.50); 2.040 (1.44); 1.988 (0.34); 1.691 (0.47); 1.681 (0.56); 1.660 (1.18); 1.651 (1.33); 1.629 (1.22); 1.621 (1.14); 1.599 (0.46); 1.590 (0.38); -0.000 (1.55); |
| I-164 | 8.443 (6.32); 7.614 (2.10); 7.556 (0.61); 7.552 (0.55); 7.539 (0.39); 7.535 (1.16); 7.531 (0.41); 7.518 (0.55); 7.514 (0.68); 7.471 (1.72); 7.466 (1.74); 7.204 (1.72); 7.184 (2.55); 7.176 (0.35); 7.164 (1.46); 6.870 (1.54); 6.850 (2.90); 6.810 (1.16); 6.808 (1.16); 6.804 (1.18); 6.789 (0.60); 6.787 (0.61); 6.784 (0.61); 5.748 (4.08); 5.394 (4.79); 4.121 (1.02); 4.087 (1.06); 3.760 (16.00); 3.304 (166.71); 3.284 (0.74); 3.274 (0.35); 3.264 (0.40); 2.997 (0.60); 2.991 (0.73); 2.961 (1.27); 2.933 (0.72); 2.928 (0.60); 2.540 (0.33); 2.523 (0.62); 2.518 (0.95); 2.510 (12.11); 2.505 (23.72); 2.501 (31.99); 2.496 (21.83); 2.492 (10.14); 2.207 (10.16); 2.070 (1.01); 2.060 (0.82); 2.034 (0.94); 2.029 (0.90); 1.987 (1.37); 1.674 (0.36); 1.653 (0.76); 1.644 (0.85); 1.622 (0.79); 1.614 (0.71); 1.193 (0.39); 1.175 (0.76); 1.157 (0.38); -0.000 (2.56); |
| I-165 | 8.631 (8.18); 8.624 (0.59); 7.576 (0.44); 7.560 (0.90); 7.555 (0.82); 7.539 (1.74); 7.522 (0.86); 7.518 (1.01); 7.501 (0.45); 7.216 (0.42); 7.207 (2.65); 7.187 (3.95); 7.179 (0.59); 7.167 (2.23); 6.493 (3.95); 5.747 (7.53); 5.418 (7.70); 5.372 (2.00); 5.289 (1.91); 5.247 (0.78); 5.183 (0.73); 4.308 (0.60); 4.276 (0.68); 4.022 (0.44); 3.921 (0.59); 3.892 (0.68); 3.568 (1.60); 3.470 (0.61); 3.455 (0.56); 3.437 (1.20); 3.420 (0.80); 3.410 (0.70); 3.372 (0.58); 3.305 (1677.54); 3.282 (7.60); 3.261 (0.44); 3.046 (0.71); 3.015 (0.39); 2.674 (1.47); 2.669 (2.00); 2.664 (1.38); 2.660 (0.75); 2.539 (2.83); 2.523 (5.77); 2.518 (8.63); 2.509 (107.78); 2.505 (210.41); 2.500 (282.72); 2.496 (192.97); 2.491 (90.13); 2.336 (0.66); 2.332 (1.41); 2.327 (1.86); 2.322 (1.35); 2.264 (0.94); 2.213 (16.00); 2.195 (1.53); 2.185 (0.88); 2.179 (0.87); 2.160 (1.26); 2.132 (0.75); 2.069 (1.97); 2.049 (0.59); 1.987 (1.67); 1.398 (0.47); 1.237 (0.56); 1.193 (0.50); 1.175 (0.92); 1.157 (0.51); 1.093 (0.66); 0.008 (0.44); -0.000 (12.60); |
| I-166 | 8.620 (8.22); 8.246 (3.25); 7.576 (0.41); 7.559 (0.88); 7.555 (0.81); 7.542 (0.57); 7.538 (1.71); 7.534 (0.62); 7.521 (0.82); 7.517 (1.00); 7.500 (0.45); 7.351 (3.03); 7.345 (3.15); 7.215 (0.43); 7.206 (4.14); 7.194 (0.61); 7.185 (5.90); 7.166 (2.23); 7.156 (0.34); 7.097 (2.19); 7.091 (2.09); 7.077 (1.51); 7.071 (1.48); 5.747 (0.34); 5.418 (7.20); 4.075 (1.41); 4.040 (1.48); 3.308 (355.53); 3.285 (1.39); 3.275 (1.03); 3.269 (1.01); 3.239 (1.65); 3.213 (0.87); 3.204 (0.78); 2.670 (0.38); 2.540 (0.51); 2.523 (1.05); 2.519 (1.60); 2.510 (20.89); 2.505 (41.08); 2.501 (55.48); 2.497 (38.22); 2.492 (17.96); 2.328 (0.39); 2.281 (0.33); 2.256 (0.64); 2.247 (0.55); 2.228 (0.57); 2.216 (0.48); 2.165 (1.86); 2.147 (16.00); 2.132 (2.43); 2.070 (0.32); -0.000 ( 1.85); |
| I-167 | 8.588 (8.85); 8.259 (3.14); 7.363 (2.96); 7.357 (3.09); 7.213 (1.99); 7.192 (2.77); 7.099 (2.20); 7.094 (2.06); 7.079 (1.54); 7.073 (1.50); 5.747 (0.49); 4.938 (0.58); 4.926 (0.70); 4.917 (1.15); 4.907 (0.73); 4.894 (0.58); 4.089 (1.35); 4.055 (1.43); 3.568 (0.54); 3.305 (731.69); 3.266 (1.34); 3.259 (1.80); 3.232 (0.86); 3.224 (0.76); 2.674 (0.61); 2.669 (0.83); 2.665 (0.60); 2.539 (1.18); 2.523 (2.39); 2.518 (3.53); 2.509 (44.88); 2.505 (88.53); 2.500 (120.50); 2.496 (82.43); 2.491 (38.66); 2.332 (0.62); 2.327 (0.90); 2.322 (0.62); 2.277 (0.62); 2.248 (0.57); 2.186 (1.91); 2.170 (16.00); 2.148 (2.34); 2.069 (1.63); 1.892 (0.96); 1.871 (1.03); 1.863 (1.06); 1.745 (0.85); 1.737 (0.93); 1.728 (0.97); 1.722 (1.03); 1.713 (1.05); 1.565 (0.52); 1.541 (1.62); 1.532 (1.04); 1.525 (1.24); 1.517 (1.87); 1.509 ( 1.32); 1.495 (0.87); 1.487 (0.72); 1.438 (0.57); 1.421 (0.68); 1.414 (1.29); 1.406 (0.92); 1.398 (1.73); 1.389 (1.13); 1.381 (1.15); 1.356 (0.59); 1.336 (0.48); 1.311 (0.56); 1.306 (0.55); -0.000 (9.72); |
| I-168 | 8.631 (13.75); 7.577 (0.66); 7.561 (1.42); 7.556 (1.24); 7.544 (0.91); 7.540 (2.74); 7.535 (0.97); 7.523 (1.27); 7.518 (1.63); 7.502 (0.73); 7.308 (2.11); 7.217 (0.61); 7.208 (4.05); 7.200 (0.63); 7.196 (0.77); 7.188 (6.02); 7.175 (5.29); 7.167 (3.82); 7.161 (2.72); 7.042 (2.40); 7.025 (5.94); 6.906 (4.42); 6.889 (2.79); 5.746 (16.00); 5.518 (1.04); 5.476 (2.95); 5.421 (11.25); 5.411 (3.50); 5.368 (1.03); 4.293 (0.86); 4.260 (0.93); 4.039 (0.77); 4.022 (0.79); 3.930 (0.78); 3.896 (0.90); 3.470 (0.65); 3.440 (1.16); 3.411 (0.65); 3.314 (1183.89); 3.081 (0.54); 3.073 (0.62); 3.045 (1.00); 3.019 (0.53); 3.012 (0.52); 2.675 (0.62); 2.670 (0.86); 2.665 (0.63); 2.540 (1.19); 2.523 (2.27); 2.519 (3.44); 2.510 (45.59); 2.506 (89.89); 2.501 (122.58); 2.497 (83.59); 2.492 (39.32); 2.376 (0.45); 2.337 (0.54); 2.332 (0.83); 2.328 (0.97); 2.323 (0.70); 2.212 (0.66); 2.176 (1.23); 2.154 (1.89); 2.125 (1.14); 2.069 (1.26); 2.050 (0.50); 1.987 (3.69); 1.398 (0.47); 1.193 ( 1.08); 1.175 (2.11 ); 1.157 ( 1.06); -0.000 (0.60); |
| I-169 | 8.581 (5.54); 7.813 (2.38); 7.419 (1.83); 7.414 (1.88); 6.891 (1.44); 6.879 (0.43); 6.870 (3.18); 6.859 (0.49); 6.840 (1.34); 6.836 (1.34); 6.819 (0.59); 6.816 (0.61); 5.747 (8.68); 4.933 (0.43); 4.920 (0.51); 4.911 (0.83); 4.901 (0.52); 4.888 (0.41); 4.053 (0.99); 4.019 (1.03); 3.819 (2.51); 3.781 (16.00); 3.299 (151.19); 3.276 (0.60); 3.262 (0.68); 3.255 (0.76); 3.226 (1.25); 3.198 (0.69); 3.191 (0.60); 2.522 (0.97); 2.517 (1.45); 2.509 (17.49); 2.504 (34.29); 2.500 (46.28); 2.495 (32.40); 2.491 (15.62); 2.275 (0.49); 2.246 (0.44); 2.233 (0.50); 2.218 (11.25); 2.181 (0.52); 2.171 (0.49); 2.153 (1.17); 2.121 (1.22); 2.087 (0.36); 2.070 (1.57); 1.892 (0.72); 1.871 (0.79); 1.864 (0.81); 1.743 (0.65); 1.736 (0.70); 1.721 (0.77); 1.713 (0.80); 1.565 (0.39); 1.556 (0.53); 1.541 (1.07); 1.533 (1.25); 1.517 (0.97); 1.509 (1.43); 1.502 (0.92); 1.486 (0.55); 1.479 (0.44); 1.433 (0.42); 1.417 (0.52); 1.409 (0.97); 1.398 (2.48); 1.384 (0.83); 1.376 (0.84); 1.351 (0.42); 1.304 (0.43); 1.298 (0.42); -0.000 (5.76); |
| I-170 | 8.596 (8.47); 6.500 (4.04); 5.748 (10.39); 5.430 (0.81); 5.387 (2.04); 5.301 (2.00); 5.259 (0.81); 4.936 (0.63); 4.924 (0.75); 4.914 (1.20); 4.904 (0.78); 4.891 (0.62); 4.319 (0.61); 4.285 (0.69); 3.944 (0.57); 3.909 (0.63); 3.488 (0.52); 3.459 (0.83); 3.428 (0.49); 3.296 (932.40); 3.272 (3.62); 3.067 (0.76); 2.678 (0.98); 2.673 (1.89); 2.668 (2.56); 2.664 (1.83); 2.659 (0.88); 2.539 (3.05); 2.522 (9.02); 2.517 (14.10); 2.508 (144.39); 2.504 (273.40); 2.499 (360.52); 2.495 (244.38); 2.490 (112.92); 2.335 (0.97); 2.331 (1.88); 2.326 (2.49); 2.322 (1.80); 2.317 (0.85); 2.225 (16.00); 2.202 (1.37); 2.175 (1.45); 2.146 (0.83); 2.069 (5.30); 1.897 (1.14); 1.873 (1.22); 1.867 (1.23); 1.748 (1.01); 1.737 (1.05); 1.716 (1.24); 1.564 (0.65); 1.558 (0.85); 1.541 (1.56); 1.534 (1.89); 1.510 (1.99); 1.487 (0.79); 1.479 (0.64); 1.436 (0.63); 1.411 (1.41); 1.403 (1.04); 1.387 (1.24); 1.379 (1.24); 1.361 (0.49); 1.354 (0.64); 1.331 (0.56); 1.306 (0.67); 1.275 (0.56); 1.236 (0.64); 0.008 (1.38); -0.000 (36.06); -0.009 (1.16); |
| I-171 | 8.616 (9.60); 8.122 (3.70); 7.577 (0.48); 7.560 (1.05); 7.556 (0.95); 7.543 (0.70); 7.539 (2.03); 7.535 (0.77); 7.522 (0.96); 7.518 (1.21); 7.501 (0.54); 7.216 (0.46); 7.207 (3.05); 7.195 (0.61); 7.187 (4.57); 7.179 (0.72); 7.167 (2.60); 7.157 (0.45); 7.052 (2.44); 7.033 (3.00); 7.013 (3.21); 6.872 (1.81); 6.869 (1.78); 6.852 (1.50); 5.747 (14.39); 5.419 (8.61); 4.068 (1.71); 4.057 (1.25); 4.039 (1.91); 4.034 (1.79); 4.022 (1.46); 3.298 (338.12); 3.275 (1.34); 3.256 (1.07); 3.250 (1.05); 3.220 (1.98); 3.194 (1.06); 3.186 (0.94); 2.673 (0.56); 2.669 (0.76); 2.664 (0.56); 2.539 (0.83); 2.522 (2.17); 2.517 (3.29); 2.509 (41.01); 2.504 (80.68); 2.500 (109.29); 2.495 (75.81); 2.491 (36.26); 2.331 (0.56); 2.326 (0.79); 2.322 (0.55); 2.270 (0.39); 2.257 (0.54); 2.238 (15.15); 2.205 (0.76); 2.194 (0.62); 2.168 (0.33); 2.143 (2.17); 2.099 (16.00); 2.070 (1.27); 1.987 (2.94); 1.398 (3.25); 1.193 (0.82); 1.175 (1.62); 1.157 (0.81); -0.000 (5.46); |
| I-172 | 8.586 (8.69); 8.134 (3.34); 7.061 (2.22); 7.042 (2.72); 7.026 (2.94); 6.874 (1.64); 6.871 (1.63); 6.855 (1.37); 5.747 (11.02); 4.941 (0.63); 4.929 (0.78); 4.919 (1.27); 4.909 (0.82); 4.896 (0.63); 4.083 (1.55); 4.048 (1.61); 3.297 (365.38); 3.273 (2.20); 3.240 (1.89); 3.213 (1.02); 3.205 (0.91); 2.673 (0.69); 2.668 (0.96); 2.664 (0.69); 2.539 (1.02); 2.522 (2.78); 2.517 (4.25); 2.508 (51.73); 2.504 (101.30); 2.499 (136.63); 2.495 (94.99); 2.490 (45.45); 2.331 (0.69); 2.326 (0.96); 2.322 (0.71); 2.242 (13.99); 2.216 (0.64); 2.162 (1.84); 2.125 (16.00); 2.069 (0.69); 1.907 (0.98); 1.892 (1.12); 1.870 (1.22); 1.862 (1.24); 1.747 (1.00); 1.738 (1.07); 1.730 (1.12); 1.722 (1.21); 1.715 (1.22); 1.577 (0.54); 1.569 (0.59); 1.553 (1.44); 1.546 (1.84); 1.530 (1.45); 1.522 (2.07); 1.500 (1.10); 1.491 (0.84); 1.440 (0.65); 1.424 (0.80); 1.416 (1.45); 1.408 (1.09); 1.398 (3.41); 1.391 (1.38); 1.383 (1.31); 1.367 (0.51); 1.359 (0.68); 1.353 (0.51); 1.339 (0.56); 1.315 (0.66); 1.309 (0.65); 1.285 (0.53); -0.000 (13.51); |
| I-173 | 8.615 (5.63); 7.806 (2.25); 7.558 (0.62); 7.554 (0.55); 7.542 (0.40); 7.537 (1.19); 7.533 (0.44); 7.521 (0.57); 7.516 (0.70); 7.396 (1.78); 7.391 (1.72); 7.206 (1.78); 7.194 (0.34); 7.186 (2.63); 7.177 (0.38); 7.165 (1.53); 6.867 (0.80); 6.847 (3.68); 6.839 (1.81); 6.834 (1.67); 6.817 (0.37); 6.813 (0.39); 5.747 (2.90); 5.415 (4.95); 4.039 (0.95); 4.003 (1.00); 3.742 (16.00); 3.303 (239.83); 3.279 (3.48); 3.242 (0.61); 3.234 (0.69); 3.206 (1.19); 3.178 (0.66); 3.172 (0.58); 2.669 (0.40); 2.539 (0.45); 2.523 (1.18); 2.518 (1.74); 2.509 (21.25); 2.505 (41.84); 2.500 (56.79); 2.496 (39.18); 2.491 (18.71); 2.327 (0.39); 2.257 (0.46); 2.215 (11.30); 2.162 (0.48); 2.151 (0.48); 2.134 (1.17); 2.102 (1.18); 2.070 (0.88); 1.987 (0.39); 1.398 (0.46); -0.000 (2.71 ); |
| I-175 | 8.362 (16.00); 7.577 (0.81); 7.560 (1.80); 7.556 (1.72); 7.543 (1.31); 7.539 (3.53); 7.535 (1.40); 7.522 (1.78); 7.518 (2.08); 7.501 (0.94); 7.300 (2.96); 7.213 (0.87); 7.204 (5.37); 7.195 (1.06); 7.192 (1.26); 7.184 (8.49); 7.176 (1.74); 7.166 (8.92); 7.155 (4.97); 7.033 (3.37); 7.018 (8.50); 6.898 (7.22); 6.883 (4.20); 5.748 (10.45); 5.431 (1.37); 5.417 (15.88); 5.390 (5.65); 5.361 (8.04); 5.320 (1.08); 4.340 (1.45); 4.306 (1.52); 3.969 (1.35); 3.934 (1.49); 3.421 (1.08); 3.412 (0.90); 3.402 (1.46); 3.393 (2.34); 3.384 (1.49); 3.374 (1.05); 3.365 (1.34); 3.355 (0.77); 3.307 (111.47); 3.284 (2.88); 3.261 (2.01); 3.231 (1.20); 2.865 (1.01); 2.854 (0.85); 2.836 (1.79); 2.808 (1.02); 2.525 (0.79); 2.512 (14.06); 2.507 (26.94); 2.503 (35.76); 2.499 (25.34); 2.494 (12.47); 2.134 (1.29); 2.099 (2.61); 2.072 (1.28); 2.063 (1.51); 1.989 (3.02); 1.803 (1.42); 1.780 (1.33); 1.773 (1.23); 1.590 (1.19); 1.580 (1.25); 1.559 (1.17); 1.551 (1.12); 1.397 (0.81); 1.194 (0.85); 1.177 (1.63); 1.159 (0.81); -0.000 (4.38); |
| I-176 | 8.746 (16.00); 7.549 (1.87); 7.545 (2.63); 7.542 (1.95); 7.530 (2.88); 7.525 (5.13); 7.522 (4.32); 7.503 (4.16); 7.499 (2.72); 7.480 (7.69); 7.475 (3.08); 7.465 (4.38); 7.461 (2.94); 7.446 (2.95); 7.440 (1.18); 7.425 (0.95); 7.315 (3.00); 7.182 (6.83); 7.164 (3.30); 7.048 (3.31); 7.028 (7.55); 6.908 (6.52); 6.892 (3.72); 5.748 (10.49); 5.462 (1.18); 5.419 (5.16); 5.383 (5.08); 5.340 (1.19); 4.386 (1.45); 4.352 (1.55); 4.041 (1.61); 4.023 (2.34); 4.014 (1.40); 4.005 (1.52); 3.979 (1.50); 3.534 (1.05); 3.524 (0.75); 3.514 (1.28); 3.505 (2.23); 3.496 (1.30); 3.486 (0.83); 3.477 (1.18); 3.468 (0.61); 3.330 (1.30); 3.306 (128.39); 3.282 (3.26); 3.271 (1.34); 2.904 (0.99); 2.873 (1.80); 2.846 (1.02); 2.524 (0.91); 2.511 (16.46); 2.507 (31.32); 2.502 (41.31); 2.498 (29.07); 2.494 (14.15); 2.207 (1.30); 2.171 (2.65); 2.136 (1.54); 1.988 (6.67); 1.882 (1.18); 1.874 (1.25); 1.851 (1.19); 1.843 (1.12); 1.667 (1.19); 1.658 (1.30); 1.636 (1.20); 1.628 (1.15); 1.194 (1.91); 1.176 (3.75); 1.158 (1.82); -0.000 (5.53); |
| I-177 | 8.378 (8.39); 7.299 (1.51); 7.288 (0.48); 7.235 (1.53); 7.217 (2.06); 7.191 (1.38); 7.173 (2.48); 7.166 (3.92); 7.154 (2.99); 7.120 (2.01); 7.101 (2.58); 7.082 (0.95); 7.033 (1.72); 7.018 (4.82); 6.898 (3.98); 6.882 (2.34); 5.747 (5.98); 5.434 (0.55); 5.392 (2.81); 5.360 (14.06); 5.319 (0.59); 4.344 (0.73); 4.312 (0.76); 4.040 (0.42); 4.022 (0.44); 3.971 (0.68); 3.937 (0.74); 3.425 (0.56); 3.415 (0.43); 3.405 (0.78); 3.396 (1.26); 3.387 (0.79); 3.377 (0.61); 3.368 (0.75); 3.359 (0.44); 3.303 (99.52); 3.280 (2.35); 3.262 (1.05); 3.233 (0.63); 3.143 (0.37); 3.038 (0.44); 2.865 (0.50); 2.851 (0.57); 2.835 (0.90); 2.808 (0.51); 2.523 (0.79); 2.509 (12.59); 2.505 (23.77); 2.500 (31.24); 2.496 (21.75); 2.492 (10.49); 2.270 (15.51); 2.226 (16.00); 2.141 (0.68); 2.103 (1.35); 2.069 (1.16); 1.987 (1.98); 1.843 (0.36); 1.833 (0.39); 1.811 (0.73); 1.804 (0.80); 1.781 (0.73); 1.624 (0.37); 1.614 (0.38); 1.592 (0.63); 1.583 (0.64); 1.562 (0.58); 1.553 (0.55); 1.193 (0.56); 1.175 (1.08); 1.158 (0.54); -0.000 (3.14); |
| I-178 | 8.259 (7.95); 6.986 (1.60); 6.896 (3.35); 6.877 (1.80); 6.832 (2.76); 6.806 (1.67); 6.786 (3.82); 6.695 (1.84); 5.260 (0.80); 5.232 (2.78); 5.202 (2.76); 5.174 (0.81); 5.058 (1.28); 4.946 (0.64); 4.939 (1.05); 4.933 (0.65); 4.473 (0.61); 4.451 (0.63); 3.882 (0.62); 3.600 (16.00); 3.349 (0.61); 3.336 (0.65); 3.330 (1.25); 3.324 (0.67); 3.311 (0.66); 3.281 (0.65); 3.277 (0.87); 3.274 (0.63); 3.021 (0.73); 2.848 (0.76); 2.844 (0.78); 2.167 (1.87); 2.087 (0.69); 1.950 (4.42); 1.946 (8.58); 1.942 (12.67); 1.938 (8.65); 1.933 (4.40); 1.917 (0.73); 1.906 (0.84); 1.903 (0.80); 1.896 (0.85); 1.893 (0.86); 1.887 (0.89); 1.881 (0.74); 1.861 (0.59); 1.854 (0.62); 1.764 (0.83); 1.758 (0.83); 1.752 (0.95); 1.747 (0.90); 1.742 (0.97); 1.737 (0.71); 1.694 (0.60); 1.564 (1.09); 1.559 (1.46); 1.554 (0.98); 1.549 (1.10); 1.543 (1.60); 1.539 (1.21); 1.528 (0.73); 1.522 (0.60); 1.457 (0.61); 1.447 (0.83); 1.441 (1.48); 1.435 (1.13); 1.430 (0.70); 1.425 (1.26); 1.419 (1.33); 1.402 (0.60); 1.354 (0.60); -0.000 (1.52); |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt warden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-d6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

### Anwendunssbeispiele

### Beispiel A

### Phytophthora-Test (Tomate) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen (I-2), (I-12), (I-13), (I-14), (I-15), (I-16), (I-17), (I-18), (I-19), (I-20), (I-25), (I-26), (I-27), (I-31), (I-32), (I-34), (I-35), (I-36), (I-41), (I-42), (I-43), (I-44), (I-45), (I-46), (I-48), (I-49), (I-51), (I-52), (I-53), (I-54), (I-55), (I-57), (I-58), (I-59), (I-61), (I-62), (I-63), (I-64), (I-65), (I-68), (I-68), (I-71), (I-72), (I-73), (I-74), (I-78), (I-79), (I-80), (I-91), (I-96), (I-111), (I-112), (I-120), (I-121), (I-122), (I-123), (I-125), (I-126), (I-127), (I-135), (I-137), (I-138), (I-140), (I-141), (I-142), (I-143), (I-144), (I-145), (I-149), (I-150), (I-151), (I-152), (I-153), (I-155), (I-156), (I-157), (I-161), (I-162), (I-163), (I-164), (I-167), (I-168), (I-169), (I-170), (I-171), (I-172) und (1-173) bei einer Konzentration an Wirkstoff von 500ppm einen Wirkungsgrad von 70% oder mehr.

### Beispiel B

### Plasmopara-Test (Rebe) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21 °C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen (I-12), (I-13), (I-14), (I-15), (I-16), (I-17), (I-18), (I-19), (I-20), (I-25), (I-26), (I-41), (I-42), (I-43), (I-46), (1-48), (I-51), (I-52), (I-53), (I-54), (I-59), (I-61), (I-62), (I-63), (I-64), (I-68), (I-69), (I-71) und (I-72) bei einer Konzentration an Wirkstoff von 100ppm einen Wirkungsgrad von 70% oder mehr.

## Patentansprüche

1. Verbindungen der Formel (**I**), 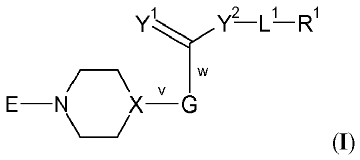 in welcher die Symbole folgende Bedeutungen haben:
E steht für 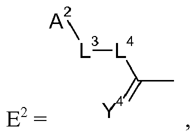
A², stehen für ein Phenyl, das bis zu drei Substituenten enthalten kann, wobei die Substituenten unabhängig voneinander ausgewählt sind aus folgender Liste:
Halogen, Cyano, Hydroxy, SH, Amino, Nitro, C₁-C₆-Alkyl, C₂-C₆-Alkenyl, C₂-C₆-Alkinyl, C₃-C₁₀-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, C₂-C₈-Dialkylamino, C₃-C₆-Cycloalkylamino, C₄-C₁₀-Cycloalkylalkoxy, C₂-C₄-Alkoxyalkyl, C₂-C₆-Alkoxyalkoxy, C₁-C₄-Hydroxyalkyl, C₂-C₄-Alkylcarbonyl, C₂-C₆-Alkoxycarbonyl, C₂-C₆-Alkylcarbonyloxy, C₂-C₆-Alkylcarbonylthio, C₂-C₆-Alkylaminocarbonyl, C₃-C₈-Dialkylaminocarbonyl, Tri(C₁-C₄-alkyl)silyl, C(=O)H, CR³=NOR⁴, Phenyl oder Benzyl
oder
A², stehen für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-l-yl, 1,2,4-Triazol-l-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu drei Substituenten enthalten kann, wobei die Substituenten unabhängig voneinander ausgewählt sind aus folgender Liste:
Substituenten am Kohlenstoff:
Halogen, Cyano, Hydroxy, SH, Amino, Nitro, C₁-C₆-Alkyl, C₂-C₆-Alkenyl, C₂-C₆-Alkinyl, C₃-C₁₀-Cycloalkyl, C₄-C₁₀-Cycloalkylalkyl, C₄-C₁₀-Alkylcycloalkyl, C₅-C₁₀-Alkylcycloalkylalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Halogenalkylthio, C₁-C₄-Halogenalkylsulfinyl, C₁-C₄-Halogenalkylsulfonyl, C₁-C₄-Alkylamino, C₂-C₈-Dialkylamino, C₃-C₆-Cycloalkylamino, C₄-C₁₀-Cycloalkylalkoxy, C₂-C₄-Alkoxyalkyl, C₂-C₆-Alkoxyalkoxy, C₁-C₄-Hydroxyalkyl, C₂-C₄-Alkylcarbonyl, C₂-C₆-Alkoxycarbonyl, C₂-C₆-Alkylcarbonyloxy, C₂-C₆-Alkylcarbonylthio, C₂-C₆-Alkylaminocarbonyl, C₃-C₈-Dialkylaminocarbonyl, Tri(C₁-C₄-alkyl)silyl, C(=O)H, CR³=NOR⁴, Phenyl oder Benzyl
Substituenten am Stickstoff:
C₁-C₆-Akl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkylsulfonyl, C(=O)H, C(=O)Me, C(=O)OMe, oder C₂-C₄-Alkoxyalkyl,
L¹ steht für (C(R¹⁵)₂)ₚ,
p steht für 0, 1 oder 2,
L³ steht für eine direkte Bindung,
L⁴ steht für Sauerstoff, CHR⁵, NR⁶ oder C(=O),
L⁸ steht für eine direkte Bindung, -O, -C(=O), -S(O)ₘ, -CHR¹⁶ oder -NR¹⁷,
m steht für 0, 1 oder 2,
Y¹, Y², Y⁴ stehen unabhängig voneinander für Schwefel oder Sauerstoff,
Y³ steht für OR⁷, SR⁸, NR⁹R¹⁰ oder R¹¹,
n steht für 0, 1 oder 2,
X steht für CR¹², Stickstoff,
G steht für 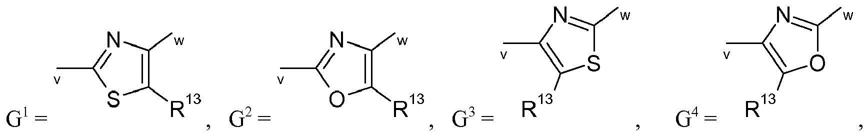 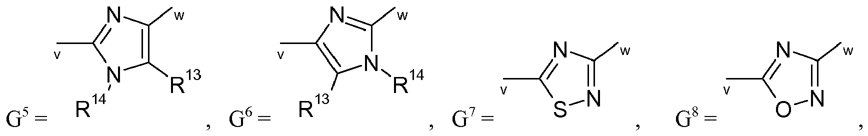 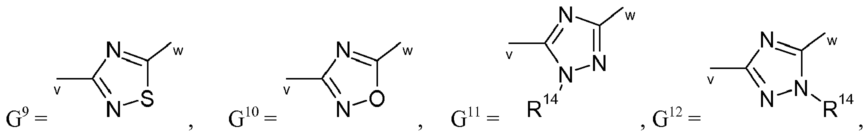 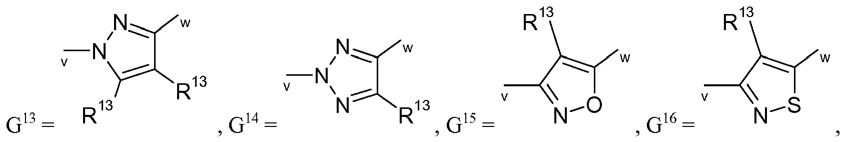 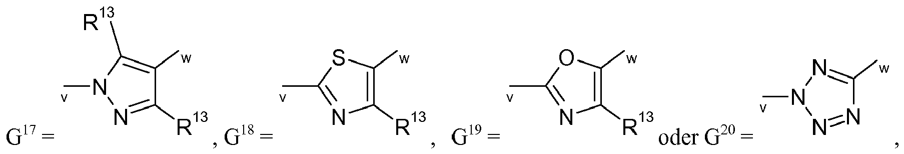
wobei die Bindung, die mit "v" identifiziert ist, direkt an X gebunden ist, und wobei die Bindung, die mit "w" identifiziert ist, direkt an C(=Y¹)Y²L¹R¹ gebunden ist,
R¹ steht für unsubstituiertes oder substituiertes C₃-C₁₀-Cycloalkyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenoxy, Hydroxy, Oxo, C₂-C₆-Alkoxycarbonyl, C₁-C₆-Alkylcarbonyloxy, C₁-C₆-Alkylcarbonyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio oder-Q,
oder
R¹ steht für unsubstituiertes oder substituiertes C₅-C₁₀-Cycloalkenyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
R¹ steht für unsubstituiertes oder substituiertes Phenyl, wobei die Substituenten unabhängig voneinander ausgewählt sind aus folgender Liste:
Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkyl-sulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -L⁸Q,
oder
R¹ steht für gesättigtes oder teilweise oder vollständig ungesättigtes, unsubstituiertes oder substituietes, Naphthyl oder Indenyl, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
R¹ steht für einen unsubstituierten oder substituierten 5- oder 6-gliedrigen Heteroarylrest, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfmyl, C₁-C₄-Alkylsulfonyl, C₁-C₄-Halogenalkylsulfonyl, Tri(C₁-C₄-alkyl)silyl oder -L⁸Q,
Substituenten am Stickstoff: C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
R¹ steht für benzokondensiertes unsubstituiertes oder substituiertes 5- oder 6- gliedriges Heteroaryl, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
R¹ steht für unsubstituiertes oder substituiertes C₅-C₁₅-Heterocyclyl, wobei L¹ an einem Kohlenstoff vom Heterocyclylrest gebunden ist und mögliche Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
Q steht für ein Phenyl, das bis zu zwei Substituenten enthalten kann, wobei die Substituenten unabhängig voneinander ausgewählt sind aus folgender Liste:
Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
oder
Q steht für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe oder Phenyl,
R² steht für C₁-C₄-Alkyl, C₁-C₄-Halogenalkyl oder C₂-C₄-Alkoxyalkyl,
R³, R⁴ stehen unabhängig voneinander für Wasserstoff, C₁-C₄-Alkyl, C₂-C₆-Alkenyl, C₂-4C₆-Alkinyl, C₁-C₃-Halogenalkyl, C₃-C₆-Cycloalkyl, Benzyl oder Phenyl,
R⁵ steht für Wasserstoff, Halogen, Cyano, Hydroxy, C(=O)H, OC(=O)H, OC(=O)Me, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂-C₄-Halogenalkylcarbonyl, C₂-C₅-Alkoxycarbonyl, C₃-C₅-Alkoxycarbonylalkyl, C₂-C₅-Alkylaminocarbonyl, C₃-C₅-Dialkylaminocarbonyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
R⁶ steht für Wasserstoff, C(=O)H, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂-C₄-Halogenalkylcarbonyl, C₂-C₅-Alkoxycarbonyl, C₃-C₅-Alkoxycarbonylalkyl, C₂-C₅-Alkylaminocarbonyl, C₃-C₅-Dialkylaminocarbonyl, C₁-C₄-Alkylsulfonyl oder C₁-C₄-Halogenalkylsulfonyl,
R⁷, R⁸ sind unabhängig voneinander ausgewählt aus folgender Liste:
C₁-C₆-Akl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₈-Alkylcycloalkyl, C₄-C₈-Cycloalkylalkyl, C₄-C₈-Halogencycloalkylalkyl, C₅-C₈-Alkylcycloalkylalkyl, C₂-C₆-Alkoxyalkyl, C₄-C₈-Cycloalkoxyalkyl, C₃-C₆-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₆-Dialkylaminoalkyl, C₂-C₆-Halogenalkylaminoalkyl, C₄-C₈-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxylcarbonyl, C₂-C₆-Alkylaminocarbonyl, C₃-C₈-Dialkylaminocarbonyl, Tri(C₁-C₄-alkyl)silyl oder C₄-C₈-Cycloalkylaminocarbonyl,
R⁹ steht für Wasserstoff, Cyano, Hydroxy, Amino, C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₄-C₈-Cycloalkylalkyl, C₂-C₆-Alkoxyalkyl, C₁-C₆-Alkoxy, C₁-C₆-Halogenalkoxy, C₁-C₆-Alkylsulfonyl, C₁-C₆-Halogenalkylsulfonyl, C₂-C₆-Alkylcarbonyl, C₂-C₆-Halogenalkylcarbonyl, C₁-C₆-Alkylamino, C₂-C₈-Dialkylamino, C₁-C₆-Halogenalkylamino oder C₂-C₈-Halogendialkylamino,
R¹⁰ steht für Wasserstoff, C₁-C₆-Akl, C₂-C₆-Alkenyl, C₂-C₆-Alkinyl, C₁-C₆-Halogenalkyl oder C₃-C₆-Cycloalkyl,
oder
R⁹, R¹⁰ bilden zusammen einen -(CH₂)₄-, -(CH₂)₅-, -(CH₂)₂S(CH₂)₂-, -(CH₂)₂S(=O)(CH₂)₂-, - (CH₂)₂S(=O)₂(CH₂)₂-, -(CH₂)₂NR³CH₂)₂- oder -(CH₂)₂O(CH₂)₂- Rest,
R¹¹ steht für Wasserstoff, Halogen, Cyano, C₁-C₄-Alkyl, C₁-C₄-Halogenalkyl, C₂-C₄-Alkoxylalkyl, C₂-C₄-Alkylcarbonyl, C₂-C₄-Alkoxycarbonyl, C₂-C₃-Alkylaminocarbonyl oder C₃-C₆-Dialkylaminocarbonyl,
R¹² steht für Wasserstoff, Halogen, Cyano, Hydroxy, OC(=O)Me, OC(=O)H, C(=O)H, C(=O)OH, C(=O)OMe, C(=O)Me, C₁-C₄-Alkyl, C₂-C₄-Alkenyl, C₂-C₄-Alkinyl, C₁-C₄-Halogenalkyl, C₁-C₄-Alkoxy oder C₁-C₄-Halogenalkoxy,
R¹³ steht für Wasserstoff, C₁-C₃-Alkyl, C₁-C₃-Halogenalkyl oder Halogen,
R¹⁴ steht für Wasserstoff oder C₁-C₃-Alkyl, C(=O)H, C(=O)Me oder C(=O)OMe,
R¹⁵ ist gleich oder verschieden unabhängig voneinander Wasserstoff, Halogen, C₁-C₄-Alkyl, C₂-C₄-Alkenyl, C₂-C₄-Alkinyl, C₁-C₄-Alkoxy, Oxo, C₃-C₆-Cycloalkyl, C₁-C₄-Halogenalkyl oder Phenyl
R¹⁶ steht für Wasserstoff, C₁-C₄-Alkyl oder C₁-C₄-Halogenalkyl,
R¹⁷ steht für Wasserstoff, C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
sowie agrochemisch wirksame Salze davon,
wobei folgende Verbindungen ausgenommen sind:
a) Verbindungen in denen,
A² für Pyrazol-1-yl steht,
X für CH steht,
G für G¹ steht,
b) Verbindungen in denen,
X für Stickstoff steht,
G für G¹³, G¹⁴, G¹⁷ oder G²⁰ steht,
c) Verbindungen, in denen,
Y⁴ für Schwefel steht,
L⁴ für C(=O) steht,

2. Verbindungen der Formel (I) nach Anspruch 1,
in welcher die Symbole folgende Bedeutungen haben,
E E²,
A² stehen außerdem für Phenyl, das bis zu zwei Substituenten enthalten kann, wobei die Substituenten unabhängig voneinander ausgewählt sind aus folgender Liste:
Halogen, Cyano, Hydroxy, Amino, Nitro, C₁-C₆-Alkyl, C₂-C₆-Alkenyl, C₂-C₆-Alkinyl, C₃-C₁₀-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylcarbonyloxy, C(=O)H oder CR³=NOR⁴
A² stehen außerdem für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus folgender Liste:
Substituenten am Kohlenstoff:
Halogen, Cyano, Hydroxy, Amino, Nitro, C₁-C₆-Alkyl, C₂-C₆-Alkenyl, C₂-C₆-Alkinyl, C₃-C₁₀-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₁-C₄-Hydroxyalkyl, C₂-C₄-Alkylcarbonyl, C₂-C₆-Alkoxycarbonyl, C₂-C₆-Alkylcarbonyloxy, C(=O)H, CR³=NOR⁴ oder Phenyl
Substituenten am Stickstoff:
C₁-C₆-Alkyl, C₁-C₄-Halogenalkyl, C₂-C₆-Alkenyl, C₂-C₆-Halogenalkenyl, C₂-C₆-Alkinyl oder C₂-C₆-Halogenalkinyl.
L¹ steht für eine direkte Bindung, -CH₂- oder -CHCH₃-,
L³ steht für eine direkte Bindung,
L⁴ steht für Sauerstoff, CHR⁵, NR⁶,
L⁸ steht für eine direkte Bindung, -O-,
Y¹ steht für Sauerstoff, Schwefel,
Y² steht für Sauerstoff, Schwefel,
Y³ steht für OR⁷, SR⁸,
Y⁴ steht für Sauerstoff, Schwefel,
X steht für CH, CF, N,
G steht für G¹, G², G³, G¹³, G¹⁴ oder G¹⁸
R¹ steht außerdem für unsubstituiertes oder substituiertes C₃-C₁₀-Cycloalkyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
R¹ steht außerdem für unsubstituiertes oder substituiertes C₅-C₁₀-Cycloalkenyl, wobei die Substituenten unabhängig voneinander ausgewählt sind aus folgender Liste:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
R¹ steht außerdem für unsubstituiertes oder substituiertes Phenyl, wobei die Substituenten unabhängig voneinander ausgewählt sind aus folgender Liste:
Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Aklsulfinyl, C₁-C₆-Halogenalkylsulfinyl, C₁-C₆-Alkyl-sulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -L⁸Q,
R¹ steht außerdem für gesättigtes oder teilweise oder vollständig ungesättigtes, unsubstituiertes oder substituietes, Naphthyl oder Indenyl, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
R¹ steht außerdem für einen unsubstituierten oder substituierten 5- oder 6-gliedrigen Heteroarylrest, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -L⁸Q,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
R¹ steht außerdem für benzokondensiertes unsubstituiertes oder substituiertes 5- oder 6-gliedriges Heteroaryl, wobei L¹ an einem Kohlenstoff vom Heteroarylrest gebunden ist und wobei die Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Akl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
R¹ steht außerdem für unsubstituiertes oder substituiertes C₅-C₁₅-Heterocyclyl, wobei L¹ an einem Kohlenstoff vom Heterocyclylrest gebunden ist und wobei mögliche Substituenten unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaimnocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
Q steht für Phenyl, das bis zu zwei Substituenten enthalten kann, wobei die Substituenten unabhängig voneinander ausgewählt sind aus folgender Liste:
Halogen, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Q steht außerdem für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste:
Substituenten am Kohlenstoff:
Halogen, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Substituenten am Stickstoff: C₁-C₆-Alkyl, C₂-C₆-Alkenyl, C₂-C₆-Alkinyl, C₃-C₆-Cycloalkyl oder Phenyl,
R³, R⁴ stehen unabhängig voneinander für Wasserstoff,
R³, R⁴ stehen unabhängig voneinander außerdem für Methyl, Ethyl, Propyl, *iso*-Propyl, Butyl, *iso*-Butyl oder *tert*-Butyl,
R⁵ steht für Wasserstoff,
R⁵ steht außerdem für Methyl, Ethyl, Propyl, *iso*-Propyl, Butyl, *iso*-Butyl oder *tert-*Butyl,
R⁶ steht für Wasserstoff,
R⁶ steht außerdem für Methyl, Ethyl, Propyl, *iso*-Propyl, Butyl, *iso*-Butyl oder *tert-*Butyl,
R⁷ steht für Methyl, Ethyl, Propyl, *iso*-Propyl, Butyl, *iso*-Butyl oder *tert*-Butyl,
R⁸ steht für Methyl, Ethyl, Propyl, *iso*-Propyl, Butyl, *iso*-Butyl oder *tert*-Butyl,
R¹³ steht für Wasserstoff,
sowie agrochemisch wirksame Salze davon.

3. Verfahren zur Bekämpfung von pflanzenpathogenen Schadpilzen, **dadurch gekennzeichnet, dass** man Verbindungen der Formel (I) gemäß einem oder mehreren der Ansprüche 1 bis 2, auf die pflanzenpathogenen Schadpilze und/oder deren Lebensraum ausbringt.

4. Mittel zur Bekämpfung von pflanzenpathogenen Schadpilzen, **gekennzeichnet durch** einen Gehalt an mindestens einer Verbindung der Formel (I) gemäß einem oder mehreren der Ansprüche 1 bis 2, neben Streckmitteln und/oder oberflächenaktiven Stoffen.

5. Verwendung von Heteroarylpiperidin und -piperazin Derivaten der Formel (I), gemäß einem oder mehreren der Ansprüche 1 bis 2 zur Bekämpfung von pflanzenpathogenen Schadpilzen.

6. Verfahren zur Herstellung von Mitteln zur Bekämpfung von pflanzenpathogenen Schadpilzen, **dadurch gekennzeichnet, dass** man Heteroarylpiperidin und -piperazin Derivate der Formel (I) gemäß einem oder mehreren der Ansprüche 1 bis 2 mit Streckmitteln und/oder oberflächenaktiven Stoffen vermischt.

## Claims

1. Compounds of the formula (I) 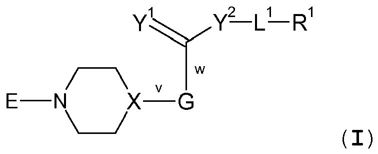 in which the symbols have the following meanings:
E represents 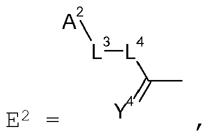
A² represents phenyl which may contain up to three substituents, where the substituents independently of one another are selected from the list below:
halogen, cyano, hydroxyl, SH, amino, nitro, C₁-C₆-alkyl, C₂-C₆-alkenyl, C₂-C₆-alkynyl, C₃-C₁₀-cycloalkyl, C₄-C₁₀₋cycloalkylalkyl, C₄-C₁₀-alkylcycloalkyl, C₅-C₁₀-alkylcycloalkylalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, C₂-C₈-dialkylamino, C₃-C₆-cycloalkylamino, C₄-C₁₀-cycloalkylalkoxy, C₂-C₄-alkoxyalkyl, C₂-C₆-alkoxyalkoxy, C₁-C₄-hydroxyalkyl, C₂-C₄-alkylcarbonyl, C₂-C₆-alkoxycarbonyl, C₂-C₆-alkylcarbonyloxy, C₂-C₆-alkylcarbonylthio, C₂-C₆-alkylaminocarbonyl, C₃-C₈-dialkylaminocarbonyl, tri(C₁-C₄-alkyl)silyl, C(=O)H, CR³=NOR⁴, phenyl or benzyl
or
A² represents a heteroaromatic radical selected from the group below: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl which may contain up to three substituents, where the substituents independently of one another are selected from the list below:
substituents at carbon:
halogen, cyano, hydroxyl, SH, amino, nitro, C₁-C₆-alkyl, C₂-C₆-alkenyl, C₂-C₆-alkynyl, C₃-C₁₀-cycloalkyl, C₄-C₁₀₋cycloalkylalkyl, C₄-C₁₀-alkylcycloalkyl, C₅-C₁₀-alkylcycloalkylalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, C₂-C₈-dialkylamino, C₃-C₆-cycloalkylamino, C₄-C₁₀-cycloalkylalkoxy, C₂-C₄-alkoxyalkyl, C₂-C₆-alkoxyalkoxy, C₁-C₄-hydroxyalkyl, C₂-C₄-alkylcarbonyl, C₂-C₆-alkoxycarbonyl, C₂-C₆-alkylcarbonyloxy, C₂-C₆-alkylcarbonylthio, C₂-C₆-alkylaminocarbonyl, C₃-C₈-dialkylaminocarbonyl, tri(C₁-C₄-alkyl)silyl, C(=O)H, CR³=NOR⁴, phenyl or benzyl
substituents at nitrogen:
C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, C₁-C₄-alkylsulfonyl, C(=O)H, C(=O)Me, C(=O)OMe or C₂-C₄-alkoxyalkyl,
L¹ represents (C(R¹⁵)₂)ₚ,
p represents 0, 1 or 2,
L³ represents a direct bond,
L⁴ represents oxygen, CHR⁵, NR⁶ or C(=O),
L⁸ represents a direct bond, -O, -C(=O), -S(O)ₘ, -CH¹⁶ or -NR¹⁷,
m represents 0, 1 or 2,
Y¹, Y², Y⁴ independently of one another represent sulphur or oxygen,
Y³ represents OR⁷, SR⁸, NR⁹R¹⁰ or R¹¹,
n represents 0, 1 or 2,
X represents CR¹² or nitrogen,
G represents 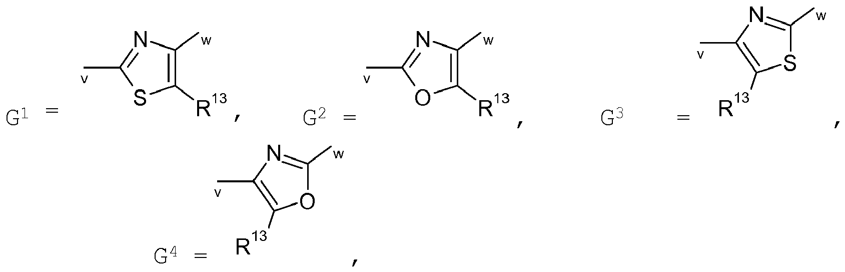 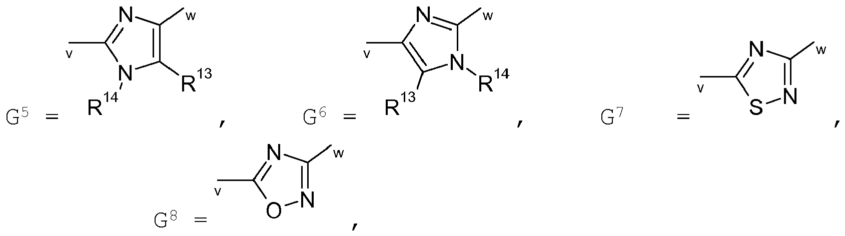 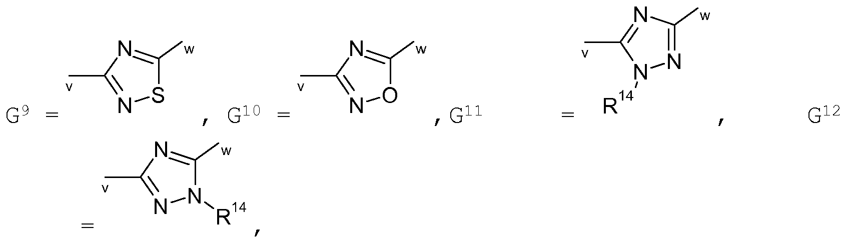 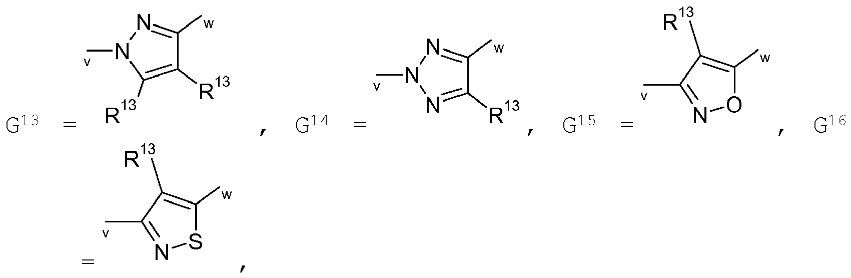 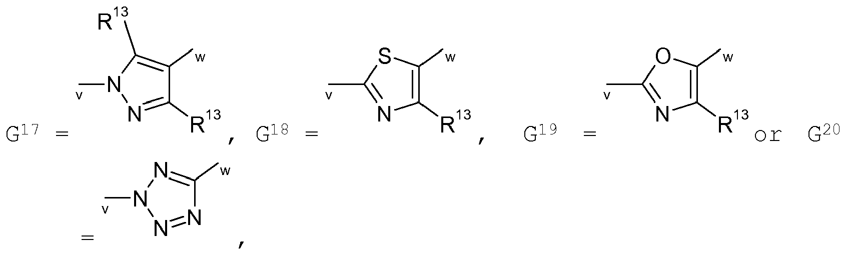 where the bond identified by "v" is attached directly to X and where the bond identified by "w" is attached directly to C(=Y¹)Y²L¹R¹,
R¹ represents unsubstituted or substituted C₃-C₁₀-cycloalkyl, where the substituents independently of one another are selected from the list below:
cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₄-alkyl)silyl, phenoxy, hydroxyl, oxo, C₂-C₆-alkoxycarbonyl, C₁-C₆-alkylcarbonyloxy, C₁-C₆-alkylcarbonyl, C₁-C₆-alkoxy, C₁-C₆-haloalkoxy, C₂-C₆-alkenyloxy, C₂-C₆-alkynyloxy, C₁-C₆-alkylthio, C₁-C₆-haloalkylthio or -Q,
or
R¹ represents unsubstituted or substituted C₅-C₁₀-cycloalkenyl, where the substituents independently of one another are selected from the list below:
cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl)silyl, phenyl, hydroxyl, oxo, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
or
R¹ represents unsubstituted or substituted phenyl, where the substituents independently of one another are selected from the list below:
halogen, cyano, hydroxyl, SH, amino, nitro, C(=O)H, C(=O)OH, CONR³R⁴, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkyl-cycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₂-C₆-alkenyloxy, C₂-C₆-haloalkenyloxy, C₂-C₆-alkynyloxy, C₂-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri(C₁-C₄-alkyl)silyl, C₁-C₆-alkylsulphonylamino, C₁-C₆-haloalkylsulphonylamino or -L⁸Q,
or
R¹ represents saturated or partially or fully unsaturated unsubstituted or substituted naphthyl or indenyl, where the substituents independently of one another are selected from the list below:
cyano, nitro, halogen, C₁-C₆-alkyl, C₁-C₄-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl) silyl, benzyl, phenyl, hydroxyl, SH, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
or
R¹ represents an unsubstituted or substituted 5-or 6-membered heteroaryl radical where L¹ is attached to a carbon of the heteroaryl radical and where the substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkyl-cycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₆-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or -L⁸Q,
substituents at nitrogen: C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe, benzyl or phenyl,
or
R¹ represents benzo-fused unsubstituted or substituted 5- or 6-membered heteroaryl where L¹ is attached to a carbon of the heteroaryl radical and where the substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or phenyl,
substituents at nitrogen: C₁-C₆-alkyl, C₂-C₆-alkenyl, C₂-C₆-alkynyl, C₁-C₆- haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe, benzyl or phenyl,
or
R¹ represents unsubstituted or substituted C₅-C₁₅-heterocyclyl where L¹ is attached to a carbon of the heterocyclyl radical and possible substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or phenyl,
substituents at nitrogen: C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe, benzyl or phenyl,
Q represents phenyl which may contain up to two substituents, where the substituents independently of one another are selected from the list below:
halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or phenyl,
or
Q represents a 5- or 6-membered heteroaryl radical which may contain up to two substituents, where the substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or phenyl,
substituents at nitrogen: C₁-C₆-alkyl, C₂-C₆-alkenyl, C₂-C₆-alkynyl, C₁-C₆- haloalkyl, C₂-C₆-haloalkenyl, C₂-C₆-haloalkynyl, C₃-C₆-cycloalkyl, C₃-C₆-halocycloalkyl, C₄-C₁₀₋alkylcycloalkyl, C₄-C₁₀-cycloalkylalkyl, C₁-C₄-alkylsulphonyl, C(=O)H, C(=O)Me, C(=O)OMe or phenyl,
R² represents C₁-C₄-alkyl, C₁-C₄-haloalkyl or C₂-C₄-alkoxyalkyl,
R³, R⁴ independently of one another represent hydrogen, C₁-C₄-alkyl, C₂-C₆-alkenyl, C₂-C₆-alkynyl, C₁-C₃-haloalkyl, C₃-C₆-cycloalkyl, benzyl or phenyl,
R⁵ represents hydrogen, halogen, cyano, hydroxyl, C(=O)H, OC(=O)H, OC(=O)Me, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₂-C₄-alkoxyalkyl, C₂-C₄-alkylthioalkyl, C₂-C₄-alkylsulphinylalkyl, C₂-C₄-alkylsulphonylalkyl, C₂-C₄-alkylcarbonyl, C₂-C₄-haloalkylcarbonyl, C₂-C₅-alkoxycarbonyl, C₃-C₅-alkoxycarbonylalkyl, C₂-C₅-alkylaminocarbonyl, C₃-C₅-dialkylaminocarbonyl, C₁-C₄-alkoxy, C₁-C₄-haloalkoxy, C₁-C₄-alkylthio, C₁-C₄-haloalkylthio, C₁-C₄-alkylsulphinyl, C₁-C₄-haloalkylsulphinyl, C₁-C₄-alkylsulphonyl or C₁-C₄-haloalkylsulphonyl,
R⁶ represents hydrogen, C(=O)H, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₂-C₄-alkoxyalkyl, C₂-C₄-alkylthioalkyl, C₂-C₄-alkylsulphinylalkyl, C₂-C₄-alkylsulphonylalkyl, C₂-C₄-alkylcarbonyl, C₂-C₄-haloalkylcarbonyl, C₂-C₅-alkoxycarbonyl, C₃-C₅-alkoxycarbonylalkyl, C₂-C₅-alkylaminocarbonyl, C₃-C₅-dialkylaminocarbonyl, C₁-C₄-alkylsulphonyl or C₁-C₄-haloalkylsulphonyl,
R⁷, R⁸ independently of one another are selected from the list below:
C₁-C₆-alkyl, C₃-C₆-alkenyl, C₃-C₆-alkynyl, C₁-C₆-haloalkyl, C₃-C₆-haloalkenyl, C₃-C₆-haloalkynyl, C₃-C₆-cycloalkyl, C₃-C₆-halocycloalkyl, C₄-C₈-alkylcycloalkyl, C₄-C₈-cycloalkylalkyl, C₄-C₈-halocycloalkylalkyl, C₅-C₈-alkylcycloalkylalkyl, C₂-C₆-alkoxyalkyl, C₄-C₈-cycloalkoxyalkyl, C₃-C₆-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₆-dialkylaminoalkyl, C₂-C₆-haloalkylaminoalkyl, C₄-C₈-cycloalkylaminoalkyl, C₂-C₆-alkylcarbonyl, C₂-C₆-haloalkylcarbonyl, C₄-C₈-cycloalkylcarbonyl, C₂-C₆-alkoxylcarbonyl, C₂-C₆-alkylaminocarbonyl, C₃-C₈-dialkylaminocarbonyl, tri (C₁-C₄-alkyl) silyl or C₄-C₈-cycloalkylaminocarbonyl,
R⁹ represents hydrogen, cyano, hydroxyl, amino, C₁-C₆-alkyl, C₃-C₆-alkenyl, C₃-C₆-alkynyl, C₁-C₆-haloalkyl, C₃-C₆-haloalkenyl, C₃-C₆-haloalkynyl, C₃-C₆-cycloalkyl, C₄-C₈-cycloalkylalkyl, C₂-C₆-alkoxyalkyl, C₁-C₆-alkoxy, C₁-C₆-haloalkoxy, C₁-C₆-alkylsulphonyl, C₁-C₆-haloalkylsulphonyl, C₂-C₆-alkylcarbonyl, C₂-C₆-haloalkylcarbonyl, C₁-C₆-alkylamino, C₂-C₈-dialkylamino, C₁-C₆-haloalkylamino or C₂-C₈-halodialkylamino,
R¹⁰ represents hydrogen, C₁-C₆-alkyl, C₂-C₆-alkenyl, C₂-C₆-alkynyl, C₁-C₆-haloalkyl or C₃-C₆-cycloalkyl,
or
R⁹, R¹⁰ together form a -(CH₂)₄-, -(CH₂)₅-, -(CH₂)₂S(CH₂)₂-, -(CH₂)₂S(=O) (CH₂)₂-, -(CH₂)₂S(=O)₂(CH₂)₂-, -(CH₂)₂NR³(CH₂)₂- or - (CH₂)₂O(CH₂)₂- radical,
R¹¹ represents hydrogen, halogen, cyano, C₁-C₄-alkyl, C₁-C₄-haloalkyl, C₂-C₄-alkoxylalkyl, C₂-C₄-alkylcarbonyl, C₂-C₄-alkoxycarbonyl, C₂-C₃-alkylaminocarbonyl or C₃-C₆-dialkylaminocarbonyl,
R¹² represents hydrogen, halogen, cyano, hydroxyl, OC(=O)Me, OC(=O)H, C(=O)H, C(=O)OH, C(=O)OMe, C(=O)Me, C₁-C₄-alkyl, C₂-C₄-alkenyl, C₂-C₄-alkynyl, C₁-C₄-haloalkyl, C₁-C₄-alkoxy or C₁-C₄-haloalkoxy,
R¹³ represents hydrogen, C₁-C₃-alkyl, C₁-C₃-haloalkyl or halogen,
R¹⁴ represents hydrogen or C₁-C₃-alkyl, C(=O)H, C(=O)Me or C(=O)OMe,
R¹⁵ are identical or different and independently of one another represent hydrogen, halogen, C₁-C₄-alkyl, C₂-C₄-alkenyl, C₂-C₄-alkynyl, C₁-C₄-alkoxy, oxo, C₃-C₆-cycloalkyl, C₁-C₄-haloalkyl or phenyl
R¹⁶ represents hydrogen, C₁-C₄-alkyl or C₁-C₄-haloalkyl,
R¹⁷ represents hydrogen, C₁-C₆-alkyl, C₁-C₆-haloalkyl, C₃-C₈-cycloalkyl, C₂-C₆-alkylcarbonyl, C₂-C₆-haloalkylcarbonyl, C₂-C₆-alkoxycarbonyl or C₂-C₆-haloalkoxycarbonyl,
and also agrochemically active salts thereof, except for the following compounds:
a) compounds in which
A² represents pyrazol-1-yl,
X represents CH,
G represents G¹,
b) compounds in which
X represents nitrogen,
G represents G¹³, G¹⁴, G¹⁷ or G²⁰,
c) compounds in which
Y⁴ represents sulphur,
L⁴ represents C(=O).

2. Compounds of the formula (I) according to Claim 1,
in which the symbols have the following meanings:
E represents E²,
A² furthermore represents phenyl which may contain up to two substituents, where the substituents independently of one another are selected from the list below:
halogen, cyano, hydroxyl, amino, nitro, C₁-C₆-alkyl, C₂-C₆-alkenyl, C₂-C₆-alkynyl, C₃-C₁₀-cycloalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphonyl, C₁-C₄-haloalkylthio, C₁-C₄-haloalkylsulphonyl, C₂-C₄-alkoxyalkyl, C₁-C₄-hydroxyalkyl, C₂-C₄-alkylcarbonyl, C₂-C₆-alkoxycarbonyl, C₂-C₆-alkylcarbonyloxy, C(=O)H or CR³=NOR⁴
A² furthermore represents a heteroaromatic radical selected from the group below: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl which may contain up to two substituents, where the substituents independently of one another are selected from the list below:
substituents at carbon:
halogen, cyano, hydroxyl, amino, nitro, C₁-C₆-alkyl, C₂-C₆-alkenyl, C₂-C₆-alkynyl, C₃-C₁₀-cycloalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphonyl, C₁-C₄-haloalkylthio, C₁-C₄-haloalkylsulphonyl, C₂-C₄-alkoxyalkyl, C₁-C₄-hydroxyalkyl, C₂-C₄-alkylcarbonyl, C₂-C₆-alkoxycarbonyl, C₂-C₆-alkylcarbonyloxy, C(=O)H, CR³=NOR⁴ or phenyl
substituents at nitrogen:
C₁-C₆-alkyl, C₁-C₄-haloalkyl, C₂-C₆-alkenyl, C₂-C₆-haloalkenyl, C₂-C₆-alkynyl or C₂-C₆-haloalkynyl.
L¹ represents a direct bond, -CH₂- or -CHCH₃-,
L³ represents a direct bond,
L⁴ represents oxygen, CHR⁵, NR⁶,
L⁸ represents a direct bond, -O-,
Y¹ represents oxygen, sulphur,
Y² represents oxygen, sulphur,
Y³ represents OR⁷, SR⁸,
Y⁴ represents oxygen, sulphur,
X represents CH, CF, N,
G represents G¹, G², G³, G¹³, G¹⁴ or G¹⁸
R¹ furthermore represents unsubstituted or substituted C₃-C₁₀-cycloalkyl, where the substituents independently of one another are selected from the list below:
cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl) silyl, phenyl, hydroxyl, oxo, C₁-C₆-alkoxy, C₁-C₆- haloalkoxy, C₂-C₆-alkenyloxy, C₂-C₆-alkynyloxy, C₁-C₆-alkylthio or C₁-C₆-haloalkylthio,
R¹ furthermore represents unsubstituted or substituted C₅-C₁₀-cycloalkenyl, where the substituents independently of one another are selected from the list below:
cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl) silyl, phenyl, hydroxyl, oxo, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
R¹ furthermore represents unsubstituted or substituted phenyl, where the substituents independently of one another are selected from the list below:
halogen, cyano, hydroxyl, SH, amino, nitro, C(=O)H, C(=O)OH, CONR³R⁴, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkylcycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₂-C₆-alkenyloxy, C₂-C₆-haloalkenyloxy, C₂-C₆-alkynyloxy, C₂-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri (C₁-C₄-alkyl)silyl, C₁-C₆-alkylsulphonylamino, C₁-C₆-haloalkylsulphonylamino or -L⁸Q,
R¹ furthermore represents saturated or partially or fully unsaturated unsubstituted or substituted naphthyl or indenyl, where the substituents independently of one another are selected from the list below:
cyano, nitro, halogen, C₁-C₆-alkyl, C₁-C₄-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₄-alkyl) silyl, benzyl, phenyl, hydroxyl, SH, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
R¹ furthermore represents an unsubstituted or substituted 5- or 6-membered heteroaryl radical where L¹ is attached to a carbon of the heteroaryl radical and where the substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆- haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or -L⁸Q,
substituents at nitrogen: C₁-C₆-alkyl, C₂-C₆-alkenyl, C₂-C₆-alkynyl, C₁-C₆- haloalkyl, C₂-C₆-haloalkenyl, C₂-C₆-haloalkynyl, C₃-C₆-cycloalkyl, C₃-C₆-halocycloalkyl, C₄-C₁₀-alkyl-cycloalkyl, C₄-C₁₀-cycloalkylalkyl or phenyl,
R¹ furthermore represents benzo-fused unsubstituted or substituted 5- or 6-membered heteroaryl where L¹ is attached to a carbon of the heteroaryl radical and where the substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri (C₁-C₄-alkyl) silyl or phenyl,
substituents at nitrogen: C₁-C₆-alkyl, C₂-C₆-alkenyl, C₂-C₆-alkynyl, C₁-C₆- haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl or phenyl,
R¹ furthermore represents unsubstituted or substituted C₅-C₁₅-heterocyclyl where L¹ is attached to a carbon of the heterocyclyl radical and where possible substituents independently of one another are selected from the list below:
substituents at carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or phenyl,
substituents at nitrogen: C₁-C₆-alkyl, C₂-C₆-alkenyl, C₂-C₆-alkynyl, C₁-C₆- haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl or phenyl,
Q represents phenyl which may contain up to two substituents, where the substituents independently of one another are selected from the list below:
halogen, cyano, hydroxyl, SH, nitro, C₁-C₆-alkyl, C₁-C₆-haloalkyl, C₃-C₆-cycloalkyl, C₁-C₄-alkoxy, C₁-C₄-haloalkoxy, C₁-C₄-alkylthio, C₁-C₄-haloalkylthio or phenyl,
Q furthermore represents furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, tetrazol-5-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-l-yl, 1,2,4-triazol-3-yl, 1,2,4-triazol-4-yl, tetrazol-5-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl or 1,2,4-triazin-3-yl which may contain up to two substituents, where the substituents independently of one another are selected from the list below:
substituents at carbon:
halogen, cyano, hydroxyl, SH, nitro, C₁-C₆-alkyl, C₁-C₆-haloalkyl, C₃-C₆-cycloalkyl, C₁-C₄-alkoxy, C₁-C₄-haloalkoxy, C₁-C₄-alkylthio, C₁-C₄-haloalkylthio or phenyl,
substituents at nitrogen: C₁-C₆-alkyl, C₂-C₆-alkenyl, C₂-C₆-alkynyl, C₃-C₆-cycloalkyl or phenyl,
R³, R⁴ independently of one another represent hydrogen,
R³, R⁴ independently of one another furthermore represent methyl, ethyl, propyl, isopropyl, butyl, isobutyl or *tert*-butyl,
R⁵ represents hydrogen,
R⁵ furthermore represents methyl, ethyl, propyl, isopropyl, butyl, isobutyl or *tert*-butyl,
R⁶ represents hydrogen,
R⁶ furthermore represents methyl, ethyl, propyl, isopropyl, butyl, isobutyl or *tert*-butyl,
R⁷ represents methyl, ethyl, propyl, isopropyl, butyl, isobutyl or *tert*-butyl,
R⁸ represents methyl, ethyl, propyl, isopropyl, butyl, isobutyl or *tert*-butyl,
R¹³ represents hydrogen,
and also agrochemically active salts thereof.

3. Method for controlling phytopathogenic harmful fungi, **characterized in that** compounds of the formula (**I**) according to Claim 1 or 2 are applied to the phytopathogenic harmful fungi and/or their habitat.

4. Composition for controlling phytopathogenic harmful fungi, **characterized in that** it comprises at least one compound of the formula **(I)** according to Claim 1 or 2, in addition to extenders and/or surfactants.

5. Use of heteroarylpiperidine and -piperazine derivatives of the formula **(I)** according to Claim 1 or 2 for controlling phytopathogenic harmful fungi.

6. Process for preparing compositions for controlling phytopathogenic harmful fungi, **characterized in that** heteroarylpiperidine and -piperazine derivatives of the formula **(I)** according to Claim 1 or 2 are mixed with extenders and/or surfactants.

## Revendications

1. Composés de formule (I), 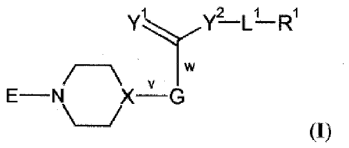 dans laquelle les symboles présentent les significations suivantes :
E représente 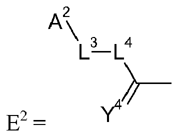
A² représente phényle, qui peut contenir jusqu'à trois substituants, les substituants étant choisis, indépendamment les uns des autres, dans la liste suivante :
halogène, cyano, hydroxy, SH, amino, nitro, C₁-C₆-alkyle, C₂-C₆-alcényle, C₂-C₆-alcynyle, C₃-C₁₀-cycloalkyle, C₄-C₁₀-cycloalkylalkyle, C₄-C₁₀-alkylcycloalkyle, C₅-C₁₀-alkylcycloalkylalkyle, C₁-C₆-halogénoalkyle, C₂-C₆-halogénoalcényle, C₂-C₆-halogénoalcynyle, C₃-C₆-halogénocycloalkyle, C₁-C₄-alcoxy, C₁-C₄-halogénoalcoxy, C₁-C₄-alkylthio, C₁-C₄-alkylsulfinyle, C₁-C₄-alkylsulfonyle, C₁-C₄-halogénoalkylthio, C₁-C₄-halogénoalkylsulfinyle, C₁-C₄-halogénoalkylsulfonyle, C₁-C₄-alkylamino, C₂-C₈-dialkylamino, C₃-C₆-cycloalkylamino, C₄-C₁₀-cycloalkylalcoxy, C₂-C₄-alcoxyalkyle, C₂-C₆-alcoxyalcoxy, C₁-C₄-hydroxyalkyle, C₂-C₄-alkylcarbonyle, C₂-C₆-alcoxycarbonyle, C₂-C₆-alkylcarbonyloxy, C₂-C₆-alkylcarbonylthio, C₂-C₆-alkylaminocarbonyle, C₃-C₈-dialkylaminocarbonyle, tri (C₁-C₄-alkyl)silyle, C(=O)H, CR³=NOR⁴, phényle ou benzyle
ou
A² représente un radical hétéroaromatique choisi dans le groupe suivant : furann-2-yle, furann-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yle ou pyrimidin-5-yle, qui peut contenir jusqu'à trois substituants, les substituants étant choisis, indépendamment les uns des autres, dans la liste suivante : substituants sur le carbone :
halogène, cyano, hydroxy, SH, amino, nitro, C₁-C₆-alkyle, C₂-C₆-alcényle, C₂-C₆-alcynyle, C₃-C₁₀-cycloalkyle, C₄-C₁₀-cycloalkylalkyle, C₄-C₁₀-alkylcycloalkyle, C₅-C₁₀-alkylcycloalkylalkyle, C₁-C₆-halogénoalkyle, C₂-C₆-halogénoalcényle, C₂-C₆-halogénoalcynyle, C₃-C₆-halogénocycloalkyle, C₁-C₄-alcoxy, C₁-C₄-halogénoalcoxy, C₁-C₄-alkylthio, C₁-C₄-alkylsulfinyle, C₁-C₄-alkylsulfonyle, C₁-C₄-halogénoalkylthio, C₁-C₄-halogénoalkylsulfinyle, C₁-C₄-halogénoalkylsulfonyle, C₁-C₄-alkylamino, C₂-C₈-dialkylamino, C₃-C₆-cycloalkylamino, C₄-C₁₀-cycloalkylalcoxy, C₂-C₄-alcoxyalkyle, C₂-C₆-alcoxyalcoxy, C₁-C₄-hydroxyalkyle, C₂-C₄-alkylcarbonyle, C₂-C₆-alcoxycarbonyle, C₂-C₆-alkylcarbonyloxy, C₂-C₆-alkylcarbonylthio, C₂-C₆-alkylaminocarbonyle, C₃-C₈-dialkylaminocarbonyle, tri (C₁-C₄-alkyl)silyle, C(=O)H, CR³=NOR⁴, phényle ou benzyle
substituants sur l'azote :
C₁-C₆-alkyle, C₂-C₆-alcényle, C₂-C₆-alcynyle, C₃-C₆-cycloalkyle, C₁-C₆-halogénoalkyle, C₂-C₆-halogénoalcényle, C₂-C₆-halogénoalcynyle, C₃-C₆-halogénocycloalkyle, C₁-C₄-alkylsulfonyle, C(=O)H, C(=O)Me, C(=O)OMe ou C₂-C₄-alcoxyalkyle,
L¹ représente (C(R¹⁵)₂)ₚ,
p vaut 0, 1 ou 2,
L³ représente une liaison directe,
L⁴ représente oxygène, CHR⁵, NR⁶ ou C(=O),
L⁸ représente une liaison directe, -O, -C(=O), -S(O)ₘ, -CHR¹⁶ ou -NR¹⁷,
m vaut 0, 1 ou 2,
Y¹, Y², Y⁴ représentent, indépendamment les uns des autres, soufre ou oxygène,
Y³ représente OR⁷, SR⁸, NR⁹R¹⁰ ou R¹¹,
n vaut 0, 1 ou 2,
X représente oxygène, CR¹², azote,
G représente 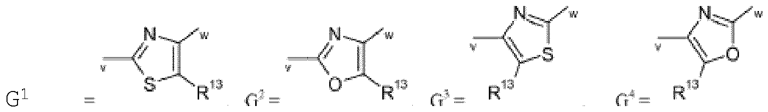 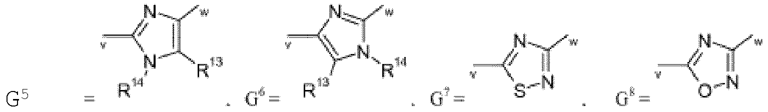 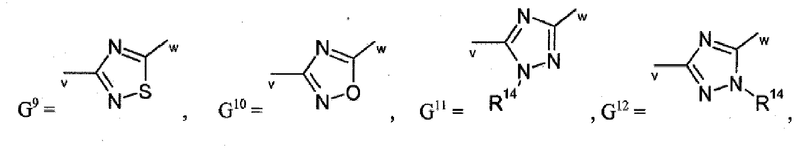 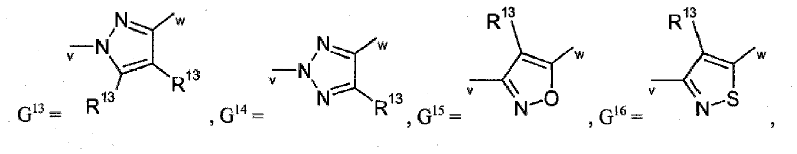 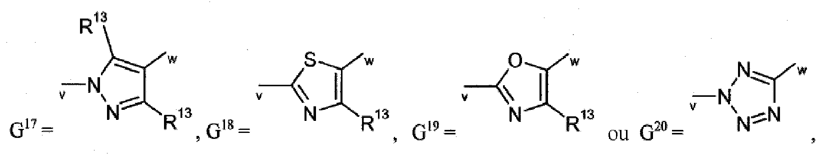
la liaison, identifiée par "v", étant liée directement à X et la liaison, identifiée par "w", étant liée directement à C(=Y¹)Y²L¹R¹,
R¹ représente C₃-C₁₀-cycloalkyle non substitué ou substitué, les substituants étant choisis, indépendamment les uns des autres, dans la liste suivante :
cyano, halogène, C₁-C₆-alkyle, C₁-C₆-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, phénoxy, hydroxy, oxo, C₂-C₆-alcoxycarbonyle, C₁-C₆-alkylcarbonyloxy, C₁-C₆-alkylcarbonyle, C₁-C₆-alcoxy, C₁-C₆-halogénoalcoxy, C₂-C₆-alcényloxy, C₂-C₆-alcynyloxy, C₁-C₆-alkylthio, C₁-C₆-halogénoalkylthio ou -Q, ou
R¹ représente C₅-C₁₀-cycloalcényle non substitué ou substitué, les substituants étant choisis, indépendamment les uns des autres, dans la liste suivante :
cyano, halogène, C₁-C₆-alkyle, C₁-C₆-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, phényle, hydroxy, oxo, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio, ou
R¹ représente phényle non substitué ou substitué, les substituants étant choisis, indépendamment les uns des autres, dans la liste suivante :
halogène, cyano, hydroxy, SH, amino, nitro, C(=O)H, C(=O)OH, CONR³R⁴, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₈-cycloalkyle, C₃-C₈-halogénocycloalkyle, C₄-C₁₀-alkylcycloalkyle, C₄-C₁₀-cycloalkylalkyle, C₆-C₁₄-cycloalkylcycloalkyle, C₄-C₁₀-halogénocycloalkylalkyle, C₅-C₁₀-alkylcycloalkylalkyle, C₃-C₈-cycloalcényle, C₃-C₈-halogénocycloalcényle, C₂-C₆-alcoxyalkyle, C₄-C₁₀-cycloalcoxyalkyle, C₃-C₈-alcoxyalcoxyalkyle, C₂-C₆-alkylthioalkyle, C₂-C₆-alkylsulfinylalkyle, C₂-C₆-alkylsulfonylalkyle, C₂-C₆-alkylaminoalkyle, C₃-C₈-dialkylaminoalkyle, C₂-C₆-halogénoalkylaminoalkyle, C₄-C₁₀-cycloalkylaminoalkyle, C₂-C₆-alkylcarbonyle, C₂-C₆-halogénoalkylcarbonyle, C₄-C₈-cycloalkylcarbonyle, C₂-C₆-alcoxycarbonyle, C₄-C₈-cycloalcoxycarbonyle, C₅-C₁₀-cycloalkylalcoxycarbonyle, C₂-C₆-alkylaminocarbonyle, C₃-C₈-dialkylaminocarbonyle, C₄-C₈-cycloalkylaminocarbonyle, C₂-C₆-halogénoalcoxyalkyle, C₁-C₆-hydroxyalkyle, C₁-C₆-alcoxy, C₁-C₆-halogénoalcoxy, C₃-C₈-cycloalcoxy, C₃-C₈-halogénocycloalcoxy, C₄-C₁₀-cycloalkylalcoxy, C₂-C₆-alcényloxy, C₂-C₆-halogénoalcényloxy, C₂-C₆-alcynyloxy, C₂-C₆-halogénoalcynyloxy, C₂-C₆-alcoxyalcoxy, C₂-C₆-alkylcarbonyloxy, C₂-C₆-halogénoalkylcarbonyloxy, C₄-C₈-cycloalkylcarbonyloxy, C₃-C₆-alkylcarbonylalcoxy, C₁-C₆-alkylthio, C₁-C₆-halogénoalkylthio, C₃-C₆-cycloalkylthio, C₁-C₆-alkylsulfinyle, C₁-C₆-halogénoalkylsulfinyle, C₁-C₆-alkylsulfonyle, C₁-C₆-halogénoalkylsulfonyle, C₃-C₈-cycloalkylsulfonyle, tri (C₁-C₄-alkyl)silyle, C₁-C₆-alkylsulfonylamino, C₁-C₆-halogénoalkylsulfonylamino ou -L⁸Q, ou
R¹ représente naphtyle ou indényle saturé ou partiellement ou totalement insaturé, non substitué ou substitué, les substituants étant choisis, indépendamment les uns des autres, dans la liste suivante :
cyano, nitro, halogène, C₁-C₆-alkyle, C₁-C₄-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, benzyle, phényle, hydroxy, SH, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio, ou
R¹ représente un radical hétéroaryle non substitué ou substitué de 5 ou 6 chaînons, L¹ étant lié à un carbone du radical hétéroaryle et les substituants étant choisis, indépendamment les uns des autres, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou -L⁸Q,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₁-C₄-alkylsulfonyle, C(=O)H, C(=O)Me, C(=O)OMe, benzyle ou phényle, ou
R¹ représente un radical hétéroaryle benzocondensé, non substitué ou substitué de 5 ou 6 chaînons, L¹ étant lié à un carbone du radical hétéroaryle et les substituants étant choisis, indépendamment les uns des autres, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou phényle,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₁-C₄-alkylsulfonyle, C(=O)H, C(=O)Me, C(=O)OMe, benzyle ou phényle, ou
R¹ représente un radical C₅-C₁₅-hétérocyclyle, non substitué ou substitué, L¹ étant lié à un carbone du radical hétérocyclyle et des substituants éventuels étant choisis, indépendamment les uns des autres, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou phényle,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₁-C₄-alkylsulfonyle, C(=O)H, C(=O)Me, C(=O)OMe, benzyle ou phényle,
Q représente phényle, qui peut contenir jusqu'à deux substituants, les substituants étant choisis, indépendamment l'un de l'autre, dans la liste suivante :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri(C₁-C₄-alkyl)silyle ou phényle, ou
Q représente un radical hétéroaryle de 5 ou 6 chaînons, qui peut contenir jusqu'à deux substituants, les substituants étant choisis, indépendamment l'un de l'autre, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou phényle,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₁-C₄-alkylsulfonyle, C(=O)H, C(=O)Me, C(=O)OMe ou phényle,
R² représente C₁-C₄-alkyle, C₁-C₄-halogénoalkyle ou C₂-C₄-alcoxyalkyle,
R³, R⁴ représentent, indépendamment l'un de l'autre, hydrogène, C₁-C₄-alkyle, C₂-C₆-alcényle, C₂-C₆-alcynyle, C₁-C₃-halogénoalkyle, C₃-C₆-cycloalkyle, benzyle ou phényle,
R⁵ représente hydrogène, halogène, cyano, hydroxy, C(=O)H, OC(=O)H, OC(=O)Me, C₁-C₄-alkyle, C₂-C₄-alcényle, C₂-C₄-alcynyle, C₁-C₄-halogénoalkyle, C₂-C₄-halogénoalcényle, C₂-C₄-halogénoalcynyle, C₂-C₄-alcoxyalkyle, C₂-C₄-alkylthioalkyle, C₂-C₄-alkylsulfinylalkyle, C₂-C₄-alkylsulfonylalkyle, C₂-C₄-alkylcarbonyle, C₂-C₄-halogénoalkylcarbonyle, C₂-C₅-alcoxycarbonyle, C₃-C₅-alcoxycarbonylalkyle, C₂-C₅-alkylaminocarbonyle, C₃-C₅-dialkylaminocarbonyle, C₁-C₄-alcoxy, C₁-C₄-halogénoalcoxy, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle ou C₁-C₄-halogénoalkylsulfonyle,
R⁶ représente hydrogène, C(=O)H, C₁-C₄-alkyle, C₂-C₄-alcényle, C₂-C₄-alcynyle, C₁-C₄-halogénoalkyle, C₂-C₄-halogénoalcényle, C₂-C₄-halogénoalcynyle, C₂-C₄-alcoxyalkyle, C₂-C₄-alkylthioalkyle, C₂-C₄-alkylsulfinylalkyle, C₂-C₄-alkylsulfonylalkyle, C₂-C₄-alkylcarbonyle, C₂-C₄-halogénoalkylcarbonyle, C₂-C₅-alcoxycarbonyle, C₃-C₅-alcoxycarbonylalkyle, C₂-C₅-alkylaminocarbonyle, C₃-C₅-dialkylaminocarbonyle, C₁-C₄-alkylsulfonyle ou C₁-C₄-halogénoalkylsulfonyle,
R⁷, R⁸ sont choisis, indépendamment l'un de l'autre, dans la liste suivante:
C₁-C₆-alkyle, C₃-C₆-alcényle, C₃-C₆-alcynyle, C₁-C₆-halogénoalkyle, C₃-C₆-halogénoalcényle, C₃-C₆-halogénoalcynyle, C₃-C₆-cycloalkyle, C₃-C₆-halogénocycloalkyle, C₄-C₈-alkylcycloalkyle, C₄-C₈-cycloalkylalkyle, C₄-C₈-halogénocycloalkylalkyle, C₅-C₈-alkylcycloalkylalkyle, C₂-C₆-alcoxyalkyle, C₄-C₈-cycloalcoxyalkyle, C₃-C₆-alcoxyalcoxyalkyle, C₂-C₆-alkylthioalkyle, C₂-C₆-alkylsulfinylalkyle, C₂-C₆-alkylsulfonylalkyle, C₂-C₆-alkylaminoalkyle, C₃-C₆-dialkylaminoalkyle, C₂-C₆-halogénoalkylaminoalkyle, C₄-C₈-cycloalkylaminoalkyle, C₂-C₆-alkylcarbonyle, C₂-C₆-halogénoalkylcarbonyle, C₄-C₈-cycloalkylcarbonyle, C₂-C₆-alcoxylcarbonyle, C₂-C₆-alkylaminocarbonyle, C₃-C₈-dialkylaminocarbonyle, tri (C₁-C₄-alkyl)silyle ou C₄-C₈-cycloalkylaminocarbonyle,
R⁹ représente hydrogène, cyano, hydroxy, amino, C₁-C₆-alkyle, C₃-C₆-alcényle, C₃-C₆-alcynyle, C₁-C₆-halogénoalkyle, C₃-C₆-halogénoalcényle, C₃-C₆-halogénoalcynyle, C₃-C₆-cycloalkyle, C₄-C₈-cycloalkylalkyle, C₂-C₆-alcoxyalkyle, C₁-C₆-alcoxy, C₁-C₆-halogénoalcoxy, C₁-C₆-alkylsulfonyle, C₁-C₆-halogénoalkylsulfonyle, C₂-C₆-alkylcarbonyle, C₂-C₆-halogénoalkylcarbonyle, C₁-C₆-alkylamino, C₂-C₈-dialkylamino, C₁-C₆-halogénoalkylamino ou C₂-C₈-halogénodialkylamino,
R¹⁰ représente hydrogène, C₁-C₆-alkyle, C₂-C₆-alcényle, C₂-C₆-alcynyle, C₁-C₆-halogénoalkyle ou C₃-C₆-cycloalkyle, ou
R⁹, R¹⁰ forment ensemble un radical -(CH₂)₄-, -(CH₂)₅-, -(CH₂)₂S(CH₂)₂-, -(CH₂)₂S(=O) (CH₂)₂-, - (CH₂)₂S (=O)₂(CH₂)₂-, - (CH₂)₂NR³ (CH₂)₂- ou - (CH₂)₂O(CH₂)₂-,
R¹¹ représente hydrogène, halogène, cyano, C₁-C₄-alkyle, C₁-C₄-halogénoalkyle, C₂-C₄-alcoxylalkyle, C₂-C₄-alkylcarbonyle, C₂-C₄-alcoxycarbonyle, C₂-C₃-alkylaminocarbonyle ou C₃-C₆-dialkylaminocarbonyle,
R¹² représente hydrogène, halogène, cyano, hydroxy, OC(=O)Me, OC(=O)H, C(=O)H, C(=O)OH, C(=O)OMe, C(=O)Me, C₁-C₄-alkyle, C₂-C₄-alcényle, C₂-C₄-alcynyle, C₁-C₄-halogénoalkyle, C₁-C₄-alcoxy ou C₁-C₄-halogénoalcoxy,
R¹³ représente hydrogène, C₁-C₃-alkyle, C₁-C₃-halogénoalkyle ou halogène,
R¹⁴ représente hydrogène ou C₁-C₃-alkyle, C(=O)H, C(=O)Me ou C(=O)OMe,
R¹⁵ sont identiques ou différents et représentent, indépendamment l'un de l'autre, hydrogène, halogène, C₁-C₄-alkyle, C₂-C₄-alcényle, C₂-C₄-alcynyle, C₁-C₄-alcoxy, oxo, C₃-C₆-cycloalkyle, C₁-C₄-halogénoalkyle ou phényle
R¹⁶ représente hydrogène, C₁-C₄-alkyle ou C₁-C₄-halogénoalkyle,
R¹⁷ représente hydrogène, C₁-C₆-alkyle, C₁-C₆-halogénoalkyle, C₃-C₈-cycloalkyle, C₂-C₆-alkylcarbonyle, C₂-C₆-halogénoalkylcarbonyle, C₂-C₆-alcoxycarbonyle ou C₂-C₆-halogénoalcoxycarbonyle,
ainsi que les sels actifs sur le plan agrochimique correspondants,
les composés suivants étant exclus :
a) composés dans lesquels
A² représente pyrazol-1-yle,
X représente CH,
G représente G¹,
b) composés dans lesquels
X représente azote,
G représente G¹³, G¹⁴, G¹⁷ ou G²⁰,
c) composés dans lesquels
Y⁴ représente soufre,
L⁴ représente C(=O).

2. Composés de formule (I) selon la revendication 1, dans laquelle les symboles ont les significations suivantes :
E E²,
A² représente en outre phényle, qui peut contenir jusqu'à deux substituants, les substituants étant choisis, indépendamment l'un de l'autre, dans la liste suivante :
halogène, cyano, hydroxy, amino, nitro, C₁-C₆-alkyle, C₂-C₆-alcényle, C₂-C₆-alcynyle, C₃-C₁₀-cycloalkyle, C₁-C₆-halogénoalkyle, C₂-C₆-halogénoalcényle, C₂-C₆-halogénoalcynyle, C₃-C₆-halogénocycloalkyle, C₁-C₄-alcoxy, C₁-C₄-halogénoalcoxy, C₁-C₄-alkylthio, C₁-C₄-alkylsulfonyle, C₁-C₄-halogénoalkylthio, C₁-C₄-halogénoalkylsulfonyle, C₂-C₄-alcoxyalkyle, C₁-C₄-hydroxyalkyle, C₂-C₄-alkylcarbonyle, C₂-C₆-alcoxycarbonyle, C₂-C₆-alkylcarbonyloxy, C(=O)H ou CR³=NOR⁴
A² représente en outre un radical hétéroaromatique choisi dans le groupe suivant : furann-2-yle, furann-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yle ou pyrimidin-5-yle, qui peut contenir jusqu'à deux substituants, les substituants étant choisis, indépendamment l'un de l'autre, dans la liste suivante :
substituants sur le carbone :
halogène, cyano, hydroxy, amino, nitro, C₁-C₆-alkyle, C₂-C₆-alcényle, C₂-C₆-alcynyle, C₃-C₁₀-cycloalkyle, C₁-C₆-halogénoalkyle, C₂-C₆ halogénoalcényle, C₂-C₆-halogénoalcynyle, C₃-C₆-halogénocycloalkyle, C₁-C₄-alcoxy, C₁-C₄-halogénoalcoxy, C₁-C₄-alkylthio, C₁-C₄-alkylsulfonyle, C₁-C₄-halogénoalkylthio, C₁-C₄-halogénoalkylsulfonyle, C₂-C₄-alcoxyalkyle, C₁-C₄-hydroxyalkyle, C₂-C₄-alkylcarbonyle, C₂-C₆-alcoxycarbonyle, C₂-C₆-alkylcarbonyloxy, C(=O)H, CR³=NOR⁴ ou phényle
substituants sur l'azote :
C₁-C₆-alkyle, C₁-C₄-halogénoalkyle, C₂-C₆-alcényle, C₂-C₆-halogénoalcényle, C₂-C₆-alcynyle ou C₂-C₆-halogénoalcynyle,
L¹ représente une liaison directe, -CH₂- ou -CHCH₃-,
L³ représente une liaison directe,
L⁴ représente oxygène, CHR⁵, NR⁶,
L⁸ représente une liaison directe, -O-
Y¹ représente oxygène, soufre,
Y² représente oxygène, soufre,
Y³ représente OR⁷, SR⁸,
Y⁴ représente oxygène, soufre,
X représente CH, CF, N,
G représente G¹, G², G³, G¹³, G¹⁴ ou G¹⁸
R¹ représente en outre C₃-C₁₀-cycloalkyle non substitué ou substitué, les substituants étant choisis, indépendamment les uns des autres, dans la liste suivante :
cyano, halogène, C₁-C₆-alkyle, C₁-C₆-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, phényle, hydroxy, oxo, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio,
R¹ représente en outre C₅-C₁₀-cycloalcényle non substitué ou substitué, les substituants étant choisis, indépendamment les uns des autres, dans la liste suivante :
cyano, halogène, C₁-C₆-alkyle, C₁-C₆-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, phényle, hydroxy, oxo, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio,
R¹ représente en outre phényle non substitué ou substitué, les substituants étant choisis, indépendamment les uns des autres, dans la liste suivante :
halogène, cyano, hydroxy, SH, amino, nitro, C(=O)H, C(=O)OH, CONR³R⁴, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₈-cycloalkyle, C₃-C₈-halogénocycloalkyle, C₄-C₁₀-alkylcycloalkyle, C₄-C₁₀-cycloalkylalkyle, C₆-C₁₄-cycloalkylcycloalkyle, C₄-C₁₀-halogénocycloalkylalkyle, C₅-C₁₀-alkylcycloalkylalkyle, C₃-C₈-cycloalcényle, C₃-C₈-halogénocycloalcényle, C₂-C₆-alcoxyalkyle, C₄-C₁₀-cycloalcoxyalkyle, C₃-C₈-alcoxyalcoxyalkyle, C₂-C₆-alkylthioalkyle, C₂-C₆-alkylsulfinylalkyle, C₂-C₆-alkylsulfonylalkyle, C₂-C₆-alkylaminoalkyle, C₃-C₈-dialkylaminoalkyle, C₂-C₆-halogénoalkylaminoalkyle, C₄-C₁₀-cycloalkylaminoalkyle, C₂-C₆-alkylcarbonyle, C₂-C₆-halogénoalkylcarbonyle, C₄-C₈-cycloalkylcarbonyle, C₂-C₆-alcoxycarbonyle, C₄-C₈-cycloalcoxycarbonyle, C₅-C₁₀-cycloalkylalcoxycarbonyle, C₂-C₆-alkylaminocarbonyle, C₃-C₈-dialkylaminocarbonyle, C₄-C₈-cycloalkylaminocarbonyle, C₂-C₆-halogénoalcoxyalkyle, C₁-C₆-hydroxyalkyle, C₁-C₆-alcoxy, C₁-C₆-halogénoalcoxy, C₃-C₈-cycloalcoxy, C₃-C₈-halogénocycloalcoxy, C₄-C₁₀-cycloalkylalcoxy, C₂-C₆-alcényloxy, C₂-C₆-halogénoalcényloxy, C₂-C₆-alcynyloxy, C₂-C₆-halogénoalcynyloxy, C₂-C₆-alcoxyalcoxy, C₂-C₆-alkylcarbonyloxy, C₂-C₆-halogénoalkylcarbonyloxy, C₄-C₈-cycloalkylcarbonyloxy, C₃-C₆-alkylcarbonylalcoxy, C₁-C₆-alkylthio, C₁-C₆-halogénoalkylthio, C₃-C₆-cycloalkylthio, C₁-C₆-alkylsulfinyle, C₁-C₆-halogénoalkylsulfinyle, C₁-C₆-alkylsulfonyle, C₁-C₆-halogénoalkylsulfonyle, C₃-C₈-cycloalkylsulfonyle, tri (C₁-C₄-alkyl)silyle, C₁-C₆-alkylsulfonylamino, C₁-C₆-halogénoalkylsulfonylamino ou -L⁸Q,
R¹ représente en outre naphtyle ou indényle saturé ou partiellement ou totalement insaturé, non substitué ou substitué, les substituants étant choisis, indépendamment les uns des autres, dans la liste suivante :
cyano, nitro, halogène, C₁-C₆-alkyle, C₁-C₄-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₄-alkyl)silyle, benzyle, phényle, hydroxy, SH, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio,
R¹ représente en outre un radical hétéroaryle non substitué ou substitué de 5 ou 6 chaînons, L¹ étant lié à un carbone du radical hétéroaryle et les substituants étant choisis, indépendamment les uns des autres, dans la liste suivante : substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cucloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou -L⁸Q,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle ou phényle,
R¹ représente en outre un radical hétéroaryle benzocondensé, non substitué ou substitué de 5 ou 6 chaînons, L¹ étant lié à un carbone du radical hétéroaryle et les substituants étant choisis, indépendamment les uns des autres, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou phényle,
substituants sur l'azote :
C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle ou phényle,
R¹ représente en outre un radical C₅-C₁₅-hétérocyclyle, non substitué ou substitué, L¹ étant lié à un carbone du radical hétérocyclyle et des substituants éventuels étant choisis, indépendamment les uns des autres, dans la liste suivante :
substituants sur le carbone :
halogène, cyano, hydroxy, SH, amino, nitro, NR³R⁴, C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁o-cycloalkylalkyle, C₆-C₁₄-cycloalkylcycloalkyle, C₅-C₁₀-alkylcycloalkylalkyle, C₂-C₄-alcoxyalkyle, C₂-C₄-alkylcarbonyle, C₂-C₆-alcoxycarbonyle, C₂-C₆-alkylaminocarbonyle, C₃-C₈-dialkylaminocarbonyle, C₁-C₄-hydroxyalkyle, C₁-C₄-alcoxy, C₁-C₄-halogénoalcoxy, C₂-C₆-alkylcarbonyloxy, C₂-C₆-alkylcarbonylthio, C₁-C₄-alkylthio, C₁-C₄-halogénoalkylthio, C₁-C₄-alkylsulfinyle, C₁-C₄-halogénoalkylsulfinyle, C₁-C₄-alkylsulfonyle, C₁-C₄-halogénoalkylsulfonyle, tri (C₁-C₄-alkyl)silyle ou phényle,
substituants sur l'azote : C₁-C₆-alkyle, C₂-C₆-alcényle, C₂-C₆-alcynyle, C₁-C₆-halogénoalkyle, C₂-C₆-halogénoalcényle, C₂-C₆-halogénoalcynyle, C₃-C₆-cycloalkyle, C₃-C₆-halogénocycloalkyle, C₄-C₁₀-alkylcycloalkyle, C₄-C₁₀-cycloalkylalkyle ou phényle,
Q représente phényle, qui peut contenir jusqu'à deux substituants, les substituants étant choisis, indépendamment l'un de l'autre, dans la liste suivante :
halogène, cyano, hydroxy, SH, nitro, C₁-C₆-alkyle, C₁-C₆-halogénoalkyle, C₃-C₆-cycloalkyle, C₁-C₄-alcoxy, C₁-C₄-halogénoalcoxy, C₁-C₄-alkylthio, C₁-C₄-halogénoalkylthio ou phényle,
Q représente en outre furann-2-yle, furann-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, tétrazol-5-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, 1,2,4-triazol-4-yle, tétrazol-5-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle ou 1,2,4-triazin-3-yle, qui peut contenir jusqu'à deux substituants, les substituants pouvant être choisis, indépendamment l'un de l'autre, dans la liste suivante
substituants sur le carbone :
halogène, cyano, hydroxy, SH, nitro, C₁-C₆-alkyle, C₁-C₆-halogénoalkyle, C₃-C₆-cycloalkyle, C₁-C₄-alcoxy, C₁-C₄-halogénoalcoxy, C₁-C₄-alkylthio, C₁-C₄-halogénoalkylthio ou phényle, substituants sur l'azote :
C₁-C₆-alkyle, C₂-C₆-alcényle, C₂-C₆-alcynyle, C₃-C₆-cycloalkyle ou phényle,
R³, R⁴ représentent, indépendamment l'un de l'autre, hydrogène ;
R³, R⁴ représentent, indépendamment l'un de l'autre, en outre méthyle, éthyle, propyle, iso-propyle, butyle, iso-butyle ou tert-butyle,
R⁵ représente hydrogène,
R⁵ représente en outre méthyle, éthyle, propyle, iso-propyle, butyle, iso-butyle ou tert-butyle,
R⁶ représente hydrogène,
R⁶ représente en outre méthyle, éthyle, propyle, iso-propyle, butyle, iso-butyle ou tert-butyle,
R⁷ représente méthyle, éthyle, propyle, iso-propyle, butyle, iso-butyle ou tert-butyle,
R⁸ représente méthyle, éthyle, propyle, iso-propyle, butyle, iso-butyle ou tert-butyle,
R¹³ représente hydrogène,
ainsi que les sels actifs sur le plan agrochimique correspondants.

3. Procédé pour lutter contre des champignons nuisibles phytopathogènes, **caractérisé en ce qu'**on épand des composés de formule (I) selon l'une ou plusieurs des revendications 1 à 2 sur les champignons nuisibles phytopathogènes et/ou leur espace de vie.

4. Agent pour lutter contre des champignons nuisibles phytopathogènes, **caractérisé par** une teneur en au moins un composé de formule (I) selon l'une ou plusieurs des revendications 1 à 2, outre des agents d'allongement et/ou des substances tensioactives.

5. Utilisation des dérivés d'hétéroarylpipéridine et d'hétéroarylpipérazine de formule (I), selon l'une ou plusieurs des revendications 1 à 2 pour lutter contre des champignons nuisibles phytopathogènes.

6. Procédé pour préparer des agents pour lutter contre des champignons nuisibles phytopathogènes, **caractérisé en ce qu'**on mélange des dérivés d'hétéroarylpipéridine et d'hétéroarylpipérazine de formule (I) selon l'une ou plusieurs des revendications 1 à 2 avec des agents d'allongement et/ou des substances tensioactives.
